(19) Europäisches Patentamt
European Patent Office
Office européen des brevets

(11) EP 3 193 604 B1

(12) EUROPEAN PATENT SPECIFICATION

(45) Date of publication and mention of the grant of the patent:
**16.06.2021 Bulletin 2021/24**

(21) Application number: **15840234.7**

(22) Date of filing: **09.09.2015**

(51) Int Cl.:
***A61P 35/00*** *(2006.01)* ***C07D 413/14*** *(2006.01)*
***C07D 413/12*** *(2006.01)* ***A61K 31/42*** *(2006.01)*

(86) International application number:
**PCT/US2015/049219**

(87) International publication number:
**WO 2016/040504 (17.03.2016 Gazette 2016/11)**

(54) **SUBSTITUTED N-(PYRROLIDIN-3-YL)ISOXAZOLE-3-CARBOXAMIDE COMPOUNDS**

SUBSTITUIERTE N-(PYRROLIDINV3EYL)ISOXAZOL-3-CARBOXAMIDVERBINDUNGEN

COMPOSÉS DE N-(PYRROLIDIN-3YL)ISOXAZOL-3-CARBOXAMIDES SUBSTITUÉS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **10.09.2014 US 201462048757 P**
**13.04.2015 US 201562146808 P**

(43) Date of publication of application:
**26.07.2017 Bulletin 2017/30**

(73) Proprietor: **Epizyme, Inc.**
**Cambridge, MA 02139 (US)**

(72) Inventors:
- **FOLEY, Megan Alene Cloonan**
**Somerville, Massachusetts 02144 (US)**
- **KUNTZ, Kevin Wayne**
**Woburn, Massachusetts 01801 (US)**
- **MITCHELL, Lorna Helen**
**Cambridge, Massachusetts 02138 (US)**
- **MUNCHHOF, Michael John**
**Salem, Connecticut 06420 (US)**
- **HARVEY, Darren Martin**
**Acton, Massachusetts 01720 (US)**

(74) Representative: **Harris, Jennifer Lucy et al**
**Kilburn & Strode LLP**
**Lacon London**
**84 Theobalds Road**
**London WC1X 8NL (GB)**

(56) References cited:
**WO-A1-2014/175370 WO-A2-2007/121920**

- **ANDREWD FERGUSON ET AL: "Structural Basis of Substrate Methylation and Inhibition of SMYD2", STRUCTURE, ELSEVIER, AMSTERDAM, NL, vol. 19, no. 9, 13 June 2011 (2011-06-13), pages 1262-1273, XP028284161, ISSN: 0969-2126, DOI: 10.1016/J.STR.2011.06.011 [retrieved on 2011-07-08]**
- **DATABASE PUBCHEM 22 October 2012 XP055417573 Retrieved from 2378974, accession no. N N N N**
- **DATABASE PUBCHEM 19 August 2012 XP055417577 Retrieved from 8952603, accession no. N N N N**
- **DATABASE PUBCHEM 28 February 2014 XP055417073 Retrieved from 2928190, accession no. N N N N**
- **DATABASE PUBCHEM 27 November 2010 XP055417579 Retrieved from 9688186, accession no. N N N N**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

SEQUENCE LISTING

<110> EPIZYME, INC. Mitchell, Lorna H Foley, Megan A Kuntz, Kevin W Munchhof, Michael J Harvey, Darren M.

<120> SUBSTITUTED PYRROLIDINE COMPOUNDS

<130> 3562.003PC02/JMC/MFG

<140> To be assigned
<141> Herewith

<150> 62/146,808
<151> 2015-04-13

<150> 62/048,757
<151> 2014-09-10

<160> 3

<170> PatentIn version 3.5

<210> 1
<211> 855
<212> PRT
<213> Artificial Sequence

<220>
<223> synthesized protein

<400> 1

```
Met Ala Pro Ile Leu Gly Tyr Trp Lys Ile Lys Gly Leu Val Gln Pro
1               5                   10                  15

Thr Arg Leu Leu Leu Glu Tyr Leu Glu Glu Lys Tyr Glu Glu His Leu
            20                  25                  30

Tyr Glu Arg Asp Glu Gly Asp Lys Trp Arg Asn Lys Lys Phe Glu Leu
        35                  40                  45

Gly Leu Glu Phe Pro Asn Leu Pro Tyr Tyr Ile Asp Gly Asp Val Lys
    50                  55                  60

Leu Thr Gln Ser Met Ala Ile Ile Arg Tyr Ile Ala Asp Lys His Asn
65                  70                  75                  80

Met Leu Gly Gly Cys Pro Lys Glu Arg Ala Glu Ile Ser Met Leu Glu
                85                  90                  95

Gly Ala Val Leu Asp Ile Arg Tyr Gly Val Ser Arg Ile Ala Tyr Ser
            100                 105                 110
```

```
Lys Asp Phe Glu Thr Leu Lys Val Asp Phe Leu Ser Lys Leu Pro Glu
        115                 120                 125

Met Leu Lys Met Phe Glu Asp Arg Leu Cys His Lys Thr Tyr Leu Asn
    130                 135                 140

Gly Asp His Val Thr His Pro Asp Phe Met Leu Tyr Asp Ala Leu Asp
145                 150                 155                 160

Val Val Leu Tyr Met Asp Pro Met Cys Leu Asp Ala Phe Pro Lys Leu
                165                 170                 175

Val Cys Phe Lys Lys Arg Ile Glu Ala Ile Pro Gln Ile Asp Lys Tyr
            180                 185                 190

Leu Lys Ser Ser Lys Tyr Ile Ala Trp Pro Leu Gln Gly Trp Gln Ala
        195                 200                 205

Thr Phe Gly Gly Gly Asp His Pro Pro Lys Ser Asp Leu Val Pro Arg
    210                 215                 220

His Asn Gln Thr Ser Leu Tyr Lys Lys Ala Gly Thr Met Asp Asp Gln
225                 230                 235                 240

Gln Ala Leu Asn Ser Ile Met Gln Asp Leu Ala Val Leu His Lys Ala
                245                 250                 255

Ser Arg Pro Ala Leu Ser Leu Gln Glu Thr Arg Lys Ala Lys Ser Ser
            260                 265                 270

Ser Pro Lys Lys Gln Asn Asp Val Arg Val Lys Phe Glu His Arg Gly
        275                 280                 285

Glu Lys Arg Ile Leu Gln Phe Pro Arg Pro Val Lys Leu Glu Asp Leu
    290                 295                 300

Arg Ser Lys Ala Lys Ile Ala Phe Gly Gln Ser Met Asp Leu His Tyr
305                 310                 315                 320

Thr Asn Asn Glu Leu Val Ile Pro Leu Thr Thr Gln Asp Asp Leu Asp
                325                 330                 335

Lys Ala Leu Glu Leu Leu Asp Arg Ser Ile His Met Lys Ser Leu Lys
            340                 345                 350

Ile Leu Leu Val Ile Asn Gly Ser Thr Gln Ala Thr Asn Leu Glu Pro
        355                 360                 365
```

```
Leu Pro Ser Leu Glu Asp Leu Asp Asn Thr Val Phe Gly Ala Glu Arg
    370                 375                 380

Lys Lys Arg Leu Ser Ile Ile Gly Pro Thr Ser Arg Asp Arg Ser Ser
385                 390                 395                 400

Pro Pro Pro Gly Tyr Ile Pro Asp Glu Leu His Gln Val Ala Arg Asn
                405                 410                 415

Gly Ser Phe Thr Ser Ile Asn Ser Glu Gly Glu Phe Ile Pro Glu Ser
            420                 425                 430

Met Glu Gln Met Leu Asp Pro Leu Ser Leu Ser Ser Pro Glu Asn Ser
        435                 440                 445

Gly Ser Gly Ser Cys Pro Ser Leu Asp Ser Pro Leu Asp Gly Glu Ser
    450                 455                 460

Tyr Pro Lys Ser Arg Met Pro Arg Ala Gln Ser Tyr Pro Asp Asn His
465                 470                 475                 480

Gln Glu Phe Ser Asp Tyr Asp Asn Pro Ile Phe Glu Lys Phe Gly Lys
                485                 490                 495

Gly Gly Thr Tyr Pro Arg Arg Tyr His Val Ser Tyr His His Gln Glu
            500                 505                 510

Tyr Asn Asp Gly Arg Lys Thr Phe Pro Arg Ala Arg Arg Thr Gln Gly
        515                 520                 525

Asn Gln Leu Thr Ser Pro Val Ser Phe Ser Pro Thr Asp His Ser Leu
    530                 535                 540

Ser Thr Ser Ser Gly Ser Ser Ile Phe Thr Pro Glu Tyr Asp Asp Ser
545                 550                 555                 560

Arg Ile Arg Arg Arg Gly Ser Asp Ile Asp Asn Pro Thr Leu Thr Val
                565                 570                 575

Met Asp Ile Ser Pro Pro Ser Arg Ser Pro Arg Ala Pro Thr Asn Trp
            580                 585                 590

Arg Leu Gly Lys Leu Leu Gly Gln Gly Ala Phe Gly Arg Val Tyr Leu
        595                 600                 605

Cys Tyr Asp Val Asp Thr Gly Arg Glu Leu Ala Val Lys Gln Val Gln
```

610 615 620

Phe Asp Pro Asp Ser Pro Glu Thr Ser Lys Glu Val Asn Ala Leu Glu
625 630 635 640

Cys Glu Ile Gln Leu Leu Lys Asn Leu Leu His Glu Arg Ile Val Gln
645 650 655

Tyr Tyr Gly Cys Leu Arg Asp Pro Gln Glu Lys Thr Leu Ser Ile Phe
660 665 670

Met Glu Tyr Met Pro Gly Gly Ser Ile Lys Asp Gln Leu Lys Ala Tyr
675 680 685

Gly Ala Leu Thr Glu Asn Val Thr Arg Lys Tyr Thr Arg Gln Ile Leu
690 695 700

Glu Gly Val His Tyr Leu His Ser Asn Met Ile Val His Arg Asp Ile
705 710 715 720

Lys Gly Ala Asn Ile Leu Arg Asp Ser Thr Gly Asn Val Lys Leu Gly
725 730 735

Asp Phe Gly Ala Ser Lys Arg Leu Gln Thr Ile Cys Leu Ser Gly Thr
740 745 750

Gly Met Lys Ser Val Thr Gly Thr Pro Tyr Trp Met Ser Pro Glu Val
755 760 765

Ile Ser Gly Gln Gly Tyr Gly Arg Lys Ala Asp Ile Trp Ser Val Ala
770 775 780

Cys Thr Val Val Glu Met Leu Thr Glu Lys Pro Pro Trp Ala Glu Phe
785 790 795 800

Glu Ala Met Ala Ala Ile Phe Lys Ile Ala Thr Gln Pro Thr Asn Pro
805 810 815

Lys Leu Pro Pro His Val Ser Asp Tyr Thr Arg Asp Phe Leu Lys Arg
820 825 830

Ile Phe Val Glu Ala Lys Leu Arg Pro Ser Ala Asp Glu Leu Leu Arg
835 840 845

His Met Phe Val His Tyr His
850 855

<210> 2
<211> 428
<212> **PRT**
<213> **Artificial** Sequence

<220>
<223> synthesized protein

<400> 2

```
Met Glu Pro Leu Lys Val Glu Lys Phe Ala Thr Ala Lys Arg Gly Asn
1               5                   10                  15

Gly Leu Arg Ala Val Thr Pro Leu Arg Pro Gly Glu Leu Leu Phe Arg
            20                  25                  30

Ser Asp Pro Leu Ala Tyr Thr Val Cys Lys Gly Ser Arg Gly Val Val
        35                  40                  45

Cys Asp Arg Cys Leu Leu Gly Lys Glu Lys Leu Met Arg Cys Ser Gln
    50                  55                  60

Cys Arg Val Ala Lys Tyr Cys Ser Ala Lys Cys Gln Lys Lys Ala Trp
65                  70                  75                  80

Pro Asp His Lys Arg Glu Cys Lys Cys Leu Lys Ser Cys Lys Pro Arg
                85                  90                  95

Tyr Pro Pro Asp Ser Val Arg Leu Leu Gly Arg Val Val Phe Lys Leu
            100                 105                 110

Met Asp Gly Ala Pro Ser Glu Ser Glu Lys Leu Tyr Ser Phe Tyr Asp
        115                 120                 125

Leu Glu Ser Asn Ile Asn Lys Leu Thr Glu Asp Lys Lys Glu Gly Leu
    130                 135                 140

Arg Gln Leu Val Met Thr Phe Gln His Phe Met Arg Glu Glu Ile Gln
145                 150                 155                 160

Asp Ala Ser Gln Leu Pro Pro Ala Phe Asp Leu Phe Glu Ala Phe Ala
                165                 170                 175

Lys Val Ile Cys Asn Ser Phe Thr Ile Cys Asn Ala Glu Met Gln Glu
            180                 185                 190

Val Gly Val Gly Leu Tyr Pro Ser Ile Ser Leu Leu Asn His Ser Cys
        195                 200                 205

Asp Pro Asn Cys Ser Ile Val Phe Asn Gly Pro His Leu Leu Leu Arg
```

```
    210                 215                 220

Ala Val Arg Asp Ile Glu Val Gly Glu Glu Leu Thr Ile Cys Tyr Leu
225                 230                 235                 240

Asp Met Leu Met Thr Ser Glu Glu Arg Arg Lys Gln Leu Arg Asp Gln
                245                 250                 255

Tyr Cys Phe Glu Cys Asp Cys Phe Arg Cys Gln Thr Gln Asp Lys Asp
            260                 265                 270

Ala Asp Met Leu Thr Gly Asp Glu Gln Val Trp Lys Glu Val Gln Glu
        275                 280                 285

Ser Leu Lys Lys Ile Glu Glu Leu Lys Ala His Trp Lys Trp Glu Gln
    290                 295                 300

Val Leu Ala Met Cys Gln Ala Ile Ile Ser Ser Asn Ser Glu Arg Leu
305                 310                 315                 320

Pro Asp Ile Asn Ile Tyr Gln Leu Lys Val Leu Asp Cys Ala Met Asp
                325                 330                 335

Ala Cys Ile Asn Leu Gly Leu Leu Glu Glu Ala Leu Phe Tyr Gly Thr
            340                 345                 350

Arg Thr Met Glu Pro Tyr Arg Ile Phe Phe Pro Gly Ser His Pro Val
        355                 360                 365

Arg Gly Val Gln Val Met Lys Val Gly Lys Leu Gln Leu His Gln Gly
    370                 375                 380

Met Phe Pro Gln Ala Met Lys Asn Leu Arg Leu Ala Phe Asp Ile Met
385                 390                 395                 400

Arg Val Thr His Gly Arg Glu His Ser Leu Ile Glu Asp Leu Ile Leu
                405                 410                 415

Leu Leu Glu Glu Cys Asp Ala Asn Ile Arg Ala Ser
            420                 425
```

<210> 3
<211> 29
<212> PRT
<213> Artificial Sequence

<220>
<223> synthesized protein

<220>
<221> MISC_FEATURE
<223> C-terminal amide cap

<400> 3

```
Ala Arg Thr Lys Gln Thr Ala Arg Lys Ser Thr Gly Gly Lys Ala Pro
1               5                   10                  15

Arg Lys Gln Leu Ala Thr Lys Ala Ala Arg Lys Ser Ala
            20                  25
```

## Description

### BACKGROUND OF THE INVENTION

#### Field of the Invention

The present disclosure provides substituted pyrrolidines as SMYD protein inhibitors, such as SMYD3 and SMYD2 inhibitors, for use in treating cancer.

#### Background

Epigenetic regulation of gene expression is an important biological determinant of protein production and cellular differentiation and plays a significant pathogenic role in a number of human diseases. Epigenetic regulation involves heritable modification of genetic material without changing its nucleotide sequence. Typically, epigenetic regulation is mediated by selective and reversible modification (*e.g.,* methylation) of DNA and proteins *(e.g.,* histones) that control the conformational transition between transcriptionally active and inactive states of chromatin. These covalent modifications can be controlled by enzymes such as methyltransferases *(e.g.,* SMYD proteins such as SMYD3 and SMYD2), many of which are associated with genetic alterations that can cause human disease, such as proliferative disorders. Thus, there is a need for the development of small molecules that are capable of inhibiting the activity of SMYD proteins such as SMYD3 and SMYD2.

ANDREW FERGUSSON ET AL, STRUCTURE, ELSEVIER, AMSTERDAM, NL, vol. 19, no. 9, ISSN 0969-2126, (20110613), pages 1262 - 1273 relates to structural basis of substrate methylation and inhibition of SMYD2.

### BRIEF SUMMARY OF THE INVENTION

In one aspect according to claim 1, the present disclosure provides a compound for use in treating cancer, the compound having Formula I below.

In another aspect according to claim 11, the present disclosure provides pharmaceutical composition for use in treating cancer, the pharmaceutical composition comprising the compound having Formula I below, or a pharmaceutically acceptable salt or hydrate thereof, and a pharmaceutically acceptable carrier.

The present disclosure provides substituted pyrrolidine compounds represented by Formula I below, and the pharmaceutically acceptable salts and solvates thereof, collectively referred to herein as "Compounds of the Disclosure."

The present disclosure also provides a Compound of the Disclosure and one or more pharmaceutically acceptable carriers.

The present disclosure also provides a method of inhibiting SMYD proteins, such as SMYD3 or SMYD2, or both, in a mammal, comprising administering to the mammal an effective amount of at least one Compound of the Disclosure.

The present disclosure also provides methods for treating a disease, disorder, or condition, *e.g.,* cancer, responsive to inhibition of SMYD proteins, such as SMYD3 or SMYD2, or both, comprising administering a therapeutically effective amount of a Compound of the Disclosure.

The present disclosure also provides the use of Compounds of the Disclosure as inhibitors of SMYD3.

The present disclosure also provides the use of Compounds of the Disclosure as inhibitors of SMYD2.

The present disclosure also provides the use of Compounds of the Dicslosure as inhibitors of SMYD proteins.

The present disclosure also provides a pharmaceutical composition for treating a disease, disorder, or condition responsive to inhibition of SMYD proteins, such as SMYD3 or SMYD2, or both, wherein the pharmaceutical composition comprises a therapeutically effective amount of a Compound of the Disclosure in a mixture with one or more pharmaceutically acceptable carriers.

The present disclosure also provides Compounds of the Disclosure for use in treating cancer in a mammal, e.g., breast, cervical, colon, kidney, liver, head and neck, skin, pancreatic, ovary, esophageal, lung, and prostate cancer.

The present disclosure also provides a Compound of the Disclosure for use in the manufacture of a medicament for treating cancer in a mammal.

The present disclosure also provides kit comprising a Compound of the Disclosure.

Additional embodiments and advantages of the disclosure will be set forth, in part, in the description that follows, and will flow from the description, or can be learned by practice of the disclosure. The embodiments and advantages of the disclosure will be realized and attained by means of the elements and combinations particularly pointed out in the appended claims.

It is to be understood that both the foregoing summary and the following detailed description are exemplary and explanatory only, and are not restrictive of the invention as claimed.

DETAILED DESCRIPTION OF THE INVENTION

The present disclosure is based on the use of Compounds of the Disclosure as inhibitors of SMYD proteins. In view of this property, the Compounds of the Disclosure are useful for treating diseases, disorders, or conditions, e.g., cancer, responsive to inhibition of SMYD proteins.

The present disclosure is also based on the use of Compounds of the Disclosure as inhibitors of SMYD3. In view of this property, the Compounds of the Disclosure are useful for treating diseases, disorders, or conditions, e.g., cancer, responsive to inhibition of SMYD3.

The present disclosure is also based on the use of Compounds of the Disclosure as inhibitors of SMYD2. In view of this property, the Compounds of the Disclosure are useful for treating diseases, disorders, or conditions, e.g., cancer, responsive to inhibition of SMYD2.

In one embodiment, Compounds of the Disclosure are compounds having Formula I:

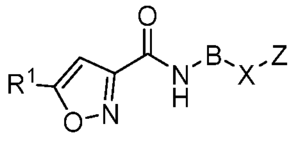

I

and the pharmaceutically acceptable salts or solvates, e.g., hydrates, thereof, wherein:

B is:

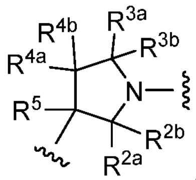

;

X is selected from the group consisting of $-S(=O)_2-$, $-S(=O)_2N(R^6)-$, $-S(=O)_2C(R^7)(H)-$, -C(=O)-, $-C(=O)N(R^6)-$, -C(=O)O-, and $-C(=O)C(R^7)(H)-$; or X is absent, i.e., Z forms a bond with the pyrrolidine nitrogen atom;

Z is selected from the group consisting of hydrogen, optionally substituted $C_{1-6}$ alkyl, haloalkyl, (cycloalkyl)alkyl, hydroxyalkyl, (amino)alkyl, (alkylamino)alkyl, (dialkylamino)alkyl, (cycloalkylamino)alkyl, (heterocyclo)alkyl, (amino)(hydroxy)alkyl, (amino)(aryl)alkyl, (hydroxy)(aryl)alkyl, (aralkylamino)alkyl, alkoxyalkyl, optionally substituted $C_{6-14}$ aryl, optionally substituted 4- to 14-membered heterocyclo, optionally substituted 5- to 14-membered heteroaryl, optionally substituted $C_{3-12}$ cycloalkyl, aralkyl, heteroaralkyl, and $-CH_2N(H)C(=O)R^{8c}$;

$R^1$ is selected from the group consisting of hydrogen, cyano, $C_{1-6}$ alkyl, haloalkyl, optionally substituted 4- to 14-membered heterocyclo, alkynyl, and $C_{3-6}$ cycloalkyl;

$R^{2a}$, $R^{2b}$, $R^{3a}$, $R^{3b}$, $R^{4a}$, and $R^{4b}$ are each independently selected from the group consisting of hydrogen, amino, alkylamino, dialkylamino, cycloalkylamino, halo, hydroxy, $C_{1-6}$ alkyl, alkoxy, haloalkyl, hydroxyalkyl, (amino)alkyl, (alkylamino)alkyl, (dialkylamino)alkyl, (cycloalkylamino)alkyl, (heterocyclo)alkyl, optionally substituted $C_{3-12}$ cycloalkyl, optionally substituted $C_{6-14}$ aryl, optionally substituted 4- to 14-membered heterocyclo, optionally substituted 5- to 14-membered heteroaryl, alkoxyalkyl, aralkyl, alkoxycarbonyl, sulfonamido, carboxamido, $-N(H)C(=O)R^{8a}$; and $-CH_2N(H)C(=O)R^{8b}$; or

$R^{2a}$ and $R^{2b}$ taken together with the carbon atom to which they are attached form a carbonyl; and $R^{3a}$, $R^{3b}$, $R^{4a}$, and $R^{4b}$ are each independently selected from the group consisting of hydrogen, hydroxy, $C_{1-6}$ alkyl, alkoxy, hydroxyalkyl, $C_{3-12}$ cycloalkyl, optionally substituted $C_{6-14}$ aryl, alkoxyalkyl, aralkyl, $-N(H)C(=O)R^{8a}$; and $-CH_2N(H)C(=O)R^{8b}$; or

$R^{3a}$ and $R^{3b}$ taken together with the carbon atom to which they are attached form a carbonyl; and $R^{2a}$, $R^{2b}$, $R^{4a}$, and $R^{4b}$ are each independently selected from the group consisting of hydrogen, hydroxy, $C_{1-6}$ alkyl, alkoxy, hydroxyalkyl, $C_{3-12}$ cycloalkyl, optionally substituted $C_{6-14}$ aryl, alkoxyalkyl, $-N(H)C(=O)R^{8a}$; and $-CH_2N(H)C(=O)R^{8b}$; or

$R^{4a}$ and $R^{4b}$ taken together with the carbon atom to which they are attached form a carbonyl; and $R^{2a}$, $R^{2b}$, $R^{3a}$, and $R^{3b}$ are each independently selected from the group consisting of hydrogen, hydroxy, $C_{1-6}$ alkyl, alkoxy, hydroxyalkyl, $C_{3-12}$ cycloalkyl, optionally substituted $C_{6-14}$ aryl, alkoxyalkyl, $-N(H)C(=O)R^{8a}$; and $-CH_2N(H)C(=O)R^{8b}$; or

$R^{2a}$ and $R^{2b}$ taken together with the carbon atom to which they are attached form a $C_{3-6}$ cycloalkyl or $C_{3-6}$ heterocyclo; and $R^{3a}$, $R^{3b}$, $R^{4a}$, and $R^{4b}$ are each independently selected from the group consisting of hydrogen, hydroxy, $C_{1-6}$

alkyl, alkoxy, hydroxyalkyl, $C_{3-12}$ cycloalkyl, optionally substituted $C_{6-14}$ aryl, alkoxyalkyl, aralkyl, $-N(H)C(=O)R^{8a}$; and $-CH_2N(H)C(=O)R^{8b}$; or

$R^{3a}$ and $R^{3b}$ taken together with the carbon atom to which they are attached form a $C_{3-6}$ cycloalkyl or $C_{3-6}$ heterocyclo; and $R^{2a}$, $R^{2b}$, $R^{4a}$, and $R^{4b}$ are each independently selected from the group consisting of hydrogen, hydroxy, $C_{1-6}$ alkyl, alkoxy, hydroxyalkyl, $C_{3-12}$ cycloalkyl, optionally substituted $C_{6-14}$ aryl, alkoxyalkyl, $-N(H)C(=O)R^{8a}$; and $-CH_2N(H)C(=O)R^{8b}$; or

$R^{4a}$ and $R^{4b}$ taken together with the carbon atom to which they are attached form a $C_{3-6}$ cycloalkyl or $C_{3-6}$ heterocyclo; and $R^{2a}$, $R^{2b}$, $R^{3a}$, and $R^{3b}$ are each independently selected from the group consisting of hydrogen, hydroxy, $C_{1-6}$ alkyl, alkoxy, hydroxyalkyl, $C_{3-12}$ cycloalkyl, optionally substituted $C_{6-14}$ aryl, alkoxyalkyl, $-N(H)C(=O)R^{8a}$; and $-CH_2N(H)C(=O)R^{8b}$;

$R^5$ is selected from the group consisting of hydrogen and $C_{1-4}$ alkyl;

$R^6$ is selected from the group consisting of hydrogen and $C_{1-4}$ alkyl;

$R^7$ is selected from the group consisting of hydrogen, $C_{1-4}$ alkyl, amino, alkylamino, dialkylamino, (amino)alkyl, (alkylamino)alkyl, (dialkylamino)alkyl, and hydroxyalkyl;

$R^{8a}$ is selected from the group consisting of $C_{1-6}$ alkyl, haloalkyl, hydroxyalkyl, (amino)alkyl, (alkylamino)alkyl, (dialkylamino)alkyl, (cycloalkylamino)alkyl, (heterocyclo)alkyl, optionally substituted $C_{6-14}$ aryl, optionally substituted 4- to 14-membered heterocyclo, optionally substituted 5- to 14-membered heteroaryl, optionally substituted $C_{3-12}$ cycloalkyl, aralkyl, and heteroaralkyl;

$R^{8b}$ is selected from the group consisting of $C_{1-6}$ alkyl, haloalkyl, hydroxyalkyl, (amino)alkyl, (alkylamino)alkyl, (dialkylamino)alkyl, (cycloalkylamino)alkyl, (heterocyclo)alkyl, optionally substituted $C_{6-14}$ aryl, optionally substituted 4- to 14-membered heterocyclo, optionally substituted 5- to 14-membered heteroaryl, optionally substituted $C_{3-12}$ cycloalkyl, aralkyl, and heteroaralkyl; and

$R^{8c}$ is selected from the group consisting of $C_{1-6}$ alkyl, haloalkyl, hydroxyalkyl, (amino)alkyl, (alkylamino)alkyl, (dialkylamino)alkyl, (cycloalkylamino)alkyl, (heterocyclo)alkyl, optionally substituted $C_{6-14}$ aryl, optionally substituted 4- to 14-membered heterocyclo, optionally substituted 5- to 14-membered heteroaryl, optionally substituted $C_{3-12}$ cycloalkyl, aralkyl, and heteroaralkyl.

In another embodiment, Compounds of the Disclosure are compounds having Formula I, and the pharmaceutically acceptable salts or solvates, e.g., hydrates, thereof, wherein Z is selected from the group consisting of hydrogen, optionally substituted $C_{1-6}$ alkyl, hydroxyalkyl, (amino)alkyl, (alkylamino)alkyl, (dialkylamino)alkyl, (cycloalkylamino)alkyl, (heterocyclo)alkyl, (amino)(hydroxy)alkyl, (amino)(aryl)alkyl, (hydroxy)(aryl)alkyl, (aralkylamino)alkyl, alkoxyalkyl, optionally substituted $C_{6-14}$ aryl, optionally substituted 4- to 14-membered heterocyclo, optionally substituted 5- to 14-membered heteroaryl, optionally substituted $C_{3-12}$ cycloalkyl, aralkyl, and heteroaralkyl.

In another embodiment, Compounds of the Disclosure are compounds having Formula I, and the pharmaceutically acceptable salts or solvates, e.g., hydrates, thereof, wherein $R^1$ is selected from the group consisting of hydrogen, $C_{1-6}$ alkyl, and $C_{3-6}$ cycloalkyl.

In another embodiment, Compounds of the Disclosure are compounds having Formula I, and the pharmaceutically acceptable salts or solvates, e.g., hydrates, thereof, wherein X is selected from the group consisting of $-S(=O)_2-$, $-S(=O)_2N(R^6)-$, $-S(=O)_2C(R^7)(H)-$, $-C(=O)-$, $-C(=O)N(R^6)-$, $-C(=O)O-$, and $-C(=O)C(R^7)(H)-$, i.e., X is not absent.

In another embodiment, Compounds of the Disclosure are compounds having Formula I, and the pharmaceutically acceptable salts or solvates, e.g., hydrates, thereof, wherein $R^1$ is selected from the group consisting of ethyl, cyclopropyl, and cyclopentyl.

In another embodiment, Compounds of the Disclosure are compounds having having Formula I, and the pharmaceutically acceptable salts or solvates, e.g., hydrates, thereof, wherein B is selected from the group consisting of:

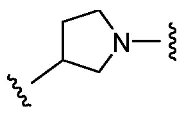 , 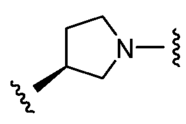 and 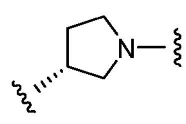 ;

and $R^1$, X, and Z are as defined above in connection with Formula I.

In another embodiment, Compounds of the Disclosure are compounds having having Formula I, and the pharmaceutically acceptable salts or solvates, e.g., hydrates, thereof, wherein B is selected from the group consisting of:

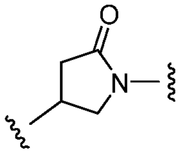 , 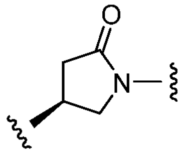 and 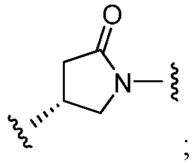 ;

and $R^1$, X, and Z are as defined above in connection with Formula **I.**

In another embodiment, Compounds of the Disclosure are compounds having having Formula **I,** and the pharmaceutically acceptable salts or solvates, e.g., hydrates, thereof, wherein B is selected from the group consisting of:

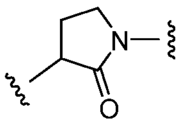 , 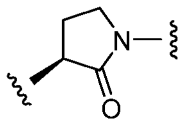 and 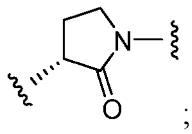 ;

and $R^1$, X, and Z are as defined above in connection with Formula I.

In another embodiment, Compounds of the Disclosure are compounds having having Formula **I,** and the pharmaceutically acceptable salts or solvates, e.g., hydrates, thereof, wherein B is selected from the group consisting of:

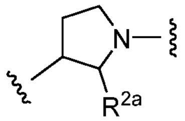 , 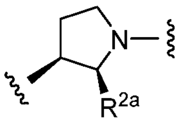 , 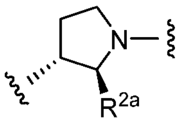 ,

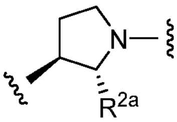 and 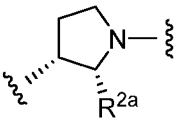 ;

$R^{2a}$ is selected from the group consisting of $C_{1-6}$ alkyl, $C_{3-12}$ cycloalkyl, and optionally substituted $C_{6-14}$ aryl; and $R^1$, X, and Z are as defined above in connection with Formula **I**. In another embodiment, $R^{2a}$ is selected from the group consisting of:

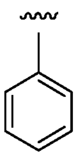 , 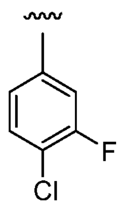 , 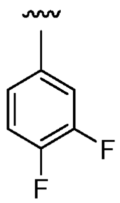 , 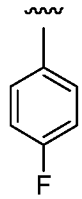 and 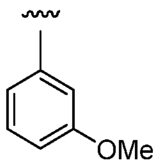 .

In another embodiment, Compounds of the Disclosure are compounds having having Formula **I,** and the pharmaceutically acceptable salts or solvates, e.g., hydrates, thereof, wherein B is selected from the group consisting of:

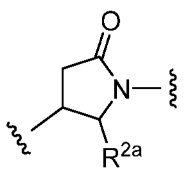 , 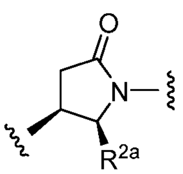 , 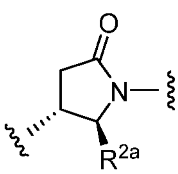 ,

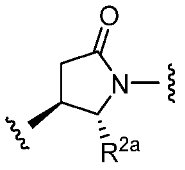 and 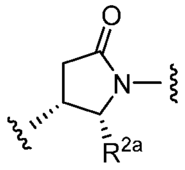 ;

$R^{2a}$ is selected from the group consisting of $C_{1-6}$ alkyl, $C_{3-12}$ cycloalkyl, and optionally substituted $C_{6-14}$ aryl; and $R^1$, X, and Z are as defined above in connection with Formula **I**. In another embodiment, $R^{2a}$ is selected from the group consisting of:

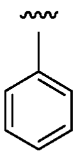 , 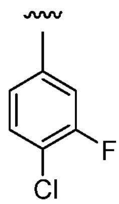 , 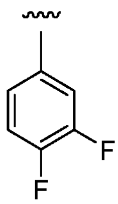 , 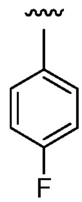 and 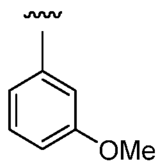 .

In another embodiment, Compounds of the Disclosure are compounds having having Formula I, and the pharmaceutically acceptable salts or solvates, e.g., hydrates, thereof, wherein B is selected from the group consisting of:

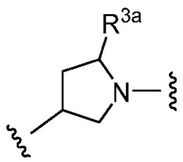 , 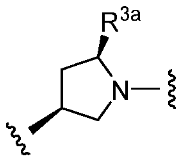 , 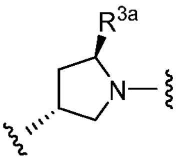 ,

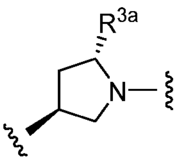 and 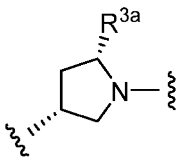 ;

$R^{3a}$ is selected from the group consisting of $C_{1-6}$ alkyl, hydroxyalkyl, $C_{3-12}$ cycloalkyl, optionally substituted $C_{6-14}$ aryl, alkoxyalkyl, and $-CH_2N(H)C(=O)R^{8b}$; and $R^1$, X, and Z are as defined above in connection with Formula **I**. In another embodiment, $R^{3a}$ is selected from the group consisting of:

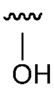 , 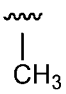 , 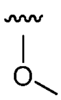 , 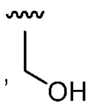 , 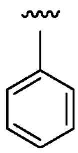 , 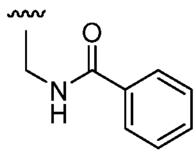 ,

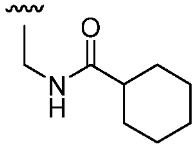 , 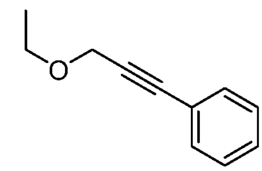 and 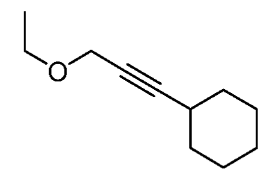

In another embodiment, Compounds of the Disclosure are compounds having having Formula **I,** and the pharmaceutically acceptable salts or solvates, e.g., hydrates, thereof, wherein B is selected from the group consisting of:

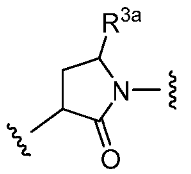 , 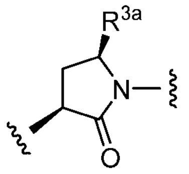 , 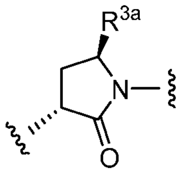 ,

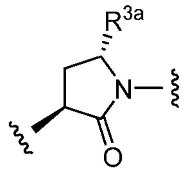 and 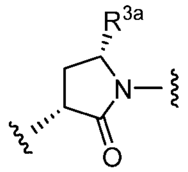 ;

$R^{3a}$ is selected from the group consisting of $C_{1-6}$ alkyl, hydroxyalkyl, $C_{3-12}$ cycloalkyl, optionally substituted $C_{6-14}$ aryl, alkoxyalkyl, and $-CH_2N(H)C(=O)R^{8b}$; and $R^1$, X, and Z are as defined above in connection with Formula **I**. In another embodiment, $R^{3a}$ is selected from the group consisting of:

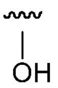 , 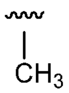 , 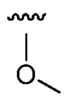 , 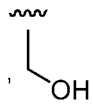 , 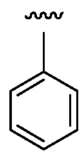 , 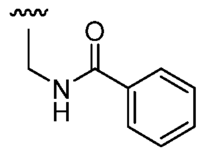 ,

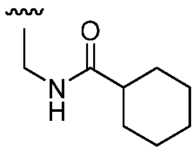 , 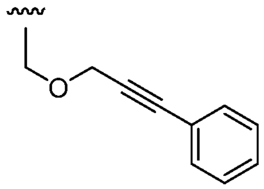 and 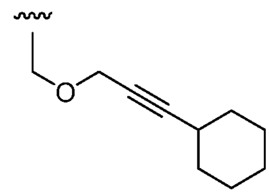 .

In another embodiment, Compounds of the Disclosure are compounds having having Formula **I,** and the pharmaceutically acceptable salts or solvates, e.g., hydrates, thereof, wherein B is selected from the group consisting of:

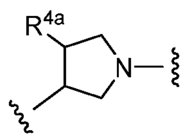 , 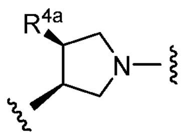 , 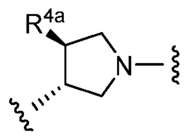 ,

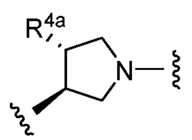 and 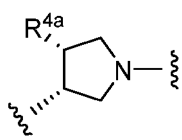 ;

$R^{4a}$ is selected from the group consisting of hydroxy, $C_{1-6}$ alkyl, alkoxy, hydroxyalkyl, $C_{3-12}$ cycloalkyl, optionally substituted $C_{6-14}$ aryl, alkoxyalkyl, aralkyl, $-N(H)C(=O)R^{8a}$, and $-CH_2N(H)C(=O)R^{8b}$; and $R^1$, X, and Z are as defined above in connection with Formula **I**. In another embodiment, $R^{4a}$ is selected from the group consisting of:

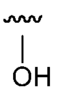 , 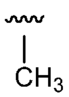 , 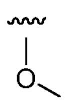 , 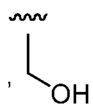 , 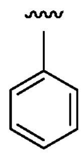 , 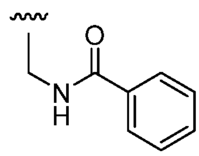 ,

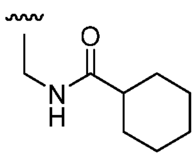, 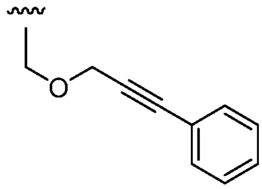 and 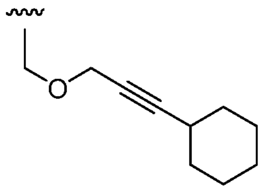

In another embodiment, Compounds of the Disclosure are compounds having having Formula **I,** and the pharmaceutically acceptable salts or solvates, e.g., hydrates, thereof, wherein B is selected from the group consisting of:

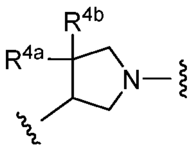, 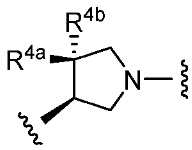, 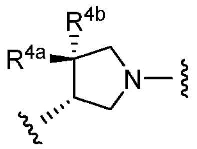,

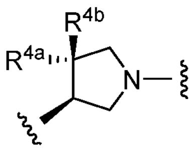 and 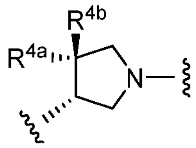;

$R^{4a}$ is $C_{1-4}$ alkyl; $R^{4b}$ is hydroxy; and $R^1$, X, and Z are as defined above in connection with Formula **I.**

In another embodiment, Compounds of the Disclosure are compounds having having Formula **I,** and the pharmaceutically acceptable salts or solvates, e.g., hydrates, thereof, wherein X is selected from the group consisting of $-S(=O)_2-$ and -C(=O)-; or X is absent; and $R^1$, X, and Z are as defined above in connection with Formula **I.** In another embodiment, X is $-S(=O)_2-$. In another embodiment, X is -C(=O)-. In another embodiment, X is absent.

In another embodiment, Compounds of the Disclosure are compounds having having Formula **I,** and the pharmaceutically acceptable salts or solvates, e.g., hydrates, thereof, wherein Z is selected from the group consisting of optionally substituted $C_{1-6}$ alkyl, hydroxyalkyl, (amino)alkyl, (alkylamino)alkyl, (dialkylamino)alkyl, (cycloalkylamino)alkyl, (heterocyclo)alkyl, optionally substituted $C_{6-14}$ aryl, optionally substituted 4- to 14-membered heterocyclo, optionally substituted 5- to 14-membered heteroaryl, optionally substituted $C_{3-12}$ cycloalkyl, aralkyl, and heteroaralkyl; and $R^1$, X, and Z are as defined above in connection with Formula **I.** In another embodiment, Z is selected from the group consisting of optionally substituted $C_{1-6}$ alkyl, (amino)alkyl, (alkylamino)alkyl, (heterocyclo)alkyl, optionally substituted $C_{6-14}$ aryl, optionally substituted 4- to 14-membered heterocyclo, optionally substituted 5- to 14-membered heteroaryl, optionally substituted $C_{3-12}$ cycloalkyl, aralkyl, and heteroaralkyl.

In another embodiment, Compounds of the Disclosure are compounds having having Formula **I,** and the pharmaceutically acceptable salts or solvates, e.g., hydrates, thereof, wherein $R^1$ is ethyl; and $R^1$, X, and Z are as defined above in connection with Formula **I.**

In another embodiment, Compounds of the Disclosure are compounds having having Formula **I,** and the pharmaceutically acceptable salts or solvates, e.g., hydrates, thereof, wherein $R^1$ is cyclopropyl; and $R^1$, X, and Z are as defined above in connection with Formula **I.**

In another embodiment, Compounds of the Disclosure are compounds having having Formula **I,** and the pharmaceutically acceptable salts or solvates, e.g., hydrates, thereof, wherein $R^1$ is cyclopentyl; and $R^1$, X, and Z are as defined above in connection with Formula **I.**

In another embodiment, Compounds of the Disclosure are compounds having having Formula **I,** and the pharmaceutically acceptable salts or solvates, e.g., hydrates, thereof, wherein $R^1$ is ethyl; X is selected from the group consisting of $-S(=O)_2-$, $-S(=O)_2N(R^6)-$, $-S(=O)_2C(R^7)(H)-$, -C(=O)-, $-C(=O)N(R^6)-$, -C(=O)O-, and $-C(=O)C(R^7)(H)-$, i.e., X is not absent; and $R^1$ and Z are as defined above in connection with Formula **I.**

In another embodiment, Compounds of the Disclosure are compounds having having Formula **I,** and the pharmaceutically acceptable salts or solvates, e.g., hydrates, thereof, wherein $R^1$ is cyclopropyl; X is selected from the group consisting of $-S(=O)_2-$, $-S(=O)_2N(R^6)-$, $-S(=O)_2C(R^7)(H)-$, -C(=O)-, $-C(=O)N(R^6)-$, -C(=O)O-, and $-C(=O)C(R^7)(H)-$, i.e., X is not absent; and $R^1$ and Z are as defined above in connection with Formula **I.**

In another embodiment, Compounds of the Disclosure are compounds having having Formula **I,** and the pharmaceutically acceptable salts or solvates, e.g., hydrates, thereof, wherein $R^1$ is cyclopentyl; X is selected from the group

consisting of -$S(=O)_2$-, -$S(=O)_2N(R^6)$-, -$S(=O)_2C(R^7)(H)$-, -C(=O)-, -$C(=O)N(R^6)$-, -C(=O)O-, and -$C(=O)C(R^7)(H)$-, i.e., X is not absent; and $R^1$ and Z are as defined above in connection with Formula **I.**

In another embodiment, Compounds of the Disclosure are compounds of Tables 1-3, and the pharmaceutically acceptable salts or solvates, e.g., hydrates, thereof, or different pharmaceutically acceptable salt thereof.

It should be appreciated that the Compounds of the Disclosure in certain embodiments are the free base, various salts, and hydrate forms, and are not limited to the particular salt listed in Tables 1-3.

Table 1

| Cpd. No. | Structure | Salt Form | Chemical Name | LCMS M+H | SMYD2 Biochem $IC_{50}$ (μM)* | SMYD3 Biochem $IC_{50}$ (μM)* |
|---|---|---|---|---|---|---|
| 1 | 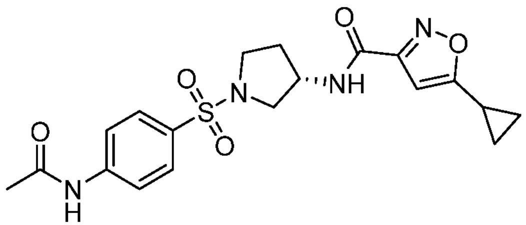 | None | (S)-N-(1-((4-acetamidophenyl)sulfonyl)pyrrolidi n-3-yl)-5-cyclopropylisoxazole-3-carboxamide | 419.4 | >50 | >100 |
| 2 | 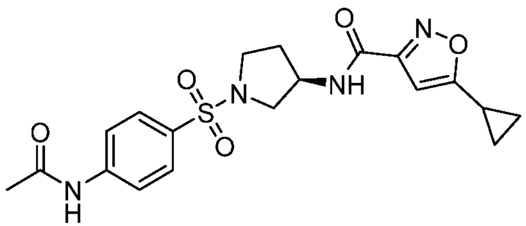 | None | (R)-N-(1-((4-acetamidophenyl)sulfonyl)pyrrolidi n-3-yl)-5-cyclopropylisoxazole-3-carboxamide | 419.3 | >50 | >100 |
| 3 | 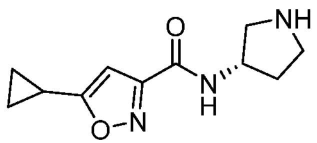 | HCl | (S)-5-cyclopropyl-N-(pyrrolidin-3- yl)isoxazole-3-carboxamide | 222.05 | 48.59574 | 80.35 |
| 4 | 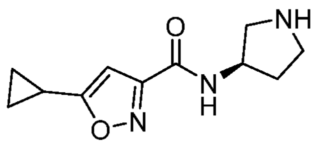 | HCl | (R)-5-cyelopropyl-N-(pyrrolidin-3-yl)isoxazole-3-carboxamide | 222 | >50 | 83.67 |

(continued)

| Cpd. No. | Structure | Salt Form | Chemical Name | LCMS M+H | SMYD2 Biochem $IC_{50}$ (μM)* | SMYD3 Biochem $IC_{50}$ (μM)* |
|---|---|---|---|---|---|---|
| 5 | 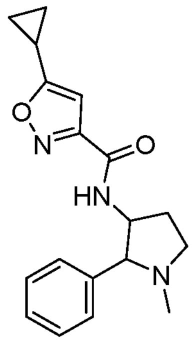 | None | 5-cyclopropyl-N-(1-methyl-2-phenylpyrrolidin-3-yl) isoxazole-3-carboxamide | 312.2 | >50 | 55.1 |
| 6 | 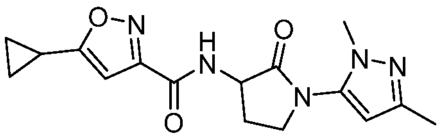 | None | 5-cyclopropyl-N-(1-(1,3-dimethyl-1H-pyrazol-5-yl)-2-oxopyrrolidin-3-yl)isoxazole-3-carboxamide | 330.2 | >50 | >200 |
| 7 | 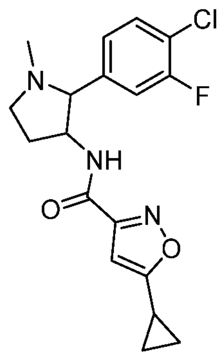 | None | N-(2-(4-chloro-3-fluorophenyl)-1-methylpyrrolidin-3-yl)-5-cyclopropylisoxazole-3-carboxamide | 364.2 | >50 | 51.5 |

(continued)

| Cpd. No. | Structure | Salt Form | Chemical Name | LCMS M+H | SMYD2 Biochem $IC_{50}$ (μM)* | SMYD3 Biochem $IC_{50}$ (μM)* |
|---|---|---|---|---|---|---|
| 8 | 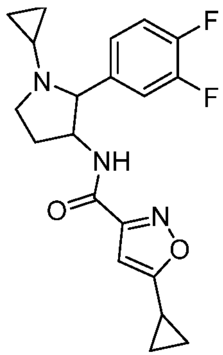 | None | 5-cyclopropyl-N-(1-cyclopropyl-2-(3,4-difluorophenyl)pyrrolidin-3-yl)isoxazole-3-carboxamide | 374.3 | >50 | 12.38 |
| 9 | 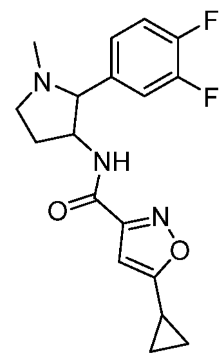 | None | 5-cyclopropyl-N-(2-(3,4-difluorophenyl)-1-methylpyrrolidin-3-yl)isoxazole-3-carboxamide | 348.1 | >50 | 67.23 |
| 10 | 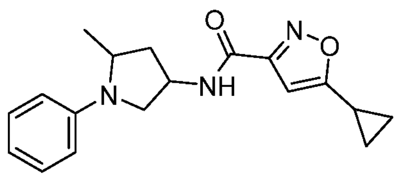 | TFA | 5-cyclopropyl-N-(5-methyl-1-phenylpyrrolidin-3-yl) isoxazole-3-carboxamide | 312.1 | >50 | 53.53 |

(continued)

| Cpd. No. | Structure | Salt Form | Chemical Name | LCMS M+H | SMYD2 Biochem $IC_{50}$ (μM)* | SMYD3 Biochem $IC_{50}$ (μM)* |
|---|---|---|---|---|---|---|
| 11 | 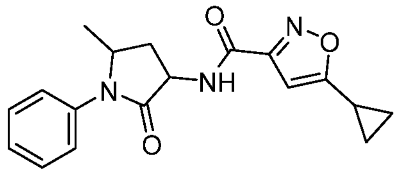 | None | 5-cyclopropyl-N-(5-methyl-2-oxo-1-phenylpyrrolidin-3-yl)isoxazole-3-carboxamide | 326.1 | >50 | 78.07 |
| 12 | 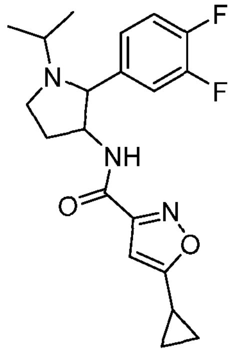 | None | 5-cyclopropyl-N-(2-(3,4-difluorophenyl)-1-isopropylpyrrolidin-3-yl)isoxazole-3-carboxamide | 376.3 | >50 | 58.6 |
| 13 | 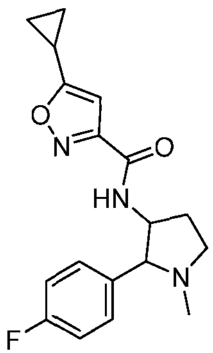 | None | 5-cyclopropyl-N-(2-(4-fluorophenyl)-1-methylpyrrolidin-3-yl)isoxazole-3 -carboxamide | 330.1 | >50 | 51.85 |

(continued)

| Cpd. No. | Structure | Salt Form | Chemical Name | LCMS M+H | SMYD2 Biochem $IC_{50}$ (µM)* | SMYD3 Biochem $IC_{50}$ (µM)* |
|---|---|---|---|---|---|---|
| 14 | 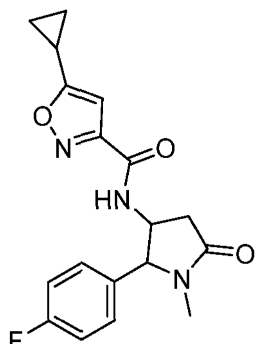 | None | 5-cyclopropyl-N-(2-(4-fluorophenyl)-1-methyl-5-oxopyrrolidin-3-yl)isoxazole-3-carboxamide | 344.1 | >50 | 58.51 |
| 15 | 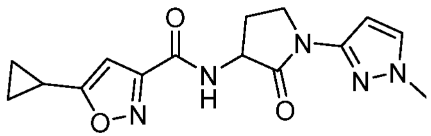 | None | 5-cyclopropyl-N-(1-(1-methyl-1H-pyrazol-3-yl)-2-oxopyrrolidin-3-yl)isoxazole-3-carboxamide | 316.1 | >50 | 157.72 |
| 16 | 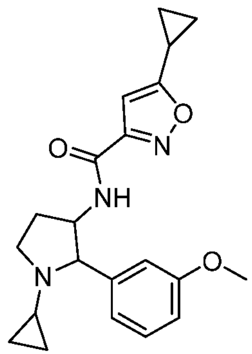 | None | 5-cyclopropyl-N-(1-cyclopropyl-2-(3-methoxyphenyl) pyrrolidin-3-yl)isoxazole-3-carboxamide | 368.3 | >50 | 77.42 |

(continued)

| Cpd. No. | Structure | Salt Form | Chemical Name | LCMS M+H | SMYD2 Biochem $IC_{50}$ (μM)* | SMYD3 Biochem $IC_{50}$ (μM)* |
|---|---|---|---|---|---|---|
| 17 | 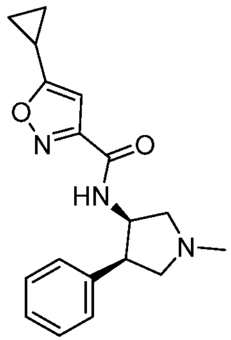 | None | (±)-*cis*-5-cyclopropyl-N-((1-methyl-4-phenylpyrrolidin-3-yl)isoxazole-3-carboxamide | 312.3 | >50 | 56.23 |
| 18 | 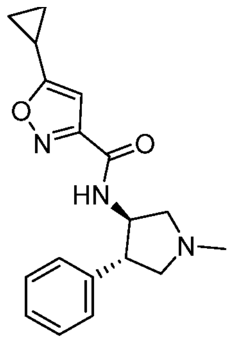 | None | (±)-*trans*-5-cyclopropyl-N-(1-methyl-4-phenylpyrrolidin-3-yl)i soxazole-3-carboxami de | 312.2 | >50 | 50.39 |
| 19 | 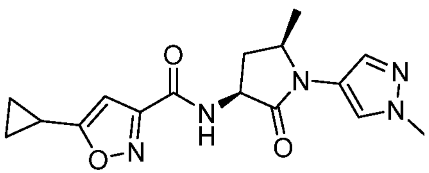 | None | (±)-*cis*-5-cyclopropyl-N-(5-methyl-1-(1-methyl-1H-pyrazol-4-yl)-2-oxopyrrolidin-3-yl)isoxazole-3-carboxamide | 330.2 | >50 | >200 |

(continued)

| Cpd. No. | Structure | Salt Form | Chemical Name | LCMS M+H | SMYD2 Biochem $IC_{50}$ (μM)* | SMYD3 Biochem $IC_{50}$ (μM)* |
|---|---|---|---|---|---|---|
| 20 | 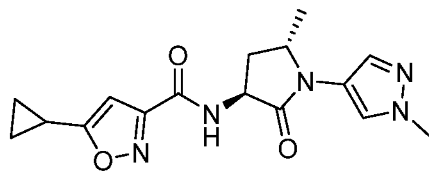 | None | (±)-*trans* 5-cyclopropyl-N-(5-methyl-1-(1-methyl-1H-pyrazol-4-yl)-2-oxopyrrolidin-3-yl)isoxazole-3-carboxamide | 330.2 | >50 | >200 |
| 21 | 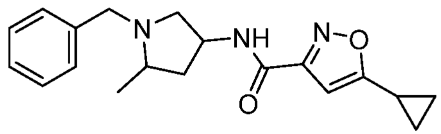 | None | N-(1-benzyl-5-methylpyrrolidin-3-yl)-5-cyclopropylisoxazole-3-carboxamide | 326.1 | 44.50781 | 54.12 |
| 22 | 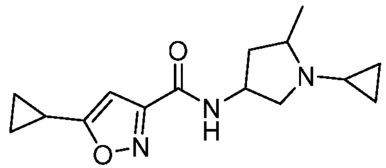 | None | 5-cyclopropyl-N-(1-cyclopropyl-5-methylpyrrolidin-3-yl)isoxazole-3-carboxamide | 276.2 | >50 | 47.06 |
| 23 | 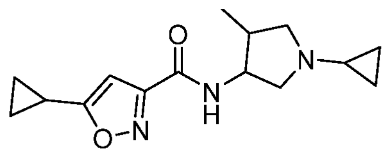 | None | 5-cyclopropyl-N-(1-cyclopropyl-4-methylpyrrolidin-3-yl)isoxazole-3-carboxamide | 276.2 | 5.17947 | 48.48 |
| 24 | 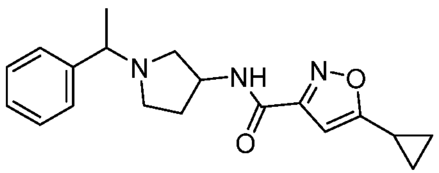 | None | 5-cyclopropyl-N-(1-(1-phenylethyl)pyrrolidin-3-yl) isoxazole-3-carboxamide | 326.1 | >50 | 38.36 |

(continued)

| Cpd. No. | Structure | Salt Form | Chemical Name | LCMS M+H | SMYD2 Biochem $IC_{50}$ (μM)* | SMYD3 Biochem $IC_{50}$ (μM)* |
|---|---|---|---|---|---|---|
| 25 | 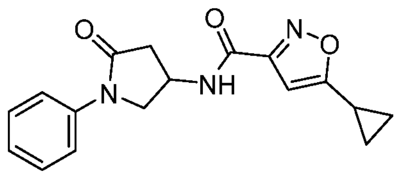 | None | 5-cyclopropyl-N-(5-oxo-1-phenylpyrrolidin-3-yl) isoxazole-3-carboxamide | 312.2 | >50 | 98.81 |
| 26 | 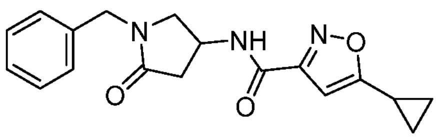 | None | N-(1-benzyl-5-oxopyrrolidin-3-yl)-5-cyclopropylisoxazole-3-carboxamide | 326.3 | >50 | 88.35 |
| 27 | 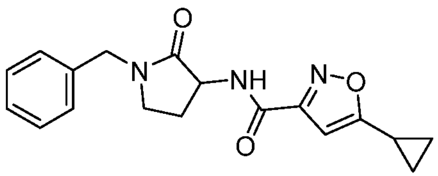 | None | N-(1-benzyl-2-oxopyrrolidin-3-yl)-5-cyclopropylisoxazole-3-carboxamide | 326.1 | >50 | >200 |
| 28 | 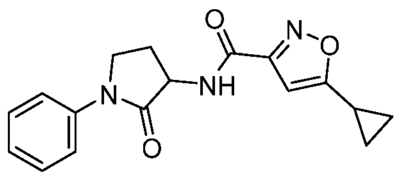 | None | 5-cyclopropyl-N-(2-oxo-1-phenylpyrrolidin-3-yl) isoxazole-3-carboxamide | 312.3 | >50 | 94.16 |
| 29 | 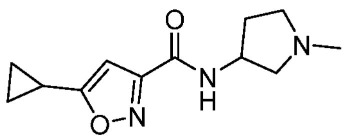 | None | 5-cyclopropyl-N-(1-methylpyrrolidin-3-yl)isoxazole-3-carboxamide | 236.2 | >50 | 84.04 |

(continued)

| Cpd. No. | Structure | Salt Form | Chemical Name | LCMS M+H | SMYD2 Biochem $IC_{50}$ (μM)* | SMYD3 Biochem $IC_{50}$ (μM)* |
|---|---|---|---|---|---|---|
| 30 | 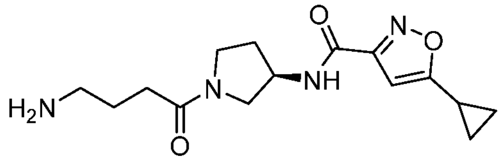 | HCl | (R)-N-(1-(4-aminobutanoyl)pyrrolidin-3-yl)-5-cyclopropylisoxazole-3-carboxamide | 307.1 | >50 | >10 |
| 31 | 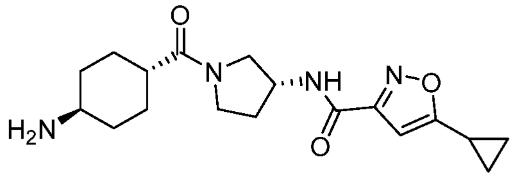 | HCl | N-((R)-1-((1r,4r)-4-aminocyclohexane-1-carbonyl) pyrrolidin-3-yl)-5-cyclopropylisoxazole-3-carboxamide | 347.2 | >50 | >10 |
| 32 | 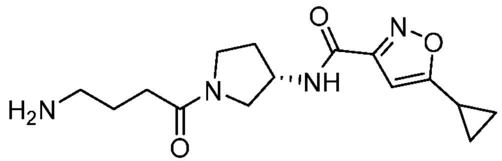 | HCl | (S)-N-(1-(4-aminobutanoyl)pyrrolidin-3-yl)-5-cyclopropylisoxazole-3-carboxamide | 307.1 | >50 | >10 |
| 33 | 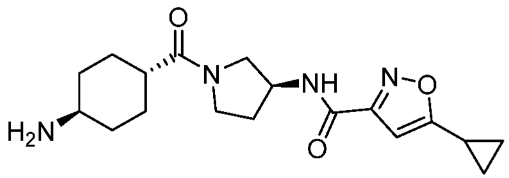 | HCl | N-((S)-1-((1r,4s)-4-aminocyclohexane-1-carbonyl) pyrrolidin-3-yl)-5-cyclopropylisoxazole-3-carboxamide | 347.15 | >50 | 3.25 |
| 34 | 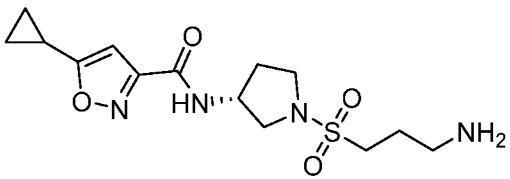 | None | (R)-N-(1-((3-aminopropyl)sulfonyl)pyrrolidin-3-yl)-5-cyclopropylisoxazole-3-carboxamide | 343.2 | >50 | 5.8 |

(continued)

| Cpd. No. | Structure | Salt Form | Chemical Name | LCMS M+H | SMYD2 Biochem $IC_{50}$ (μM)* | SMYD3 Biochem $IC_{50}$ (μM)* |
|---|---|---|---|---|---|---|
| 35 | 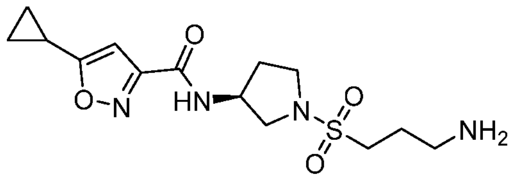 | None | (S)-N-(1-((3-aminopropyl)sulfonyl)pyrrolidin-3-yl)-5-cyclopropylisoxazole-3-carboxamide | 343 | >50 | 4.43 |
| 36 | 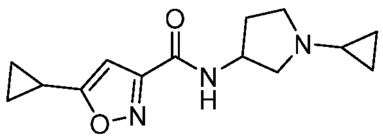 | None | 5-cyclopropyl-N-(1-cyclopropylpyrrolidin-3-yl) isoxazole-3-carboxamide | 262.3 | 32.4951 | |
| 37 | 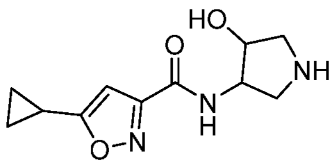 | None | 5-cyclopropyl-N-(4-hydroxypyrrolidin-3-yl)isoxazole-3-carboxamide | 238.2 | 36.11231 | |
| 38 | 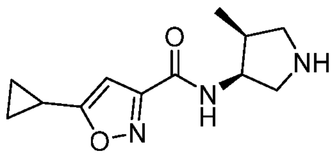 | TFA | (±)-*cis*-5-cyclopropyl-N-(4-methylpyrrolidin-3-yl) isoxazole-3-carboxamide | 236.3 | 15.4 | |
| 39 | 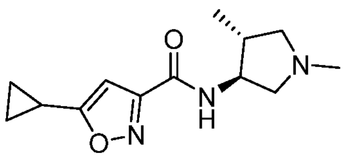 | None | (±)-*trans*-5-cyclopropyl-N-(1,4-dimethylpyrrolidin-3-yl)isoxazole-3-carboxamide | 250.4 | 33.6 | |
| 40 | 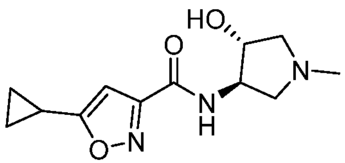 | None | (±)-*trans*-5-cyclopropyl-N-(-4-hydroxy-1-methylpyrrolidin-3 - yl)isoxazole-3-carboxamide | 252.2 | >50 | |

(continued)

| Cpd. No. | Structure | Salt Form | Chemical Name | LCMS M+H | SMYD2 Biochem $IC_{50}$ (μM)* | SMYD3 Biochem $IC_{50}$ (μM)* |
|---|---|---|---|---|---|---|
| 41 | 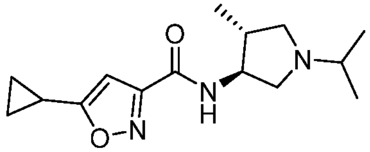 | TFA | 5-cyclopropyl-N-((3 S,4R)-1-isopropyl-4-methylpyrrolidin-3-yl)isoxazole-3-carboxamide | 278.3 | 18.2 | |
| 42 | 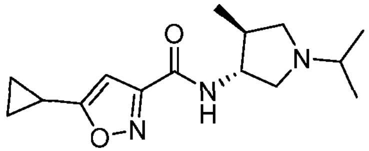 | TFA | 5-cyclopropyl-N-((3R,4S)-1-isopropyl-4-methylpyrrolidin-3-yl)isoxazole-3-carboxamide | 278.3 | 1.9 | |
| 43 | 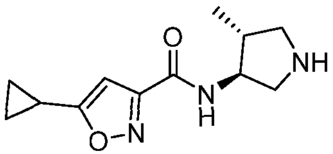 | TFA | (±)-*trans*-5-cyclopropyl-N-(4-methylpyrrolidin-3-yl) isoxazole-3-carboxamide | 236.4 | >50 | |
| 44 | 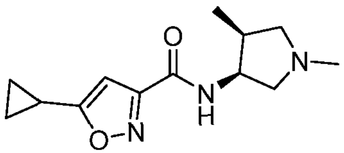 | None | (±)-*cis*-5-cyclopropyl-N-(1,4-dimethylpyrrolidin-3-yl) isoxazole-3-carboxamide | 249.9 | 4.6 | |
| 45 | 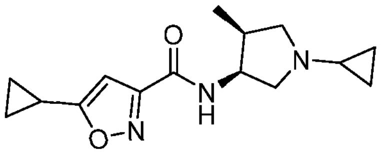 | TFA | (±)-*cis*-5-cyclopropyl-N-(1-cyclopropyl-4-methylpyrrolidin-3-yl)isoxazole-3-carboxamide | 275.9 | 19.17286 | |
| 46 | 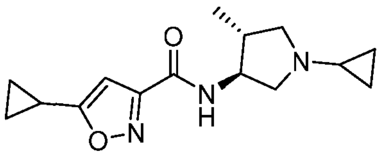 | TFA | (±)-*trans*-5-cyclopropyl-N-(1-cyclopropyl-4-methylpyrrolidin-3-yl)isoxazole-3-carboxamide | 276.2 | 2.74789 | |

(continued)

| Cpd. No. | Structure | Salt Form | Chemical Name | LCMS M+H | SMYD2 Biochem $IC_{50}$ (μM)* | SMYD3 Biochem $IC_{50}$ (μM)* |
|---|---|---|---|---|---|---|
| 47 | 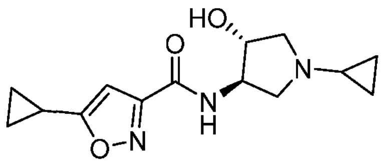 | None | (±)-*trans*-5-cyclopropyl-N-(1-cyclopropyl-4-hydroxypyrrolidin-3-yl)isoxazole-3-carboxamide | 278.2 | >50 | |
| 48 | 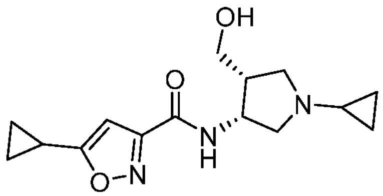 | None | (±)-*cis*-5-cyclopropyl-N-(1-cyclopropyl-4-(hydroxymethyl)pyrrolidin-3 - yl)isoxazole-3-carboxamide | 292.3 | 8.4585 | |
| 49 | 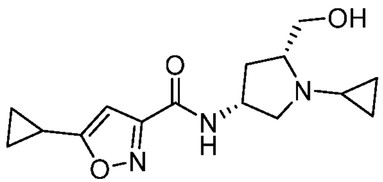 | None | (±)-*cis*-5-cyclopropyl-N-(1-cyclopropyl-5-(hydroxymethyl)pyrroli din-3 - yl)isoxazole-3-carboxamide | 292.3 | >50 | |
| 50 | 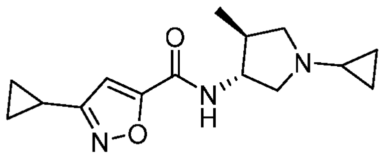 | None | (±)-*trans*-3-cyclopropyl-N-(1-cyclopropyl-4-methylpyrrolidin-3-yl)isoxazole-5-carboxamide | 276.7 | >50 | |
| 51 | 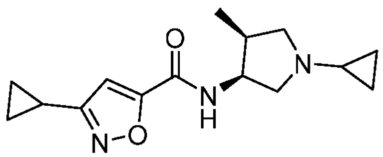 | None | (±)-*cis*-3-cyclopropyl-N-(1-cyclopropyl-4-methylpyrrolidin-3-yl)isoxazole-5-carboxamide | 276.3 | 7.9 | |

(continued)

| Cpd. No. | Structure | Salt Form | Chemical Name | LCMS M+H | SMYD2 Biochem $IC_{50}$ (μM)* | SMYD3 Biochem $IC_{50}$ (μM)* |
|---|---|---|---|---|---|---|
| 52 | 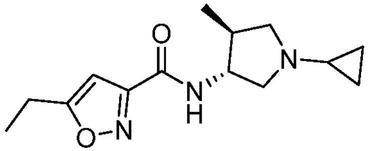 | TFA | (±)-*trans*-N-(1-cyclopropyl-4-methylpyrrolidin-3-yl)-5-ethylisoxazole-3-carboxamide | 264.3 | >50 | |
| 53 | 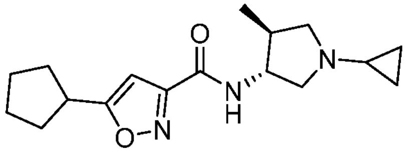 | TFA | (±)-*trans*-5-cyclopentyl-N-(1-cyclopropyl-4-methylpyrrolidin-3-yl)isoxazole-3-carboxamide | 304.3 | >50 | |
| 54 | 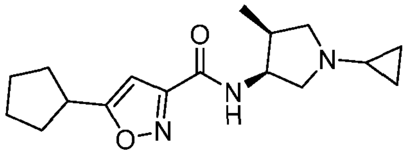 | TFA | (±)-*cis*-5-cyclopentyl-N-(1-cyclopropyl-4-methylpyrrolidin-3-yl)isoxazole-3-carboxamide | 304 | >50 | |
| 112 | 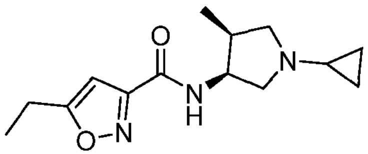 | TFA | (±)-*cis*-N-(1-cyclopropyl-4-methylpyrrolidin-3-yl)-5-ethylisoxazole-3-carboxamide | 264.3 | 9.4 | |

* $IC_{50}$ values are an average of n=1 to n=50

Table 2

| Cpd. No. | Structure | Chemical Name | SMYD2 Biochem $IC_{50}$ (μM)* |
|---|---|---|---|
| **55** | 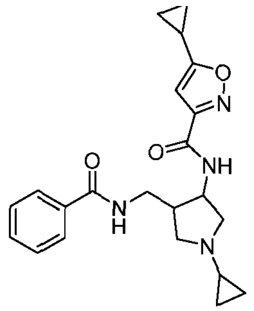 | N-(4-(benzamidomethyl)-1-cyclopropylpyrrolidin-3-yl)-5-cyclopropylisoxazole-3-carboxamide | |
| **56** | 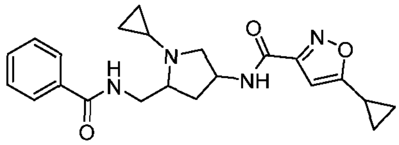 | N-(5-(benzamidomethyl)-1-cyclopropylpyrrolidin-3-yl)-5-cyclopropylisoxazole-3-carboxamide | |
| **57** | 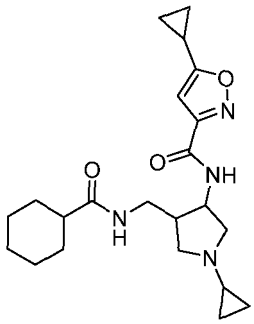 | N-(4-(cyclohexanecarboxamido methyl)-1-cyclopropylpyrrolidin-3-yl)-5-cyclopropylisoxazole-3- carboxamide | |
| **58** | 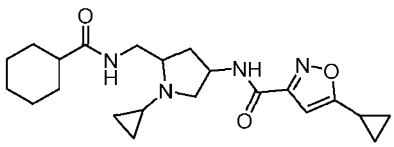 | N-(5-(cyclohexanecarboxamido methyl)-1-cyclopropylpyrrolidin-3-yl)-5-cyclopropylisoxazole-3-carboxamide | |
| **59** | 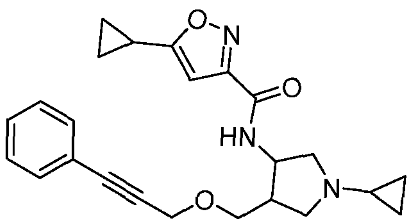 | 5-cyclopropyl-N-(1-cyclopropyl-4-(((3-phenylprop-2-yn-1-yl)oxy)methyl) pyrrolidin-3-yl)isoxazole-3-carboxamide | |
| **60** | 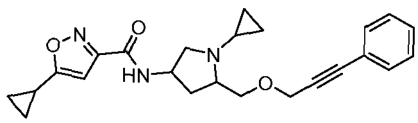 | 5-cyclopropyl-N-(1-cyclopropyl-5-(((3-phenylprop-2-yn-1-yl)oxy)methyl) pyrrolidin-3-yl)isoxazole-3-carboxamide | |

(continued)

| Cpd. No. | Structure | Chemical Name | SMYD2 Biochem $IC_{50}$ (μM)* |
|---|---|---|---|
| **61** | 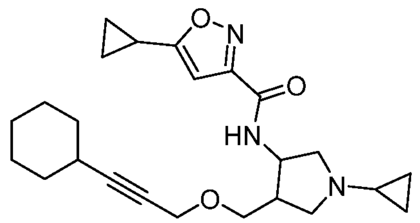 | N-(4-(((3-cyclohexylprop-2-yn-1-yl)oxy)methyl)-1-cyclopropylpyrrolidin-3-yl)-5-cyclopropylisoxazole-3-carboxamide | |
| **62** | 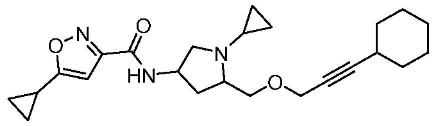 | N-(5-(((3-cyclohexylprop-2-yn-1-yl)oxy)methyl)-1-cyclopropylpyrrolidin-3-yl)-5-cyclopropylisoxazole-3-carboxamide | |
| **63** | 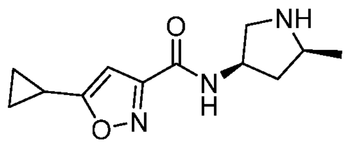 | (±)-*cis*5-cyclopropyl-N-(5-methylpyrrolidin-3-yl)isoxazole-3-carboxamide | >50 |
| **64** | 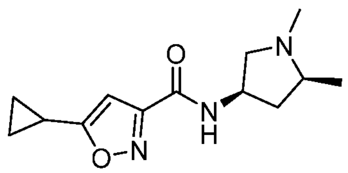 | (±)-*cis*-5-cyclopropyl-N-(1,5-dimethylpyrrolidin-3-yl)isoxazole-3-carboxamide | >50 |
| **65** | 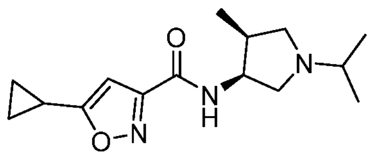 | (±)-*cis*-5-cyclopropyl-N-(1-isopropyl-4-methylpyrrolidin-3-yl)isoxazole-3-carboxamide | 2.4 |
| **66** | 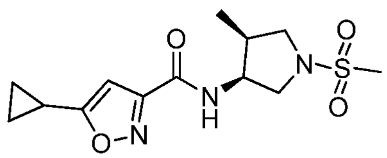 | (±)-*cis*-5-cyclopropyl-N-(4-methyl-1-(methylsulfonyl)pyrrolidi n-3-yl)isoxazole-3-carboxamide | >50 |
| **67** | 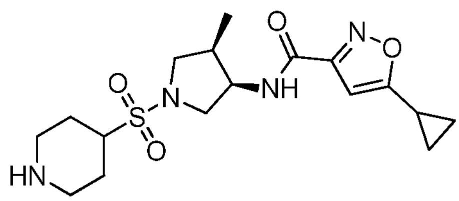 | (±)-*cis*5-cyclopropyl-N-(4-methyl-1-(piperidin-4-ylsulfonyl)pyrrolidin-3-yl) isoxazole-3-carboxamide | >50 |
| **68** | 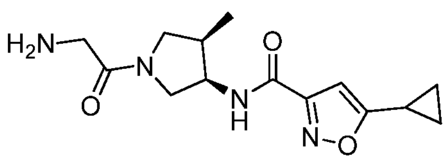 | (±)-*cis*-5-cyclopropyl-N-(1-glycyl-4-methylpyrrolidin-3-yl)isoxazole-3-carboxamide | >50 |

(continued)

| Cpd. No. | Structure | Chemical Name | SMYD2 Biochem $IC_{50}$ (μM)* |
|---|---|---|---|
| **69** | 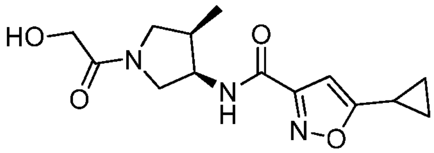 | (±)-*cis*-5-cyclopropyl-N-(1-(2-hydroxyacetyl)-4-methylpyrrolidin-3-yl) isoxazole-3-carboxamide | >50 |
| **70** | 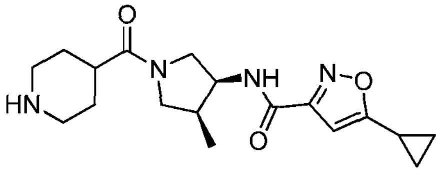 | (±)-*cis*-5-cyclopropyl-N-(4-methyl-1-(piperidine-4-carbonyl)pyrrolidin-3-yl) isoxazole-3-carboxamide | >50 |
| **71** | 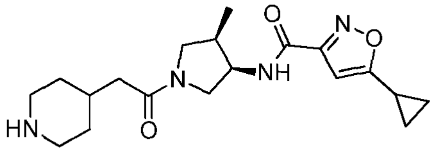 | (±)-*cis*-5-cyclopropyl-N-(4-methyl-1-(2-(piperidin-4-yl)acetyl)pyrrolidin-3-yl) isoxazole-3-carboxamide | >50 |
| **72** | 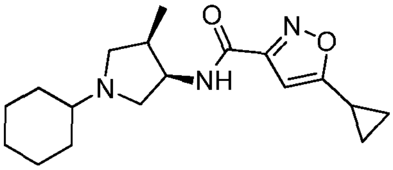 | (±)-*cis*-N-(1-cyclohexyl-4-methylpyrrolidin-3-yl)-5-cyclopropylisoxazole-3-carboxamide | 18.4 |
| **73** | 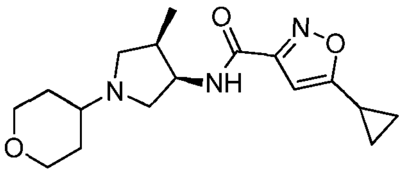 | (±)-*cis*-5-cyclopropyl-N-(4-methyl-1-(tetrahydro-2H-pyran-4-yl)pyrrolidin-3-yl) isoxazole-3-carboxamide | 40.7 |
| **74** | 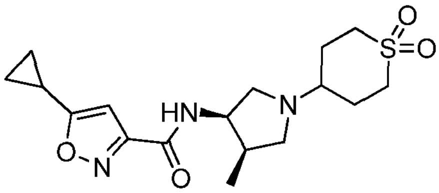 | (±)-*cis*-5-cyclopropyl-N-(1-(1,1-dioxidotetrahydro-2H-thiopyran-4-yl)-4-methylpyrrolidin-3-yl)isoxazole-3-carboxamide | 14.0 |
| **75** | 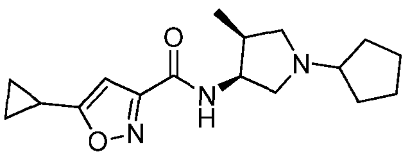 | (±)-*cis*-N-(1-cyclopentyl-4-methylpyrrolidin-3-yl)-5-cyclopropylisoxazole-3-carboxamide | 5.0 |
| **76** | 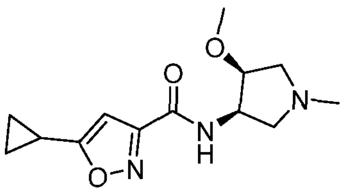 | (±)-*cis*-5-cyclopropyl-N-(4-methoxy-1-methylpyrrolidin-3-yl)isoxazole-3-carboxamide | |

(continued)

| Cpd. No. | Structure | Chemical Name | SMYD2 Biochem $IC_{50}$ (μM)* |
|---|---|---|---|
| 77 | 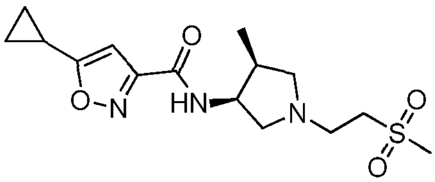 | (±)-*cis*-cyclopropyl-N-((3S)-4-methyl-1-(2-(methylsulfonyl)ethyl)pyr rolidin-3-yl) isoxazole-3-carboxamide | >50 |
| 78 | 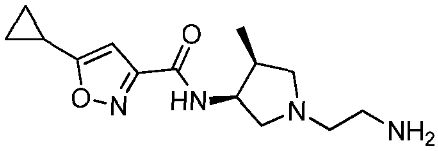 | (±)-*cis*-N-(1-(2-aminoethyl)-4-methylpyrrolidin-3-yl)-5-cyclopropylisoxazole-3-carboxamide | 27.5 |
| 79 | 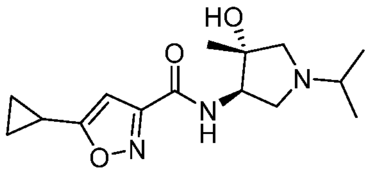 | (±)-*trans*-5-cyclopropyl-N-(4-hydroxy-1-isopropyl-4-methylpyrrolidin-3-yl)isoxazole-3-carboxamide | |
| 80 | 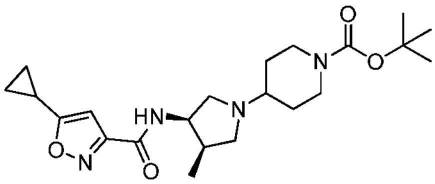 | (±)-*cis*-tert-butyl4-(3-(5-cyclopropylisoxazole-3-carboxamido)-4-methylpyrrolidin-1-yl)piperidine-1-carboxylate | 30.5 |
| 81 | 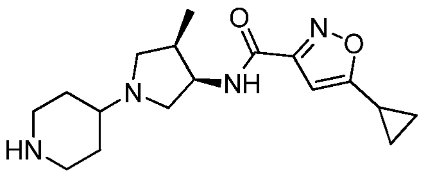 | (±)-*cis*-5-cyclopropyl-N-(4-methyl-1-(piperidin-4-yl)pyrrolidin-3-yl)isoxazole-3-carboxamide | >50 |
| 82 | 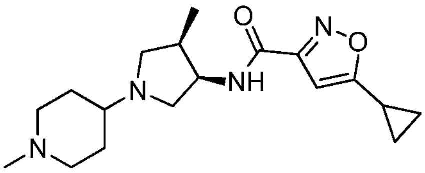 | (±)-*cis*-5-cyclopropyl-N-(4-methyl-1-(1-methylpiperidin-4-yl)pyrrolidin-3-yl) isoxazole-3-carboxamide | >50 |
| 83 | 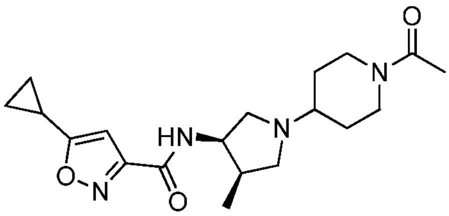 | (±)-*cis*-N-(1-(1-acetylpiperidin-4-yl)-4-methylpyrrolidin-3-yl)-5-cyclopropylisoxazole-3-carboxamide | >50 |
| 84 | 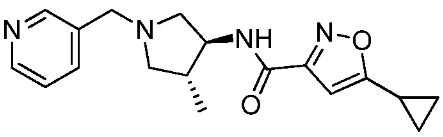 | (±)-*trans*-5-cyclopropyl-N-(4-methyl-1-(pyridin-3- ylmethyl)pyrrolidin-3-yl) isoxazole-3-carboxamide | >50 |

(continued)

| Cpd. No. | Structure | Chemical Name | SMYD2 Biochem $IC_{50}$ (μM)* |
|---|---|---|---|
| **85** | 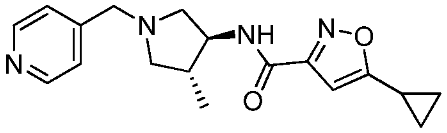 | (±)-*trans*-5-cyclopropyl-N-(4-methyl-1-(pyridin-4-ylmethyl)pyrrolidin-3-yl) isoxazole-3-carboxamide | >50 |
| **86** | 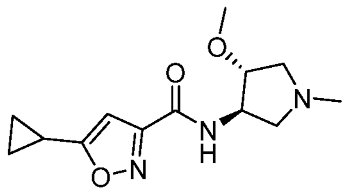 | 5-cyclopropyl-N-((3R,4R)-4-methoxy-1-methylpyrrolidin-3-yl)isoxazole-3-carboxamide | |
| **87** | 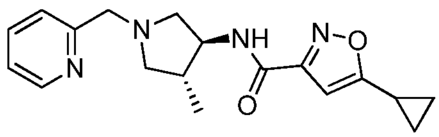 | (±)-*trans*-5-cyclopropyl-N-(4-methyl-1-(pyridin-2-ylmethyl)pyrrolidin-3-yl) isoxazole-3-carboxamide | >50 |
| **88** | 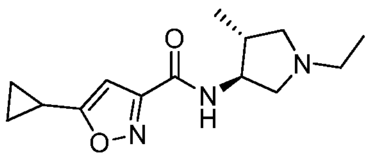 | (±)-*trans*-5-cyclopropyl-N-(1-ethyl-4-methylpyrrolidin-3-yl)isoxazole-3-carboxamide | 34.2 |
| **89** | 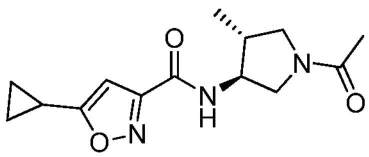 | (±)-*trans*-N-(1-acetyl-4-methylpyrrolidin-3-yl)-5-cyclopropylisoxazole-3-carboxamide | >50 |
| **90** | 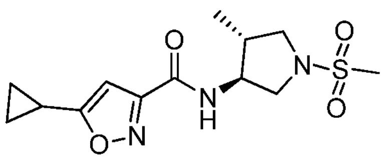 | (±)-*trans*-5-cyclopropyl-N-(4-methyl-1-(methylsulfonyl)pyrrolidi n-3-yl)isoxazole-3-carboxamide | >50 |
| **91** | 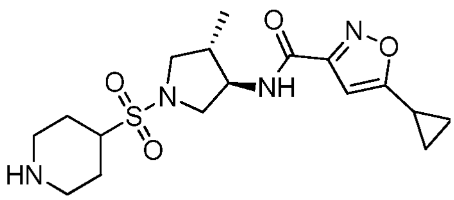 | (±)-*trans*-5-cyclopropyl-N-(4-methyl-1-(piperidin-4-ylsulfonyl)pyrrolidin-3-yl) isoxazole-3-carboxamide | >50 |
| **92** | 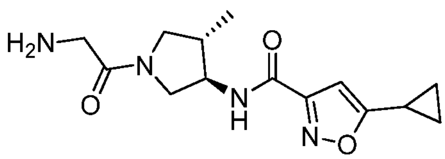 | (±)-*trans*-5-cyclopropyl-N-(1-glycyl-4-methylpyrrolidin-3-yl)isoxazole-3-carboxamide | >50 |
| **93** | 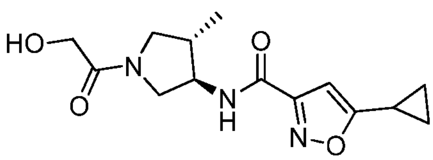 | (±)-*trans*-5-cyclopropyl-N-(1-(2-hydroxyacetyl)-4-methylpyrrolidin-3-yl) isoxazole-3-carboxamide | >50 |

(continued)

| Cpd. No. | Structure | Chemical Name | SMYD2 Biochem $IC_{50}$ (μM)* |
|---|---|---|---|
| **94** | 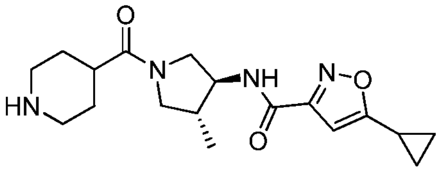 | (±)-*trans*-5-cyclopropyl-N-(4-methyl-1-(piperidine-4-carbonyl)pyrrolidin-3-yl) isoxazole-3-carboxamide | >50 |
| **95** | 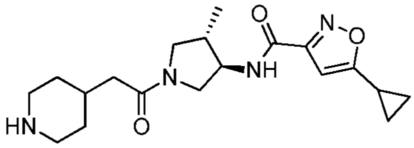 | (±)-*trans*-5-cyclopropyl-N-(4-methyl-1-(2-(piperidin-4-yl)acetyl)pyrrolidin-3-yl) isoxazole-3-carboxamide | >50 |
| **96** | 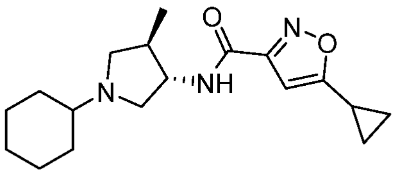 | (±)-*trans*-N-(1-cyclohexyl-4-methylpyrrolidin-3-yl)-5-cyclopropylisoxazole-3-carboxamide | >50 |
| **97** | 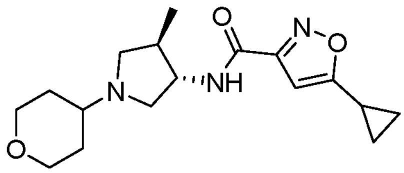 | (±)-*trans*-5-cyclopropyl-N-(4-methyl-1-(tetrahydro-2H-pyran-4-yl)pyrrolidin-3-yl) isoxazole-3-carboxamide | >50 |
| **98** | 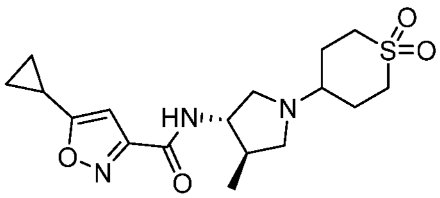 | (±)-*trans*-5-cyclopropyl-N-(1-(1,1-dioxidotetrahydro-2H-thiopyran-4-yl)-4-methylpyrrolidin-3-yl)isoxazole-3-carboxamide | |
| **99** | 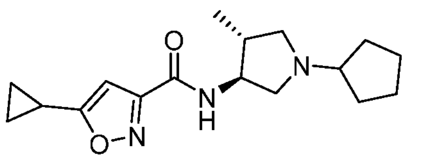 | (±)-*trans*-N-(1-cyclopentyl-4-methylpyrrolidin-3-yl)-5-cyclopropylisoxazole-3-carboxamide | 48.7 |
| **100** | 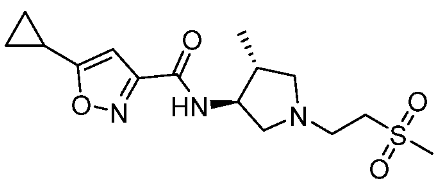 | (±)-*trans*-5-cyclopropyl-N-(4-methyl-1-(2-(methylsulfonyl)ethyl)pyr rolidin-3-yl) isoxazole-3-carboxamide | >50 |
| **101** | 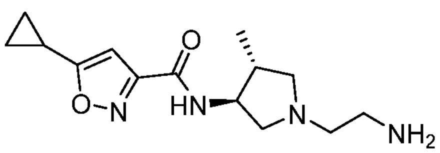 | (±)-*trans*-N-(1-(2-aminoethyl)-4-methylpyrrolidin-3-yl)-5-cyclopropylisoxazole-3-carboxamide | >50 |

(continued)

| Cpd. No. | Structure | Chemical Name | SMYD2 Biochem $IC_{50}$ (μM)* |
|---|---|---|---|
| **102** | 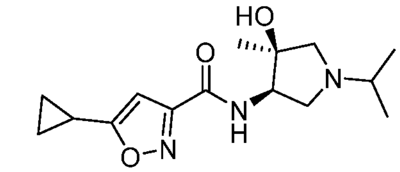 | (±)-*cis*-5-cyclopropyl-N-(4-hydroxy-1-isopropyl-4-methylpyrrolidin-3-yl)isoxazole-3-carboxamide | |
| **103** | 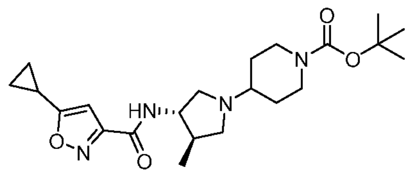 | (±)-*trans*-tert-butyl4-((3S,4R)-3-(5-cyclopropylisoxazole-3-carboxamido)-4-methylpyrrolidin-1-yl)piperidine-1-carboxylate | >50 |
| **104** | 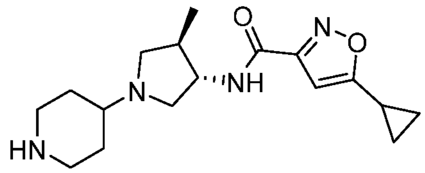 | (±)-*trans*-5-cyclopropyl-N-((3 S,4R)-4-methyl-1-(piperidin-4-yl)pyrrolidin-3-yl) isoxazole-3-carboxamide | >50 |
| **105** | 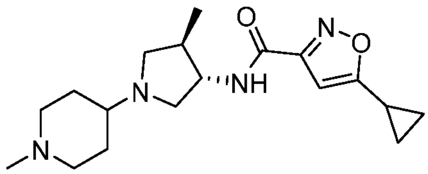 | (±)-*trans*-5-cyclopropyl-N-((3 S,4R)-4-methyl-1-(1-methylpiperidin-4-yl)pyrrolidin-3-yl)isoxazole-3-carboxamide | >50 |
| **106** | 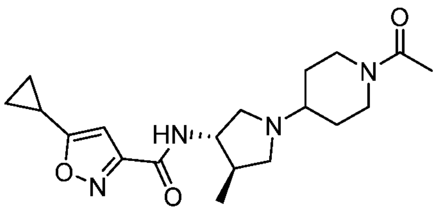 | (±)-*trans*-N-(1-(1-acetylpiperidin-4-yl)-4-methylpyrrolidin-3-yl)-5-cyclopropylisoxazole-3-carboxamide | >50 |
| **107** | 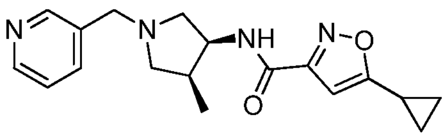 | (±)-*cis*-5-cyclopropyl-N-(4-methyl-1-(pyridin-3- ylmethyl)pyrrolidin-3-yl) isoxazole-3-carboxamide | 17.7 |
| **108** | 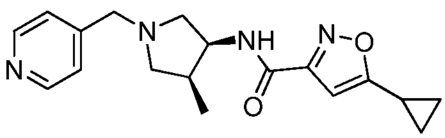 | (±)-*cis*-5-cyclopropyl-N-(4-methyl-1-(pyridin-4-ylmethyl)pyrrolidin-3-yl) isoxazole-3-carboxamide | 22.6 |
| **109** | 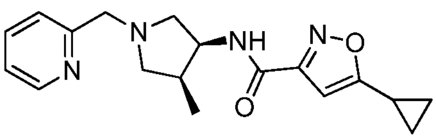 | (±)-*cis*-5-cyclopropyl-N-(4-methyl-1-(pyridin-2-ylmethyl)pyrrolidin-3-yl) isoxazole-3-carboxamide | 31.8 |

(continued)

| Cpd. No. | Structure | Chemical Name | SMYD2 Biochem $IC_{50}$ (μM)* |
|---|---|---|---|
| **110** | 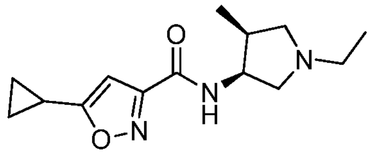 | (±)-*cis*-5-cyclopropyl-N-(1-ethyl-4-methylpyrrolidin-3-yl)isoxazole-3-carboxamide | 3.0 |
| **111** | 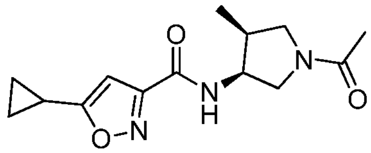 | (±)-*cis*-N-(1 -acetyl-4-methylpyrrolidin-3-yl)-5-cyclopropylisoxazole-3-carboxamide | >50 |
| * $IC_{50}$ values are an average of n=1 to n=50 | | | |

Table 3

| Cpd. No. | Structure | Salt Form | Chemical Name | LCMS M+H | SMYD2 Biochem $IC_{50}$ (μM)* |
|---|---|---|---|---|---|
| 113 | 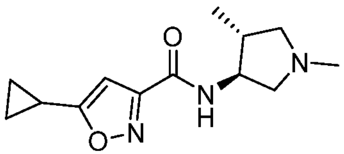 | None | (±)-*trans*-5-cyclopropyl-N-(1,4-dimethylpyrrolidin-3-yl)-1,2-oxazole-3-carboxamide | 250 | |
| 114 | 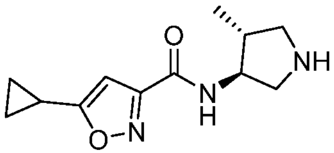 | None | (±)-*trans*-5-cyclopropyl-N-(4-methylpyrrolidin-3-yl)-1,2-oxazole-3-carboxamide | 236.1 | |
| 115 | 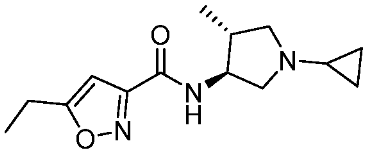 | TFA | (±)-*trans*-N-(1-cyclopropyl-4-methylpyrrolidin-3-yl)-5-ethyl-1,2-oxazole-3-carboxamide | | |
| 116 | 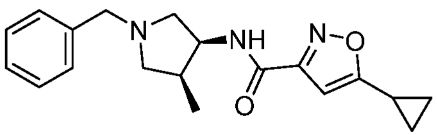 | TFA | (±)-*cis*-N-([1-benzyl-4-methylpyrrolidin-3-yl)-5-cyclopropyl-1,2-oxazole-3-carboxamide | | 4.2 |
| 117 | 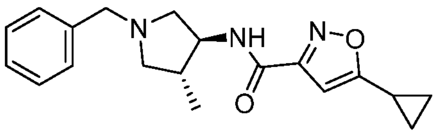 | TFA | (±)-*trans*-N-(1-benzyl-4-methylpyrrolidin-3-yl)-5-cyclopropyl-1,2-oxazole-3-carboxamide | | >50 |
| 118 | 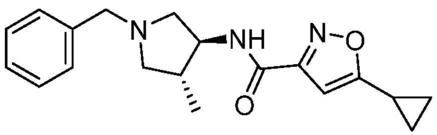 | None | (±)-*trans*-N-(1-benzyl-4-methylpyrrolidin-3-yl)-5-cyclopropyl-1,2-oxazole-3-carboxamide | 326.2 | > 50.0 |

(continued)

| Cpd. No. | Structure | Salt Form | Chemical Name | LCMS M+H | SMYD2 Biochem $IC_{50}$ (μM)* |
|---|---|---|---|---|---|
| 119 | 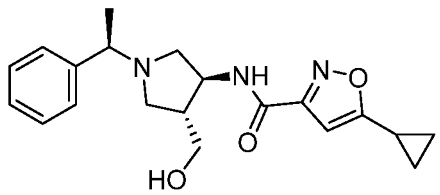 | None | 5-cyclopropyl-N-[(3 S,4R)-4-(hydroxymethyl)-1-[(1R)-1-phenylethyl]pyrrolidin-3-yl]-1,2-oxazole-3-carboxamide | | 38.2 |
| 120 | 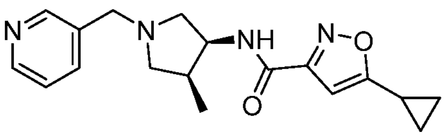 | None | (±)-*cis*-5-cyclopropyl-N-[4-methyl-1-(pyridin-3-ylmethyl) pyrrolidin-3-yl]-1,2-oxazole-3-carboxamide | 327.1 | 10.63005 |
| 121 | 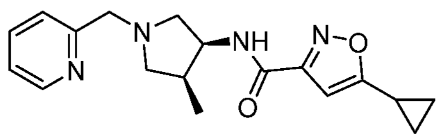 | None | (±)-*cis*-5-cyclopropyl-N-[4-m ethyl-1 - (pyridin-2-ylmethyl) pyrrolidin-3-yl]-1,2-oxazole-3-carboxamide | 327 | 15.66503 |
| 122 | 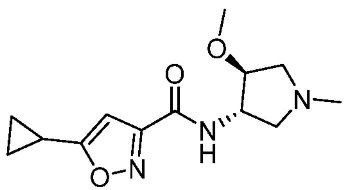 | None | (±)-*trans*-5-cyclopropyl-N-(4-methoxy-1-methylpyrrolidin-3-yl)-1,2-oxazole-3-carboxamide | 266.2 | > 50 |
| 123 | 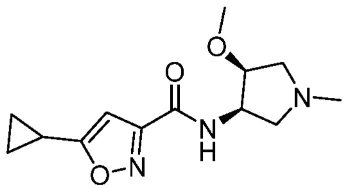 | None | (±)-*cis*-5-cyclopropyl-N-(4-methoxy-1-methylpyrrolidin-3-yl)-1,2-oxazole-3-carboxamide | 266.3 | 49.8 |

(continued)

| Cpd. No. | Structure | Salt Form | Chemical Name | LCMS M+H | SMYD2 Biochem $IC_{50}$ (μM)* |
|---|---|---|---|---|---|
| 124 | 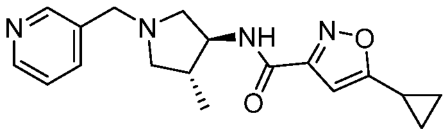 | None | (±)-*trans*-5-cyclopropyl-N-[4-methyl-1-(pyridin-3-ylmethyl) pyrrolidin-3-yl]-1,2-oxazole-3-carboxamide | 327.1 | > 50 |
| 125 | 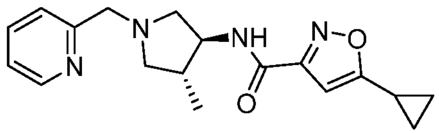 | None | (±)-*trans*-5-cyclopropyl-N-[4-methyl-1-(pyridin-2-ylmethyl) pyrrolidin-3-yl]-1,2-oxazole-3-carboxamide | 327.1 | > 50 |
| 126 | 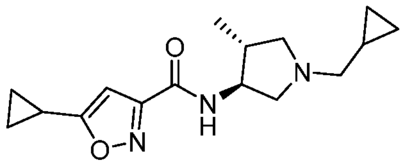 | None | (±)-*trans*-5-cyclopropyl-N-[1-(cyclopropylmethyl)-4-methylpyrrolidin-3-yl]-1,2-oxazole-3-carboxamide | 290.1 | > 50 |
| 127 | 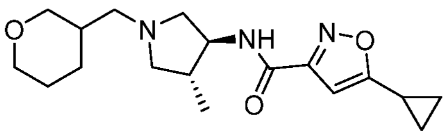 | None | (±)-*trans*-5-cyclopropyl-N-[4-methyl-1-(oxan-3-ylmethyl) pyrrolidin-3-yl]-1,2-oxazole-3-carboxamide | 334.2 | > 50 |
| 128 | 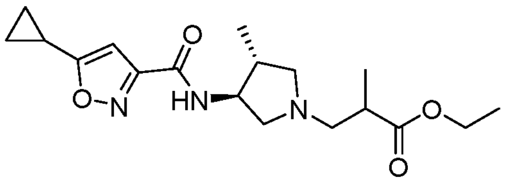 | None | *trans*-ethyl 3-[3-(5-cyclopropyl-1,2-oxazole-3-amido)-4-methylpyrrolidin-1-yl]-2-methylpropanoate | 350.2 | > 50 |
| 129 | 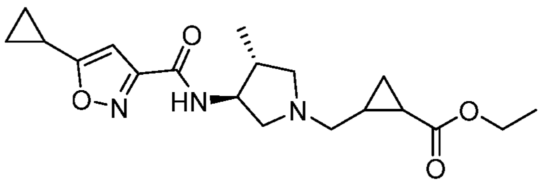 | None | *trans*-ethyl 2-{[3-(5-cyclopropyl-1,2-oxazole-3-amido)-4-methylpyrrolidin-1-yl]methyl}cyclopropane-1-carboxylate | 362.1 | |

(continued)

| Cpd. No. | Structure | Salt Form | Chemical Name | LCMS M+H | SMYD2 Biochem $IC_{50}$ (μM)* |
|---|---|---|---|---|---|
| 130 | 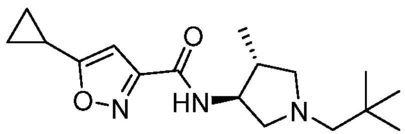 | None | (±)-*trans*-5-cyclopropyl-N-[(3S,4R)-1-(2,2-dimethylpropyl)-4-methylpyrrolidin-3-yl]-1,2-oxazole-3-carboxamide | 306.2 | > 50 |
| 131 | 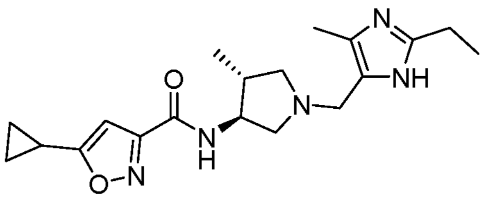 | None | (±)-*trans*-5-cyclopropyl-N-[1-[(2-ethyl-4-methyl-1H-imidazol-5-yl)methyl]-4-methylpyrrolidin-3-yl]-1,2-oxazole-3-carboxamide | 358.2 | > 50 |
| 132 | 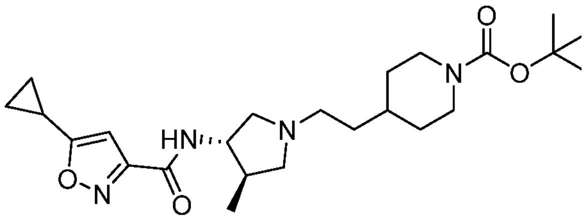 | None | (±)-*trans*-tert-butyl 4-{2-(5-cyclopropyl-1,2-oxazole-3-amido)-4-methylpyrrolidin-1-yl]ethyl}piperidine-1-carboxylate | 447.2 | > 50.0 |
| 133 | 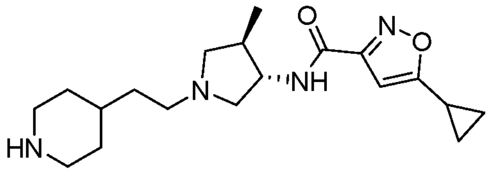 | None | (±)-*trans*-5-cyclopropyl-N-[4-methyl-1-[2-(piperidin-4-yl)ethyl] pyrrolidin-3-yl]-1,2-oxazole-3-carboxamide | 347.2 | > 50.0 |
| 134 | 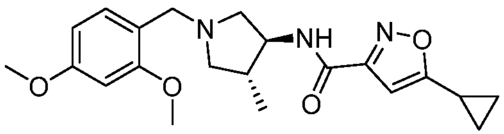 | None | (±)-*trans*--cyclopropyl-N-[1-[(2,4-dimethoxyphenyl)methyl]-4-methylpyrrolidin-3-yl]-1,2-oxazole-3-carboxamide | 386.1 | > 50.0 |

(continued)

| Cpd. No. | Structure | Salt Form | Chemical Name | LCMS M+H | SMYD2 Biochem $IC_{50}$ (μM)* |
|---|---|---|---|---|---|
| 135 | 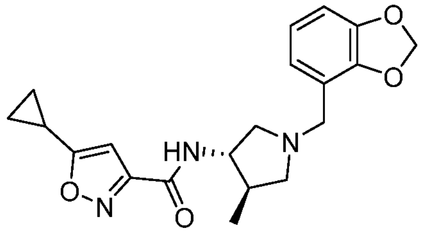 | None | (±)-*trans*-N-[1-(2H-1,3-benzodioxol-4-ylmethyl)-4-methylpyrrolidin-3-yl]-5-cyclopropyl-1,2-oxazole-3-carboxamide | 370.1 | > 50.0 |
| 136 | 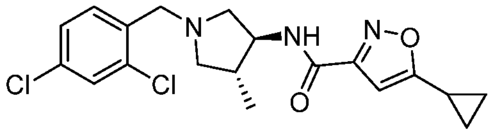 | None | (±)-*trans*-5-cyclopropyl-N-[1-[(2,4-dichlorophenyl)methyl]-4-methylpyrrolidin-3-yl]-1,2-oxazole-3-carboxamide | 394.1 | > 50.0 |
| 137 | 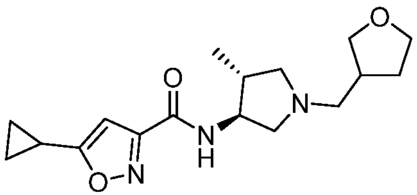 | None | (±)-*trans*-5-cyclopropyl-N-[4-methyl-1-(oxolan-3-ylmethyl) pyrrolidin-3-yl]-1,2-oxazole-3-carboxamide | 320.1 | > 50.0 |
| 138 | 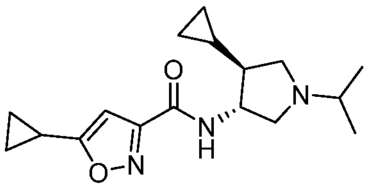 | None | (±)-*trans*-5-cyclopropyl-N-[4-cyclopropyl-1-(propan-2-yl) pyrrolidin-3-yl]-1,2-oxazole-3-carboxamide | 304.3 | 48.07835 |
| 139 | 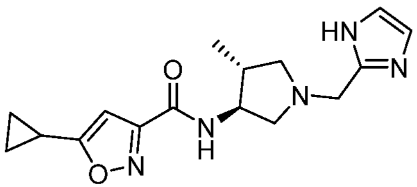 | None | (±)-*trans*-5-cyclopropyl-N-[1-(1H-imidazol-2-ylmethyl)-4-methylpyrrolidin-3-yl]-1,2-oxazole-3-carboxamide | 316.1 | > 50.0 |

(continued)

| Cpd. No. | Structure | Salt Form | Chemical Name | LCMS M+H | SMYD2 Biochem $IC_{50}$ (μM)* |
|---|---|---|---|---|---|
| 140 | 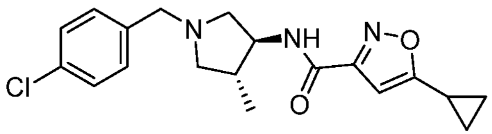 | None | (±)-*trans*-N-{1-[(4-chlorophenyl)methyl]-4-methylpyrrolidin-3-yl]-5-cyclopropyl-1,2-oxazole-3-carboxamide | 360.1 | > 50.0 |
| 141 | 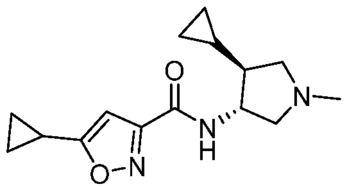 | None | (±)-*trans*-5-cyclopropyl-N-[4-cyclopropyl-1-methylpyrrolidin-3-yl]-1,2-oxazole-3-carboxamide | 276.1 | > 50.0 |
| 142 | 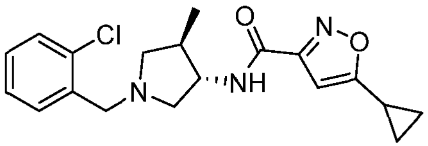 | None | (±)-*trans*-N-1-[(2-chlorophenyl)methyl]-4-methylpyrrolidin-3-yl]-5-cyclopropyl-1,2-oxazole-3-carboxamide | 360.1 | 27.50903 |
| 143 | 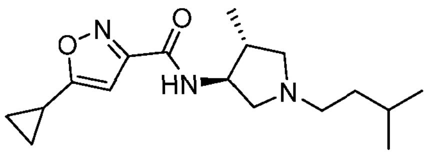 | None | (±)-*trans*-5-cyclopropyl-N-[4-methyl-1-(3-methylbutyl)pyrrolidin-3-yl]-1,2-oxazole-3-carboxamide | 306.2 | > 50.0 |
| 144 | 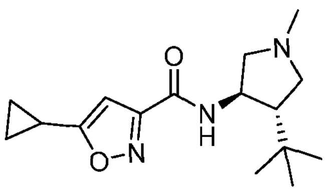 | None | (±)-*trans*-N-[4-tert-butyl-1-methylpyrrolidin-3-yl]-5-cyclopropyl-1,2-oxazole-3-carboxamide | 292 | > 50.0 |

(continued)

| Cpd. No. | Structure | Salt Form | Chemical Name | LCMS M+H | SMYD2 Biochem $IC_{50}$ (μM)* |
|---|---|---|---|---|---|
| 146 | 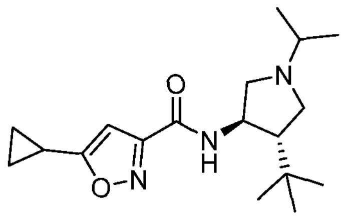 | None | (±)-*trans*-N-[4-tert-butyl-1-(propan-2-yl)pyrrolidin-3-yl]-5-cyclopropyl-1,2-oxazole-3-carboxamide | 320.2 | > 50.0 |
| 148 | 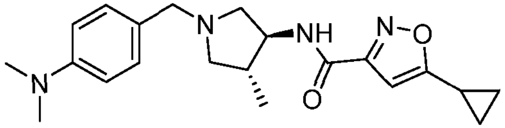 | None | (±)-*trans*-5-cyclopropyl-N-[1-{ [4-(dimethylamino)phenyl] methyl}-4-methylpyrrolidin-3-yl]-1,2-oxazole-3-carboxamide | 369.2 | 36.97206 |
| 149 | 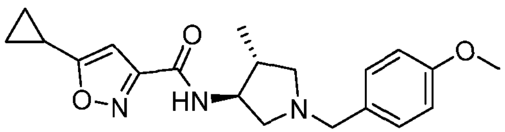 | None | (±)-*trans*-5-cyclopropyl-N-[1-[(4-methoxyphenyl)methyl]-4-methylpyrrolidin-3-yl]-1,2-oxazole-3-carboxamide | 356.2 | 45.59912 |
| 150 | 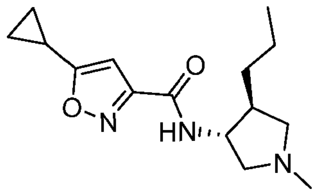 | None | (±)-*trans*-5-cyclopropyl-N-[1 -methyl-4-propylpyrrolidin-3-yl]-1,2-oxazole-3-carboxamide | 278.2 | 44.85866 |
| 151 | 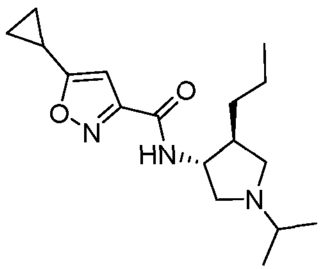 | None | (±)-*trans*-5-cyclopropyl-N-[1-(propan-2-yl)-4-propylpyrrolidin-3-yl]-1,2-oxazole-3-carboxamide | 306.2 | > 50.0 |

(continued)

| Cpd. No. | Structure | Salt Form | Chemical Name | LCMS M+H | SMYD2 Biochem $IC_{50}$ (μM)* |
|---|---|---|---|---|---|
| 152 | 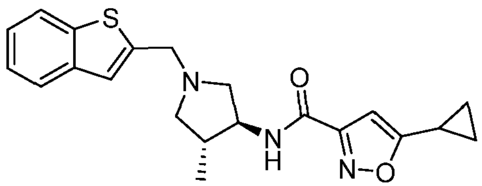 | None | (±)-*trans*-N-[1-(1-benzothiophen-2-ylmethyl)-4-methylpyrrolidin-3-yl]-5-cyclopropyl-1,2-oxazole-3-carboxamide | 382.1 | > 50.0 |
| 153 | 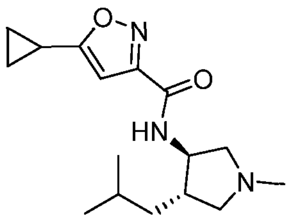 | None | (±)-*trans*-5-cyclopropyl-N-[1 -methyl-4-(2-methylpropyl) pyrrolidin-3-yl]-1,2-oxazole-3-carboxamide | 292.1 | 39.11627 |
| 154 | 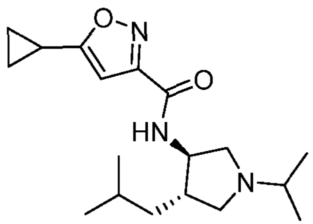 | None | (±)-*trans*-5-cyclopropyl-N-[4-(2-methylpropyl)-1 -(propan-2-yl) pyrrolidin-3-yl]-1,2-oxazole-3-carboxamide | 320.2 | > 50.0 |
| 155 | 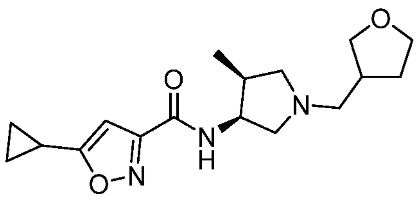 | None | (±)-*cis*-5-cyclopropyl-N-[4-methyl-1-(oxolan-3-ylmethyl) pyrrolidin-3-yl]-1,2-oxazole-3-carboxamide | 320.1 | 4.48876 |

(continued)

| Cpd. No. | Structure | Salt Form | Chemical Name | LCMS M+H | SMYD2 Biochem $IC_{50}$ (μM)* |
|---|---|---|---|---|---|
| 156 | 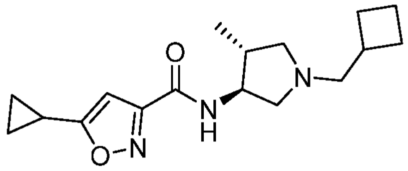 | None | (±)-*trans*-N-[1-(cyclobutylmethyl)-4-methylpyrrolidin-3-yl]-5-cyclopropyl-1,2-oxazole-3-carboxamide | 304.2 | > 50.0 |
| 157 | 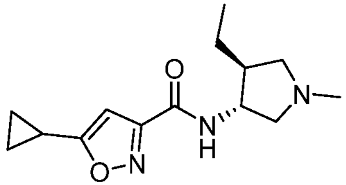 | None | (±)-*trans*-5-cyclopropyl-N-[4-ethyl-1-methylpyrrolidin-3-yl]-1,2-oxazole-3-carboxamide | 264.2 | 45.27925 |
| 158 | 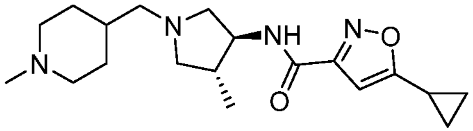 | None | (±)-*trans*-5-cyclopropyl-N-[4-methyl-1-[(1-methylpiperidin-4-yl) methyl]pyrrolidin-3-yl]-1,2-oxazole-3-carboxamide | 347.2 | > 50.0 |
| 159 | 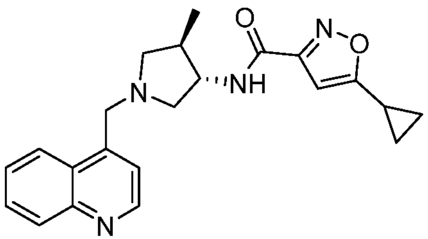 | None | (±)-*trans*-5-cyclopropyl-N-[4-methyl-1-(quinolin-4-ylmethyl) pyrrolidin-3 - yl]-1,2-oxazole-3-carboxamide | 377 | > 50.0 |
| 160 | 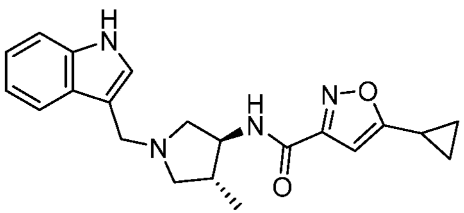 | None | (±)-*trans*-5-cyclopropyl-N-[1-(1H-indol-3-ylmethyl)-4-methylpyrrolidin-3-yl]-1,2-oxazole-3-carboxamide | 365 | > 50.0 |

(continued)

| Cpd. No. | Structure | Salt Form | Chemical Name | LCMS M+H | SMYD2 Biochem $IC_{50}$ (μM)* |
|---|---|---|---|---|---|
| 161 | 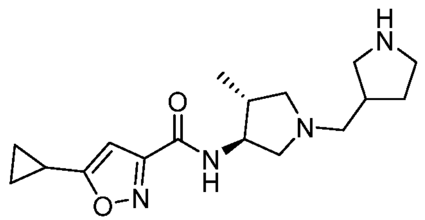 | None | (±)-*trans*-5-cyclopropyl-N-[4-methyl-1-(pyrrolidin-3-ylmethyl) pyrrolidin-3-yl]-1,2-oxazole-3-carboxamide | 319.2 | > 50.0 |
| 162 | 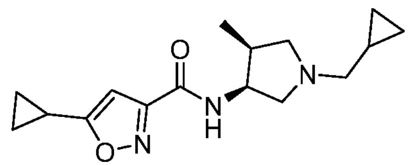 | None | (±)-*cis*-5-cyclopropyl-N-[ 1-(cyclopropylmethyl)-4-methylpyrrolidin-3-yl]-1,2-oxazole-3-carboxamide | 290.2 | 1.41532 |
| 163 | 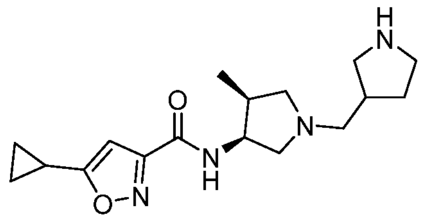 | None | (±)-*cis*-5-cyclopropyl-N-[4-methyl-1-(pyrrolidin-3-ylmethyl) pyrrolidin-3-yl]-1,2-oxazole-3-carboxamide | 319.2 | > 50.0 |
| 164 | 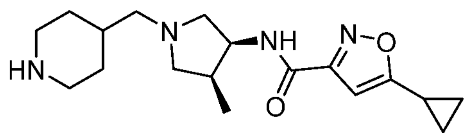 | None | (±)-*cis*-5-cyclopropyl-N-[4-methyl-1-(piperidin-4-ylmethyl) pyrrolidin-3-yl]-1,2-oxazole-3-carboxamide | 333.2 | 4.78469 |
| 165 | 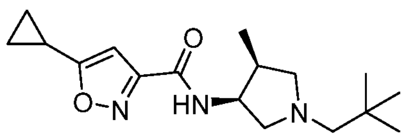 | None | (±)-*cis*-5-cyclopropyl-N-[ 1-(2,2-dimethylpropyl)-4-methylpyrrolidin-3-yl]-1,2-oxazole-3-carboxamide | 306 | 11.26278 |

(continued)

| Cpd. No. | Structure | Salt Form | Chemical Name | LCMS M+H | SMYD2 Biochem $IC_{50}$ (μM)* |
|---|---|---|---|---|---|
| 166 | 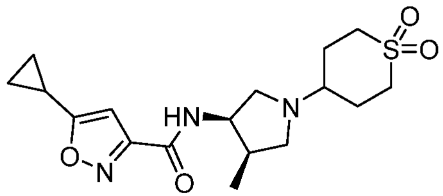 | HCO OH | (±)-*cis*-5-cyclopropyl-N-(1-(1,1-dioxidotetrahydro-2H-thiopyran-4-yl)-4-methylpyrrolidin-3-yl)isoxazole-3-carboxamide | 368 | 19.8 |
| 168 | 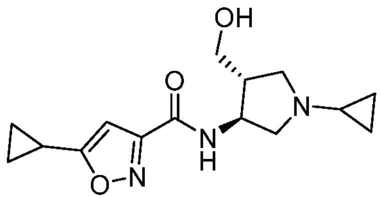 | None | 5-cyclopropyl-N-[(3S,4R)-1-cyclopropyl-4-(hydroxymethyl) pyrrolidin-3-yl]-1,2-oxazole-3-carboxamide | 292.2 | 43.22971 |
| 169 | 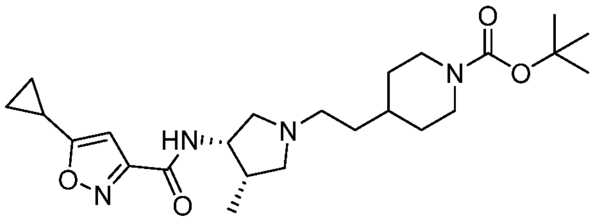 | None | (±)-*cis*-tert-butyl 4-{2-[3-(5-cyclopropyl-1,2-oxazole-3-amido)-4-methylpyrrolidin-1-yl]ethyl}piperidine-1-carboxylate | 447.2 | 6.92417 |
| 170 | 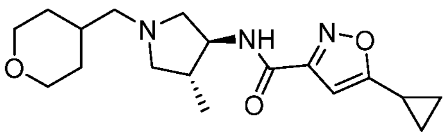 | None | (±)-*trans*-5-cyclopropyl-N-[4-methyl-1-(oxan-4-ylmethyl) pyrrolidin-3-yl]-1,2-oxazole-3-carboxamide | 334 | > 50.0 |

(continued)

| Cpd. No. | Structure | Salt Form | Chemical Name | LCMS M+H | SMYD2 Biochem $IC_{50}$ (μM)* |
|---|---|---|---|---|---|
| 171 | 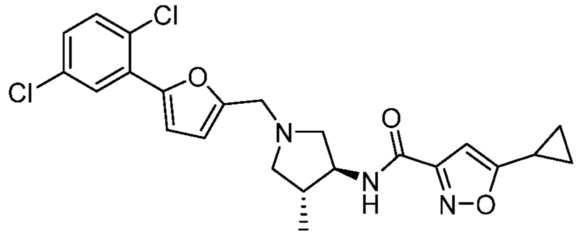 | None | (±)-*trans*-5-cyclopropyl-N-[1-{[5-(2,5-dichlorophenyl)furan-2-yl] methyl}-4-methylpyrrolidin-3-yl]-1,2-oxazole-3-carboxamide | 460.1 | > 50.0 |
| 172 | 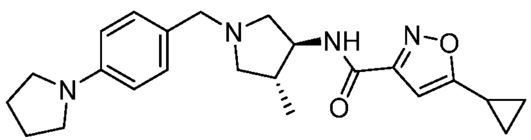 | None | (±)-*trans*-5-cyclopropyl-N-[4-methyl-1-{[4-(pyrrolidin-1-yl) phenyl]methyl}pyrrolidin-3-yl]-1,2-oxazole-3-carboxamide | 395.2 | 29.53714 |
| 173 | 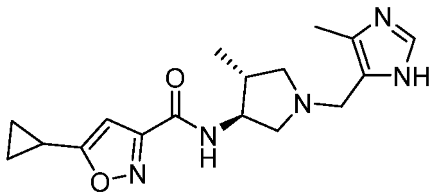 | None | (±)-*trans*-5-cyclopropyl-N-[4-methyl-1-[(4-methyl-1H-imidazol-5-yl)methyl]pyrrolidin-3-yl]-1,2-oxazole-3-carboxamide | 330.2 | > 50.0 |
| 174 | 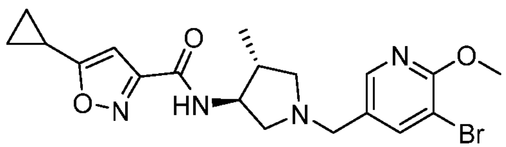 | None | (±)-*trans*-N-[1-[(5-bromo-6-methoxypyridin-3-yl)methyl]-4-methylpyrrolidin-3-yl]-5-cyclopropyl-1,2-oxazole-3-carboxamide | 435.1 | > 50.0 |
| 175 | 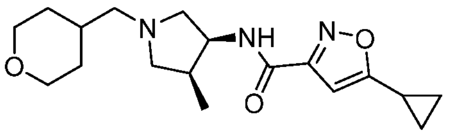 | None | (±)-*cis*-5-cyclopropyl-N-[4-methyl-1-(oxan-4-ylmethyl)pyrrolidin-3-yl]-1,2-oxazole-3-carboxamide | 334.2 | 3.95227 |

(continued)

| Cpd. No. | Structure | Salt Form | Chemical Name | LCMS M+H | SMYD2 Biochem $IC_{50}$ (μM)* |
|---|---|---|---|---|---|
| 176 | 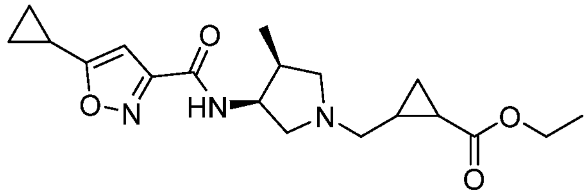 | None | *cis*-ethyl 2-{ [3-(5-cyclopropyl-1,2-oxazole-3-amido)-4-methylpyrrolidin-1-yl]methyl}cyclopropane-1-carboxylate | 362.2 | 5.28026 |
| 177 | 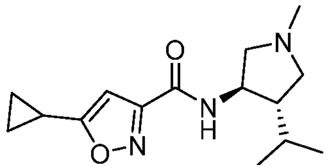 | None | (±)-*trans*-5-cyclopropyl-N-[1-methyl-4-(propan-2-yl)pyrrolidin-3-yl]-1,2-oxazole-3-carboxamide | 278.2 | > 50.0 |
| 178 | 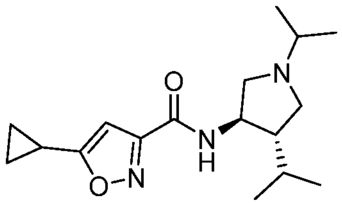 | None | (±)-*trans*-N-[1,4-bis(propan-2-yl)pyrrolidin-3-yl]-5-cyclopropyl-1,2-oxazole-3-carboxamide | 306.2 | > 50.0 |
| 179 | 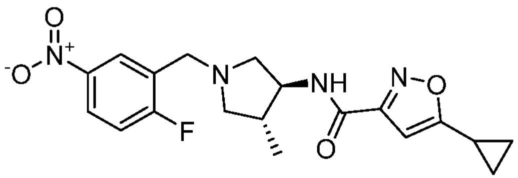 | None | (±)-*trans*-5-cyclopropyl-N-[1-[(2-fluoro-5-nitrophenyl)methyl]-4-methylpyrrolidin-3-yl]-1,2-oxazole-3-carboxamide | 389.2 | > 50.0 |
| 180 | 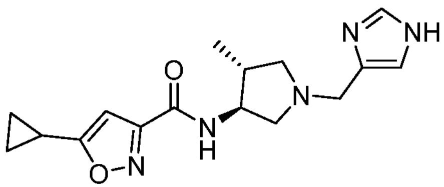 | None | (±)-*trans*-5-cyclopropyl-N-[1-(1H-imidazol-4-ylmethyl)-4-methylpyrrolidin-3-yl]-1,2-oxazole-3-carboxamide | 316.2 | 42.51127 |

(continued)

| Cpd. No. | Structure | Salt Form | Chemical Name | LCMS M+H | SMYD2 Biochem $IC_{50}$ (μM)* |
|---|---|---|---|---|---|
| 181 | 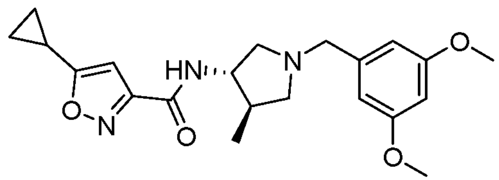 | None | (±)-*trans*-5-cyclopropyl-N-[1-[(3,5-dimethoxyphenyl)methyl]-4-methylpyrrolidin-3-yl]-1,2-oxazole-3-carboxamide | 386.3 | > 50.0 |
| 182 | 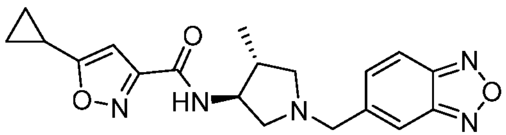 | None | N-[(3 S,4R)-1-(2,1,3-benzoxadiazol-5-ylmethyl)-4-methylpyrrolidin-3-yl]-5-cyclopropyl-1,2-oxazole-3-carboxamide | 368.1 | > 50.0 |
| 183 | 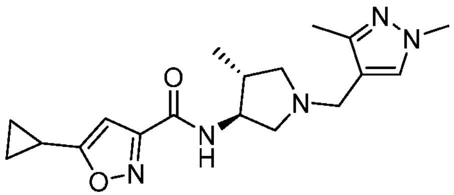 | None | (±)-*trans*-5-cyclopropyl-N-[1-[(1,3-dimethyl-1H-pyrazol-4-yl)methyl]-4-methylpyrrolidin-3-yl]-1,2-oxazole-3-carboxamide | 344.2 | > 50.0 |
| 184 | 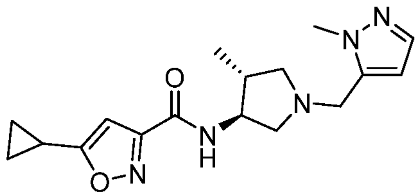 | None | (±)-*trans*-5-cyclopropyl-N-[4-methyl-1-[(1-methyl-1H-pyrazol-5-yl)methyl]pyrrolidin-3-yl]-1,2-oxazole-3-carboxamide | 330.1 | > 50.0 |
| 185 | 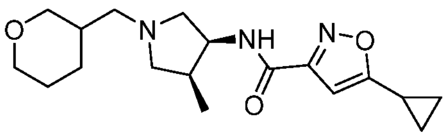 | None | (±)-*cis*-5-cyclopropyl-N-[4-methyl-1-(oxan-3-ylmethyl)pyrrolidin-3-yl]-1,2-oxazole-3-carboxamide | 334.1 | 4.91521 |

(continued)

| Cpd. No. | Structure | Salt Form | Chemical Name | LCMS M+H | SMYD2 Biochem $IC_{50}$ (μM)* |
|---|---|---|---|---|---|
| 186 | 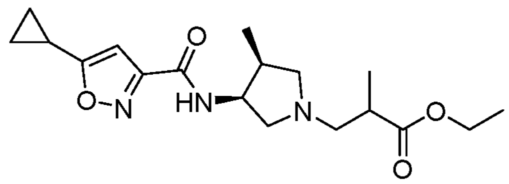 | None | *cis*-ethyl 3-[3-(5-cyclopropyl-1,2-oxazole-3-amido)-4-methylpyrrolidin-1-yl]-2-methylpropanoate | 350.1 | 20.83071 |
| 187 | 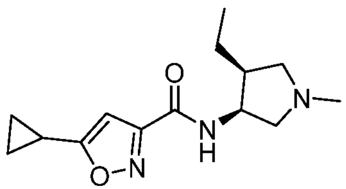 | None | (±)-*cis*-5-cyclopropyl-N-[4-ethyl-1-methylpyrrolidin-3-yl]-1,2-oxazole-3-carboxamide | 264.1 | 18.11728 |
| 188 | 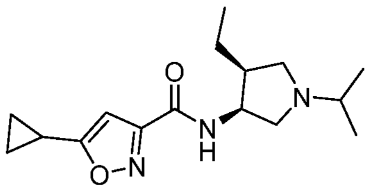 | None | (±)-*cis*-5-cyclopropyl-N-[4-ethyl-1-(propan-2-yl)pyrrolidin-3-yl]-1,2-oxazole-3-carboxamide | 292.2 | 15.93961 |
| 189 | 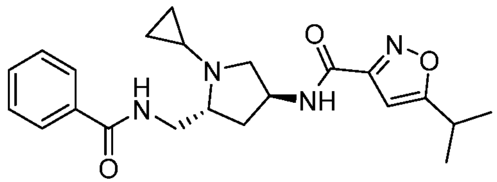 | None | (±)-*trans*-N-[1-cyclopropyl-5-[(phenylformamido)methyl] pyrrolidin-3-yl]-5-(propan-2-yl)-1,2-oxazole-3-carboxamide | 397.05 | > 50 |
| 190 | 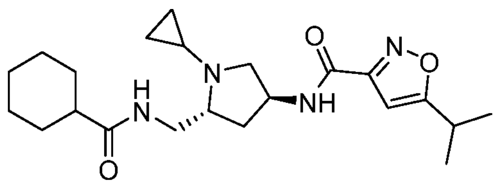 | None | (±)-*trans*-N-[5-[(cyclohexylformamido)methyl]-1 -cyclopropylpyrrolidin-3-yl]-5-(propan-2-yl)-1,2-oxazole-3-carboxamide | 403.05 | > 50 |

(continued)

| Cpd. No. | Structure | Salt Form | Chemical Name | LCMS M+H | SMYD2 Biochem $IC_{50}$ (μM)* |
|---|---|---|---|---|---|
| 191 | 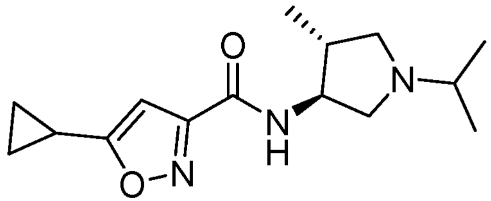 | None | (±)-*trans*-5-cyclopropyl-N-[4-methyl-1-(propan-2-yl)pyrrolidin-3-yl]-1,2-oxazole-3-carboxamide | 278.05 | 40.3 |
| 192 | 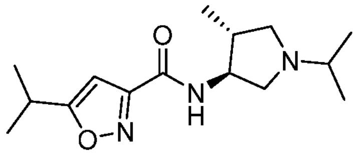 | None | (±)-*trans*-N-[4-methyl-1-(propan-2-yl)pyrrolidin-3-yl]-5-(propan-2-yl)-1,2-oxazole-3-carboxamide | 280.2 | > 50.0 |
| 193 | 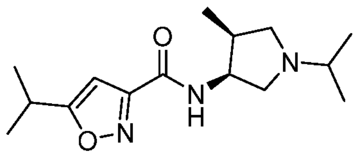 | None | (±)-*cis*-N-[4-methyl-1-(propan-2-yl)pyrrolidin-3-yl]-5-(propan-2-yl)-1,2-oxazole-3-carboxamide | 280 | 7.85157 |
| 194 | 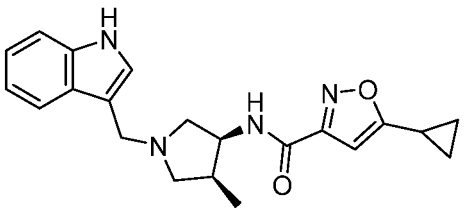 | None | (±)-*cis*-5-cyclopropyl-N-[ 1 -(1H-indol-3-ylmethyl)-4-methylpyrrolidin-3-yl]-1,2-oxazole-3-carboxamide | 365 | 6.86265 |
| 195 | 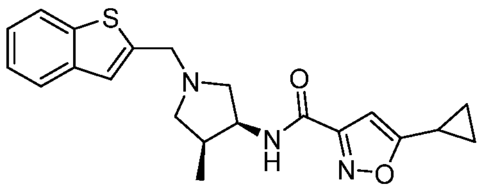 | None | (±)-*cis*-N-[1-(1-benzothiophen-2-ylmethyl)-4-methylpyrrolidin-3-yl]-5-cyclopropyl-1,2-oxazole-3-carboxamide | 381.9 | 33.4307 |

(continued)

| Cpd. No. | Structure | Salt Form | Chemical Name | LCMS M+H | SMYD2 Biochem $IC_{50}$ (μM)* |
|---|---|---|---|---|---|
| 196 | 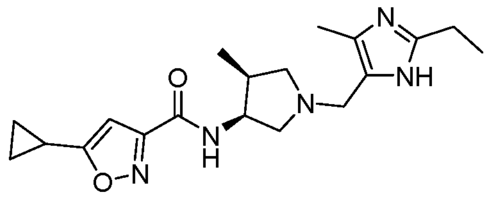 | None | (±)-*cis*-5-cyclopropyl-N-(1-[(2-ethyl-4-methyl-1H-imidazol-5-yl) methyl]-4-methylpyrrolidin-3-yl)-1,2-oxazole-3-carboxamide | 358.2 | 35.45364 |
| 197 | 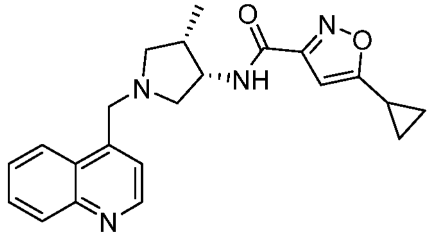 | None | (±)-*cis*-5-cyclopropyl-N-[4-methyl-1-(quinolin-4-ylmethyl) pyrrolidin-3-yl]-1,2-oxazole-3-carboxamide | 377 | > 50.0 |
| 198 | 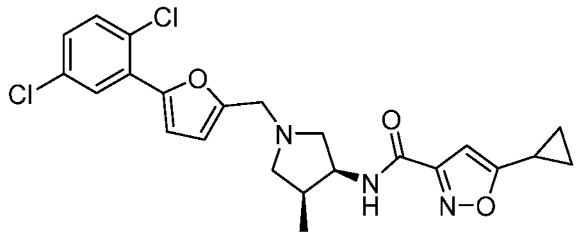 | None | (±)-*cis*-5-cyclopropyl-N-(1-{[5-(2,5-dichlorophenyl)furan-2-yl] methyl}-4-methylpyrrolidin-3-yl)-1,2-oxazole-3-carboxamide | 459.9 | > 50.0 |
| 199 | 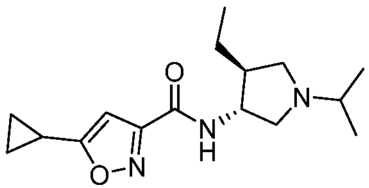 | None | (±)-*trans*-5-cyclopropyl-N-[4-ethyl-1-(propan-2-yl)pyrrolidin-3-yl]-1,2-oxazole-3-carboxamide | 292.2 | 25.7354 |

(continued)

| Cpd. No. | Structure | Salt Form | Chemical Name | LCMS M+H | SMYD2 Biochem $IC_{50}$ (μM)* |
|---|---|---|---|---|---|
| 200 | 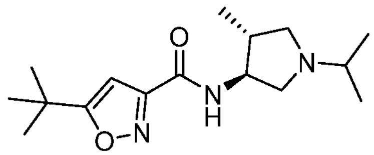 | None | (±)-*trans*-5-tert-butyl-N-[4-methyl-1-(propan-2-yl)pyrrolidin-3-yl]-1,2-oxazole-3-carboxamide | 294.2 | > 50.0 |
| 201 | 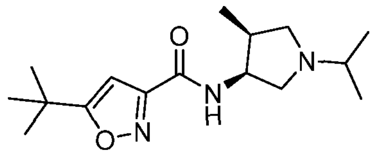 | None | (±)-*cis*-5 -tert-butyl-N-[4-methyl-1-(propan-2-yl)pyrrolidin-3-yl]-1,2-oxazole-3-carboxamide | 294.2 | > 50.0 |
| 202 | 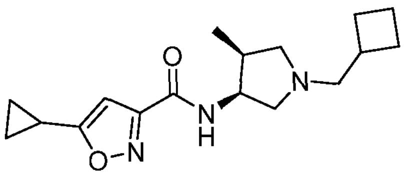 | None | (±)-*cis*-N-[ 1-(cyclobutylmethyl)-4-methylpyrrolidin-3-yl]-5-cyclopropyl-1,2-oxazole-3-carboxamide | 304.2 | 1.63077 |
| 203 | 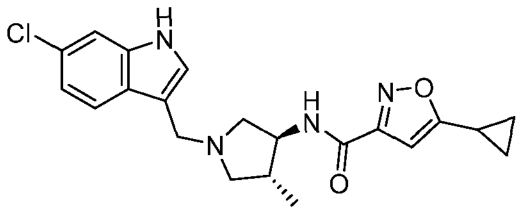 | None | (±)-*trans*-N-[1-[(6-chloro-1H-indol-3-yl)methyl]-4-methylpyrrolidin-3-yl]-5-cyclopropyl-1,2-oxazole-3-carboxamide | 399.1 | 48.27382 |
| 204 | 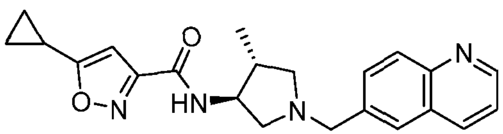 | None | (±)-*trans*-5-cyclopropyl-N-[4-methyl-1-(quinolin-6-ylmethyl) pyrrolidin-3 - yl]-1,2-oxazole-3-carboxamide | 377.1 | > 50.0 |

(continued)

| Cpd. No. | Structure | Salt Form | Chemical Name | LCMS M+H | SMYD2 Biochem $IC_{50}$ (μM)* |
|---|---|---|---|---|---|
| 205 | 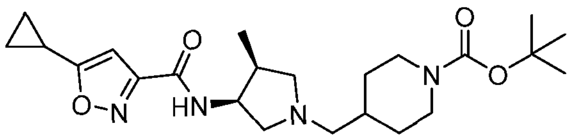 | None | (±)-*cis*-tert-butyl 4-{[3-(5-cyclopropyl-1,2-oxazole-3-amido)-4-methylpyrrolidin-1-yl]methyl}piperidine-1-carboxylate | 433.3 | 7.69173 |
| 206 | 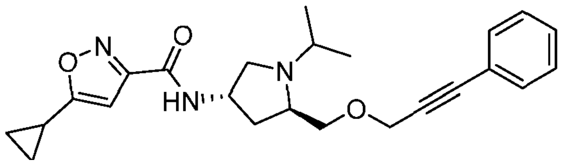 | None | (±)-*trans*-5-cyclopropyl-N-[5-{[(3-phenylprop-2-yn-1-yl)oxy] methyl}-(propan-2-yl)pyrrolidin-3-yl]-1,2-oxazole-3-carboxamide | 408 | |
| 207 | 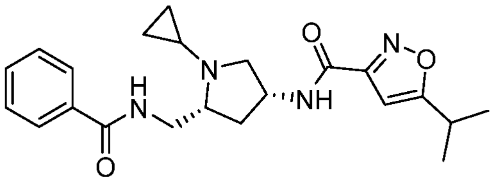 | None | (±)-*cis*-N-[ 1-cyclopropyl-5-[(phenylformamido)methyl]pyrrolidin-3-yl]-5-(propan-2-yl)-1,2-oxazole-3-carboxamide | 397.55 | |
| 208 | 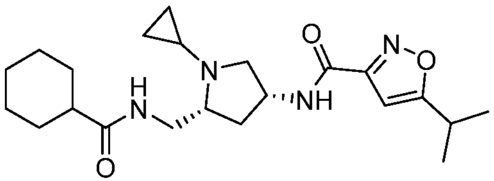 | None | (±)-*cis*-N-[5-[(cyclohexylformamido)methyl]-1 -cyclopropylpyrrolidin-3-yl]-5-(propan-2-yl)-1,2-oxazole-3-carboxamide | 403.05 | |
| 209 | 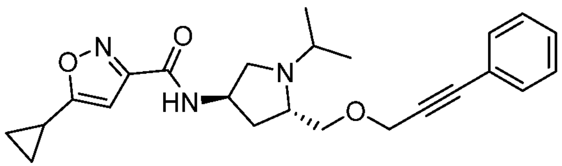 | None | (±)-*trans*-5-cyclopropyl-N-[5-{[(3-phenylprop-2-yn-1-yl)oxy] methyl}-1-(propan-2-yl)pyrrolidin-3-yl]-1,2-oxazole-3-carboxamide | 408.05 | |

(continued)

| Cpd. No. | Structure | Salt Form | Chemical Name | LCMS M+H | SMYD2 Biochem $IC_{50}$ (μM)* |
|---|---|---|---|---|---|
| 210 | 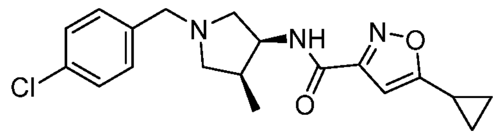 | None | (±)-*cis*-N-[1 -[(4-chlorophenyl)methyl]-4-methylpyrrolidin-3-yl]-5-cyclopropyl-1,2-oxazole-3-carboxamide | 360.1 | 4.31614 |
| 211 | 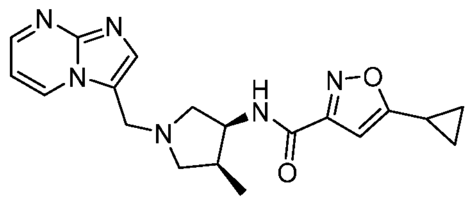 | None | (±)-*cis*-5 -cyclopropyl-N-[ 1-{imidazo[1,2-a]pyrimidin-3-ylmethyl}-4-methylpyrrolidin-3-yl]-1,2-oxazole-3-carboxamide | 367.2 | > 50.0 |
| 212 | 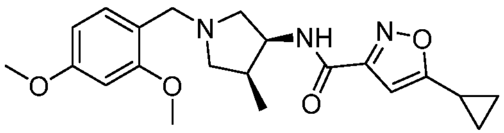 | None | (±)-*cis*-5-cyclopropyl-N-[1-[(2,4-dimethoxyphenyl)methyl]-4-methylpyrrolidin-3-yl]-1,2-oxazole-3-carboxamide | 386.1 | 5.23053 |
| 213 | 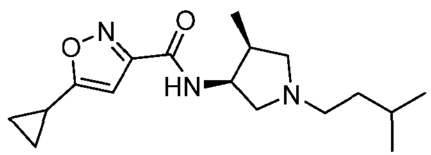 | None | (±)-*cis*-5-cyclopropyl-N-[4-methyl-1-(3-methylbutyl)pyrrolidin-3-yl]-1,2-oxazole-3-carboxamide | 306.2 | 1.61827 |
| 214 | 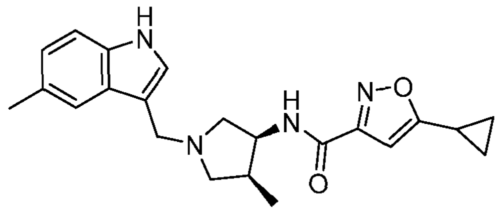 | None | (±)-*cis*-5-cyclopropyl-N-[4-methyl-1-[(5-methyl-1H-indol-3-yl) methyl]pyrrolidin-3-yl]-1,2-oxazole-3-carboxamide | 379.1 | 29.51428 |

(continued)

| Cpd. No. | Structure | Salt Form | Chemical Name | LCMS M+H | SMYD2 Biochem $IC_{50}$ (μM)* |
|---|---|---|---|---|---|
| 215 | 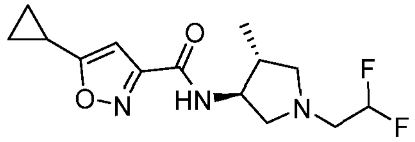 | None | (±)-*trans*-5-cyclopropyl-N-[1-(2,2-difluoroethyl)-4-methylpyrrolidin-3-yl]-1,2-oxazole-3-carboxamide | 300.1 | > 50.0 |
| 216 | 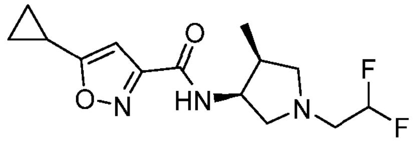 | None | (±)-*cis*-5-cyclopropyl-N-[1-(2,2-difluoroethyl)-4-methylpyrrolidin-3-yl]-1,2-oxazole-3-carboxamide | 300.1 | > 50.0 |
| 217 | 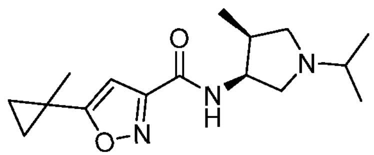 | None | (±)-*cis*-N-[4-methyl-1-(propan-2-yl)pyrrolidin-3-yl]-5-(1-methylcyclopropyl)-1,2-oxazole-3-carboxamide | 292.2 | 22.25498 |
| 218 | 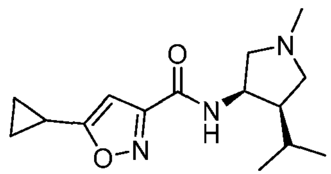 | None | (±)-*cis*-5-cyclopropyl-N-[1-methyl-4-(propan-2-yl)pyrrolidin-3-yl]-1,2-oxazole-3-carboxamide | 278.1 | 37.43706 |
| 219 | 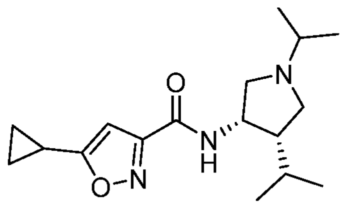 | None | (±)-*cis*-N-[1,4-bis(propan-2-yl)pyrrolidin-3-yl]-5-cyclopropyl-1,2-oxazole-3-carboxamide | 306.3 | > 50.0 |

(continued)

| Cpd. No. | Structure | Salt Form | Chemical Name | LCMS M+H | SMYD2 Biochem $IC_{50}$ (μM)* |
|---|---|---|---|---|---|
| 220 | 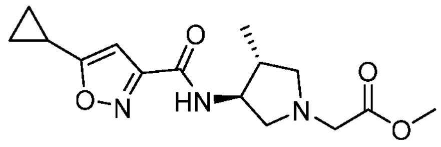 | None | (±)-*trans*-methyl 2-[3-(5-cyclopropyl-1,2-oxazole-3-amido)-4-methylpyrrolidin-1-yl]acetate | 308 | > 50.0 |
| 221 | 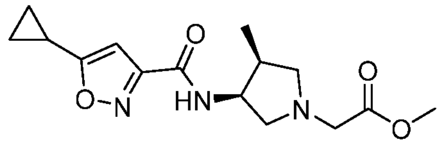 | None | (±)-*cis*-methyl 2-[3-(5-cyclopropyl-1,2-oxazole-3-amido)-4-methylpyrrolidin-1-yl]acetate | 308 | > 50.0 |
| 222 | 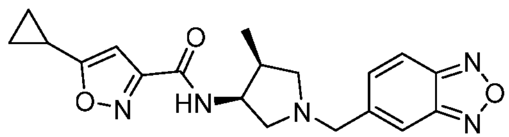 | None | (±)-*cis*-N-[1-(2,1,3-benzoxadiazol-5-ylmethyl)-4-methylpyrrolidin-3-yl]-5-cyclopropyl-1,2-oxazole-3-carboxamide | 368.1 | 41.62391 |
| 223 | 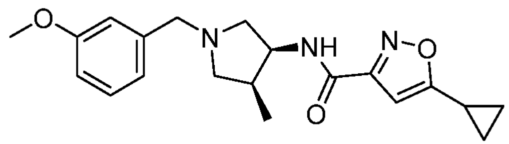 | None | (±)-*cis*-5-cyclopropyl-N-[1-[(3-methoxyphenyl)methyl]-4-methylpyrrolidin-3-yl]-1,2-oxazole-3-carboxamide | 356.1 | 4.9583 |
| 224 | 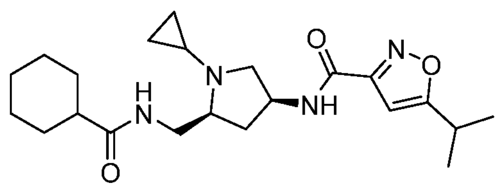 | None | (±)-*cis*-N-[5-[(cyclohexylformamido)methyl]-1 -cyclopropylpyrrolidin-3-yl]-5-(propan-2-yl)-1,2-oxazole-3-carboxamide | 403.3 | > 50 |

(continued)

| Cpd. No. | Structure | Salt Form | Chemical Name | LCMS M+H | SMYD2 Biochem $IC_{50}$ (μM)* |
|---|---|---|---|---|---|
| 225 | 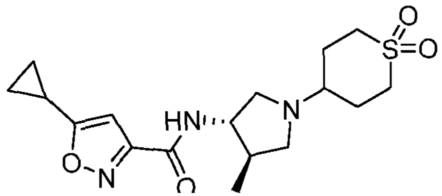 | None | (±)-*trans*-5-cyclopropyl-N-[1-(1,1-dioxo-1??-thian-4-yl)-4-methylpyrrolidin-3-yl]-1,2-oxazole-3-carboxamide | 368 | > 50 |
| 226 | 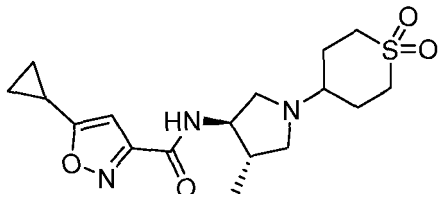 | None | (±)-*trans*-5-cyclopropyl-N-(1-(1,1-dioxidotetrahydro-2H-thiopyran-4-yl)-4-methylpyrrolidin-3-yl)isoxazole-3-carboxamide | 368 | |
| 227 | 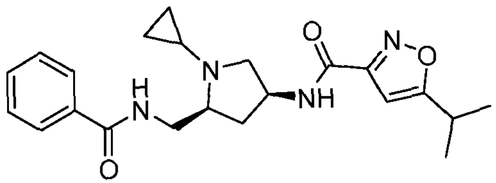 | None | (±)-*cis*-N-[1-cyclopropyl-5-[(phenylformamido)methyl]pyrrolidin-3-yl]-5-(propan-2-yl)-1,2-oxazole-3-carboxamide | 397.3 | > 50 |
| 228 | 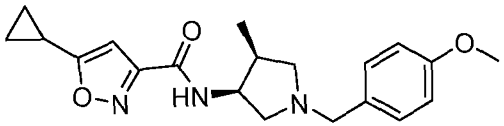 | None | (±)-*cis*-5-cyclopropyl-N-[1-[(4-methoxyphenyl)methyl]-4-methylpyrrolidin-3-yl]-1,2-oxazole-3-carboxamide | 356.1 | 3.62844 |
| 229 | 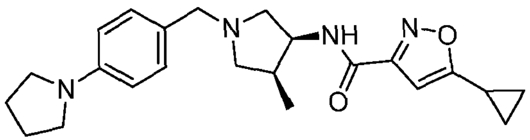 | None | (±)-*cis*-5-cyclopropyl-N-[4-methyl-1-{[4-(pyrrolidin-1-yl)phenyl]methyl}pyrrolidin-3-yl]-1,2-oxazole-3-carboxamide | 395.3 | 3.31567 |

(continued)

| Cpd. No. | Structure | Salt Form | Chemical Name | LCMS M+H | SMYD2 Biochem $IC_{50}$ (μM)* |
|---|---|---|---|---|---|
| 230 | 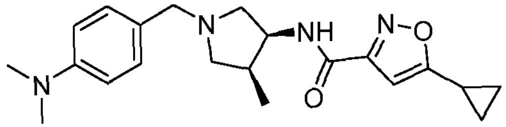 | None | (±)-*cis*-5-cyclopropyl-N-[1 -{[4-(dimethylamino)phenyl]methyl}-4-methylpyrrolidin-3-yl]-1,2-oxazole-3-carboxamide | 369.2 | 4.08446 |
| 231 | 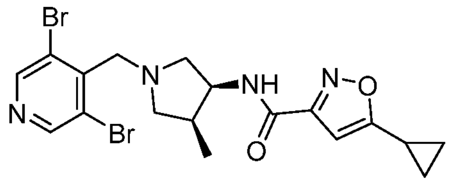 | None | (±)-*cis*-5-cyclopropyl-N-[1-[(3,5-dibromopyridin-4-yl)methyl]-4-methylpyrrolidin-3-yl]-1,2-oxazole-3-carboxamide | 485 | > 50.0 |
| 232 | 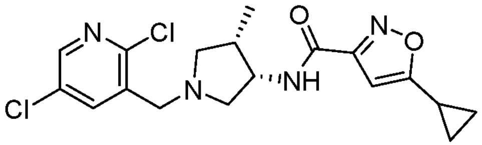 | None | (±)-*cis*-5-cyclopropyl-N-[1-[(2,5-dichloropyridin-3-yl)methyl]-4-methylpyrrolidin-3-yl]-1,2-oxazole-3-carboxamide | 395 | 36.14122 |
| 233 | 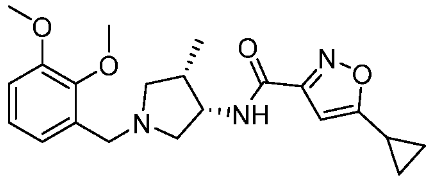 | None | (±)-*cis*-5-cyclopropyl-N-[1-[(2,3-dimethoxyphenyl)methyl]-4-methylpyrrolidin-3-yl]-1,2-oxazole-3-carboxamide | 386.2 | 2.27393 |
| 234 | 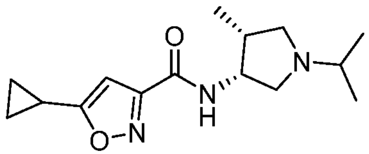 | None | 5-cyclopropyl-N-[(3R,4R)-4-methyl-1-(propan-2-yl)pyrrolidin-3-yl]-1,2-oxazole-3-carboxamide | 278.2 | 0.68185 |

(continued)

| Cpd. No. | Structure | Salt Form | Chemical Name | LCMS M+H | SMYD2 Biochem $IC_{50}$ (μM)* |
|---|---|---|---|---|---|
| 235 | 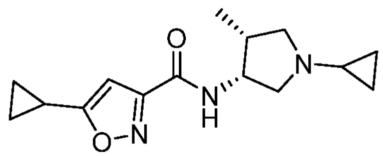 | None | 5-cyclopropyl-N-[(3R,4R)-1-cyclopropyl-4-methylpyrrolidin-3-yl]-1,2-oxazole-3-carboxamide | 276.2 | 1.15216 |
| 236 | 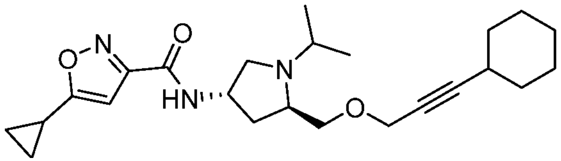 | None | (±)-*trans*-N-[5-{[(3-cyclohexylprop-2-yn-1-yl)oxy]methyl}-1-(propan-2-yl)pyrrolidin-3-yl]-5-cyclopropyl-1,2-oxazole-3-carboxamide | 414.1 | > 50 |
| 237 | 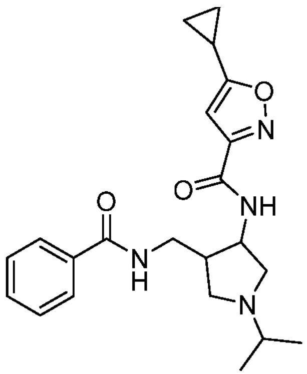 | None | 5-cyclopropyl-N-{4-[(phenylformamido)methyl]-1-(propan-2-yl) pyrrolidin-3-yl}-1,2-oxazole-3-carboxamide | 397.05 | 10.5 |
| 238 | 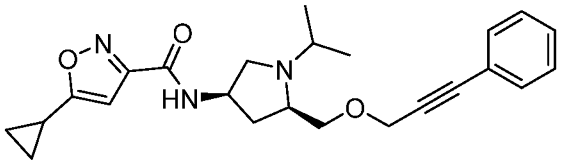 | None | (±)-*cis*-5-cyclopropyl-N-[5-{ [(3-phenylprop-2-yn-1-yl)oxy] methyl}-1-(propan-2-yl)pyrrolidin-3-yl]-1,2-oxazole-3-carboxamide | 408.05 | 39.7 |

(continued)

| Cpd. No. | Structure | Salt Form | Chemical Name | LCMS M+H | SMYD2 Biochem $IC_{50}$ (μM)* |
|---|---|---|---|---|---|
| 239 | 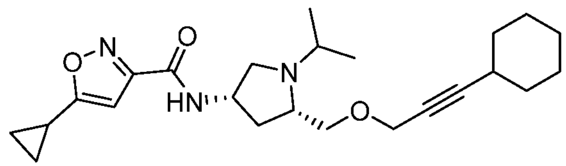 | None | (±)-*cis*-N-[5-{[(3-cyclohexylprop-2-yn-1-yl)oxy]methyl}-1-(propan-2-yl)pyrrolidin-3-yl]-5-cyclopropyl-1,2-oxazole-3-carboxamide | 414.3 | > 50.0 |
| 240 | 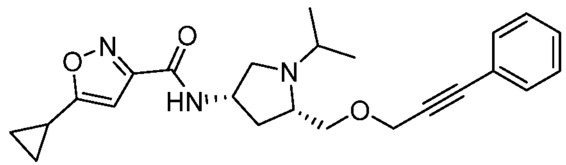 | None | (±)-*cis*-5-cyclopropyl-N-[(5-{[(3-phenylprop-2-yn-1-yl)oxy] methyl}-1-(propan-2-yl)pyrrolidin-3-yl]-1,2-oxazole-3-carboxamide | 408.3 | > 50.0 |
| 241 | 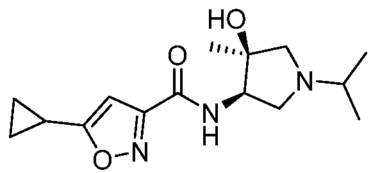 | None | (±)-*cis*-5-cyclopropyl-N-[4-hydroxy-4-methyl-1-(propan-2-yl) pyrrolidin-3-yl]-1,2-oxazole-3-carboxamide | 294 | 24.8 |
| 242 | 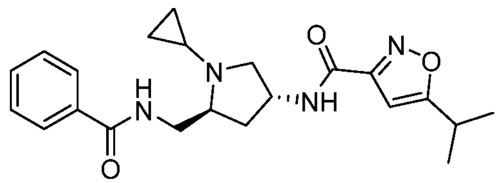 | None | (±)-*trans*-N-[1-cyclopropyl-5-[(phenylformamido)methyl] pyrrolidin-3-yl]-5-(propan-2-yl)-1,2-oxazole-3-carboxamide | 397.05 | > 50.0 |
| 243 | 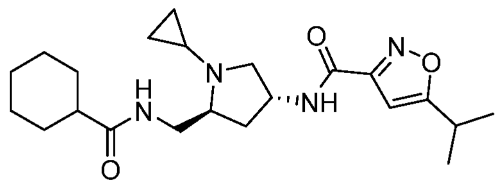 | None | (±)-*trans*-N-[5-[(cyclohexylformamido)methyl]-1 -cyclopropylpyrrolidin-3-yl]-5-(propan-2-yl)-1,2-oxazole-3-carboxamide | 403.5 | > 50.0 |

(continued)

| Cpd. No. | Structure | Salt Form | Chemical Name | LCMS M+H | SMYD2 Biochem $IC_{50}$ (μM)* |
|---|---|---|---|---|---|
| 244 | 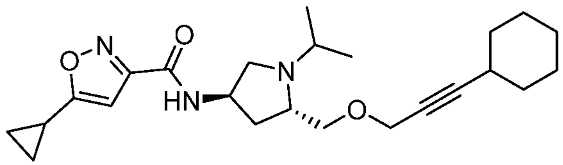 | None | (±)-*trans*-N-[5-{[(3-cyclohexylprop-2-yn-1-yl)oxy]methyl}-1-(propan-2-yl)pyrrolidin-3-yl]-5-cyclopropyl-1,2-oxazole-3-carboxamide | 414 | > 50.0 |
| 245 | 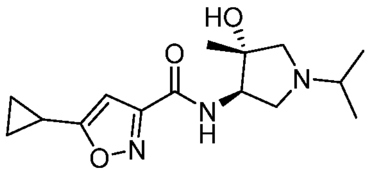 | None | 5-cyclopropyl-N-[(3R,4R)-4-hydroxy-4-methyl-1-(propan-2-yl) pyrrolidin-3-yl]-1,2-oxazole-3-carboxamide | 294 | 6.8 |
| 246 | 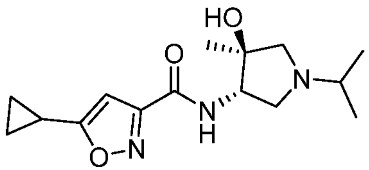 | None | 5-cyclopropyl-N-[(3 S,4S)-4-hydroxy-4-methyl-1-(propan-2-yl) pyrrolidin-3-yl]-1,2-oxazole-3-carboxamide | 294 | > 50.0 |
| 247 | 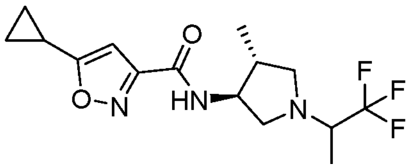 | None | trans-5-cyclopropyl-N-[(3 S,4R)-4-methyl-1-(1,1,1-trifluoropropan-2-yl)pyrrolidin-3-yl]-1,2-oxazole-3-carboxamide | 332.2 | > 50.0 |
| 248 | 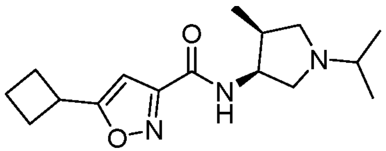 | None | (±)-*cis*-5-cyclobutyl-N-[4-methyl-1-(propan-2-yl)pyrrolidin-3-yl]-1,2-oxazole-3-carboxamide | 292.2 | 10.48654 |

(continued)

| Cpd. No. | Structure | Salt Form | Chemical Name | LCMS M+H | SMYD2 Biochem $IC_{50}$ (μM)* |
|---|---|---|---|---|---|
| 249 | 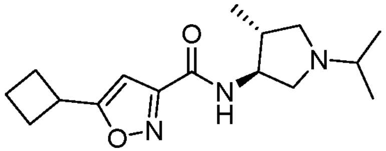 | None | (±)-*trans*-5-cyclobutyl-N-[4-methyl-1-(propan-2-yl)pyrrolidin-3-yl]-1,2-oxazole-3-carboxamide | 292.2 | > 50.0 |
| 250 | 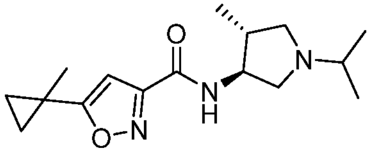 | None | (±)-*trans*-N-[4-methyl-1-(propan-2-yl)pyrrolidin-3-yl]-5-(1-methylcyclopropyl)-1,2-oxazole-3-carboxamide | 292.2 | > 50.0 |
| 251 | 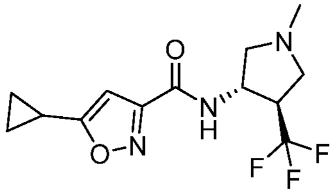 | None | (±)-*trans*-5-cyclopropyl-N-[1-methyl-4-(trifluoromethyl) pyrrolidin-3-yl]-1,2-oxazole-3-carboxamide | 304.1 | > 50.0 |
| 252 | 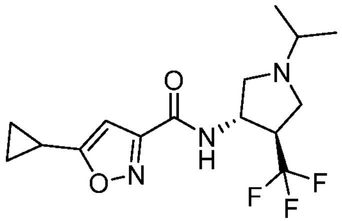 | None | (±)-*trans*-5-cyclopropyl-N-[1-(propan-2-yl)-4-(trifluoromethyl) pyrrolidin-3-yl]-1,2-oxazole-3-carboxamide | 332.1 | > 50.0 |
| 253 | 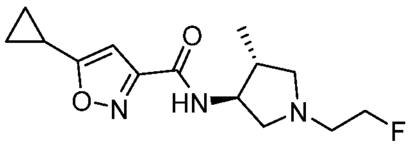 | None | (±)-*trans*-5-cyclopropyl-N-[1-(2-fluoroethyl)-4-methylpyrrolidin-3-yl]-1,2-oxazole-3-carboxamide | 282.2 | > 50.0 |

(continued)

| Cpd. No. | Structure | Salt Form | Chemical Name | LCMS M+H | SMYD2 Biochem $IC_{50}$ (μM)* |
|---|---|---|---|---|---|
| 254 | 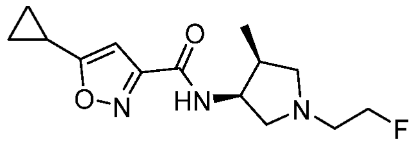 | None | (±)-*cis*-5-cyclopropyl-N-[ 1-(2-fluoroethyl)-4-methylpyrrolidin-3-yl]-1,2-oxazole-3-carboxamide | 282.2 | 5.71585 |
| 255 | 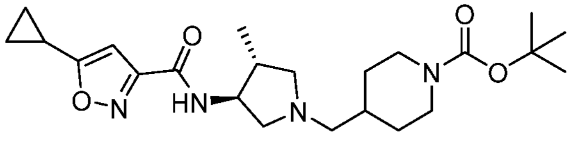 | None | (±)-*trans*-tert-butyl 4-{[3-(5-cyclopropyl-1,2-oxazole-3-amido)-4-methylpyrrolidin-1-yl]methyl}piperidine-1-carboxylate | 433.4 | > 50.0 |
| 256 | 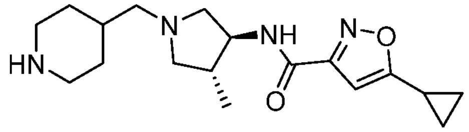 | None | (±)-*trans*-5-cyclopropyl-N-[4-methyl-1-(piperidin-4-ylmethyl) pyrrolidin-3-yl]-1,2-oxazole-3-carboxamide | 333.2 | > 50.0 |
| 257 | 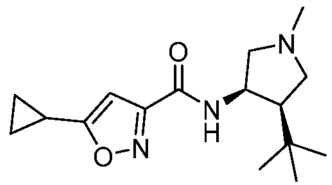 | None | (±)-*cis*-N-[4-tert-butyl-1-methylpyrrolidin-3-yl]-5-cyclopropyl-1,2-oxazole-3-carboxamide | 292.3 | > 50.0 |
| 258 | 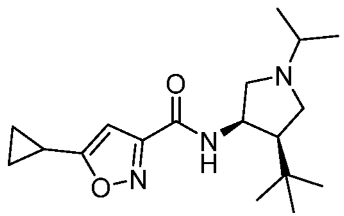 | None | (±)-cis-N-[4-tert-butyl- I -(propan-2-yl)pyrrolidin-3-yl]-5-cyclopropyl-1,2-oxazole-3-carboxamide | 320.3 | > 50.0 |

(continued)

| Cpd. No. | Structure | Salt Form | Chemical Name | LCMS M+H | SMYD2 Biochem $IC_{50}$ (μM)* |
|---|---|---|---|---|---|
| 259 | 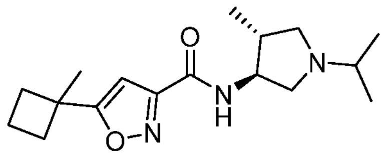 | None | (±)-*trans*-N-[4-methyl-1-(propan-2-yl)pyrrolidin-3-yl]-5-(1-methylcyclobutyl)-1,2-oxazole-3-carboxamide | 306.3 | > 50.0 |
| 260 | 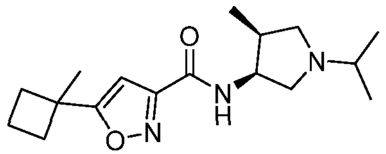 | None | (±)-*cis*-N-[4-methyl-1-(propan-2-yl)pyrrolidin-3-yl]-5-(1-methylcyclobutyl)-1,2-oxazole-3-carboxamide | 306.3 | > 50.0 |
| 261 | 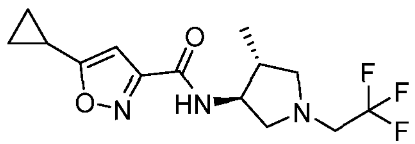 | None | (±)-*trans*-5-cyclopropyl-N-[4-methyl-1-(2,2,2-trifluoroethyl) pyrrolidin-3-yl]-1,2-oxazole-3-carboxamide | 318.2 | > 50.0 |
| 262 | 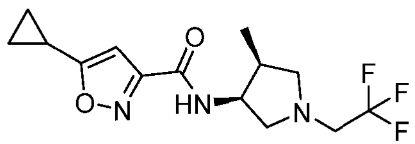 | None | (±)-*cis*-5-cyclopropyl-N-[4-methyl-1-(2,2,2-trifluoroethyl) pyrrolidin-3-yl]-1,2-oxazole-3-carboxamide | 318.2 | > 50.0 |
| 263 | 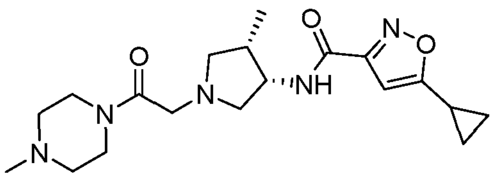 | None | (±)-*cis*-5-cyclopropyl-N-[4-methyl-1-[2-(4-methylpiperazin-1-yl)-2-oxoethyl]pyrrolidin-3-yl]-1,2-oxazole-3-carboxamide | 376.2 | > 50.0 |
| 264 | 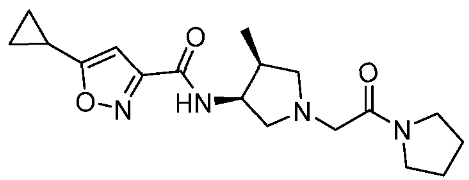 | None | (±)-*cis*-5-cyclopropyl-N-[4-methyl-1-[2-oxo-2-(pyrrolidin-1-yl) ethyl]pyrrolidin-3-yl]-1,2-oxazole-3-carboxamide | 347.1 | > 50.0 |

(continued)

| Cpd. No. | Structure | Salt Form | Chemical Name | LCMS M+H | SMYD2 Biochem $IC_{50}$ (μM)* |
|---|---|---|---|---|---|
| 265 | 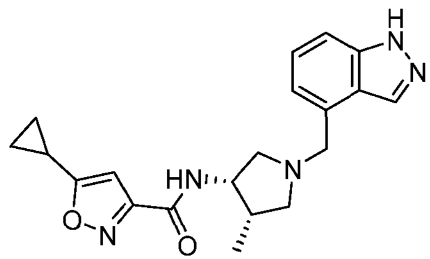 | None | (±)-*cis*-5-cyclopropyl-N-[1-(1H-indazol-4-ylmethyl)-4-methylpyrrolidin-3-yl]-1,2-oxazole-3-carboxamide | 365.9 | 4.89057 |
| 266 | 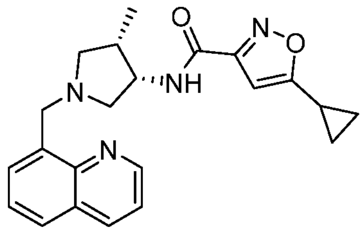 | None | (±)-*cis*-5-cyclopropyl-N-[4-methyl-1-(quinolin-8-ylmethyl) pyrrolidin-3-yl]-1,2-oxazole-3-carboxamide | 377.2 | 12.32177 |
| 267 | 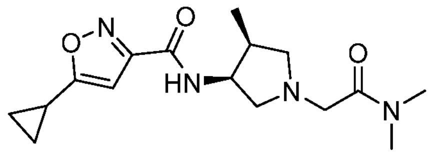 | None | (±)-*cis*-5-cyclopropyl-N-[ 1-[(dimethylcarbamoyl)methyl]-4-methylpyrrolidin-3-yl]-1,2-oxazole-3-carboxamide | 321.1 | > 50.0 |
| 268 | 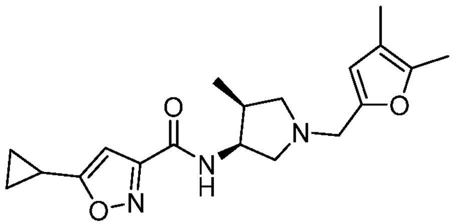 | None | (±)-*cis*-5-cyclopropyl-N-{1-[(4,5-dimethylfuran-2-yl)methyl]-4-methylpyrrolidin-3-yl]-1,2-oxazole-3-carboxamide | 344.1 | 5.3002 |

(continued)

| Cpd. No. | Structure | Salt Form | Chemical Name | LCMS M+H | SMYD2 Biochem $IC_{50}$ (μM)* |
|---|---|---|---|---|---|
| 269 | 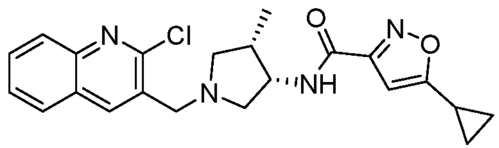 | None | (±)-*cis*-N-[1-[(2-chloroquinolin-3-yl)methyl]-4-methylpyrrolidin-3-yl]-5-cyclopropyl-1,2-oxazole-3-carboxamide | 411.1 | 4.16657 |
| 270 | 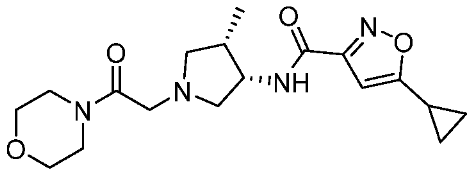 | None | (±)-*cis*-5-cyclopropyl-N-[4-methyl-1-[2-(morpholin-4-yl)-2-oxoethyl]pyrrolidin-3-yl]-1,2-oxazole-3-carboxamide | 363.1 | > 50.0 |
| 271 | 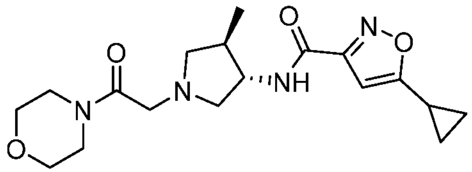 | None | (±)-*trans*-5-cyclopropyl-N-[4-methyl-1-[2-(morpholin-4-yl)-2-oxoethyl]pyrrolidin-3-yl]-1,2-oxazole-3-carboxamide | 363.1 | > 50.0 |
| 272 | 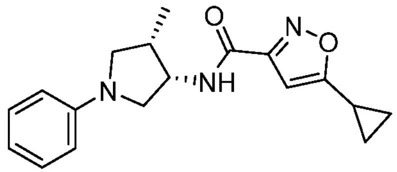 | None | (±)-*cis*-5-cyclopropyl-N-[4-methyl-1-phenylpyrrolidin-3-yl]-1,2-oxazole-3-carboxamide | 312.3 | > 50.0 |
| 273 | 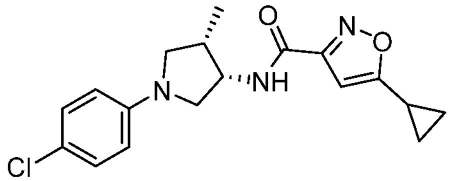 | None | (±)-*cis*-N-[1-(4-chlorophenyl)-4-methylpyrrolidin-3-yl]-5-cyclopropyl-1,2-oxazole-3-carboxamide | 346.1 | > 50.0 |

(continued)

| Cpd. No. | Structure | Salt Form | Chemical Name | LCMS M+H | SMYD2 Biochem $IC_{50}$ (μM)* |
|---|---|---|---|---|---|
| 274 | 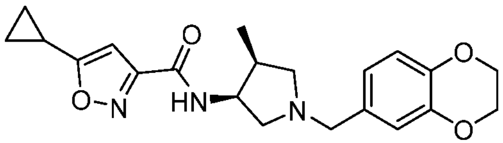 | None | (±)-*cis*-5-cyclopropyl-N-[1-(2,3-dihydro-1,4-benzodioxin-6-ylmethyl)-4-methylpyrrolidin-3-yl]-1,2-oxazole-3-carboxamide | 384.2 | 6.30276 |
| 275 | 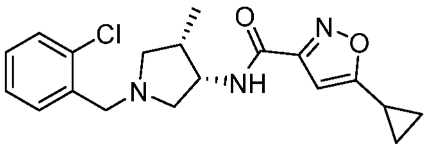 | None | (±)-*cis*-N-{1-[(2-chlorophenyl)methyl]-4-methylpyrrolidin-3-yl}-5-cyclopropyl-1,2-oxazole-3-carboxamide | 360.1 | 3.02348 |
| 276 | 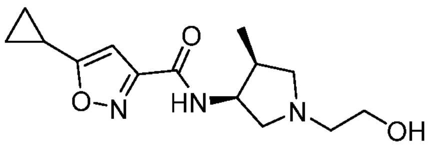 | None | (±)-*cis*-5-cyclopropyl-N-[ 1-(2-hydroxyethyl)-4-methylpyrrolidin-3-yl]-1,2-oxazole-3-carboxamide | 280.1 | 5.85391 |
| 277 | 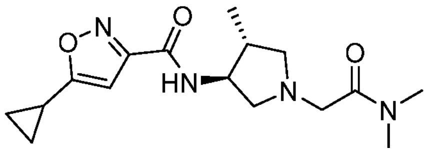 | None | (±)-*trans*-5-cyclopropyl-N-{1-[(dimethylcarbamoyl)methyl]-4-methylpyrrolidin-3-yl}-1,2-oxazole-3-carboxamide | 321.1 | > 50.0 |
| 278 | 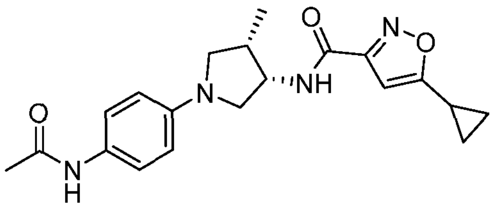 | None | (±)-*cis*-5-cyclopropyl-N-[ 1 -(4-acetamidophenyl)-4-methylpyrrolidin-3-yl]-1,2-oxazole-3-carboxamide | 369.2 | > 50.0 |

(continued)

| Cpd. No. | Structure | Salt Form | Chemical Name | LCMS M+H | SMYD2 Biochem $IC_{50}$ (μM)* |
|---|---|---|---|---|---|
| 279 | 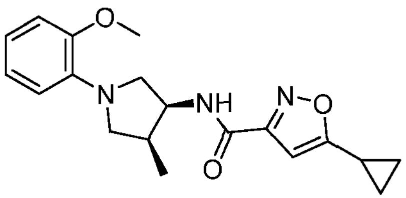 | None | (±)-*cis*-5-cyclopropyl-N-[1-(2-methoxyphenyl)-4-methylpyrrolidin-3-yl]-1,2-oxazole-3-carboxamide | 342.2 | > 50.0 |
| 280 | 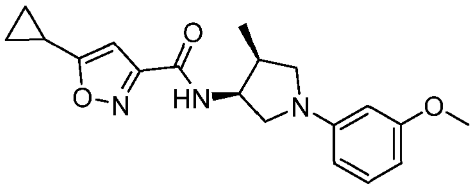 | None | (±)-*cis*-5-cyclopropyl-N-[1-(3-methoxyphenyl)-4-methylpyrrolidin-3-yl]-1,2-oxazole-3-carboxamide | 342.1 | > 50.0 |
| 281 | 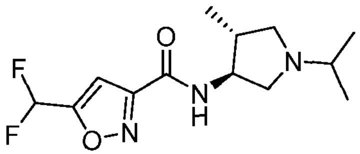 | None | (±)-*trans*-5-(difluoromethyl)-N-[4-methyl-1-(propan-2-yl) pyrrolidin-3-yl]-1,2-oxazole-3-carboxamide | 288.1 | > 50.0 |
| 282 | 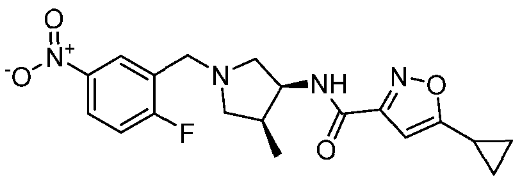 | None | (±)-*cis*-5-cyclopropyl-N-{ 1-[(2-fluoro-5 -nitrophenyl)methyl] -4-methylpyrrolidin-3-yl }-1,2-oxazole-3-carboxamide | 389.1 | 26.74083 |
| 283 | 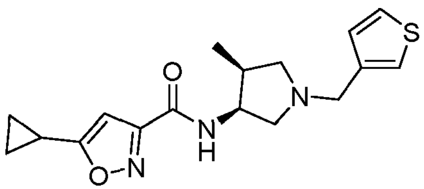 | None | (±)-*cis*-5-cyclopropyl-N-[4-methyl-1-(thiophen-3-ylmethyl) pyrrolidin-3-yl]-1,2-oxazole-3-carboxamide | 332.1 | 1.85244 |

(continued)

| Cpd. No. | Structure | Salt Form | Chemical Name | LCMS M+H | SMYD2 Biochem $IC_{50}$ (μM)* |
|---|---|---|---|---|---|
| 284 | 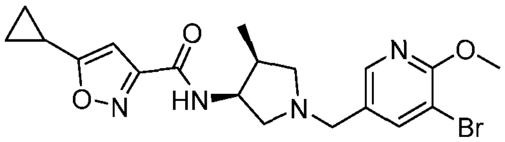 | None | (±)-*cis*-N-{ 1-[(5-bromo-6-methoxypyridin-3-yl)methyl]-4-methylpyrrolidin-3-yl}-5-cyclopropyl-1,2-oxazole-3-carboxamide | 435.1 | 40.52054 |
| 285 | 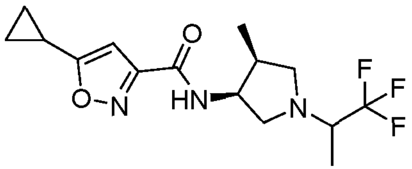 | None | *trans*-5-cyclopropyl-N-[4-methyl-1-(1,1,1-trifluoropropan-2-yl) pyrrolidin-3-yl]-1,2-oxazole-3-carboxamide | 332.1 | > 50.0 |
| 286 | 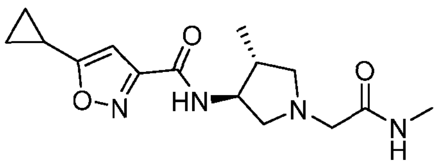 | None | (±)-*trans*-5-cyclopropyl-N-{4-methyl-1-[(methylcarbamoyl) methyl]pyrrolidin-3-yl}-1,2-oxazole-3-carboxamide | 307.1 | > 50.0 |
| 287 | 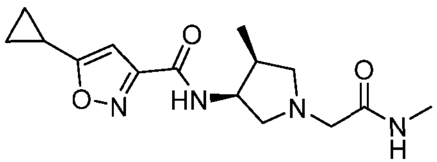 | None | (±)-*cis*-5-cyclopropyl-N-{4-methyl-1-[(methylcarbamoyl)methyl] pyrrolidin-3-yl}-1,2-oxazole-3-carboxamide | 307.2 | > 50.0 |
| 288 | 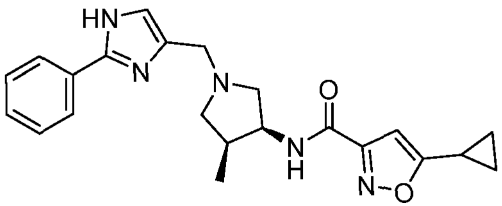 | None | (±)-*cis*-5-cyclopropyl-N-{4-methyl-1-[(2-phenyl-1H-imidazol-4-yl)methyl]pyrrolidin-3-yl}-1,2-oxazole-3-carboxamide | 392.2 | 21.0547 |

(continued)

| Cpd. No. | Structure | Salt Form | Chemical Name | LCMS M+H | SMYD2 Biochem $IC_{50}$ (μM)* |
|---|---|---|---|---|---|
| 289 | 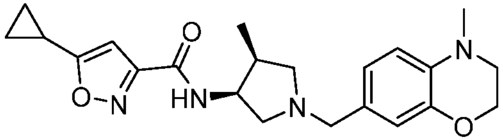 | None | (±)-*cis*-5-cyclopropyl-N-{ 4-methyl-1-[(4-methyl-3,4-dihydro-2H-1,4-benzoxazin-7-yl)methyl]pyrrolidin-3-yl }-1,2-oxazole-3-carboxamide | 397 | 6.07589 |
| 290 | 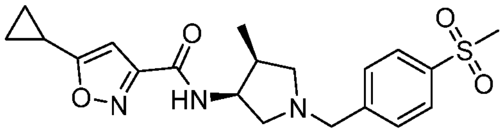 | None | (±)-*cis*-5-cyclopropyl-N-{ 1-[(4-methanesulfonylphenyl)methyl]-4-methylpyrrolidin-3-yl }-1,2-oxazole-3-carboxamide | 403.9 | 10.54803 |
| 291 | 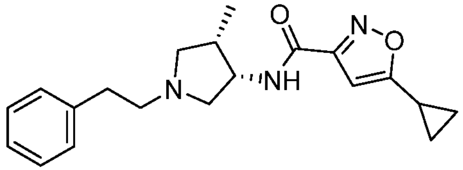 | None | (±)-*cis*-5-cyclopropyl-N-[4-methyl-1-(2-phenylethyl)pyrrolidin-3-yl]-1,2-oxazole-3-carboxamide | 340.1 | 2.45921 |
| 292 | 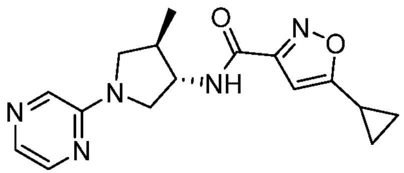 | None | (±)-*trans*-5-cyclopropyl-N-[4-methyl-1-(pyrazin-2-yl)pyrrolidin-3-yl]-1,2-oxazole-3-carboxamide | 314.2 | > 50.0 |
| 293 | 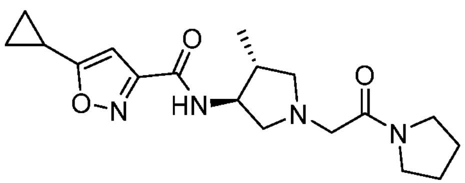 | None | (±)-*trans*-5-cyclopropyl-N-{4-methyl-1-[2-oxo-2-(pyrrolidin-1-yl) ethyl]pyrrolidin-3-yl}-1,2-oxazole-3-carboxamide | 347.1 | > 50.0 |

(continued)

| Cpd. No. | Structure | Salt Form | Chemical Name | LCMS M+H | SMYD2 Biochem $IC_{50}$ (μM)* |
|---|---|---|---|---|---|
| 294 | 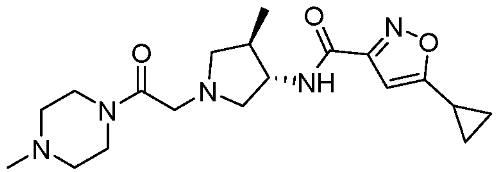 | None | (±)-*trans*-5-cyclopropyl-N-{4-methyl-1-[2-(4-methylpiperazin-1-yl)-2-oxoethyl]pyrrolidin-3-yl}-1,2-oxazole-3-carboxamide | 376.2 | > 50.0 |
| 295 | 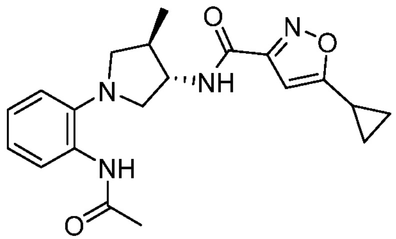 | None | (±)-*trans*-5-cyclopropyl-N-[1-(2-acetamidophenyl)-4-methylpyrrolidin-3-yl]-1,2-oxazole-3-carboxamide | 369.2 | > 50.0 |
| 296 | 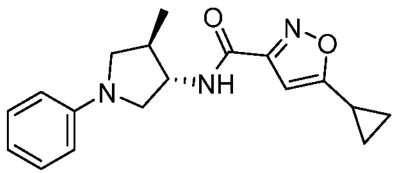 | None | (±)-*trans*-5-cyclopropyl-N-(4-methyl-1-phenylpyrrolidin-3-yl)-1,2-oxazole-3-carboxamide | 312.1 | > 50.0 |
| 297 | 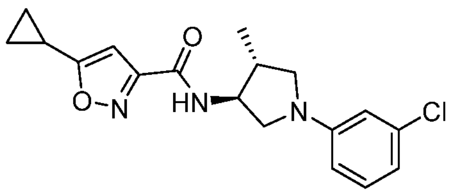 | None | (±)-*trans*-N-[1-(3-chlorophenyl)-4-methylpyrrolidin-3-yl]-5-cyclopropyl-1,2-oxazole-3-carboxamide | 346.1 | > 50.0 |
| 298 | 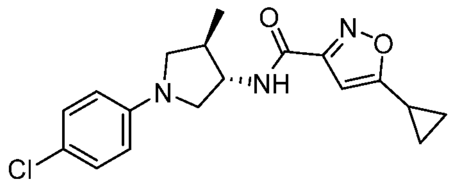 | None | (±)-*trans*-N-[1-(4-chlorophenyl)-4-methylpyrrolidin-3-yl]-5-cyclopropyl-1,2-oxazole-3-carboxamide | 346.2 | > 50.0 |

(continued)

| Cpd. No. | Structure | Salt Form | Chemical Name | LCMS M+H | SMYD2 Biochem $IC_{50}$ (μM)* |
|---|---|---|---|---|---|
| 299 | 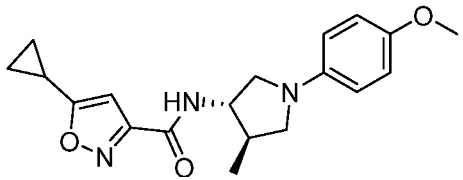 | None | (±)-*trans*-5-cyclopropyl-N-[1-(4-methoxyphenyl)-4-methylpyrrolidin-3-yl]-1,2-oxazole-3-carboxamide | 342.2 | > 50.0 |
| 300 | 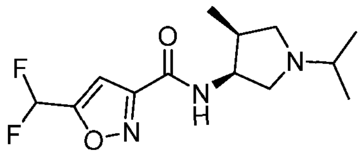 | None | (±)-*cis*-5-(difluoromethyl)-N-[4-methyl-1-(propan-2-yl)pyrrolidin-3-yl]-1,2-oxazole-3-carboxamide | 288.1 | 9.58991 |
| 301 | 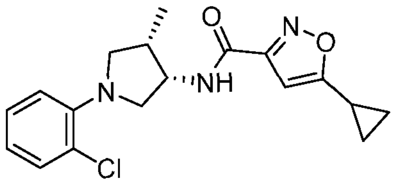 | None | (±)-*cis*-N-[1-(2-chlorophenyl)-4-methylpyrrolidin-3-yl]-5-cyclopropyl-1,2-oxazole-3-carboxamide | 346.1 | > 50.0 |
| 302 | 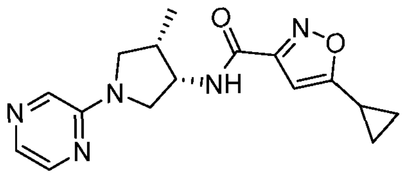 | None | (±)-*cis*-5-cyclopropyl-N-[4-methyl- 1-(pyrazin-2-yl)pyrrolidin-3-yl]-1,2-oxazole-3-carboxamide | 314.2 | > 50.0 |
| 303 | 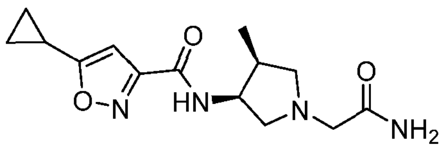 | None | (±)-*cis*-N-[1-(carbamoylmethyl)-4-methylpyrrolidin-3-yl]-5-cyclopropyl-1,2-oxazole-3-carboxamide | 293.1 | > 50.0 |

(continued)

| Cpd. No. | Structure | Salt Form | Chemical Name | LCMS M+H | SMYD2 Biochem $IC_{50}$ (μM)* |
|---|---|---|---|---|---|
| 304 | 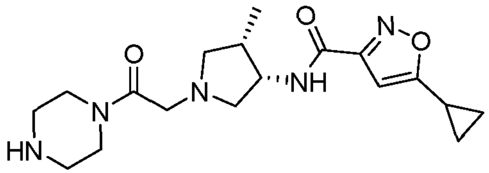 | None | (±)-*cis*-5-cyclopropyl-N-{4-methyl-1-[2-oxo-2-(piperazin-1-yl) ethyl]pyrrolidin-3-yl}-1,2-oxazole-3-carboxamide | 362.1 | > 50.0 |
| 305 | 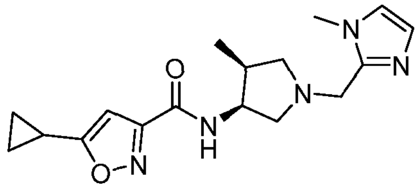 | None | (±)-*cis*-5-cyclopropyl-N-{4-methyl-1-[(1-methyl-1H-imidazol-2-yl)methyl]pyrrolidin-3-yl}-1,2-oxazole-3-carboxamide | 330.2 | 41.08368 |
| 306 | 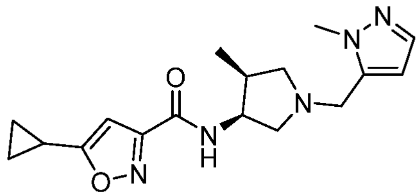 | None | (±)-*cis*-5-cyclopropyl-N-{ 4-methyl-1-[(1-methyl-1H-pyrazol-5-yl) methyl]pyrrolidin-3-yl }-1,2-oxazole-3-carboxamide | 330.2 | 41.18263 |
| 307 | 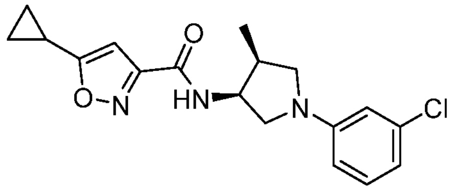 | None | (±)-*cis*-N-[1-(3-chlorophenyl)-4-methylpyrrolidin-3-yl]-5-cyclopropyl-1,2-oxazole-3-carboxamide | 346.1 | > 50.0 |
| 308 | 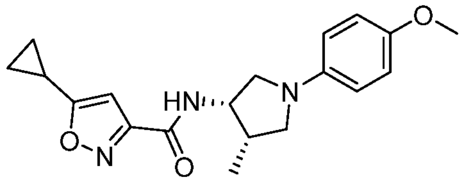 | None | (±)-*cis*-5-cyclopropyl-N-[ 1-(4-methoxyphenyl)-4-methylpyrrolidin-3-yl]-1,2-oxazole-3-carboxamide | 342.2 | > 50.0 |

(continued)

| Cpd. No. | Structure | Salt Form | Chemical Name | LCMS M+H | SMYD2 Biochem $IC_{50}$ (μM)* |
|---|---|---|---|---|---|
| 309 | 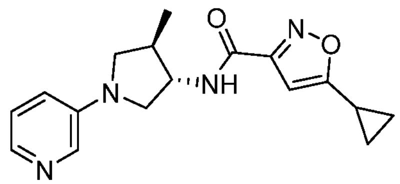 | None | (±)-trans-5-cyclopropyl-N-[4-methyl-1-(pyridin-3-yl)pyrrolidin-3 -yl]-1,2-oxazole-3-carboxamide | 313.2 | > 50.0 |
| 310 | 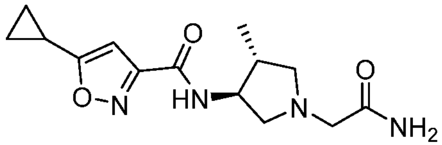 | None | (±)-*trans*-N-[1-(carbamoylmethyl)-4-methylpyrrolidin-3-yl]-5-cyclopropyl-1,2-oxazole-3-carboxamide | 293.1 | > 50.0 |
| 311 | 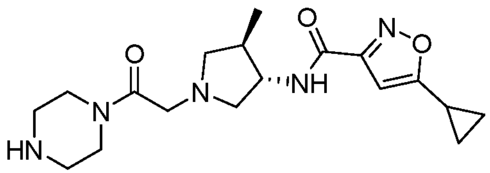 | N one | (±)-*trans*-5-cyclopropyl-N-{4-methyl-1-[2-oxo-2-(piperazin-1-yl) ethyl]pyrrolidin-3-yl}-1,2-oxazole-3-carboxamide | 362.2 | > 50.0 |
| 312 | 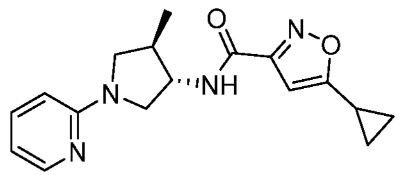 | None | (±)-*trans*-5-cyclopropyl-N-[4-methyl-1-(pyridin-2-yl)pyrrolidin-3-yl]-1,2-oxazole-3-carboxamide | 313.1 | > 50.0 |
| 313 | 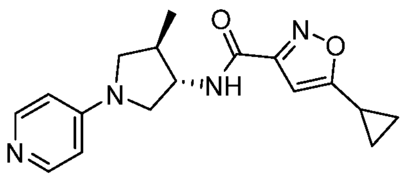 | None | (±)-*trans*-5-cyclopropyl-N-[4-methyl-1-(pyridin-4-yl)pyrrolidin-3-yl]-1,2-oxazole-3-carboxamide | 313.1 | > 50.0 |

(continued)

| Cpd. No. | Structure | Salt Form | Chemical Name | LCMS M+H | SMYD2 Biochem $IC_{50}$ (μM)* |
|---|---|---|---|---|---|
| 314 | 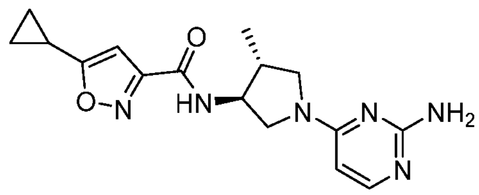 | None | (±)-*trans*-N-[1-(2-aminopyrimidin-4-yl)-4-methylpyrrolidin-3-yl]-5-cyclopropyl-1,2-oxazole-3-carboxamide | 329.1 | > 50.0 |
| 315 | 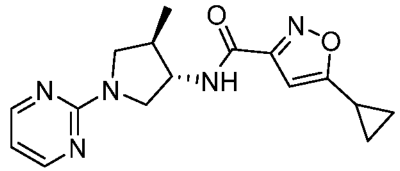 | None | (±)-*trans*-5-cyclopropyl-N-[4-methyl-1-(pyrimidin-2-yl)pyrrolidin-3-yl]-1,2-oxazole-3-carboxamide | 314.2 | > 50.0 |
| 316 | 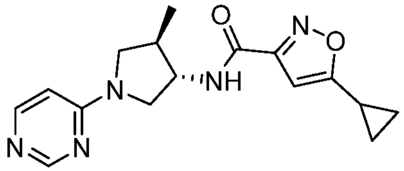 | None | (±)-*trans*-5-cyclopropyl-N-[4-methyl-1-(pyrimidin-4-yl)pyrrolidin-3-yl]-1,2-oxazole-3-carboxamide | 314.1 | > 50.0 |
| 317 | 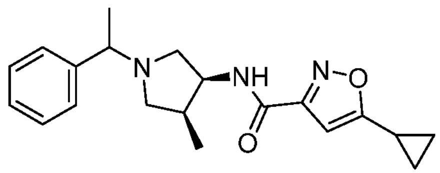 | None | (±)-*cis*-5-cyclopropyl-N-[4-methyl- 1-(1-phenylethyl)pyrrolidin-3-yl]-1,2-oxazole-3-carboxamide | 340.2 | 1.13639 |
| 318 | 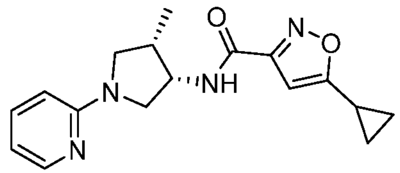 | None | (±)-*cis*-5-cyclopropyl-N-[4-methyl-1-(pyridin-2-yl)pyrrolidin-3-yl]-1,2-oxazole-3-carboxamide | 313.1 | > 50.0 |

(continued)

| Cpd. No. | Structure | Salt Form | Chemical Name | LCMS M+H | SMYD2 Biochem $IC_{50}$ (μM)* |
|---|---|---|---|---|---|
| 319 | 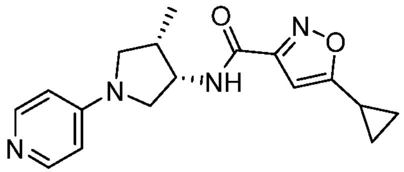 | None | (±)-*cis*-5-cyclopropyl-N-[4-methyl-1-(pyridin-4-yl)pyrrolidin-3-yl]-1,2-oxazole-3-carboxamide | 313.2 | > 50.0 |
| 320 | 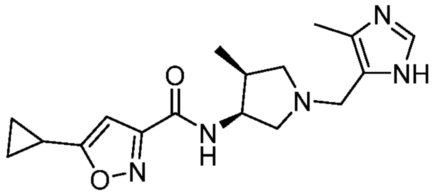 | None | (±)-*cis*-5-cyclopropyl-N-{4-methyl-1-[(4-methyl-1H-imidazol-5-yl)methyl]pyrrolidin-3-yl}-1,2-oxazole-3-carboxamide | 330.2 | 11.64992 |
| 321 | 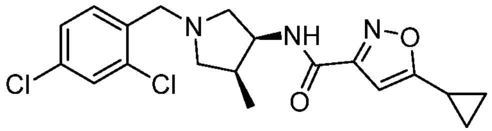 | None | (±)-*cis*-5-cyclopropyl-N-{ 1-[(2,4-dichlorophenyl)methyl]-4-methylpyrrolidin-3-yl }-1,2-oxazole-3-carboxamide | 394.1 | 4.63779 |
| 322 | 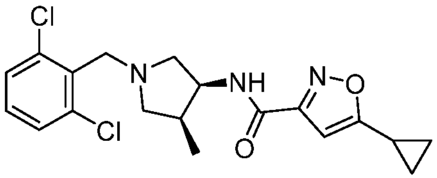 | None | (±)-*cis*-5-cyclopropyl-N-{ 1-[(2,6-dichlorophenyl)methyl]-4-methylpyrrolidin-3-yl}-1,2-oxazole-3-carboxamide | 394.1 | > 50.0 |
| 323 | 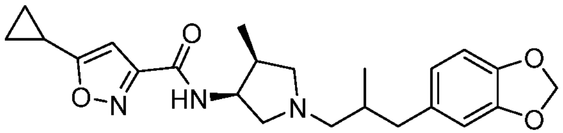 | None | *cis*-N-{1- [2-(2H-1,3-benzodioxol-5-ylmethyl)propyl]-4-methylpyrrolidin-3-yl }-5-cyclopropyl-1,2-oxazole-3-carboxamide | 412.2 | 9.31186 |

(continued)

| Cpd. No. | Structure | Salt Form | Chemical Name | LCMS M+H | SMYD2 Biochem $IC_{50}$ (μM)* |
|---|---|---|---|---|---|
| 324 | 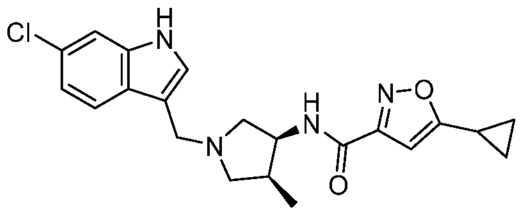 | None | (±)-*cis*-N-{1-[(6-chloro-1H-indol-3-yl)methyl]-4-methylpyrrolidin-3-yl}-5-cyclopropyl-1,2-oxazole-3-carboxamide | 399.2 | 9.33601 |
| 325 | 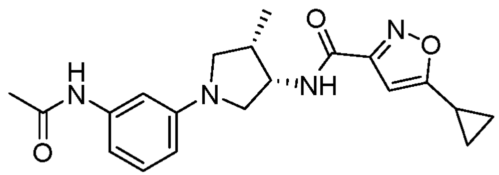 | None | (±)-*cis*-5-cyclopropyl-N-[1-(3-acetamidophenyl)-4-methylpyrrolidin-3-yl]-1,2-oxazole-3-carboxamide | 369.2 | > 50.0 |
| 326 | 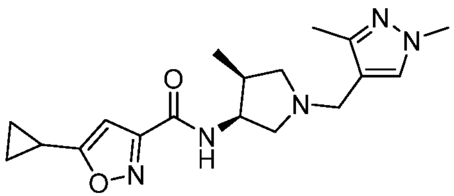 | None | (±)-*cis*-5-cyclopropyl-N-{1-[(1,3-dimethyl-1H-pyrazol-4-yl)methyl]-4-methylpyrrolidin-3-yl}-1,2-oxazole-3-carboxamide | 344.2 | 1.6713 |
| 327 | 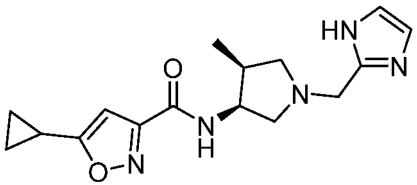 | None | (±)-*cis*-5-cyclopropyl-N-[1-(1H-imidazol-2-ylmethyl)-4-methylpyrrolidin-3-yl]-1,2-oxazole-3-carboxamide | 316.2 | 49.70017 |
| 328 | 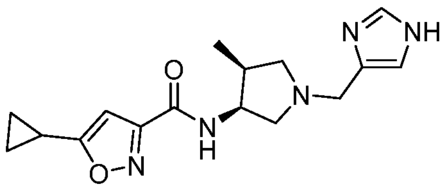 | None | (±)-*cis*-5-cyclopropyl-N-[1-(1H-imidazol-4-ylmethyl)-4-methylpyrrolidin-3-yl]-1,2-oxazole-3-carboxamide | 316.1 | 2.65204 |

(continued)

| Cpd. No. | Structure | Salt Form | Chemical Name | LCMS M+H | SMYD2 Biochem $IC_{50}$ (μM)* |
|---|---|---|---|---|---|
| 329 | 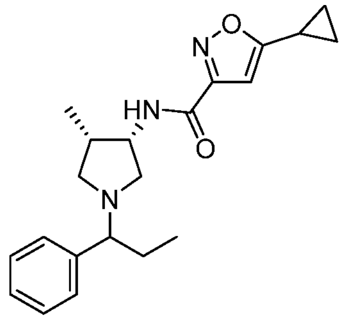 | None | *cis*-5-cyclopropyl-N-[4-methyl-1-(1-phenylpropyl)pyrrolidin-3-yl]-1,2-oxazole-3-carboxamide | 354.2 | 1.14896 |
| 330 | 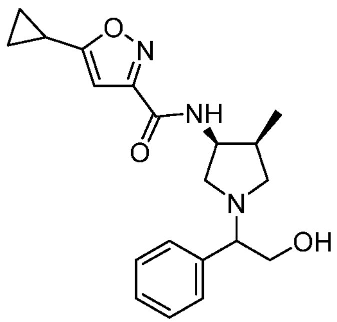 | None | *cis*-5-cyclopropyl-N-[1-(2-hydroxy-1-phenylethyl)-4-methylpyrrolidin-3-yl]-1,2-oxazole-3-carboxamide | 356.2 | 9.44083 |
| 331 | 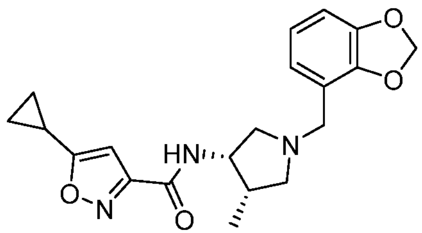 | None | (±)-*cis*-N-[1-(2H-1,3-benzodioxol-4-ylmethyl)-4-methylpyrrolidin-3-yl]-5-cyclopropyl-1,2-oxazole-3-carboxamide | 370.1 | 3.57432 |

(continued)

| Cpd. No. | Structure | Salt Form | Chemical Name | LCMS M+H | SMYD2 Biochem $IC_{50}$ (μM)* |
|---|---|---|---|---|---|
| 332 | 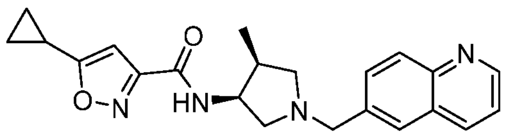 | None | (±)-*cis*-5-cyclopropyl-N-[4-methyl-1-(quinolin-6-ylmethyl) pyrrolidin-3-yl]-1,2-oxazole-3-carboxamide | 377.2 | 24.18657 |
| 333 | 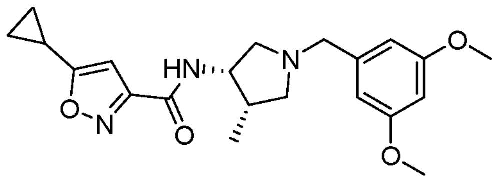 | None | (±)-*cis*-5-cyclopropyl-N-{ 1-[(3,5-dimethoxyphenyl)methyl]-4-methylpyrrolidin-3-yl}-1,2-oxazole-3-carboxamide | 386.2 | > 50.0 |
| 334 | 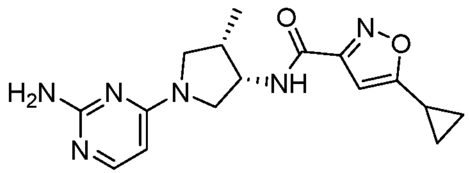 | None | (±)-*cis*-N-[1-(2-aminopyrimidin-4-yl)-4-methylpyrrolidin-3 -yl]-5-cyclopropyl-1,2-oxazole-3-carboxamide | 329.2 | > 50.0 |
| 335 | 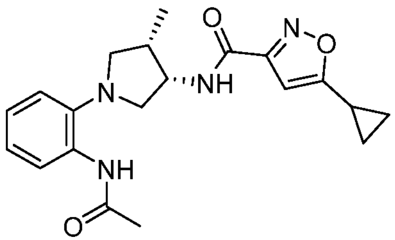 | None | (±)-*cis*-5-cyclopropyl-N-[1-(2-acetamidophenyl)-4-methylpyrrolidin-3-yl]-1,2-oxazole-3-carboxamide | 369.1 | > 50.0 |
| 336 | 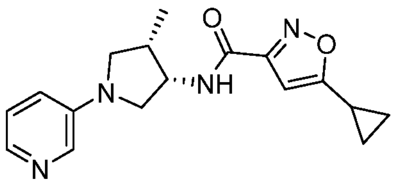 | None | (±)-*cis*-5-cyclopropyl-N-[4-methyl-1-(pyridin-3-yl)pyrrolidin-3-yl]-1,2-oxazole-3-carboxamide | 313.2 | > 50.0 |

(continued)

| Cpd. No. | Structure | Salt Form | Chemical Name | LCMS M+H | SMYD2 Biochem $IC_{50}$ (μM)* |
|---|---|---|---|---|---|
| 337 | 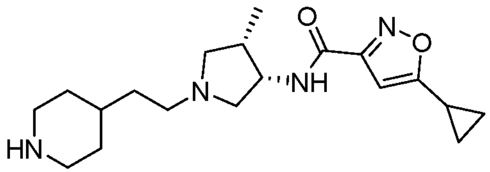 | None | (±)-*cis*-5-cyclopropyl-N-{4-methyl-1-[2-(piperidin-4-yl)ethyl] pyrrolidin-3-yl}-1,2-oxazole-3-carboxamide | 347.2 | 10.59618 |
| 338 | 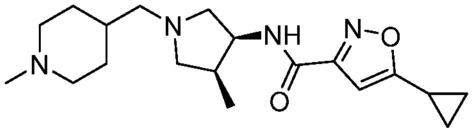 | None | (±)-*cis*-5-cyclopropyl-N-{4-methyl-1-[(1-methylpiperidin-4-yl) methyl]pyrrolidin-3-yl}-1,2-oxazole-3-carboxamide | 347.2 | > 50.0 |
| 339 | 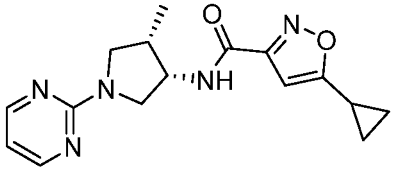 | None | (±)-*cis*-5-cyclopropyl-N-[4-methyl-1-(pyrimidin-2-yl)pyrrolidin-3-yl]-1,2-oxazole-3-carboxamide | 314.2 | > 50.0 |
| 340 | 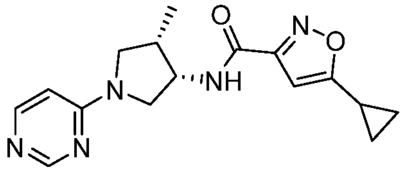 | None | (±)-*cis*-5-cyclopropyl-N-[4-methyl-1-(pyrimidin-4-yl)pyrrolidin-3-yl]-1,2-oxazole-3-carboxamide | 314.1 | > 50.0 |
| 341 | 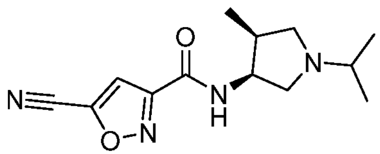 | None | (±)-*cis*-5-cyano-N-[4-m ethyl-1- (propan-2-yl)pyrrolidin-3-yl]-1,2-oxazole-3-carboxamide | 263.1 | 22.7502 |

(continued)

| Cpd. No. | Structure | Salt Form | Chemical Name | LCMS M+H | SMYD2 Biochem $IC_{50}$ (μM)* |
|---|---|---|---|---|---|
| 342 | 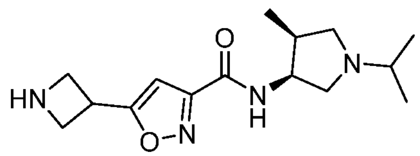 | None | (±)-*cis*-5-(azetidin-3-yl)-N-[4-methyl-1-(propan-2-yl)pyrrolidin-3-yl]-1,2-oxazole-3-carboxamide | 293.2 | > 50.0 |
| 343 | 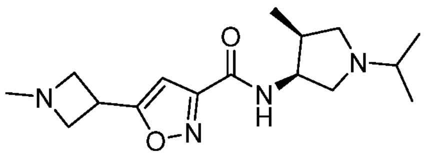 | None | (±)-*cis*-N-[4-methyl-1-(propan-2-yl)pyrrolidin-3-yl]-5-(1-methylazetidin-3-yl)-1,2-oxazole-3-carboxamide | 307.2 | > 50.0 |
| 344 | 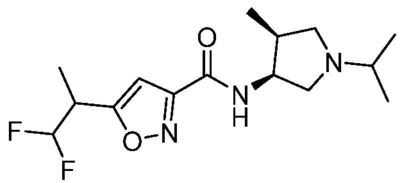 | None | *cis*-5-(1,1-difluoropropan-2-yl)-N-[4-methyl-1-(propan-2-yl) pyrrolidin-3-yl]-1,2-oxazole-3-carboxamide | 316.2 | 10.24088 |
| 345 | 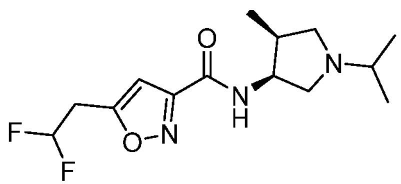 | None | (±)-*cis*-5-(2,2-difluoroethyl)-N-[4-methyl-1-(propan-2-yl) pyrrolidin-3-yl]-1,2-oxazole-3-carboxamide | 302.1 | 5.84333 |
| 346 | 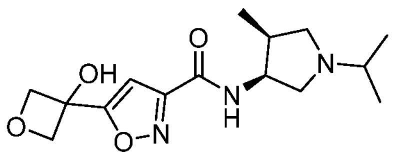 | None | (±)-*cis*-5-(3-hydroxyoxetan-3-yl)-N- [4-methyl-1-(propan-2-yl) pyrrolidin-3-yl]-1,2-oxazole-3-carboxamide | 310.2 | > 50.0 |

(continued)

| Cpd. No. | Structure | Salt Form | Chemical Name | LCMS M+H | SMYD2 Biochem $IC_{50}$ (μM)* |
|---|---|---|---|---|---|
| 347 | 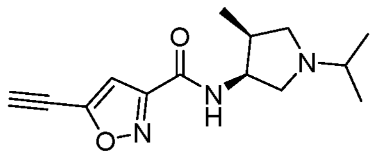 | None | (±)-*cis*-5-ethynyl-N-[4-methyl-1-(propan-2-yl)pyrrolidin-3-yl]-1,2-oxazole-3-carboxamide | 262.2 | |
| 348 | 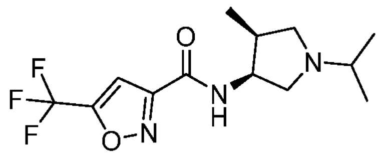 | None | (±)-*cis*-N-[4-methyl-1-(propan-2-yl)pyrrolidin-3-yl]-5-(trifluoromethyl)-1,2-oxazole-3-carboxamide | 306.1 | 6.67878 |
| 349 | 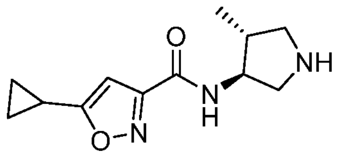 | None | (±)-*trans*-5-cyclopropyl-N-(4-methylpyrrolidin-3-yl)-1,2-oxazole-3-carboxamide | 236.2 | |
| 350 | 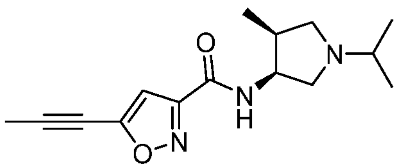 | None | (±)-*cis*-N-(1-isopropyl-4-methylpyrrolidin-3-yl)-5-(prop-1-yn-1-yl)isoxazole-3-carboxamide | | |
| 351 | 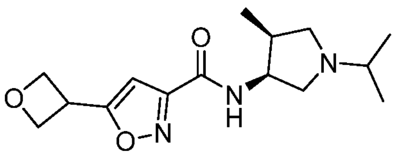 | None | (±)-*cis*-N-(1-isopropyl-4-methylpyrrolidin-3-yl)-5-(oxetan-3-yl) isoxazole-3-carboxamide | | |

(continued)

| Cpd. No. | Structure | Salt Form | Chemical Name | LCMS M+H | SMYD2 Biochem $IC_{50}$ (μM)* |
|---|---|---|---|---|---|
| 352 | 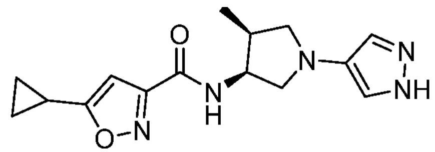 | None | (±)-*cis*-5-cyclopropyl-N-(4-methyl-1-(1 H-pyrazol-4-yl)pyrroli din-3 - yl)isoxazole-3-carboxamide | | |
| * $IC_{50}$ values are an average of n=1 to n=50 | | | | | |

In another embodiment, a Compound of the Disclosure is a compound having Formula I, provided that the compound is not:

| Structure | Name |
|---|---|
| 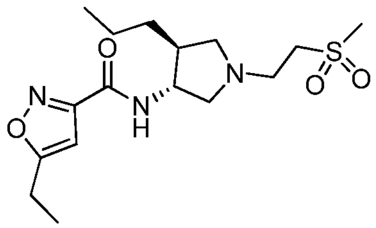 | (±)-*trans*-5-ethyl-N-(1-(2-(methylsulfonyl)ethyl)-4-propylpyrrolidin-3-yl) isoxazole-3-carboxamide |
| 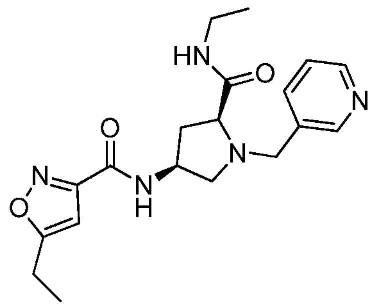 | 5-ethyl-N-((3S,5S)-5-(ethylcarbamoyl)-1-(pyridin-3-ylmethyl)pyrrolidin-3-yl)isoxazole-3-carboxamide |
| 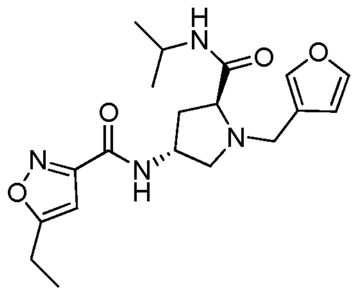 | 5-ethyl-N-((3R,5S)-1-(furan-3-ylmethyl)-5-(isopropylcarbamoyl)pyrrolidin-3-yl)isoxazole-3-carboxamide |
| 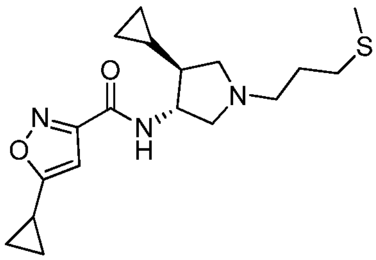 | (±)-*trans*-5-cyclopropyl-N-(4-cyclopropyl-1-(3-(methylthio)propyl) pyrrolidin-3-yl)isoxazole-3-carboxamide |
| 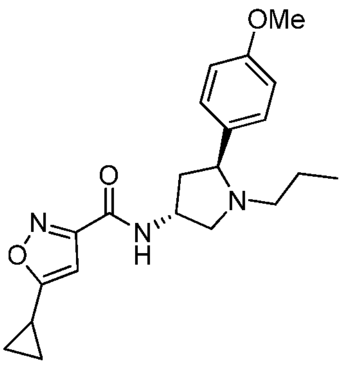 | (±)-*trans*-5-cyclopropyl-N-(5-(4-methoxyphenyl)-1-propylpyrrolidin-3-yl) isoxazole-3-carboxamide |

(continued)

| Structure | Name |
|---|---|
| 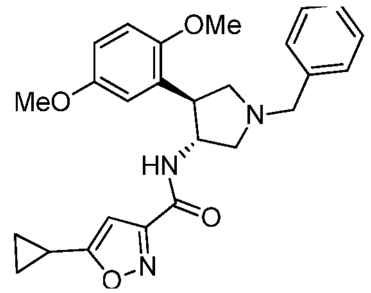 | (±)-*trans*-N-(1-benzyl-4-(2,5-dimethoxyphenyl)pyrrolidin-3-yl)-5-cyclopropylisoxazole-3-carboxamide |

In some embodiments, the disclosure relates to pharmaceutical compositions for use in treating cancer, comprising one or more of the following compounds:

| Structure | Name |
|---|---|
| 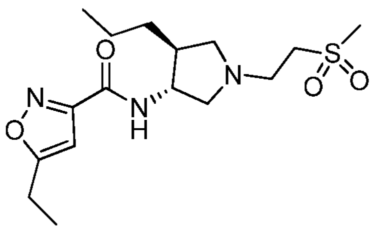 | (±)-*trans*-5-ethyl-N-(1-(2-(methylsulfonyl)ethyl)-4-propylpyrrolidin-3-yl) isoxazole-3-carboxamide |
| 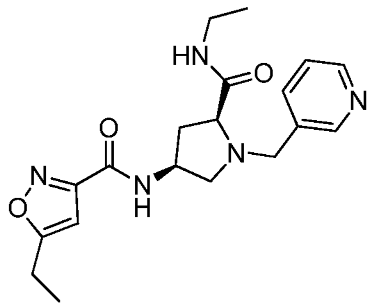 | 5-ethyl-N-((3 S,5 S)-5-(ethylcarbamoyl)-1-(pyridin-3-ylmethyl)pyrrolidin-3-yl)isoxazole-3-carboxamide |
| 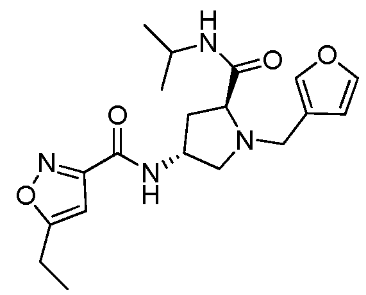 | 5-ethyl-N-((3R,5S)-1-(furan-3-ylmethyl)-5-(isopropylcarbamoyl)pyrrolidin-3-yl)isoxazole-3-carboxamide |
| 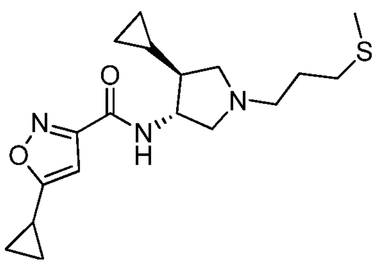 | (±)-*trans*-5-cyclopropyl-N-(4-cyclopropyl-1-(3-(methylthio)propyl) pyrrolidin-3-yl)isoxazole-3-carboxamide |

(continued)

| Structure | Name |
|---|---|
| 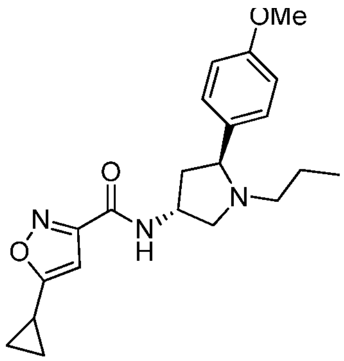 | (±)-*trans*-5-cyclopropyl-N-(5-(4-methoxyphenyl)-1-propylpyrrolidin-3-yl) isoxazole-3-carboxamide |
| 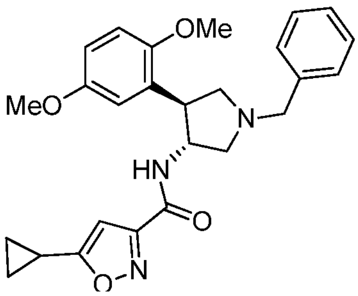 | (±)-*trans*-N-(1-benzyl-4-(2,5-dimethoxyphenyl)pyrrolidin-3-yl)-5-cyclopropylisoxazole-3-carboxamide |

and a pharmaceutically acceptable carier.

In some embodiments, the disclosure relates to a compound for use in treating cancer, wherein the compound is selected from:

| Structure | Name |
|---|---|
| 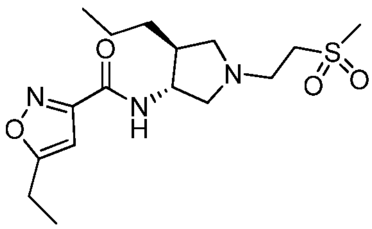 | (±)-*trans*-5-ethyl-N-(1-(2-(methylsulfonyl)ethyl)-4-propylpyrrolidin-3-yl) isoxazole-3-carboxamide |
| 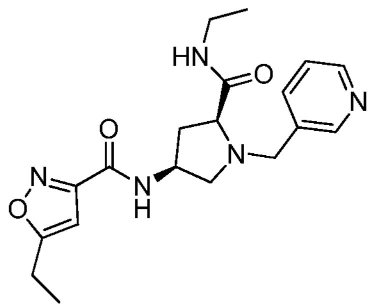 | 5-ethyl-N-((3S,5S)-5-(ethylcarbamoyl)-1-(pyridin-3-ylmethyl)pyrrolidin-3-yl) isoxazole-3-carboxamide |

(continued)

| Structure | Name |
|---|---|
| 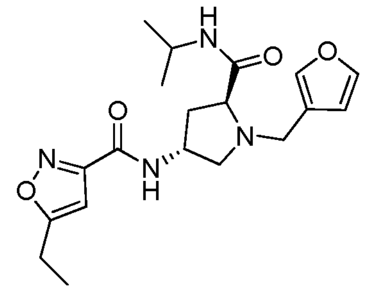 | 5-ethyl-N-((3R,5S)-1-(furan-3-ylmethyl)-5-(isopropylcarbamoyl)pyrrolidin-3-yl)isoxazole-3-carboxamide |
| 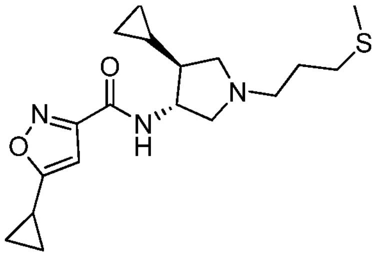 | (±)-*trans*-5-cyclopropyl-N-(4-cyclopropyl-1-(3-(methylthio)propyl) pyrrolidin-3-yl)isoxazole-3-carboxamide |
| 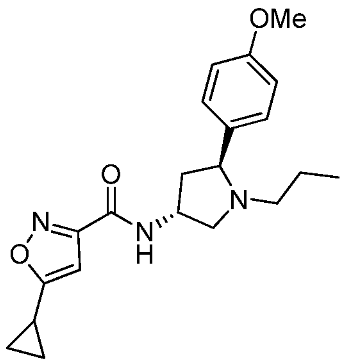 | (±)-*trans*-5-cyclopropyl-N-(5-(4-methoxyphenyl)-1-propylpyrrolidin-3-yl) isoxazole-3-carboxamide |
| 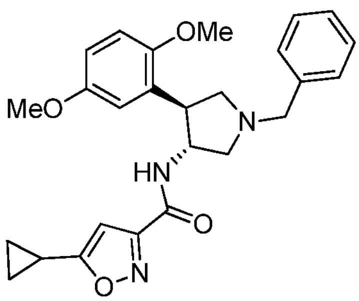 | (±)-*trans*-N-(1-benzyl-4-(2,5-dimethoxyphenyl)pyrrolidin-3-yl)-5-cyclopropylisoxazole-3-carboxamide |

Definitions

For the purpose of the present disclosure, the term "alkyl" as used by itself or as part of another group refers to a straight- or branched-chain aliphatic hydrocarbon containing one to twelve carbon atoms *(i.e.,* $C_{1-12}$ alkyl) or the number of carbon atoms designated *(i.e.,* a $C_1$ alkyl such as methyl, a $C_2$ alkyl such as ethyl, a $C_3$ alkyl such as propyl or isopropyl, etc.). In one embodiment, the alkyl group is chosen from a straight chain $C_{1-10}$ alkyl group. In another embodiment, the alkyl group is chosen from a branched chain $C_{3-10}$ alkyl group. In another embodiment, the alkyl group is chosen from a straight chain $C_{1-6}$ alkyl group. In another embodiment, the alkyl group is chosen from a branched chain $C_{3-6}$ alkyl group. In another embodiment, the alkyl group is chosen from a straight chain $C_{1-4}$ alkyl group. In another embodiment, the alkyl group is chosen from a branched chain $C_{3-4}$ alkyl group. In another embodiment, the alkyl group is chosen from a straight or branched chain $C_{3-4}$ alkyl group. In another embodiment, the alkyl group is partially or completely deuterated, *i.e.,* one or more hydrogen atoms of the alkyl group are replaced with deuterium atoms. Non-

limiting exemplary $C_{1-10}$ alkyl groups include methyl (including $-CD_3$), ethyl, propyl, isopropyl, butyl, *sec*-butyl, *tert*-butyl, *iso*-butyl, 3-pentyl, hexyl, heptyl, octyl, nonyl, and decyl. Non-limiting exemplary $C_{1-4}$ alkyl groups include methyl, ethyl, propyl, isopropyl, butyl, *sec*-butyl, *tert*-butyl, and *iso*-butyl.

For the purpose of the present disclosure, the term "optionally substituted alkyl" as used by itself or as part of another group means that the alkyl as defined above is either unsubstituted or substituted with one, two, or three substituents independently chosen from nitro, haloalkoxy, aryloxy, aralkyloxy, alkylthio, sulfonamido, alkylcarbonyl, arylcarbonyl, alkylsulfonyl, arylsulfonyl, ureido, guanidino, carboxy, alkoxycarbonyl, and carboxyalkyl. In one embodiment, the alkyl is a $C_{1-4}$ alkyl. In one embodiment, the optionally substituted alkyl is substituted with two substituents. In another embodiment, the optionally substituted alkyl is substituted with one substituent. Non-limiting exemplary optionally substituted alkyl groups include $-CH_2CH_2NO_2$, $-CH_2CH_2CO_2H$, $-CH_2CH_2SO_2CH_3$, $-CH_2CH_2COPh$, and $-CH_2C_6H_{11}$.

For the purpose of the present disclosure, the term "cycloalkyl" as used by itself or as part of another group refers to saturated and partially unsaturated (containing one or two double bonds) cyclic aliphatic hydrocarbons containing one to three rings having from three to twelve carbon atoms *(i.e.,* $C_{3-12}$ cycloalkyl) or the number of carbons designated. In one embodiment, the cycloalkyl group has two rings. In one embodiment, the cycloalkyl group has one ring. In another embodiment, the cycloalkyl group is chosen from a $C_{3-8}$ cycloalkyl group. In another embodiment, the cycloalkyl group is chosen from a $C_{3-6}$ cycloalkyl group. Non-limiting exemplary cycloalkyl groups include cyclopropyl, cyclobutyl, cyclopentyl, cyclohexyl, cycloheptyl, cyclooctyl, norbornyl, decalin, adamantyl, cyclohexenyl, and spiro[3.3]heptane.

For the purpose of the present disclosure, the term "optionally substituted cycloalkyl" as used by itself or as part of another group means that the cycloalkyl as defined above is either unsubstituted or substituted with one, two, or three substituents independently chosen from halo, nitro, cyano, hydroxy, amino, alkylamino, dialkylamino, haloalkyl, hydroxyalkyl, alkoxy, haloalkoxy, aryloxy, aralkyl, aralkyloxy, alkylthio, carboxamido, sulfonamido, alkoxycarbonyl, alkylcarbonyl, arylcarbonyl, alkylsulfonyl, arylsulfonyl, ureido, guanidino, carboxy, carboxyalkyl, alkyl, optionally substituted cycloalkyl, alkenyl, alkynyl, optionally substituted aryl, optionally substituted heteroaryl, optionally substituted heterocyclo, alkoxyalkyl, (amino)alkyl, hydroxyalkylamino, (alkylamino)alkyl, (dialkylamino)alkyl, (cyano)alkyl, (carboxamido)alkyl, mercaptoalkyl, (heterocyclo)alkyl, or (heteroaryl)alkyl. In another embodiment, the optionally substituted cycloalkyl is substituted with one, two, or three substituents independently chosen from halo, nitro, cyano, hydroxy, amino, alkylamino, dialkylamino, haloalkyl, hydroxyalkyl, alkoxy, haloalkoxy, aryloxy, aralkyl, aralkyloxy, alkylthio, carboxamido, sulfonamido, alkylcarbonyl, arylcarbonyl, alkylsulfonyl, arylsulfonyl, ureido, guanidino, carboxy, carboxyalkyl, alkyl, optionally substituted cycloalkyl, alkenyl, alkynyl, optionally substituted aryl, optionally substituted heteroaryl, optionally substituted heterocyclo, alkoxyalkyl, (amino)alkyl, hydroxyalkylamino, (alkylamino)alkyl, (dialkylamino)alkyl, (cyano)alkyl, (carboxamido)alkyl, mercaptoalkyl, (heterocyclo)alkyl, or (heteroaryl)alkyl. In one embodiment, the optionally substituted cycloalkyl is substituted with two substituents. In another embodiment, the optionally substituted cycloalkyl is substituted with one substituent. In one embodiment, the optionally substituted cycloalkyl is substituted with at least one amino, alkylamino, or dialkylamino group. Non-limiting exemplary optionally substituted cycloalkyl groups include:

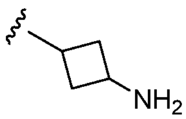 , 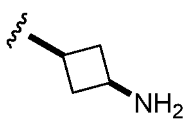 , 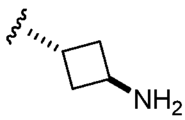 , 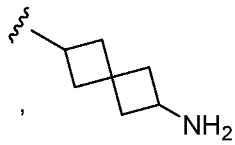 ,

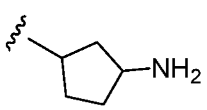 , 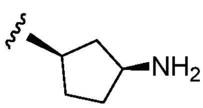 , 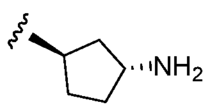 , 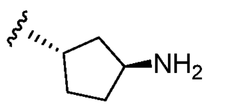 ,

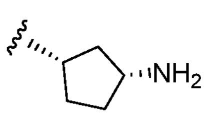 , 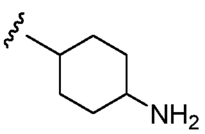 , 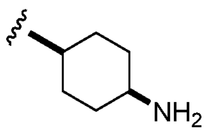 , 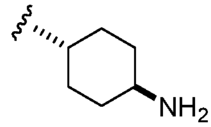 ,

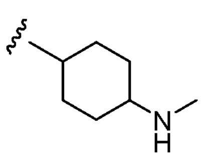 , 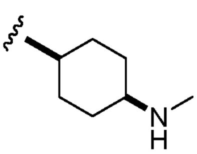 , 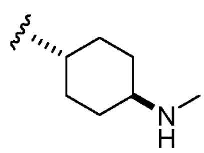 , 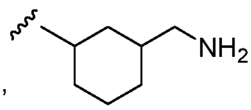 ,

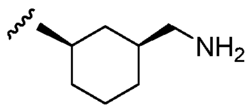, 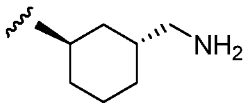, 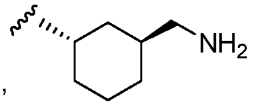, 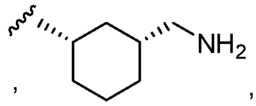,

and

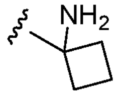

For the purpose of the present disclosure, the term "cycloalkenyl" as used by itself or part of another group refers to a partially unsaturated cycloalkyl group as defined above. In one embodiment, the cycloalkenyl has one carbon-to-carbon double bond. In another embodiment, the cycloalkenyl group is chosen from a $C_{4-8}$ cycloalkenyl group. Exemplary cycloalkenyl groups include cyclopentenyl and cyclohexenyl.

For the purpose of the present disclosure, the term "optionally substituted cycloalkenyl" as used by itself or as part of another group means that the cycloalkenyl as defined above is either unsubstituted or substituted with one, two, or three substituents independently chosen from halo, nitro, cyano, hydroxy, amino, alkylamino, dialkylamino, haloalkyl, monohydroxyalkyl, dihydroxyalkyl, alkoxy, haloalkoxy, aryloxy, aralkyloxy, alkylthio, carboxamido, sulfonamido, alkylcarbonyl, arylcarbonyl, alkylsulfonyl, arylsulfonyl, ureido, guanidino, carboxy, carboxyalkyl, alkyl, optionally substituted cycloalkyl, alkenyl, alkynyl, optionally substituted aryl, optionally substituted heteroaryl, optionally substituted heterocyclo, alkoxyalkyl, (amino)alkyl, hydroxyalkylamino, (alkylamino)alkyl, (dialkylamino)alkyl, (cyano)alkyl, (carboxamido)alkyl, mercaptoalkyl, (heterocyclo)alkyl, and (heteroaryl)alkyl. In one embodiment, the optionally substituted cycloalkenyl is substituted with two substituents. In another embodiment, the optionally substituted cycloalkenyl is substituted with one substituent. In another embodiment, the cycloalkenyl is unsubstituted.

For the purpose of the present disclosure, the term "alkenyl" as used by itself or as part of another group refers to an alkyl group as defined above containing one, two or three carbon-to-carbon double bonds. In one embodiment, the alkenyl group is chosen from a $C_{2-6}$ alkenyl group. In another embodiment, the alkenyl group is chosen from a $C_{2-4}$ alkenyl group. Non-limiting exemplary alkenyl groups include ethenyl, propenyl, isopropenyl, butenyl, *sec*-butenyl, pentenyl, and hexenyl.

For the purpose of the present disclosure, the term "optionally substituted alkenyl" as used herein by itself or as part of another group means the alkenyl as defined above is either unsubstituted or substituted with one, two or three substituents independently chosen from halo, nitro, cyano, hydroxy, amino, alkylamino, dialkylamino, haloalkyl, hydroxyalkyl, alkoxy, haloalkoxy, aryloxy, aralkyloxy, alkylthio, carboxamido, sulfonamido, alkylcarbonyl, arylcarbonyl, alkylsulfonyl, arylsulfonyl, ureido, guanidino, carboxy, carboxyalkyl, alkyl, optionally substituted cycloalkyl, alkenyl, alkynyl, optionally substituted aryl, optionally substituted heteroaryl, or optionally substituted heterocyclo.

For the purpose of the present disclosure, the term "alkynyl" as used by itself or as part of another group refers to an alkyl group as defined above containing one to three carbon-to-carbon triple bonds. In one embodiment, the alkynyl has one carbon-to-carbon triple bond. In one embodiment, the alkynyl group is chosen from a $C_{2-6}$ alkynyl group. In another embodiment, the alkynyl group is chosen from a $C_{2-4}$ alkynyl group. Non-limiting exemplary alkynyl groups include ethynyl, propynyl, butynyl, 2-butynyl, pentynyl, and hexynyl groups.

For the purpose of the present disclosure, the term "optionally substituted alkynyl" as used herein by itself or as part of another group means the alkynyl as defined above is either unsubstituted or substituted with one, two or three substituents independently chosen from halo, nitro, cyano, hydroxy, amino, alkylamino, dialkylamino, haloalkyl, hydroxyalkyl, alkoxy, haloalkoxy, aryloxy, aralkyloxy, alkylthio, carboxamido, sulfonamido, alkylcarbonyl, arylcarbonyl, alkylsulfonyl, arylsulfonyl, ureido, guanidino, carboxy, carboxyalkyl, alkyl, optionally substituted cycloalkyl, alkenyl, alkynyl, optionally substituted aryl, optionally substituted heteroaryl, or optionally substituted heterocyclo.

For the purpose of the present disclosure, the term "haloalkyl" as used by itself or as part of another group refers to an alkyl group substituted by one or more fluorine, chlorine, bromine and/or iodine atoms. In one embodiment, the alkyl group is substituted by one, two, or three fluorine and/or chlorine atoms. In another embodiment, the haloalkyl group is chosen from a $C_{1-4}$ haloalkyl group. Non-limiting exemplary haloalkyl groups include fluoromethyl, difluoromethyl, trifluoromethyl, pentafluoroethyl, 1,1-difluoroethyl, 2,2-difluoroethyl, 2,2,2-trifluoroethyl, 3,3,3-trifluoropropyl, 4,4,4-trifluorobutyl, and trichloromethyl groups.

For the purpose of the present disclosure, the term "hydroxyalkyl" as used by itself or as part of another group refers to an alkyl group substituted with one or more, e.g., one, two, or three, hydroxy groups. In one embodiment, the hydroxyalkyl group is a monohydroxyalkyl group, *i.e.,* substituted with one hydroxy group. In another embodiment, the hydroxyalkyl group is a dihydroxyalkyl group, *i.e.,* substituted with two hydroxy groups. In another embodiment, the

hydroxyalkyl group is chosen from a $C_{1-4}$ hydroxyalkyl group. Non-limiting exemplary hydroxyalkyl groups include hydroxymethyl, hydroxyethyl, hydroxypropyl and hydroxybutyl groups, such as 1-hydroxyethyl, 2-hydroxyethyl, 1,2-dihydroxyethyl, 2-hydroxypropyl, 3-hydroxypropyl, 3-hydroxybutyl, 4-hydroxybutyl, 2-hydroxy-1-methylpropyl, and 1,3-dihydroxyprop-2-yl.

For the purpose of the present disclosure, the term "alkoxy" as used by itself or as part of another group refers to an optionally substituted alkyl, optionally substituted cycloalkyl, optionally substituted alkenyl or optionally substituted alkynyl attached to a terminal oxygen atom. In one embodiment, the alkoxy group is chosen from a $C_{1-4}$ alkoxy group. In another embodiment, the alkoxy group is chosen from a $C_{1-4}$ alkyl attached to a terminal oxygen atom, e.g., methoxy, ethoxy, and *tert*-butoxy.

For the purpose of the present disclosure, the term "alkylthio" as used by itself or as part of another group refers to a sulfur atom substituted by an optionally substituted alkyl group. In one embodiment, the alkylthio group is chosen from a $C_{1-4}$ alkylthio group. Non-limiting exemplary alkylthio groups include -SCH3, and $-SCH_2CH_3$.

For the purpose of the present disclosure, the term "alkoxyalkyl" as used by itself or as part of another group refers to an alkyl group substituted with an alkoxy group. Non-limiting exemplary alkoxyalkyl groups include methoxymethyl, methoxyethyl, methoxypropyl, methoxybutyl, ethoxymethyl, ethoxyethyl, ethoxypropyl, ethoxybutyl, propoxymethyl, iso-propoxymethyl, propoxyethyl, propoxypropyl, butoxymethyl, tert-butoxymethyl, isobutoxymethyl, sec-butoxymethyl, and pentyloxymethyl.

For the purpose of the present disclosure, the term "haloalkoxy" as used by itself or as part of another group refers to a haloalkyl attached to a terminal oxygen atom. Non-limiting exemplary haloalkoxy groups include fluoromethoxy, difluoromethoxy, trifluoromethoxy, and 2,2,2-trifluoroethoxy.

For the purpose of the present disclosure, the term "heteroalkyl" as used by itself or part of another group refers to a stable straight or branched chain hydrocarbon radical containing 1 to 10 carbon atoms and at least two heteroatoms, which can be the same or different, selected from O, N, or S, wherein: 1) the nitrogen atom(s) and sulfur atom(s) can optionally be oxidized; and/or 2) the nitrogen atom(s) can optionally be quaternized. The heteroatoms can be placed at any interior position of the heteroalkyl group or at a position at which the heteroalkyl group is attached to the remainder of the molecule. In one embodiment, the heteroalkyl group contains two oxygen atoms. In one embodiment, the heteroalkyl contains one oxygen and one nitrogen atom. In one embodiment, the heteroalkyl contains two nitrogen atoms. Non-limiting exemplary heteroalkyl groups include $-CH_2OCH_2CH_2OCH_3$, $-OCH_2CH_2OCH_2CH_2OCH_3$, $-CH_2NHCH_2CH_20CH_2$, $-OCH_2CH_2NH_2$, -NHCH2CH2N(H)CH3, $-NHCH_2CH_2OCH_3$ and $-OCH_2CH_2OCH_3$.

For the purpose of the present disclosure, the term "aryl" as used by itself or as part of another group refers to a monocyclic or bicyclic aromatic ring system having from six to fourteen carbon atoms (*i.e.,* $C_{6-14}$ aryl). Non-limiting exemplary aryl groups include phenyl (abbreviated as "Ph"), naphthyl, phenanthryl, anthracyl, indenyl, azulenyl, biphenyl, biphenylenyl, and fluorenyl groups. In one embodiment, the aryl group is chosen from phenyl or naphthyl. In one embodiment, the aryl group is phenyl.

For the purpose of the present disclosure, the term "optionally substituted aryl" as used herein by itself or as part of another group means that the aryl as defined above is either unsubstituted or substituted with one to five substituents independently selected from the group consisting of halo, nitro, cyano, hydroxy, amino, alkylamino, dialkylamino, haloalkyl, hydroxyalkyl, alkoxy, haloalkoxy, aryloxy, heteroaryloxy, aralkyl, aralkyloxy, alkylthio, carboxamido, sulfonamido, alkylcarbonyl, arylcarbonyl, alkylsulfonyl, arylsulfonyl, ureido, guanidino, carboxy, carboxyalkyl, alkyl, optionally substituted cycloalkyl, alkenyl, alkynyl, optionally substituted aryl, optionally substituted heteroaryl, optionally substituted heterocyclo, alkoxyalkyl, (amino)alkyl, hydroxyalkylamino, (alkylamino)alkyl, (dialkylamino)alkyl, (cyano)alkyl, (carboxamido)alkyl, mercaptoalkyl, (heterocyclo)alkyl, (cycloalkylamino)alkyl, ($C_{1-4}$ haloalkoxy)alkyl, (heteroaryl)alkyl, $-N(R^{43})(R^{44})$, and $-N(H)C(=O)-R^{45}$, wherein $R^{43}$ is hydrogen or $C_{1-4}$ alkyl; $R^{44}$ is alkoxyalkyl, (heterocyclo)alkyl, (amino)alkyl, (alkylamino)alkyl, or (dialkylamino)alkyl; and $R^{45}$ is alkyl, optionally substituted aryl or optionally substituted heteroaryl. In one embodiment, the optionally substituted aryl is an optionally substituted phenyl. In one embodiment, the optionally substituted phenyl has four substituents. In another embodiment, the optionally substituted phenyl has three substituents. In another embodiment, the optionally substituted phenyl has two substituents. In another embodiment, the optionally substituted phenyl has one substituent. In another embodiment, the optionally substituted phenyl has one amino, alkylamino, dialkylamino, (amino)alkyl, (alkylamino)alkyl, or (dialkylamino)alkyl substituent. Non-limiting exemplary substituted aryl groups include 2-methylphenyl, 2-methoxyphenyl, 2-fluorophenyl, 2-chlorophenyl, 2-bromophenyl, 3-methylphenyl, 3-methoxyphenyl, 3-fluorophenyl, 3-chlorophenyl, 4-methylphenyl, 4-ethylphenyl, 4-methoxyphenyl, 4-fluorophenyl, 4-chlorophenyl, 2,6-di-fluorophenyl, 2,6-dichlorophenyl, 2-methyl, 3-methoxyphenyl, 2-ethyl, 3-methoxyphenyl, 3,4-dimethoxyphenyl, 3,5-di-fluorophenyl 3,5-di-methylphenyl, 3,5-dimethoxy, 4-methylphenyl, 2-fluoro-3-chlorophenyl, 3-chloro-4-fluorophenyl, and 2-phenylpropan-2-amine. The term optionally substituted aryl is meant to include groups having fused optionally substituted cycloalkyl and fused optionally substituted heterocyclo rings. Examples include:

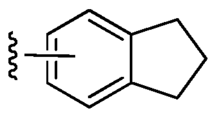 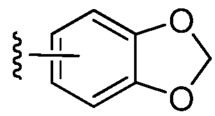 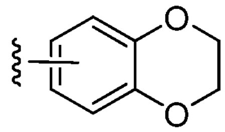 .

For the purpose of the present disclosure, the term "aryloxy" as used by itself or as part of another group refers to an optionally substituted aryl attached to a terminal oxygen atom. A non-limiting exemplary aryloxy group is PhO-.

For the purpose of the present disclosure, the term "heteroaryloxy" as used by itself or as part of another group refers to an optionally substituted heteroaryl attached to a terminal oxygen atom.

For the purpose of the present disclosure, the term "aralkyloxy" or "arylalkyloxy" as used by itself or as part of another group refers to an aralkyl group attached to a terminal oxygen atom. A non-limiting exemplary aralkyloxy group is $PhCH_2O$-.

For the purpose of the present disclosure, the term "heteroaryl" or "heteroaromatic" refers to monocyclic and bicyclic aromatic ring systems having 5 to 14 ring atoms *(i.e.,* a 5- to 14-membered heteroaryl) and 1, 2, 3, or 4 heteroatoms independently chosen from oxygen, nitrogen or sulfur. In one embodiment, the heteroaryl has three heteroatoms. In another embodiment, the heteroaryl has two heteroatoms. In another embodiment, the heteroaryl has one heteroatom. In one embodiment, the heteroaryl has 5 ring atoms, e.g., thienyl, i.e., four carbon atoms and one sulfur atom. In another embodiment, the heteroaryl has 6 ring atoms, e.g., pyridyl, i.e., five carbon atoms and one nitrogen atom. Non-limiting exemplary heteroaryl groups include thienyl, benzo[b]thienyl, naphtho[2,3-b]thienyl, thianthrenyl, furyl, benzofuryl, pyranyl, isobenzofuranyl, benzooxazonyl, chromenyl, xanthenyl, 2*H*-pyrrolyl, pyrrolyl, imidazolyl, pyrazolyl, pyridyl, pyrazinyl, pyrimidinyl, pyridazinyl, isoindolyl, 3*H*-indolyl, indolyl, indazolyl, purinyl, isoquinolyl, quinolyl, phthalazinyl, naphthyridinyl, cinnolinyl, quinazolinyl, pteridinyl, 4a*H*-carbazolyl, carbazolyl, β-carbolinyl, phenanthridinyl, acridinyl, pyrimidinyl, phenanthrolinyl, phenazinyl, thiazolyl, isothiazolyl, phenothiazolyl, isoxazolyl, furazanyl, and phenoxazinyl. In one embodiment, the heteroaryl is chosen from thienyl (*e.g.*, thien-2-yl and thien-3-yl), furyl *(e.g.,* 2-furyl and 3-furyl), pyrrolyl *(e.g.,* 1H-pyrrol-2-yl and 1H-pyrrol-3-yl), imidazolyl *(e.g.,* 2H-imidazol-2-yl and 2H-imidazol-4-yl), pyrazolyl *(e.g.,* 1H-pyrazol-3-yl, 1H-pyrazol-4-yl, and 1H-pyrazol-5-yl), pyridyl (*e.g.*, pyridin-2-yl, pyridin-3-yl, and pyridin-4-yl), pyrimidinyl (*e.g.*, pyrimidin-2-yl, pyrimidin-4-yl, and pyrimidin-5-yl), thiazolyl *(e.g.,* thiazol-2-yl, thiazol-4-yl, and thiazol-5-yl), isothiazolyl *(e.g.,* isothiazol-3-yl, isothiazol-4-yl, and isothiazol-5-yl), oxazolyl (*e.g.*, oxazol-2-yl, oxazol-4-yl, and oxazol-5-yl) and isoxazolyl (*e.g.*, isoxazol-3-yl, isoxazol-4-yl, and isoxazol-5-yl). The term "heteroaryl" is also meant to include possible N-oxides. Exemplary N-oxides include pyridyl N-oxide.

For the purpose of the present disclosure, the term "optionally substituted heteroaryl" as used by itself or as part of another group means that the heteroaryl as defined above is either unsubstituted or substituted with one to four substituents, e.g., one or two substituents, independently chosen from halo, nitro, cyano, hydroxy, amino, alkylamino, dialkylamino, haloalkyl, hydroxyalkyl, alkoxy, haloalkoxy, aralkyl aryloxy, aralkyloxy, alkylthio, carboxamido, sulfonamido, alkylcarbonyl, arylcarbonyl, alkylsulfonyl, arylsulfonyl, ureido, guanidino, carboxy, carboxyalkyl, alkyl, optionally substituted cycloalkyl, alkenyl, alkynyl, optionally substituted aryl, optionally substituted heteroaryl, optionally substituted heterocyclo, alkoxyalkyl, (amino)alkyl, hydroxyalkylamino, (alkylamino)alkyl, (dialkylamino)alkyl, (cyano)alkyl, (carboxamido)alkyl, mercaptoalkyl, (heterocyclo)alkyl, (heteroaryl)alkyl, -N($R^{43}$)($R^{44}$), or -N(H)C(=O)-$R^{45}$, wherein $R^{43}$ is hydrogen or $C_{1-4}$ alkyl; $R^{44}$ is alkoxyalkyl, (heterocyclo)alkyl, (amino)alkyl, (alkylamino)alkyl, or (dialkylamino)alkyl; and $R^{45}$ is alkyl, optionally substituted aryl, or optionally substituted heteroaryl. In one embodiment, the optionally substituted heteroaryl has one substituent. In one embodiment, the substituent is amino, alkylamino, dialkylamino, (amino)alkyl, hydroxyalkylamino, (alkylamino)alkyl, (dialkylamino)alkyl, (heterocyclo)alkyl, -N($R^{43}$)($R^{44}$), or -N(H)C(=O)-$R^{45}$. In one embodiment, the optionally substituted is an optionally substituted pyridyl, *i.e.,* 2-, 3-, or 4-pyridyl. Any available carbon or nitrogen atom can be substituted.

For the purpose of the present disclosure, the term "heterocycle" or "heterocyclo" as used by itself or as part of another group refers to saturated and partially unsaturated (*e.g.*, containing one or two double bonds) cyclic groups containing one, two, or three rings having from three to fourteen ring members *(i.e.,* a 3- to 14-membered heterocyclo) and at least one heteroatom. Each heteroatom is independently selected from the group consisting of oxygen, sulfur, including sulfoxide and sulfone, and/or nitrogen atoms, which can be quaternized. The term "heterocyclo" is meant to include cyclic ureido groups such as imidazolidinyl-2-one, cyclic amide groups such as β-lactam, γ-lactam, δ-lactam and ε-lactam, and cyclic carbamate groups such as oxazolidinyl-2-one. The term "heterocyclo" is also meant to include groups having fused optionally substituted aryl groups, e.g., indolinyl, indolinyl-2-one, benzo[d]oxazolyl-2(3H)-one. In one embodiment, the heterocyclo group is chosen from a 4-, 5-, 6-, 7- or 8-membered cyclic group containing one ring and one or two oxygen and/or nitrogen atoms. In one embodiment, the heterocyclo group is chosen from a 5- or 6-membered cyclic group containing one ring and one or two nitrogen atoms. In one embodiment, the heterocyclo group is chosen from a 8-, 9-, 10-, 11-, or 12-membered cyclic group containing two rings and one or two nitrogen atoms. The heterocyclo can be optionally linked to the rest of the molecule through a carbon or nitrogen atom. Non-limiting exemplary

heterocyclo groups include 2-oxopyrrolidin-3-yl, 2-imidazolidinone, piperidinyl, morpholinyl, piperazinyl, pyrrolidinyl, 8-azabicyclo[3.2.1]octane (nortropane), 6-azaspiro[2.5]octane, 6-azaspiro[3.4]octane, indolinyl, indolinyl-2-one, 1,3-dihydro-2H-benzo[d]imidazol-2-one

For the purpose of the present disclosure, the term "optionally substituted heterocyclo" as used herein by itself or part of another group means the heterocyclo as defined above is either unsubstituted or substituted with one to four substituents independently selected from halo, nitro, cyano, hydroxy, amino, alkylamino, dialkylamino, haloalkyl, hydroxyalkyl, alkoxy, haloalkoxy, aryloxy, aralkyl aralkyloxy, alkylthio, carboxamido, sulfonamido, alkoxycarbonyl, alkylcarbonyl, arylcarbonyl, alkylsulfonyl, arylsulfonyl, ureido, guanidino, carboxy, carboxyalkyl, alkyl, optionally substituted cycloalkyl, alkenyl, alkynyl, optionally substituted aryl, optionally substituted heteroaryl, optionally substituted heterocyclo, alkoxyalkyl, (amino)alkyl, hydroxyalkylamino, (alkylamino)alkyl, (dialkylamino)alkyl, (cyano)alkyl, (carboxamido)alkyl, mercaptoalkyl, (heterocyclo)alkyl, and (heteroaryl)alkyl. In another embodiment, the optionally substituted heterocyclo is substituted with one to four substituents independently selected from halo, nitro, cyano, hydroxy, amino, alkylamino, dialkylamino, haloalkyl, hydroxyalkyl, alkoxy, haloalkoxy, aryloxy, aralkyl aralkyloxy, alkylthio, carboxamido, sulfonamido, alkylcarbonyl, arylcarbonyl, alkylsulfonyl, arylsulfonyl, ureido, guanidino, carboxy, carboxyalkyl, alkyl, cycloalkyl, alkenyl, alkynyl, aryl, heteroaryl, heterocyclo, alkoxyalkyl, (amino)alkyl, hydroxyalkylamino, (alkylamino)alkyl, (dialkylamino)alkyl, (cyano)alkyl, (carboxamido)alkyl, mercaptoalkyl, (heterocyclo)alkyl, and (heteroaryl)alkyl. Substitution may occur on any available carbon or nitrogen atom, and may form a spirocycle. In one embodiment, the optionally substituted heterocyclo is substituted with at least one amino, alkylamino, or dialkylamino group. Non-limiting exemplary optionally substituted heterocyclo groups include:

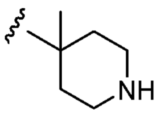, 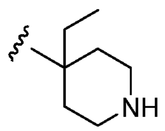, 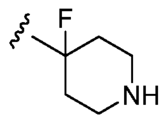, 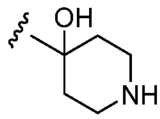,

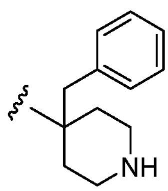, 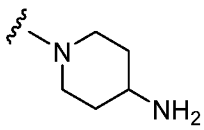, 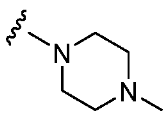, 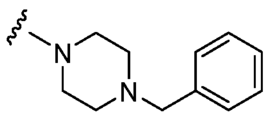,

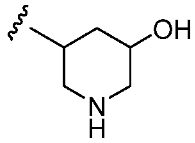, 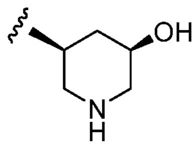, 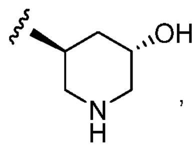, 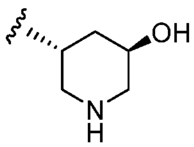,

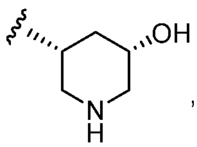, 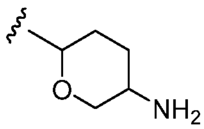, 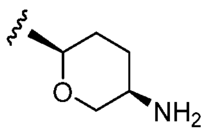, 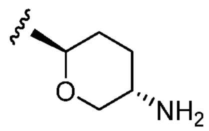

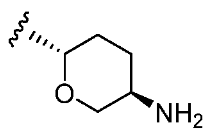 and 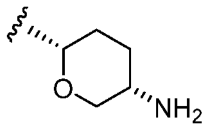

For the purpose of the present disclosure, the term "amino" as used by itself or as part of another group refers to $-NH_2$.

For the purpose of the present disclosure, the term "alkylamino" as used by itself or as part of another group refers to $-NHR^{22}$, wherein $R^{22}$ is $C_{1-6}$ alkyl. In one embodiment, $R^{22}$ is $C_{1-4}$ alkyl. Non-limiting exemplary alkylamino groups include - $N(H)CH_3$ and $-N(H)CH_2CH_3$.

For the purpose of the present disclosure, the term "dialkylamino" as used by itself or as part of another group

refers to $-NR^{23a}R^{23b}$, wherein $R^{23a}$ and $R^{23b}$ are each independently $C_{1-6}$ alkyl. In one embodiment, $R^{23a}$ and $R^{23b}$ are each independently $C_{1-4}$ alkyl. Non-limiting exemplary dialkylamino groups include $-N(CH_3)_2$ and $-N(CH_3)CH_2CH(CH_3)_2$.

For the purpose of the present disclosure, the term "hydroxyalkylamino" as used by itself or as part of another group refers to $-NHR^{24}$, wherein $R^{24}$ is hydroxyalkyl.

For the purpose of the present disclosure, the term "cycloalkylamino" as used by itself or as part of another group refers to $-NR^{25a}R^{25b}$, wherein $R^{25a}$ is optionally substituted cycloalkyl and $R^{25b}$ is hydrogen or $C_{1-4}$ alkyl.

For the purpose of the present disclosure, the term "aralkylamino" as used by itself or as part of another group refers to $-NR^{26a}R^{26b}$, wherein $R^{26a}$ is aralkyl and $R^{26b}$ is hydrogen or $C_{1-4}$ alkyl. Non-limiting exemplary aralkylamino groups include $-N(H)CH_2Ph$ and $-N(CH_3)CH_2Ph$.

For the purpose of the present disclosure, the term "(amino)alkyl" as used by itself or as part of another group refers to an alkyl group substituted with an amino group. In one embodiment, the alkyl is a $C_{1-4}$ alkyl. Non-limiting exemplary (amino)alkyl groups include $-CH_2NH_2$, $-C(NH_2)(H)CH_3$, $-CH_2CH_2NH_2$, $-CH_2C(NH_2)(H)CH_3$, $-CH_2CH_2CH_2NH_2$, $-CH_2CH_2CH_2CH_2NH_2$, and $-CH_2C(CH_3)_2CH_2NH_2$

For the purpose of the present disclosure, the term "(alkylamino)alkyl" as used by itself or as part of another group refers to an alkyl group substituted with an alkylamino group. In one embodiment, the alkyl is a $C_{1-4}$ alkyl. A non-limiting exemplary (alkylamino)alkyl group is -CH2CH2N(H)CH3.

For the purpose of the present disclosure, the term "(dialkylamino)alkyl" as used by itself or as part of another group refers to an alkyl group substituted by a dialkylamino group. In one embodiment, the alkyl is a $C_{1-4}$ alkyl. Non-limiting exemplary (dialkylamino)alkyl groups are $-CH_2CH_2N(CH_3)_2$.

For the purpose of the present disclosure, the term "(cycloalkylamino)alkyl" as used by itself or as part of another group refers to an alkyl group substituted by a cycloalkylamino group. In one embodiment, the alkyl is a $C_{1-4}$ alkyl. Non-limiting exemplary (cycloalkylamino)alkyl groups include $-CH_2N(H)$cyclopropyl, $-CH_2N(H)$cyclobutyl, and $-CH_2N(H)$cyclohexyl.

For the purpose of the present disclosure, the term "(aralkylamino)alkyl" as used by itself or as part of another group refers to an alkyl group substituted with an aralkylamino group. In one embodiment, the alkyl is a $C_{1-4}$ alkyl. A non-limiting exemplary (aralkylamino)alkyl group is $-CH_2CH_2CH_2N(H)CH_2Ph$

For the purpose of the present disclosure, the term "(cyano)alkyl" as used by itself or as part of another group refers to an alkyl group substituted with one or more cyano, *e.g.,* -CN, groups. In one embodiment, the alkyl is a $C_{1-4}$ alkyl. Non-limiting exemplary (cyano)alkyl groups include $-CH_2CH_2CN$, $-CH_2CH_2CH_2CN$, and -CH2CH2CH2CH2CN.

For the purpose of the present disclosure, the term "(amino)(hydroxy)alkyl" as used by itself or as part of another group refers to an alkyl group substituted with one amino, alkylamino, or dialkylamino group and one hydroxy group. In one embodiment, the alkyl is a $C_{1-6}$ alkyl. In another embodiment, the alkyl is a $C_{1-4}$ alkyl. Non-limiting exemplary (amino)(hydroxy)alkyl groups include:

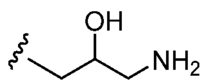 , 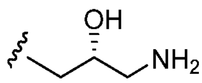 , 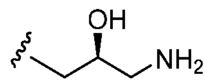

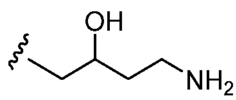 , 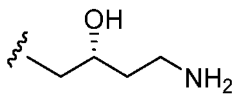 and 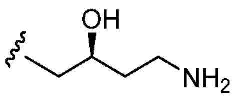 .

For the purpose of the present disclosure, the term "(amino)(aryl)alkyl" as used by itself or as part of another group refers to an alkyl group substituted with one amino, alkylamino, or dialkylamino group and one optionally substituted aryl group. In one embodiment, the alkyl is a $C_{1-6}$ alkyl. In one embodiment, the optionally substituted aryl group is an optionally substituted phenyl. Non-limiting exemplary (amino)(aryl)alkyl groups include:

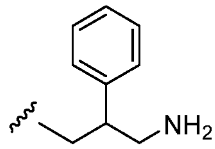 , 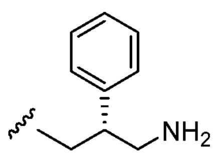 , 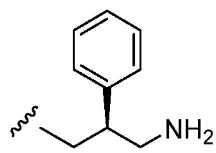

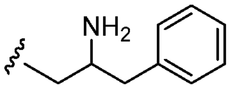, 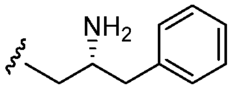 and 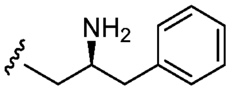.

For the purpose of the present disclosure, the term "(cycloalkyl)alkyl" as used by itself or as part of another group refers to an alkyl group substituted with one optionally substituted cycloalkyl group. In one embodiment, the alkyl is a $C_{1-4}$ alkyl. In one embodiment, the cycloalkyl is a $C_{3-6}$ cycloalkyl. In one embodiment, the optionally substituted cycloalkyl group is substituted with an amino or (amino)alkyl group. Non-limiting exemplary (cycloalkyl)alkyl groups include:

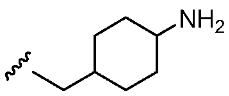, 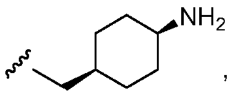, 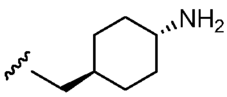, 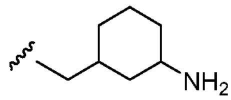,

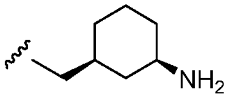, 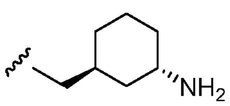, 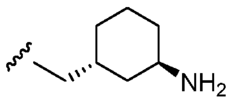, 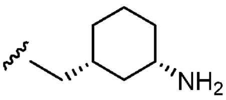,

and

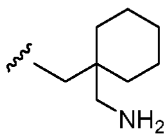

For the purpose of the present disclosure, the term "(hydroxy)(aryl)alkyl" as used by itself or as part of another group refers to an alkyl group substituted with one hydroxy group and one optionally substituted aryl group. In one embodiment, the alkyl is a $C_{1-6}$ alkyl. In one embodiment, the optionally substituted aryl group is an optionally substituted phenyl. Non-limiting exemplary (hydroxy)(aryl)alkyl groups include:

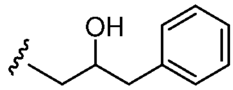, 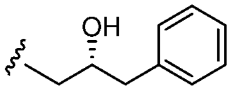 and 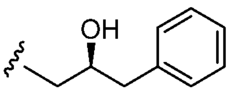.

For the purpose of the present disclosure, the term "carboxamido" as used by itself or as part of another group refers to a radical of formula $-C(=O)NR^{26a}R^{26b}$, wherein $R^{26a}$ and $R^{26b}$ are each independently hydrogen, optionally substituted alkyl, optionally substituted aryl, or optionally substituted heteroaryl, or $R^{26a}$ and $R^{26b}$ taken together with the nitrogen to which they are attached from a 3- to 8-membered heterocyclo group. In one embodiment, $R^{26a}$ and $R^{26b}$ are each independently hydrogen or optionally substituted alkyl. Non-limiting exemplary carboxamido groups include $-CONH_2$, $-CON(H)CH_3$, $CON(CH_3)_2$, and CON(H)Ph.

For the purpose of the present disclosure, the term "(carboxamido)alkyl" as used by itself or as part of another group refers to an alkyl group substituted with a carboxamido group. Non-limiting exemplary (carboxamido)alkyl groups include $-CH_2CONH_2$, $-C(H)CH_3-CONH_2$, and $-CH_2CON(H)CH_3$.

For the purpose of the present disclosure, the term "sulfonamido" as used by itself or as part of another group refers to a radical of the formula $-SO_2NR^{27a}R^{27b}$, wherein $R^{27a}$ and $R^{27b}$ are each independently hydrogen, optionally substituted alkyl, or optionally substituted aryl, or $R^{27a}$ and $R^{27b}$ taken together with the nitrogen to which they are attached from a 3- to 8-membered heterocyclo group. Non-limiting exemplary sulfonamido groups include $-SO_2NH_2$, $-SO_2N(H)CH_3$, and $-SO_2N(H)Ph$.

For the purpose of the present disclosure, the term "alkylcarbonyl" as used by itself or as part of another group refers to a carbonyl group, *i.e.,* -C(=O)-, substituted by an alkyl group. A non-limiting exemplary alkylcarbonyl group is $-COCH_3$.

For the purpose of the present disclosure, the term "arylcarbonyl" as used by itself or as part of another group refers to a carbonyl group, *i.e.,* -C(=O)-, substituted by an optionally substituted aryl group. A non-limiting exemplary

arylcarbonyl group is -COPh.

For the purpose of the present disclosure, the term "alkylsulfonyl" as used by itself or as part of another group refers to a sulfonyl group, *i.e.,* $-SO_2-$, substituted by any of the above-mentioned optionally substituted alkyl groups. A non-limiting exemplary alkylsulfonyl group is $-SO_2CH_3$.

For the purpose of the present disclosure, the term "arylsulfonyl" as used by itself or as part of another group refers to a sulfonyl group, *i.e.,* $-SO_2-$, substituted by any of the above-mentioned optionally substituted aryl groups. A non-limiting exemplary arylsulfonyl group is $-SO_2Ph$.

For the purpose of the present disclosure, the term "mercaptoalkyl" as used by itself or as part of another group refers to any of the above-mentioned alkyl groups substituted by a -SH group.

For the purpose of the present disclosure, the term "carboxy" as used by itself or as part of another group refers to a radical of the formula -COOH.

For the purpose of the present disclosure, the term "carboxyalkyl" as used by itself or as part of another group refers to any of the above-mentioned alkyl groups substituted with a -COOH. A non-limiting exemplary carboxyalkyl group is $-CH_2CO_2H$.

For the purpose of the present disclosure, the term "alkoxycarbonyl" as used by itself or as part of another group refers to a carbonyl group, *i.e.,* -C(=O)-, substituted by an alkoxy group. Non-limiting exemplary alkoxycarbonyl groups are $-CO_2Me$ and $-CO_2Et$.

For the purpose of the present disclosure, the term "aralkyl" or "arylalkyl" as used by itself or as part of another group refers to an alkyl group substituted with one, two, or three optionally substituted aryl groups. In one embodiment, the aralkyl group is a $C_{1-4}$ alkyl substituted with one optionally substituted aryl group. Non-limiting exemplary aralkyl groups include benzyl, phenethyl, $-CHPh_2$, $-CH_2(4\text{-OH-Ph})$, and $-CH(4\text{-F-Ph})_2$.

For the purpose of the present disclosure, the term "ureido" as used by itself or as part of another group refers to a radical of the formula $-NR^{30a}-C(=O)-NR^{30b}R^{30c}$, wherein $R^{22a}$ is hydrogen, alkyl, or optionally substituted aryl, and $R^{30b}$ and $R^{30c}$ are each independently hydrogen, alkyl, or optionally substituted aryl, or $R^{30b}$ and $R^{30c}$ taken together with the nitrogen to which they are attached form a 4- to 8-membered heterocyclo group. Non-limiting exemplary ureido groups include $-NH-C(C=O)-NH_2$ and $-NH-C(C=O)-NHCH_3$.

For the purpose of the present disclosure, the term "guanidino" as used by itself or as part of another group refers to a radical of the formula $-NR^{28a}-C(=NR^{29})-NR^{28b}R^{28c}$, wherein $R^{28a}$, $R^{28b}$, and $R^{28c}$ are each independently hydrogen, alkyl, or optionally substituted aryl, and $R^{29}$ is hydrogen, alkyl, cyano, alkylsulfonyl, alkylcarbonyl, carboxamido, or sulfonamido. Non-limiting exemplary guanidino groups include $-NH-C(C=NH)-NH_2$, $-NH-C(C=NCN)-NH_2$, and $-NH-C(C=NH)-NHCH_3$.

For the purpose of the present disclosure, the term "(heterocyclo)alkyl" as used by itself or as part of another group refers to an alkyl group substituted with one, two, or three optionally substituted heterocyclo groups. In one embodiment, the (heterocyclo)alkyl is a $C_{1-4}$ alkyl substituted with one optionally substituted heterocyclo group. The heterocyclo can be linked to the alkyl group through a carbon or nitrogen atom. Non-limiting exemplary (heterocyclo)alkyl groups include:

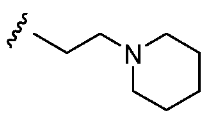 , 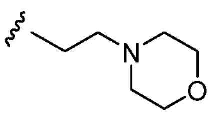 , 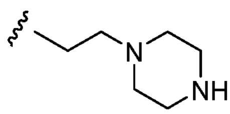 , 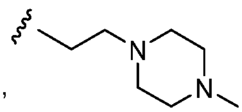 ,

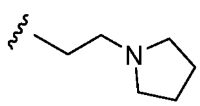 , 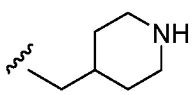 , 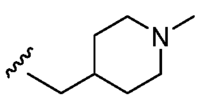 and 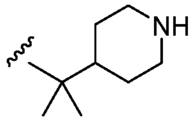 .

For the purpose of the present disclosure, the term "(heteroaryl)alkyl" or "heteroaralkyl" as used by itself or as part of another group refers to an alkyl group substituted with one, two, or three optionally substituted heteroaryl groups. In one embodiment, the (heteroaryl)alkyl group is a $C_{1-4}$ alkyl substituted with one optionally substituted heteroaryl group. Non-limiting exemplary (heteroaryl)alkyl groups include:

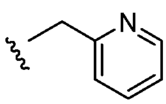 , 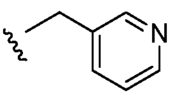 , 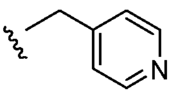 ,

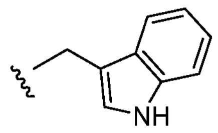 and 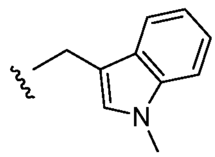 .

For the purpose of the present disclosure, the term "alkylcarbonylamino" as used by itself or as part of another group refers to an alkylcarbonyl group attached to an amino. A non-limiting exemplary alkylcarbonylamino group is $-NHCOCH_3$.

The present disclosure encompasses any of the Compounds of the Disclosure being isotopically-labelled *(i.e.,* radiolabeled) by having one or more atoms replaced by an atom having a different atomic mass or mass number. Examples of isotopes that can be incorporated into the disclosed compounds include isotopes of hydrogen, carbon, nitrogen, oxygen, phosphorous, fluorine and chlorine, such as $^{2}H$ (or deuterium (D)), $^{3}H$, $^{11}C$, $^{13}C$, $^{14}C$, $^{15}N$, $^{18}O$, $^{17}O$, $^{31}P$, $^{32}P$, $^{35}S$, $^{18}F$, and $^{36}Cl$, respectively, e.g., $^{3}H$, $^{11}C$, and $^{14}C$. In one embodiment, provided is a composition for use in treating cancer, wherein substantially all of the atoms at a position within the Compound of the Disclosure are replaced by an atom having a different atomic mass or mass number. In another embodiment, provided is a composition for use in treating cancer, wherein a portion of the atoms at a position within the Compound of the disclosure are replaced, *i.e.,* the Compound of the Disclosure is enriched at a position with an atom having a different atomic mass or mass number." Isotopically-labelled Compounds of the Disclosure can be prepared by methods known in the art.

Compounds of the Disclosure may contain one or more asymmetric centers and may thus give rise to enantiomers, diastereomers, and other stereoisomeric forms. The present disclosure is meant to encompass the use of all such possible forms, as well as their racemic and resolved forms and mixtures thereof. The individual enantiomers can be separated according to methods known in the art in view of the present disclosure. When the compounds described herein contain olefinic double bonds or other centers of geometric asymmetry, and unless specified otherwise, it is intended that they include both E and Z geometric isomers. All tautomers are intended to be encompassed by the present disclosure as well.

As used herein, the term "stereoisomers" is a general term for all isomers of individual molecules that differ only in the orientation of their atoms in space. It includes enantiomers and isomers of compounds with more than one chiral center that are not mirror images of one another (diastereomers).

The term "chiral center" or "asymmetric carbon atom" refers to a carbon atom to which four different groups are attached.

The terms "enantiomer" and "enantiomeric" refer to a molecule that cannot be superimposed on its mirror image and hence is optically active wherein the enantiomer rotates the plane of polarized light in one direction and its mirror image compound rotates the plane of polarized light in the opposite direction.

The term "racemic" refers to a mixture of equal parts of enantiomers and which mixture is optically inactive.

The term "absolute configuration" refers to the spatial arrangement of the atoms of a chiral molecular entity (or group) and its stereochemical description, e.g., R or S.

The stereochemical terms and conventions used in the specification are meant to be consistent with those described in Pure & Appl. Chem 68:2193 (1996), unless otherwise indicated.

The term "enantiomeric excess" or "ee" refers to a measure for how much of one enantiomer is present compared to the other. For a mixture of *R* and S enantiomers, the percent enantiomeric excess is defined as $|R - S|*100$, where R and S are the respective mole or weight fractions of enantiomers in a mixture such that R + S = 1. With knowledge of the optical rotation of a chiral substance, the percent enantiomeric excess is defined as $([\alpha]_{obs}/[\alpha]_{max})*100$, where $[\alpha]_{obs}$ is the optical rotation of the mixture of enantiomers and $[\alpha]_{max}$ is the optical rotation of the pure enantiomer. Determination of enantiomeric excess is possible using a variety of analytical techniques, including NMR spectroscopy, chiral column chromatography or optical polarimetry.

The terms "enantiomerically pure" or "enantiopure" refer to a sample of a chiral substance all of whose molecules (within the limits of detection) have the same chirality sense.

The terms "enantiomerically enriched" or "enantioenriched" refer to a sample of a chiral substance whose enantiomeric ratio is greater than 50:50. Enantiomerically enriched compounds may be enantiomerically pure.

The terms "a" and "an" refer to one or more.

The term "about," as used herein, includes the recited number $\pm$ 10%. Thus, "about 10" means 9 to 11.

The present disclosure encompasses the preparation and use of salts of the Compounds of the Disclosure, including non-toxic pharmaceutically acceptable salts. Examples of pharmaceutically acceptable addition salts include inorganic and organic acid addition salts and basic salts. The pharmaceutically acceptable salts include, but are not limited to, metal salts such as sodium salt, potassium salt, cesium salt and the like; alkaline earth metals such as calcium salt, magnesium salt and the like; organic amine salts such as triethylamine salt, pyridine salt, picoline salt, ethanolamine

salt, triethanolamine salt, dicyclohexylamine salt, N,N'-dibenzylethylenediamine salt and the like; inorganic acid salts such as hydrochloride, hydrobromide, phosphate, sulphate and the like; organic acid salts such as citrate, lactate, tartrate, maleate, fumarate, mandelate, acetate, dichloroacetate, trifluoroacetate, oxalate, formate and the like; sulfonates such as methanesulfonate, benzenesulfonate, p-toluenesulfonate and the like; and amino acid salts such as arginate, asparginate, glutamate and the like. The term "pharmaceutically acceptable salt" as used herein, refers to any salt, e.g., obtained by reaction with an acid or a base, of a Compound of the Disclosure that is physiologically tolerated in the target patient *(e.g.,* a mammal, *e.g.,* a human).

Acid addition salts can be formed by mixing a solution of the particular Compound of the Disclosure with a solution of a pharmaceutically acceptable non-toxic acid such as hydrochloric acid, fumaric acid, maleic acid, succinic acid, acetic acid, citric acid, tartaric acid, carbonic acid, phosphoric acid, oxalic acid, dichloroacetic acid, or the like. Basic salts can be formed by mixing a solution of the compound of the present disclosure with a solution of a pharmaceutically acceptable non-toxic base such as sodium hydroxide, potassium hydroxide, choline hydroxide, sodium carbonate and the like.

The present disclosure encompasses the preparation and use of solvates of Compounds of the Disclosure. Solvates typically do not significantly alter the physiological activity or toxicity of the compounds, and as such may function as pharmacological equivalents. The term "solvate" as used herein is a combination, physical association and/or solvation of a compound of the present disclosure with a solvent molecule such as, *e.g.* a disolvate, monosolvate or hemisolvate, where the ratio of solvent molecule to compound of the present disclosure is about 2:1, about 1:1 or about 1:2, respectively. This physical association involves varying degrees of ionic and covalent bonding, including hydrogen bonding. In certain instances, the solvate can be isolated, such as when one or more solvent molecules are incorporated into the crystal lattice of a crystalline solid. Thus, "solvate" encompasses both solution-phase and isolatable solvates. Compounds of the Disclosure can be present as solvated forms with a pharmaceutically acceptable solvent, such as water, methanol, ethanol, and the like, and it is intended that the disclosure includes both solvated and unsolvated forms of Compounds of the Disclosure. One type of solvate is a hydrate. A "hydrate" relates to a particular subgroup of solvates where the solvent molecule is water. Solvates typically can function as pharmacological equivalents. Preparation of solvates is known in the art. See, for example, M. Caira et al, J. Pharmaceut. Sci., 93(3):601-611 (2004), which describes the preparation of solvates of fluconazole with ethyl acetate and with water. Similar preparation of solvates, hemisolvates, hydrates, and the like are described by E.C. van Tonder et al., AAPS Pharm. Sci. Tech., 5(1):Article 12 (2004), and A.L. Bingham et al., Chem. Commun. 603-604 (2001). A typical, non-limiting, process of preparing a solvate would involve dissolving a Compound of the Disclosure in a desired solvent (organic, water, or a mixture thereof) at temperatures above 20°C to about 25°C, then cooling the solution at a rate sufficient to form crystals, and isolating the crystals by known methods, e.g., filtration. Analytical techniques such as infrared spectroscopy can be used to confirm the presence of the solvent in a crystal of the solvate.

Since Compounds of the Disclosure are inhibitors of SMYD proteins, such as SMYD3 and SMYD2, a number of diseases, conditions, or disorders mediated by SMYD proteins, such as SMYD3 and SMYD2, can be treated by employing these compounds. The present disclosure is thus directed generally to a method for treating a disease, condition, or disorder responsive to the inhibition of SMYD proteins, such as SMYD3 and SMYD2, in an animal suffering from, or at risk of suffering from, the disorder, the method comprising administering to the animal an effective amount of one or more Compounds of the Disclosure.

The present disclosure is further directed to a method of inhibiting SMYD proteins in an animal in need thereof, the method comprising administering to the animal a therapeutically effective amount of at least one Compound of the Disclosure.

The present disclosure is further directed to a method of inhibiting SMYD3 in an animal in need thereof, the method comprising administering to the animal a therapeutically effective amount of at least one Compound of the Disclosure.

The present disclosure is further directed to a method of inhibiting SMYD2 in an animal in need thereof, the method comprising administering to the animal a therapeutically effective amount of at least one Compound of the Disclosure.

As used herein, the terms "treat," "treating," "treatment," and the like refer to eliminating, reducing, or ameliorating a disease or condition, and/or symptoms associated therewith. Although not precluded, treating a disease or condition does not require that the disease, condition, or symptoms associated therewith be completely eliminated. As used herein, the terms "treat," "treating," "treatment," and the like may include "prophylactic treatment," which refers to reducing the probability of redeveloping a disease or condition, or of a recurrence of a previously-controlled disease or condition, in a subject who does not have, but is at risk of or is susceptible to, redeveloping a disease or condition or a recurrence of the disease or condition. The term "treat" and synonyms contemplate administering a therapeutically effective amount of a Compound of the Disclosure to an individual in need of such treatment.

Within the meaning of the disclosure, "treatment" also includes relapse prophylaxis or phase prophylaxis, as well as the treatment of acute or chronic signs, symptoms and/or malfunctions. The treatment can be orientated symptomatically, for example, to suppress symptoms. It can be effected over a short period, be oriented over a medium term, or can be a long-term treatment, for example within the context of a maintenance therapy.

The term "therapeutically effective amount" or "effective dose" as used herein refers to an amount of the active ingredient(s) that is(are) sufficient, when administered by a method of the disclosure, to efficaciously deliver the active ingredient(s) for the treatment of condition or disease of interest to an individual in need thereof. In the case of a cancer or other proliferation disorder, the therapeutically effective amount of the agent may reduce (i.e., retard to some extent and preferably stop) unwanted cellular proliferation; reduce the number of cancer cells; reduce the tumor size; inhibit (i.e., retard to some extent and preferably stop) cancer cell infiltration into peripheral organs; inhibit (i.e., retard to some extent and preferably stop) tumor metastasis; inhibit, to some extent, tumor growth; modulate protein methylation in the target cells; and/or relieve, to some extent, one or more of the symptoms associated with the cancer. To the extent the administered compound or composition prevents growth and/or kills existing cancer cells, it may be cytostatic and/or cytotoxic.

The term "container" means any receptacle and closure therefore suitable for storing, shipping, dispensing, and/or handling a pharmaceutical product.

The term "insert" means information accompanying a pharmaceutical product that provides a description of how to administer the product, along with the safety and efficacy data required to allow the physician, pharmacist, and patient to make an informed decision regarding use of the product. The package insert generally is regarded as the "label" for a pharmaceutical product.

The term "disease" or "condition" or "disorder" denotes disturbances and/or anomalies that as a rule are regarded as being pathological conditions or functions, and that can manifest themselves in the form of particular signs, symptoms, and/or malfunctions. As demonstrated below, Compounds of the Disclosure inhibit SMYD proteins, such as SMYD3 and SMYD2 and can be used in treating diseases and conditions such as proliferative diseases, wherein inhibition of SMYD proteins, such as SMYD3 and SMYD2 provides a benefit.

The Compounds of the Disclosure can be used to treat a "SMYD protein mediated disorder" *(e.g.,* a SMYD3-mediated disorder or a SMYD2-mediated disorder). A SMYD protein mediated disorder is any pathological condition in which a SMYD protein is know to play a role. A SMYD-mediated disorder may be a proliferative disease.

Inhibiting SMYD proteins, such as SMYD3 and SMYD2, may be the inhibition of the activity of one or more activities of SMYD proteins such as SMYD3 and SMYD2. The activity of the SMYD proteins such as SMYD3 and SMYD2 may be the ability of the SMYD protein such as SMYD3 or SMYD2 to transfer a methyl group to a target protein (e.g., histone). It should be appreciated that the activity of the one or more SMYD proteins such as SMYD3 and SMYD2 may be inhibited *in vitro* or *in vivo.* Examplary levels of inhibition of the activity one or more SMYD proteins such as SMYD3 and SMYD2 include at least 10% inhibition, at least 20% inhibition, at least 30% inhibition, at least 40% inhibition, at least 50% inhibition, at least 60% inhibition, at least 70% inhibition, at least 80% inhibition, at least 90% inhibition, and up to 100% inhibition.

The SMYD (SET and MYND domain) family of lysine methyltransferases (KMTs) plays pivotal roles in various cellular processes, including gene expression regulation and DNA damage response. The family of human SMYD proteins consists of SMYD1, SMYD2, SMYD3, SMYD4 and SMYD5. SMYD1, SMYD2, and SMYD3 share a high degree of sequence homology and, with the exception of SMYD5, human SMYD proteins harbor at least one C-terminal tetratrico peptide repeat (TPR) domain. (See *e.g.,* Abu-Farha et al. JMol Cell Biol (2011) 3 (5) 301-308). The SMYD proteins have been found to be linked to various cancers (See *e.g.,* Hamamoto et al. Nat Cell. Biol. 2004, 6: 731-740), Hu et al. Canncer Research 2009, 4067-4072, and Komatsu et al. Carcinogenesis 2009, 301139-1146.)

SMYD3 is a protein methyltransferase found to be expressed at high levels in a number of different cancers (Hamamoto, R., et al., Nat. Cell Biol., 6(8):731-40 (2004)). SMYD3 likely plays a role in the regulation of gene transcription and signal transduction pathways critical for survival of breast, liver, prostate and lung cancer cell lines (Hamamoto, R., et al., Nat. Cell Biol., 6(8):731-40 (2004); Hamamoto, R., et al., Cancer Sci., 97(2):113-8 (2006); Van Aller, G.S., et al., Epigenetics, 7(4):340-3 (2012); Liu, C., et al., J. Natl. Cancer Inst., 105(22):1719-28 (2013); Mazur, P.K., et al., Nature, 510(7504):283-7 (2014)).

Genetic knockdown of SMYD3 leads to a decrease in proliferation of a variety of cancer cell lines (Hamamoto, R., et al., Nat. Cell Biol., 6(8):731-40 (2004); Hamamoto, R., et al., Cancer Sci., 97(2):113-8 (2006); Van Aller, G.S., et al., Epigenetics, 7(4):340-3 (2012); Liu, C., et al., J. Natl. Cancer Inst., 105(22):1719-28 (2013); Mazur, P.K., et al., Nature, 510(7504):283-7 (2014)). Several studies employing RNAi-based technologies have shown that ablation of SMYD3 in hepatocellular carcinoma cell lines greatly reduces cell viability and that its pro-survival role is dependent on its catalytic activity (Hamamoto, R., et al., Nat. Cell Biol., 6(8):731-40 (2004); Van Aller, G.S., et al., Epigenetics, 7(4):340-3 (2012)). Moreover, SMYD3 has also been shown to be a critical mediator of transformation resulting from gain of function mutations in the oncogene, KRAS for both pancreatic and lung adenocarcinoma in mouse models. The dependence of KRAS on SMYD3 was also shown to be dependent on its catalytic activity (Mazur, P.K., et al., Nature, 510(7504):283-7 (2014)). SMYD3 function has also been implicated in colerectal cancers and RNAi mediated knockdown of SMYD3 has been shown to impair colerectal cell proliferation. (Peserico et al., Cell Physiol. 2015 Feb 28. doi: 10.1002/jcp.24975.

[Epub ahead of print]).

Furthermore, SMYD3 function has also been shown to play a role in immunology and development. For instance, de Almeida reported that SMYD3 plays a role in generation of inducible regulatory T cells (iTreg) cells. In a mouse model of respiratory syncytial virus (RSV) infection, a model in which iTreg cells have a critical role in regulating lung pathogenesis, SMYD3-/- mice demonstrated exacerbation of RSV-induced disease related to enhanced proinflammatory responses and worsened pathogenesis within the lung (de Almeida et al. Mucosal Immunol. 2015 Feb 11. doi: 10.1038/mi.2015.4. [Epub ahead of print]). In addition, as to development, Proserpio et al. have shown the importance of SMYD3 in the regulation of skeletal muscle atrophy (Proserpio et al. Genes Dev. 2013 Jun 1;27(11):1299-312), while Fujii et al. have elucidated the role of SMYD3 in cardiac and skeletal muscle development (Fujii et al. PLoS One. 2011;6(8):e23491).

SMYD2 (SET and MYND domain-containing protein 2) was first characterized as protein that is a member of a sub-family of SET domain containing proteins which catalyze the site-specific transfer of methyl groups onto substrate proteins. SMYD2 was initially shown to have methyltransferase activity towards lysine 36 on histone H3 (H3K36) but has subsequently been shown to have both histone and non-histone methyltrasferase activity.

SMYD2 has been implicated in the pathogenesis of multiple cancers. It has been shown to be over-expressed, compared to matched normal samples, in tumors of the breast, cervix, colon, kidney, liver, head and neck, skin, pancreas, ovary, esophagus and prostate, as well as hematologic malignancies such as AML, B- and T-ALL, CLL and MCL, suggesting a role for SMYD2 in the biology of these cancers. More specifically, studies using genetic knock-down of SMYD2 have demonstrated anti-proliferative effects in esophageal squamous cell carcinoma (ESCC), bladder carcinoma and cervical carcinoma cell lines. (See *e.g.,* Komatsu et al., Carcinogenesis 2009, 30, 1139, and Cho et al., Neoplasia. 2012 Jun;14(6):476-86). Moreover, high expression of SMYD2 has been shown to be a poor prognostic factor in both ESCC and pediatric ALL. (See e.g., Komatsu et al. Br J Cancer. 2015 Jan 20;112(2):357-64, and Sakamoto et al., Leuk Res. 2014 Apr;38(4):496-502). Recently, Nguyen et al., have shown that a small molecule inhibitor of SMYD2 (LLY-507) inhibited the proliferation of several esophageal, liver and breast cancer cell lines in a dose-dependent manner. (Nguyen et al. J Biol Chem. 2015 Mar 30. pii: jbc.M114.626861. [Epub ahead of print]).

SMYD2 has also been implicated in immunology. For instance, Xu et al. have shown that SMYD2 is a negative regulator of macrophage activation by suppressing Interleukin-6 and TNF-alpha production. (Xu et al., J. Biol. Chem. 2015 Feb 27;290(9):5414-23).

The present disclosure also provides a method of treating cancer in a patient comprising administering a therapeutically effective amount of a Compound of the Disclosure. While not being limited to a specific mechanism, Compounds of the Disclosure can treat cancer by inhibiting SMYD proteins, such as SMYD3 and SMYD2. Examples of treatable cancers include, but are not limited to, adrenal cancer, acinic cell carcinoma, acoustic neuroma, acral lentigious melanoma, acrospiroma, acute eosinophilic leukemia, acute erythroid leukemia, acute lymphoblastic leukemia, acute megakaryoblastic leukemia, acute monocytic leukemia, acute promyelocytic leukemia, adenocarcinoma, adenoid cystic carcinoma, adenoma, adenomatoid odontogenic tumor, adenosquamous carcinoma, adipose tissue neoplasm, adrenocortical carcinoma, adult T-cell leukemia/lymphoma, aggressive NK-cell leukemia, AIDS-related lymphoma, alveolar rhabdomyosarcoma, alveolar soft part sarcoma, ameloblastic fibroma, anaplastic large cell lymphoma, anaplastic thyroid cancer, angioimmunoblastic T-cell lymphoma, angiomyolipoma, angiosarcoma, astrocytoma, atypical teratoid rhabdoid tumor, B-cell chronic lymphocytic leukemia, B-cell prolymphocytic leukemia, B-cell lymphoma, basal cell carcinoma, biliary tract cancer, bladder cancer, blastoma, bone cancer, Brenner tumor, Brown tumor, Burkitt's lymphoma, breast cancer, brain cancer, carcinoma, carcinoma in situ, carcinosarcoma, cartilage tumor, cementoma, myeloid sarcoma, chondroma, chordoma, choriocarcinoma, choroid plexus papilloma, clear-cell sarcoma of the kidney, craniopharyngioma, cutaneous T-cell lymphoma, cervical cancer, colorectal cancer, Degos disease, desmoplastic small round cell tumor, diffuse large B-cell lymphoma, dysembryoplastic neuroepithelial tumor, dysgerminoma, embryonal carcinoma, endocrine gland neoplasm, endodermal sinus tumor, enteropathy-associated T-cell lymphoma, esophageal cancer, fetus in fetu, fibroma, fibrosarcoma, follicular lymphoma, follicular thyroid cancer, ganglioneuroma, gastrointestinal cancer, germ cell tumor, gestational choriocarcinoma, giant cell fibroblastoma, giant cell tumor of the bone, glial tumor, glioblastoma multiforme, glioma, gliomatosis cerebri, glucagonoma, gonadoblastoma, granulosa cell tumor, gynandroblastoma, gallbladder cancer, gastric cancer, hairy cell leukemia, hemangioblastoma, head and neck cancer, hemangiopericytoma, hematological malignancy, hepatoblastoma, hepatosplenic T-cell lymphoma, Hodgkin's lymphoma, non-Hodgkin's lymphoma, invasive lobular carcinoma, intestinal cancer, kidney cancer, laryngeal cancer, lentigo maligna, lethal midline carcinoma, leukemia, leydig cell tumor, liposarcoma, lung cancer, lymphangioma, lymphangiosarcoma, lymphoepithelioma, lymphoma, acute lymphocytic leukemia, acute myelogeous leukemia, chronic lymphocytic leukemia, liver cancer, small cell lung cancer, non-small cell lung cancer, MALT lymphoma, malignant fibrous histiocytoma, malignant peripheral nerve sheath tumor, malignant triton tumor, mantle cell lymphoma, marginal zone B-cell lymphoma, mast cell leukemia, mediastinal germ cell tumor, medullary carcinoma of the breast, medullary thyroid cancer, medulloblastoma, melanoma, meningioma, merkel cell cancer, mesothelioma, metastatic urothelial carcinoma, mixed Mullerian tumor, mucinous tumor, multiple myeloma, muscle tissue neoplasm, mycosis fungoides, myxoid liposarcoma, myxoma, myxosarcoma, nasopharyngeal carcinoma, neurinoma, neuroblastoma, neurofibroma, neuroma, nodular melanoma, ocular cancer, oligoastrocytoma, oligodendroglioma, oncocytoma, optic nerve sheath meningioma, optic nerve tumor, oral cancer, osteosarcoma, ovarian cancer, Pancoast tumor, papillary thyroid cancer, paraganglioma, pinealoblastoma, pineocytoma, pituicytoma, pituitary adenoma, pituitary tumor, plasmacytoma, polyembryoma, precursor T-lymphoblastic lymphoma, primary central nervous system lymphoma, primary effusion lymphoma, preimary peritoneal cancer, prostate cancer, pancreatic cancer, pharyngeal cancer, pseudomyxoma periotonei, renal cell carcinoma, renal medullary carcinoma, retinoblastoma, rhabdomyoma, rhabdomyosarcoma, Richter's transformation, rectal cancer, sarcoma, Schwannomatosis, seminoma, Sertoli cell tumor, sex cord-gonadal stromal tumor, signet ring cell carcinoma, skin cancer, small blue round cell tumors, small cell carcinoma, soft tissue sarcoma, somatostatinoma, soot wart, spinal tumor, splenic marginal zone lymphoma, squamous cell carcinoma, synovial sarcoma, Sezary's disease, small intestine cancer, squamous carcinoma, stomach cancer, T-cell lymphoma, testicular cancer, thecoma, thyroid cancer, transitional cell carcinoma, throat cancer, urachal cancer, urogenital cancer, urothelial carcinoma, uveal melanoma, uterine cancer, verrucous carcinoma, visual pathway glioma, vulvar cancer, vaginal cancer, Waldenstrom's macroglobulinemia, Warthin's tumor, and Wilms' tumor.

In another embodiment, the cancer is breast, cervix, colon, kidney, liver, head and neck, skin, pancreas, ovary, esophagus, or prostate cancer.

In another embodiment, the cancer is a hematologic malignancy such as acute myeloid leukemia (AML), B- and T-acute lymphoblastic leukemia (ALL), chronic lymphocytic leukemia (CLL), or mantle cell lymphoma (MCL).

In another embodiment, the cancer is esophageal squamous cell carcinoma (ESCC), bladder carcinoma, or cervical carcinoma.

In another embodiment, the cancer is a leukemia, for example a leukemia selected from acute monocytic leukemia, acute myelogenous leukemia, chronic myelogenous leukemia, chronic lymphocytic leukemia and mixed lineage leukemia (MLL). In another embodiment the cancer is NUT-midline carcinoma. In another embodiment the cancer is multiple myeloma. In another embodiment the cancer is a lung cancer such as small cell lung cancer (SCLC). In another embodiment the cancer is a neuroblastoma. In another embodiment the cancer is Burkitt's lymphoma. In another embodiment the cancer is cervical cancer. In another embodiment the cancer is esophageal cancer. In another embodiment the cancer is ovarian cancer. In another embodiment the cancer is colorectal cancer. In another embodiment, the cancer is prostate cancer. In another embodiment, the cancer is breast cancer.

The present disclosure also provides a therapeutic method of modulating protein methylation, gene expression, cell proliferation, cell differentiation and/or apoptosis *in vivo* in the cancers mentioned above by administering a therapeutically effective amount of a Compound of the Disclosure to a subject in need of such therapy.

Compounds of the Disclosure can be administered to a mammal in the form of a raw chemical without any other components present. Compounds of the Disclosure can also be administered to a mammal as part of a pharmaceutical composition containing the compound combined with a suitable pharmaceutically acceptable carrier. Such a carrier can be selected from pharmaceutically acceptable excipients and auxiliaries. The term "pharmaceutically acceptable carrier" or "pharmaceutically acceptable vehicle" encompasses any of the standard pharmaceutical carriers, solvents, surfactants, or vehicles. Suitable pharmaceutically acceptable vehicles include aqueous vehicles and nonaqueous vehicles. Standard pharmaceutical carriers and their formulations are described in Remington's Pharmaceutical Sciences, Mack Publishing Co., Easton, PA, 19th ed. 1995.

Pharmaceutical compositions for use in treating cancer within the scope of the present disclosure include all compositions where a Compound of the Disclosure is combined with one or more pharmaceutically acceptable carriers. In one embodiment, the Compound of the Disclosure is present in the composition for use in an amount that is effective to achieve its intended therapeutic purpose. While individual needs may vary, a determination of optimal ranges of effective amounts of each compound is within the skill of the art. Typically, a Compound of the Disclosure can be administered to a mammal, e.g., a human, orally at a dose of from about 0.0025 to about 1500 mg per kg body weight of the mammal, or an equivalent amount of a pharmaceutically acceptable salt or solvate thereof, per day to treat the particular disorder. A useful oral dose of a Compound of the Disclosure administered to a mammal is from about 0.0025 to about 50 mg per kg body weight of the mammal, or an equivalent amount of the pharmaceutically acceptable salt or solvate thereof. For intramuscular injection, the dose is typically about one-half of the oral dose.

A unit oral dose may comprise from about 0.01 mg to about 1 g of the Compound of the Disclosure, e.g., about 0.01 mg to about 500 mg, about 0.01 mg to about 250 mg, about 0.01 mg to about 100 mg, 0.01 mg to about 50 mg, *e.g.,* about 0.1 mg to about 10 mg, of the compound. The unit dose can be administered one or more times daily, *e.g.,* as one or more tablets or capsules, each containing from about 0.01 mg to about 1 g of the compound, or an equivalent amount of a pharmaceutically acceptable salt or solvate thereof.

A pharmaceutical composition for use in treating cancer of the present disclosure can be administered to any patient that may experience the beneficial effects of a Compound of the Disclosure. Foremost among such patients are mammals, e.g., humans and companion animals, although the disclosure is not intended to be so limited. In one embodiment, the patient is a human.

A pharmaceutical composition of the present disclosure can be administered by any means that achieves its

intended purpose. For example, administration can be by the oral, parenteral, subcutaneous, intravenous, intramuscular, intraperitoneal, transdermal, intranasal, transmucosal, rectal, intravaginal or buccal route, or by inhalation. The dosage administered and route of administration will vary, depending upon the circumstances of the particular subject, and taking into account such factors as age, gender, health, and weight of the recipient, condition or disorder to be treated, kind of concurrent treatment, if any, frequency of treatment, and the nature of the effect desired.

In one embodiment, a pharmaceutical composition for use of the present disclosure can be administered orally. In another embodiment, a pharmaceutical composition for use of the present disclosure can be administered orally and is formulated into tablets, dragees, capsules, or an oral liquid preparation. In one embodiment, the oral formulation comprises extruded multiparticulates comprising the Compound of the Disclosure.

Alternatively, a pharmaceutical composition of the present disclosure can be administered rectally, and is formulated in suppositories.

Alternatively, a pharmaceutical composition of the present disclosure can be administered by injection.

Alternatively, a pharmaceutical composition of the present disclosure can be administered transdermally.

Alternatively, a pharmaceutical composition of the present disclosure can be administered by inhalation or by intranasal or transmucosal administration.

Alternatively, a pharmaceutical composition of the present disclosure can be administered by the intravaginal route.

A pharmaceutical composition of the present disclosure can contain from about 0.01 to 99 percent by weight, e.g., from about 0.25 to 75 percent by weight, of a Compound of the Disclosure, e.g., about 1%, about 5%, about 10%, about 15%, about 20%, about 25%, about 30%, about 35%, about 40%, about 45%, about 50%, about 55%, about 60%, about 65%, about 70%, or about 75% by weight of a Compound of the Disclosure.

A pharmaceutical composition of the present disclosure is manufactured in a manner which itself will be known in view of the instant disclosure, for example, by means of conventional mixing, granulating, dragee-making, dissolving, extrusion, or lyophilizing processes. Thus, pharmaceutical compositions for oral use can be obtained by combining the active compound with solid excipients, optionally grinding the resulting mixture and processing the mixture of granules, after adding suitable auxiliaries, if desired or necessary, to obtain tablets or dragee cores.

Suitable excipients include fillers such as saccharides (for example, lactose, sucrose, mannitol or sorbitol), cellulose preparations, calcium phosphates (for example, tricalcium phosphate or calcium hydrogen phosphate), as well as binders such as starch paste (using, for example, maize starch, wheat starch, rice starch, or potato starch), gelatin, tragacanth, methyl cellulose, hydroxypropylmethylcellulose, sodium carboxymethylcellulose, and/or polyvinyl pyrrolidone. If desired, one or more disintegrating agents can be added, such as the above-mentioned starches and also carboxymethyl-starch, cross-linked polyvinyl pyrrolidone, agar, or alginic acid or a salt thereof, such as sodium alginate.

Auxiliaries are typically flow-regulating agents and lubricants such as, for example, silica, talc, stearic acid or salts thereof (e.g., magnesium stearate or calcium stearate), and polyethylene glycol. Dragee cores are provided with suitable coatings that are resistant to gastric juices. For this purpose, concentrated saccharide solutions can be used, which may optionally contain gum arabic, talc, polyvinyl pyrrolidone, polyethylene glycol and/or titanium dioxide, lacquer solutions and suitable organic solvents or solvent mixtures. In order to produce coatings resistant to gastric juices, solutions of suitable cellulose preparations such as acetylcellulose phthalate or hydroxypropylmethyl-cellulose phthalate can be used. Dye stuffs or pigments can be added to the tablets or dragee coatings, for example, for identification or in order to characterize combinations of active compound doses.

Examples of other pharmaceutical preparations that can be used orally include push-fit capsules made of gelatin, or soft, sealed capsules made of gelatin and a plasticizer such as glycerol or sorbitol. The push-fit capsules can contain a compound in the form of granules, which can be mixed with fillers such as lactose, binders such as starches, and/or lubricants such as talc or magnesium stearate and, optionally, stabilizers, or in the form of extruded multiparticulates. In soft capsules, the active compounds are preferably dissolved or suspended in suitable liquids, such as fatty oils or liquid paraffin. In addition, stabilizers can be added.

Possible pharmaceutical preparations for rectal administration include, for example, suppositories, which consist of a combination of one or more active compounds with a suppository base. Suitable suppository bases include natural and synthetic triglycerides, and paraffin hydrocarbons, among others. It is also possible to use gelatin rectal capsules consisting of a combination of active compound with a base material such as, for example, a liquid triglyceride, polyethylene glycol, or paraffin hydrocarbon.

Suitable formulations for parenteral administration include aqueous solutions of the active compound in a water-soluble form such as, for example, a water-soluble salt, alkaline solution, or acidic solution. Alternatively, a suspension of the active compound can be prepared as an oily suspension. Suitable lipophilic solvents or vehicles for such as suspension may include fatty oils (for example, sesame oil), synthetic fatty acid esters (for example, ethyl oleate), triglycerides, or a polyethylene glycol such as polyethylene glycol-400 (PEG-400). An aqueous suspension may contain one or more substances to increase the viscosity of the suspension, including, for example, sodium carboxymethyl cellulose, sorbitol, and/or dextran. The suspension may optionally contain stabilizers.

The present disclosure also provides kits which comprise a Compound of the Disclosure (or a composition comprising a Compound of the Disclosure) packaged in a manner that facilitates their use to practice methods of the present disclosure. The kit may include a Compound of the Disclosure (or a composition comprising a Compound of the Disclosure) packaged in a container, such as a sealed bottle or vessel, with a label affixed to the container or included in the kit that describes use of the compound or composition to practice the method of the disclosure. The compound or composition may be packaged in a unit dosage form. The kit further can include a device suitable for administering the composition according to the intended route of administration.

General Synthesis of Compounds

Compounds of the Disclosure are prepared using methods known to those skilled in the art in view of this disclosure, or by the illustrative methods shown in the General Schemes below. In the General Schemes, $R^1$, $R^{2a}$, $R^{3a}$, $R^{4a}$, and Z of Formulae A-D are as defined in connection with Formula I, unless otherwise indicated. In any of the General Schemes, suitable protecting can be employed in the synthesis, for example, when Z is (amino)alkyl or any other group that may group that may require protection. (See, Wuts, P. G. M.; Greene, T. W., "Greene's Protective Groups in Organic Synthesis", 4th Ed., J. Wiley & Sons, NY, 2007).

General Scheme 1

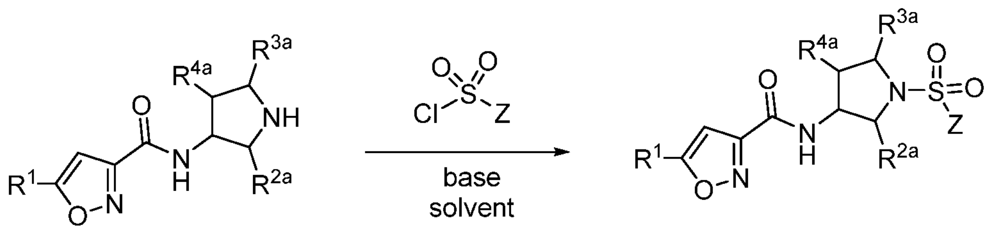


A

B

Compound A is converted to compound B (i.e, a compound having Formula I, wherein $R^{2b}$, $R^{3b}$, $R^{4b}$, and $R^5$ are each hydrogen, and X is $-S(=O)_2-$) by coupling with a suitable sulfonyl chloride ($Z-SO_2Cl$) in the presence of a suitable base such as TEA or DIPEA in a suitable solvent such as dichloromethane, acetonitrile, or DMF.

General Scheme 2

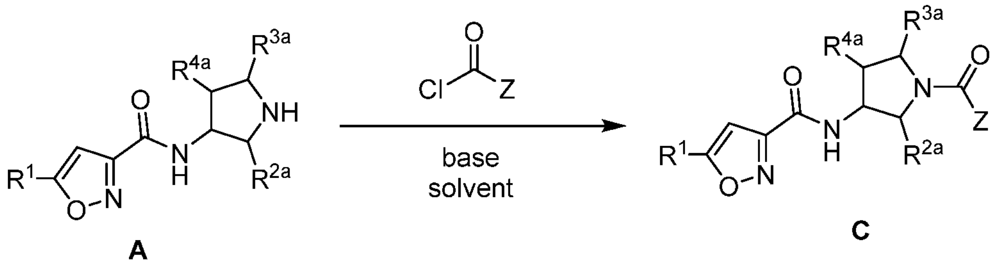


C

A

Compound A is converted to compound C (i.e, a compound having Formula I, wherein $R^{2b}$, $R^{3b}$, $R^{4b}$, and $R^5$ are each hydrogen, and X is -C(=O)-) by coupling with a suitable acide chloride (Z-COCl) in the presence of a suitable base such as TEA or DIPEA in a suitable solvent such as dichloromethane, acetonitrile, or DMF, or by coupling with a suitable carboxylic acid ($Z-CO_2H$) in the presence of a suitable coupling reagent such as HATU and a suitable base such as TEA or DIPEA in a suitable solvent such as dichloromethane, acetonitrile, or DMF.

General Scheme 3

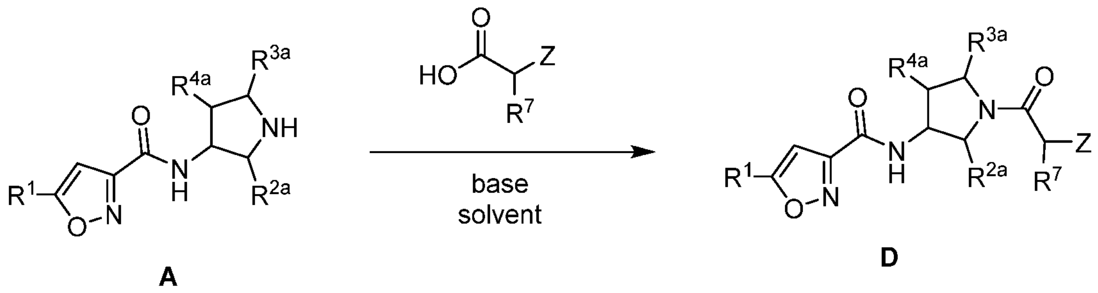


Compound A is converted to compound D (i.e, a compound having Formula I, wherein $R^{2b}$, $R^{3b}$, $R^{4b}$, and $R^5$ are each hydrogen, and X is -C(=O)C($R^7$)(H)-) by coupling with a suitable carboxylic acid (Z-C(H)$R^7$-$CO_2$H) in the presence of a suitable coupling reagent such as HATU and a suitable base such as TEA or DIPEA in a suitable solvent such as dichloromethane, acetonitrile, or DMF.

EXAMPLES

General Synthetic Methods

General methods and experimental procedures for preparing and characterizing compounds of Tables 1-3 are set forth in the general schemes above and the examples below. Wherever needed, reactions were heated using conventional hotplate apparatus or heating mantle or microwave irradiation equipment. Reactions were conducted with or without stirring, under atmospheric or elevated pressure in either open or closed vessels. Reaction progress was monitored using conventional techniques such as TLC, HPLC, UPLC, or LCMS using instrumentation and methods described below. Reactions were quenched and crude compounds isolated using conventional methods as described in the specific examples provided. Solvent removal was carried out with or without heating, under atmospheric or reduced pressure, using either a rotary or centrifugal evaporator.

Compound purification was carried out as needed using a variety of traditional methods including, but not limited to, preparative chromatography under acidic, neutral, or basic conditions using either normal phase or reverse phase HPLC or flash columns or Prep-TLC plates. Compound purity and mass confirmations were conducted using standard HPLC and / or UPLC and / or MS spectrometers and / or LCMS and / or GC equipment *(i.e.,* including, but not limited to the following instrumentation: Waters Alliance 2695 with 2996 PDA detector connected with ZQ detector and ESI source; Shimadzu LDMS-2020; Waters Acquity H Class with PDA detector connected with SQ detector and ESI source; Agilent 1100 Series with PDA detector; Waters Alliance 2695 with 2998 PDA detector; AB SCIEX API 2000 with ESI source; Agilent 7890 GC).

Compound structure confirmations were carried out using standard 300 or 400 MHz NMR spectrometers with nOe's conducted whenever necessary.

The following abbreviations are used herein:

| Abbreviation | Meaning |
|---|---|
| ACN | Acetonitrile |
| atm. | Atmosphere |
| DCM | Dichloromethane |
| DHP | Dihydropyran |
| DIBAL | diisobutyl aluminum hydride |
| DIEA | diisopropyl ethylamine |
| DMF | dimethyl formamide |
| DMF-DMA | dimethyl formamide dimethyl acetal |
| DMSO | dimethyl sulfoxide |
| Dppf | 1,1'-bis(diphenylphosphino)ferrocene |

(continued)

| Abbreviation | Meaning |
|---|---|
| EA | ethyl acetate |
| ESI | electrospray ionization |
| EtOH | Ethanol |
| FA | formic acid |
| GC | gas chromatography |
| H | Hour |
| Hex | Hexanes |
| HMDS | hexamethyl disilazide |
| HPLC | high performance liquid chromatography |
| IPA | Isopropanol |
| LCMS | liquid chromatography / mass spectrometry |
| MeOH | Methanol |
| Min | Minutes |
| NBS | *N*-bromo succinimide |
| NCS | *N*-chloro succinimide |
| NIS | *N*-iodo succinimide |
| NMR | nuclear magnetic resonance |
| nOe | nuclear Overhauser effect |
| Prep. | Preparative |
| PTSA | *para*-toluene sulfonic acid |
| Rf | retardation factor |
| rt | room temperature |
| RT | retention time |
| sat. | Saturated |
| SGC | silica gel chromatography |
| TBAF | tetrabutyl ammonium fluoride |
| TEA | Triethylamine |
| TFA | trifluoroacetic acid |
| THF | Tetrahydrofuran |
| TLC | thin layer chromatography |
| UPLC | ultra performance liquid chromatography |

EXAMPLE 1

Synthesis of 5-cyclopropylisoxazole-3-carboxylic acid

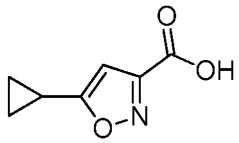

Step 1: Synthesis of ethyl 4-cyclopropyl-2,4-dioxobutanoate

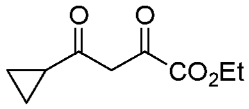

Into a 10-L 3-necked round-bottom flask purged and maintained with an inert atmosphere of nitrogen Na (164 g, 1.20 equiv) was added in portions to ethanol (5 L). A solution of $(CO_2Et)_2$ (869 g, 1.00 equiv) and 1-cyclopropylethan-1-one (500 g, 5.94 mol, 1.00 equiv) was added dropwise with stirring at 0-20 °C. The resulting solution was stirred for 1 h at 20-30 °C and then for an additional 1 h at 80°C. The resulting solution was diluted with 15 L of $H_2O$. The pH was adjusted to 2 with hydrochloric acid (12N). The resulting mixture was extracted with ethyl acetate and the organic layers combined and washed with $NaHCO_3$ (sat. aq.). The extract was concentrated under vacuum yielding 820 g (crude) of ethyl 4-cyclopropyl-2,4-dioxobutanoate as yellow oil. TLC (ethyl acetate/petroleum ether =1/5): Rf= 0.5.

Step 2: Synthesis of ethyl 5-cyclopropylisoxazole-3-carboxylate

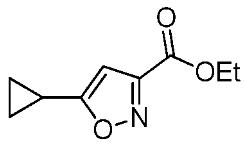

Into a 10 L round-bottom flask, was placed a solution of ethyl 4-cyclopropyl-2,4-dioxobutanoate (177 g) in ethanol (1.1 L) and $NH_2OH$-HCl (200 g). The resulting solution was stirred for 1 h at 20-30 °C. The resulting solution was allowed to react, with stirring, for an additional 1 h at 80°C. The resulting mixture was concentrated under vacuum. The residue was purified on a silica gel column with ethyl acetate/petroleum ether (1/10). This resulted in 143 g (the two step yield was 66.3%) of ethyl 5-cyclopropylisoxazole-3-carboxylate as a yellow oil. TLC (ethyl acetate/petroleum ether =1/5): Rf= 0.2.

Step 3: Synthesis of 5-cyclopropylisoxazole-3-carboxylic acid

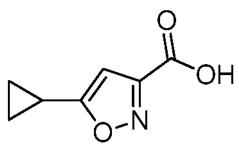

Into a 10-L round-bottom flask was placed ethyl 5-cyclopropylisoxazole-3-carboxylate (280 g, 1.55 mol, 1.00 equiv) and a solution of sodium hydroxide (74.3 g, 1.20 equiv) in water (4 L). The resulting solution was stirred for 1 h at room temperature. The resulting mixture was washed with ether. The pH value of the aqueous solution was adjusted to 2-3 with hydrochloric acid (12N). The resulting solution was extracted with ethyl acetate and the organic layers combined and concentrated under vacuum. This resulted in 220 g (93%) of 5-cyclopropylisoxazole-3-carboxylic acid as an off-white solid. $^1H$-NMR (300 MHz $CDCl_3$): $\delta$ 8.42 (brs, 1H), 6.37 (s, 1H), 2.16-2.05 (m, 1H), 1.29-1.12 (m, 2H), 1.12-0.99 (m, 2H); LCMS m/z = 153.9 $[M+H]^+$.

EXAMPLE 2

Synthesis of (±)-*cis*-5-cyclopropyl-N-(1-cyclopropyl-4-methylpyrrolidin-3-yl)isoxazole-3-carboxamide (Cpd. No. 45) and (±)-*trans*-5-cyclopropyl-N-(1-cyclopropyl-4-methylpyrrolidin-3-yl)isoxazole-3-carboxamide (Cpd. No. 46)

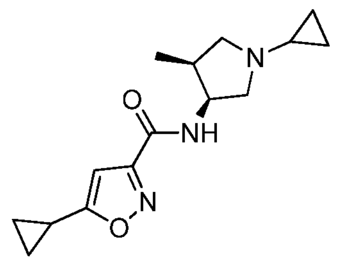

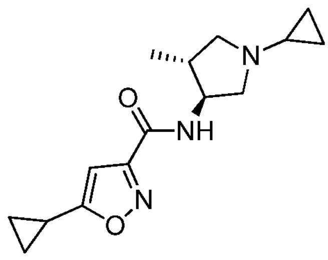

To a solution of 5-cyclopropylisoxazole-3-carboxylic acid (100mg, 0.653mmol) in DMF (2ml) was added HATU (370 mg, 0.980 mmol). The reaction stirred at room temperature for 30 minutes and then was cooled to 0°C. 1Cyclopropyl-4-methylpyrrolidin-3-amine (109 mg, 0.781 mmol) was added followed by DIPEA (252 mg, 1.960 mmol). The reaction stirred at ambient temperature for 2 hours. After completion of the reaction the reaction mixture was poured into 50 ml of water. The aqueous phase was extracted with ethyl acetate (3 x 25ml). The combined organic extracts were washed with brine, dried over sodium sulfate and concentrated under vacuum. The material was purified using column chromatography. The product was eluted at 2% MeOH in DCM. Appropriate fractions were combined and concentrated under vacuum to get 150 mg (83.79 %) of 5-cyclopropyl-N-(1-cyclopropyl-4-methylpyrrolidin-3-yl)isoxazole-3-carboxamide as a mixture of enantiomers and diastereomers. Cis and trans isomers were seperated out by chiral preparative HPLC using 0.1% TFA in hexanes/isopropanol as mobile phase to afford 35 mg of (±)-*cis*-5-cyclopropyl-N-(1-cyclopropyl-4-methylpyrrolidin-3-yl)isoxazole-3-carboxamide (Fraction-1) and 49 mg of (±)-*trans*-5 -cyclopropyl-N-(1-cyclopropyl-4-methylpyrrolidin-3-yl)isoxazole-3-carboxamide (Fraction-2).

(±)-*cis*-5-cyclopropyl-N-(1-cyclopropyl-4-methylpyrrolidin-3-yl)isoxazole-3-carboxamide: 1H NMR (400 MHz, MeOD): δ 6.41 (s, 1H), 4.33 (bs, 1H), 3.94-3.66 (m, 3H), 3.15-3.00 (m, 2H), 2.56-2.53 (m, 1H), 2.22-2.15 (m, 1H), 1.37-1.33 (d, J = 18.4 Hz, 3H), 1.23 (d, J = 6.8 Hz, 2H), 1.00-0.99 (m, 5H); LCMS: *m/z* = 277.23 $[M+H]^+$.

(±)-*trans*-5-cyclopropyl-N-(1-cyclopropyl-4-methylpyrrolidin-3-yl)isoxazole-3-carboxamide: 1H NMR (400 MHz, MeOD): δ 6.42 (s, 1H), 4.15 (bs, 1H), 3.77-3.66 (m, 3H), 3.52-3.37 (m, 2H), 3.04 (bs, 2H), 2.62 (bS, 1H), 2.22-2.26 (m, 1H), 1.19-1.12 (m, 2H), 1.09 (d, J = 7.2 Hz, 3H), 1.01-0.98 (m, 5H), 0.97-0.90 (m, 2H); LCMS: *m/z* = 276.18 $[M+H]^+$.

EXAMPLE 3

Synthesis of (±)-*cis* 5-cyclopropyl-N-(1,4-dimethylpyrrolidin-3-yl)isoxazole-3-carboxamide and (±)-*trans*-5-cyclopropyl-N-(1,4-dimethylpyrrolidin-3-yl)isoxazole-3-carboxamide (Cpd. Nos. 39 and 44)

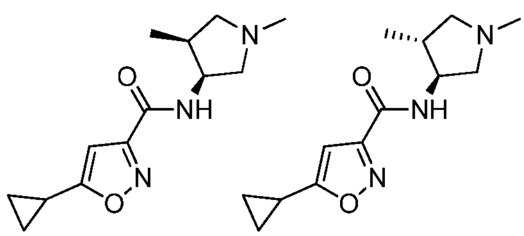

To a solution of 5-cyclopropylisoxazole-3-carboxylic acid (300 mg, 1.9 mmol) in DMF (3 ml), was added HATU (1.117 g, 2.9 mmol). The reaction mixture was stirred for 20 min at room temperature. After being cooled to 0 °C 1,4-dimethylpyrrolidin-3-amine (250 mg, 2.1 mmol) and DIPEA (380 mg, 2.9 mmol) were added to the reaction mixture. It was stirred at room temperature for 2 hrs. Completion of the reaction was confirmed by TLC. After completion, water (30 mL) was added and the product was extracted with ethyl acetate (2 x 30 mL). The combined organic layer was washed with brine, dried over sodium sulfate and concentrated under vacuum to get the crude product which was purified

by column chromatography using 1% MeOH in DCM. Distillation of the pure fractions afforded 392 mg of a mixture of diastereomers and enantiomers. The cis and trans isomers were separated by chiral prep. HPLC using 0.1% diethyamine in n-Heptane: IPA as the mobile phase. Evaporation of pure fractions afforded pure diastereomers. Yields of the isomers were 28 mg, 5.73 %) and (54 mg, 11.06 %). The NMR spectra and LC/MS of the isomers were $^1$H NMR (400 MHz, MeOD): δ 6.39 (s, 1H), 4.71-4.65 (m, 1H), 3.12 (dd, J = 7.2, 10.4 Hz, 1H), 2.98-2.94 (dd, J = 7.6, 9.2 Hz, 1H), 2.60-2.51 (m, 1H), 2.41 (s, 3H), 2.28 (t, J = 9.2 Hz, 1H), 2.21-2.14 (m, 1H), 1.18-1.13 (m, 2H), 1.00-0.98 (m, 5H): LCMS: *m/z* = 249.9 $[M+H]^+$, and $^1$H NMR (400 MHz, MeOD): δ 6.38 (s, 1H), 4.16-4.12 (m, 1H), 3.02 (dd, J = 7.6, 8.8 Hz, 1H), 2.88 (dd, J = 7.6,10.4 Hz, 1H), 2.70 (dd, J = 5.2, 10 Hz, 1H), 2.39 (s, 3H), 2.28-2.15 (m, 3H), 1.18-1.12 (m, 5H), 1.00-0.97 (m, 2H); LCMS: *m/z* = 250.40 $[M+H]^+$.

EXAMPLE 4

Synthesis of (±)-*cis*-5-cyclopropyl-N-(1-cyclopropyl-4-(hydroxymethyl)pyrrolidin-3-yl)isoxazole-3-carboxamide (Cpd. No. 48)

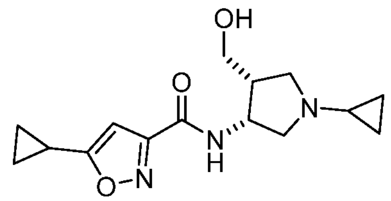

Step 1: Synthesis of methyl N-allyl-N-(tert-butoxycarbonyl)glycinate

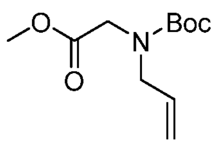

To a solution of BOC-Gly-OMe (25 g, 132 mmol) in N,N-dimethylformamide (250 mL) at -10°C under nitrogen was added sodium hydride (60% in mineral oil) (13 g, 198 mmol) portion wise over period of 30 min. The solution was allowed to stir at -10°C for 15 min and then allyl bromide (24 g, 198 mmol) was added over period of 10 min. The reaction mixture was then stirred at 0-5 °C for three hours at which point TLC (30% ethyl acetate hexane) showed complete consumption of starting material. The reaction was quenched slowly with aqueous saturated ammonium chloride (200 mL) then further diluted with water (500 mL) and extracted with diethyl ether (3 x 500mL). The combined organic layers were washed with water (3 x 500 mL) to remove any DMF then dried over sodium sulfate and evaporated to afford methyl N-allyl-N-(tert-butoxycarbonyl)glycinate (25g, 82.64 %) as a pale yellow oil. The material was used without further purification. $^1$H NMR (400 MHz, CDCl3): δ 5.85-5.74 (m, 1H), 5.19-5.07 (m, 2H), 3.97-3.82 (m, 4H), 3.74 (s,3H), 1.48-1.45 (d, J = 12 Hz, 9H).

Step-2: Synthesis of tert-butyl allyl(2-oxoethyl)carbamate

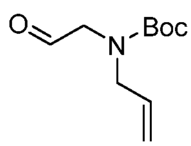

To a solution of methyl N-allyl-N-(tert-butoxycarbonyl)glycinate (2 g, 8.7 mmol) in mixture of n-hexane (40 mL) and diethyl ether (10 mL) at -78 °C was added DIBAL-H (1M in cyclohexane (0.185 g, 13 mmol) keeping the internal

temperature below -65 °C. The reaction was further stirred for an hour at -78 °C. Once the reaction appeared complete by TLC (30% ethyl acetate/hexanes), the reaction mixture was quenched with methanol (2 mL) followed by 200 mL of sat aq. solution of potassium sodium tartarate. The mixture was stirred for 1 hr at room temperature. The organic layer was removed and the aqueous layer was extracted with diethyl ether. The combined organic layer was dried over sodium sulfate and evaporated to afford the tert-butyl allyl(2-oxoethyl)carbamate (1.8g, quantitative) as pale yellow oil which was used without further purification.

Step 3: Synthesis of (E)-benzaldehyde oxime

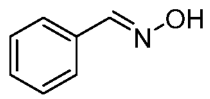

To a solution containing mixture of benzaldehyde (15 g, 141 mmol) and hydroxylamine.HCl (29.5 g, 420 mmol) in ethanol (450 mL) was added sodium hydroxide (50 g, 1270 mmol, 20% aqueous solution). The reaction mixture stirred at room temperature for 30min and then at 75 °C for 1hr. The reaction was allowed to cool to room temperature and was then quenched with mixture of water and conc. HCl. The reaction was concentrated to remove the ethanol and the remaining material was extracted with DCM (3 x 250mL). The combined organic layer was dried over sodium sulfate and concentrated to get (E)-benzaldehyde oxime (16g, 94%) as an oil. The material was used without further purification.

Step 4: Synthesis of N-benzylhydroxylamine

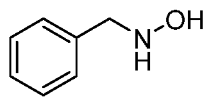

To a solution of (E)-benzaldehyde oxime (16 g, 130 mmol) and sodium cyanoborohydride (15 g, 190 mmol) in methanol (200ml) at 0 °C was added conc. HCl (20 mL, 260 mmol) drop wise. After the addition was complete the reaction mixture stirred at room temperature for 3 hrs. The reaction mixture was brought to pH 9 with 6N NaOH and concentrated under vacuum to remove MeOH. The desired product was extracted in DCM and the organic layer was washed with brine, dried over sodium sulfate and concentrated to give an oil. The material was triturated using ethyl acetate and hexanes to afford N-benzylhydroxylamine as a white solid. (8 g, 49%) $^1$H NMR (400 MHz, DMSO-$d_6$): δ 7.35-7.20 (m, 5H), 5.99 (s, 1H), 3.861 (s, 2H). LCMS: *m/z* = 124.0 $[M+H]^+$.

Step 5: Synthesis of tert-butyl 1-benzyltetrahydro-1H-pyrrolo[3,4-c]isoxazole-5(3H)-carboxylate

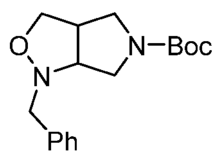

To a suspension of N-benzylhydroxylamine (1.33 g, 10 mmol) in DCM (40 mL) was added $MgBr_2$ (2 g, 10 mmol). After stirring the mixture for 15 min, 2-propanol (0.65g, 10 mmol) was added and after stirring for an additional 10 min. a solution of tert-butyl allyl(2-oxoethyl)carbamate (1.8 g, 9 mmol) in DCM (10 mL) was added. The mixture was stirred at 35 °C for 3 hrs, poured into 5% aqueous $NaHCO_3$/ice and extracted with DCM. The combined organic extract was washed with water, brine, dried over $Na_2SO_4$ and evaporated. The residue was purified by flash column chromatography on silica gel, eluting with ethyl acetate-hexanes 1:4, to yield tert-butyl 1-benzyltetrahydro-1H-pyrrolo[3,4-c]isoxazole-5(3H)-carboxylate as an oil (1.8 g, 66%). $^1$H NMR (400 MHz, CDCl3): δ 7.40-7.30 (m, 5H), 4.25 (s, 1H), 4.15-3.24 (m, 9H), 1.46 (s, 9H); LCMS: *m/z* = 305.2 $[M+H]^+$.

Step 6: Synthesis of (±)-*cis*-tert-butyl-3-amino-4-(hydroxymethyl)pyrrolidine-1-carboxylate

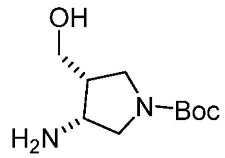

To a solution of tert-butyl 1-benzyltetrahydro-1H-pyrrolo[3,4-c]isoxazole-5(3H)-carboxylate (1.8 g, 5.9 mmol) in methanol (50 mL) was added 20 wt. % $Pd(OH)_2$ oncCarbon (500 mg). Hydrogen gas was bubbled through the solution until the starting material was consumed as judged by TLC. The reaction mixture was filtered through a pad of celite and the filtrate was evaporated under vacuum to afford (±)-*cis*-tert-butyl-3-amino-4-(hydroxymethyl)pyrrolidine-1-carboxylate as an oil. (1.4 g, quant.). $^1H$ NMR (400 MHz, CDCl3): δ 3.83-3.81 (m, 2H), 3.68 (s, 1H), 3.58-3.48 (m, 1H), 3.46-3.41 (m, 2H), 3.30-3.19 (m, 2H), 2.39-2.31 (m, 1H), 1.46 (s, 9H); LCMS: *m/z* = 217.1 $[M+H]^+$.

Step 7: Synthesis of (±)-*cis*-tert-butyl-3-(5-cyclopropylisoxazole-3-carboxamido)-4-(hydroxymethyl)pyrrolidine-1-carboxylate

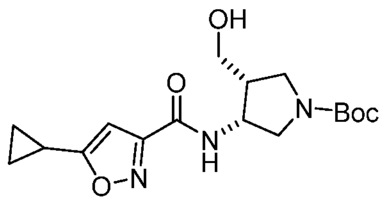

To a solution of 5-cyclopropylisoxazole-3-carboxylic acid (420 mg, 2.7 mmol) in DMF (2.0 mL) at 0 °C, was added HATU (1.31g, 3.4mmol). The reaction was stirred for 20 min at room temperature. After being cooled to 0 °C, tert-butyl (*cis*-racemic)-3-amino-4-(hydroxymethyl)pyrrolidine-1-carboxylate (500 mg, 2.3 mmol) and DIPEA (1.5 g, 11 mmol) were added to the reaction mixture. It was further stirred at room temperature for 2 hrs. Completion of the reaction was confirmed by TLC. After completion, water (50 mL) was added and the product was extracted with ethyl acetate (2 x 30mL). The combined organic layer was washed with brine, dried over sodium sulfate and concentrated under vacuum to get crude product which further purified by flash column chromatography using 70-80% EA:Hex as the eluent system to afford (±)-*cis*tert-butyl-3-(5-cyclopropylisoxazole-3-carboxamido)-4-(hydroxymethyl)pyrrolidine-1-carboxylate as an oil. (500 mg, 61%). $^1H$NMR (400 MHz, CDCl3): δ 7.23-7.09 (m, 1H), 6.36 (s, 1H), 4.68-4.61 (m, 1H), 3.49-3.71 (m, 6H), 2.98-2.82 (m, 1H), 2.72-2.58 (bs, 1H), 2.15-2.06 (m, 1H), 1.45 (s, 9H), 1.18-1.12 (m, 2H), 1.04-1.01 (m, 2H); LCMS: m/z = 352.4 $[M+H]^+$.

Step-8: Synthesis of (±)-*cis*-5-cyclopropyl-N-(4-(hydroxymethyl)pyrrolidin-3-yl)isoxazole-3-carboxamide

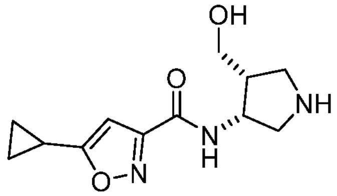

To a solution of (±)-*cis*-tert-butyl-3-(5-cyclopropylisoxazole-3-carboxamido)-4-(hydroxymethyl)pyrrolidine-1-carboxylate (250 mg, 0.71 mmol) in DCM (4 mL) was added trifluoroacetic acid (811 mg, 7.1 mmol). The reaction stirred at room temperature for 2 hrs. The reaction mixture was concentrated to dryness and the residue was used without further purification (800mg).

Step-9: Synthesis of (±)-*cis*-5-cyclopropyl-N-(1-cyclopropyl-4-(hydroxymethyl)pyrrolidin-3-yl)isoxazole-3-carboxamide

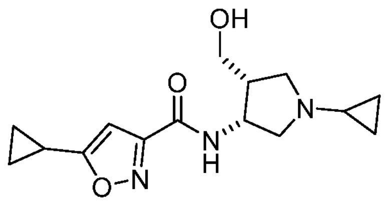

To a solution of (±)-*cis*-5-cyclopropyl-N-(4-(hydroxymethyl)pyrrolidin-3-yl)isoxazole-3-carboxamide (250 mg, 0.68 mmol) in methanol (5 mL) were added (1-ethoxy cyclopropoxy)trimethylsilane (131 mg, 0.75 mmol), acetic acid (0.3 mL) and sodium cyanoborohydride (210 mg, 3.4 mmol). The reaction mixture was heated to 60 °C overnight. After being cooled to room temperature the reaction mixture was quenched with sat. sodium bicarbonate and extracted in DCM. The organic layer was dried over sodium sulfate and concentrated under vacuum to get crude product which was subjected to purification by flash column chromatography, using silica gel and 2%MeOH: DCM as eluent system, to afford (±)-*cis*-5-cyclopropyl-N-(1-cyclopropyl-4-(hydroxymethyl)pyrrolidin-3-yl)isoxazole-3-carboxamide as an oil. (120 mg, 60%). $^1$H NMR (400 MHz, CDCl3): δ 7.38 (s, 1H), 6.34 (s, 1H), 4.75-4.70 (m, 1H), 3.64-3.55 (m, 2H), 2.97-2.87 (m,2H), 2.76-2.74 (m, 2H), 2.65-2.59 (m, 1H), 2.12-2.06 (m, 1H) , 1.73-1.69 (m, 1H), 1.16-1.11 (m, 2H), 1.02-0.98 (m, 2H), 0.47-0.38 (m, 4H); LCMS: *m/z* = 292.3 [M+H]$^+$.

EXAMPLE 8

SMYD3 Biochemical Assay

General Materials

S-adenosylmethionine (SAM), S-adenosylhomocysteine (SAH), Tris, Tween20, dimethylsulfoxide (DMSO), bovine skin gelatin (BSG), and Tris(2-carboxyethyl)phosphine hydrochloride solution (TCEP) were purchased from Sigma-Aldrich at the highest level of purity possible. $^3$H-SAM was purchase from American Radiolabeled Chemicals with a specific activity of 80 Ci/mmol. 384-well opaque white OptiPlates and SPA beads (Perkin Elmer, catalog # RPNQ0013) were purchased from PerkinElmer.

Substrates

N-terminally GST-tagged MEKK2 (MAP3K2) protein corresponding to reference sequence AAF63496.3 was purchased from Life Technologies (catalog # PV4010). This protein was expressed in High Five insect cells and purified to >85 % purity. Protein identity was confirmed by MS/MS analysis after proteolytic digestion. The protein sequence used was:

MAPILGYWKIKGLVQPTRLLLEYLEEKYEEHLYERDEGDKWRNK
KFELGLEFPNLPYYIDGDVKLTQSMAIIRYIADKHNMLGGCPKERA
EISMLEGAVLDIRYGVSRIAYSKDFETLKVDFLSKLPEMLKMFEDR
LCHKTYLNGDHVTHPDFMLYDALDVVLYMDPMCLDAFPKLVCF
KKRIEAIPQIDKYLKSSKYIAWPLQGWQATFGGGDHPPKSDLVPRH
NQTSLYKKAGTMDDQQALNSIMQDLAVLHKASRPALSLQETRKA
KSSSPKKQNDVRVKFEHRGEKRILQFPRPVKLEDLRSKAKIAFGQS
MDLHYTNNELVIPLTTQDDLDKALELLDRSIHMKSLKILLVINGST

QATNLEPLPSLEDLDNTVFGAERKKRLSIIGPTSRDRSSPPPGYIPDE
LHQVARNGSFTSINSEGEFIPESMEQMLDPLSLSSPENSGSGSCPSL
DSPLDGESYPKSRMPRAQSYPDNHQEFSDYDNPIFEKFGKGGTYPR
RYHVSYHHQEYNDGRKTFPRARRTQGNQLTSPVSFSPTDHSLSTSS
GSSIFTPEYDDSRIRRRGSDIDNPTLTVMDISPPSRSPRAPTNWRLG
KLLGQGAFGRVYLCYDVDTGRELAVKQVQFDPDSPETSKEVNAL
ECEIQLLKNLLHERIVQYYGCLRDPQEKTLSIFMEYMPGGSIKDQL
KAYGALTENVTRKYTRQILEGVHYLHSNMIVHRDIKGANILRDST
GNVKLGDFGASKRLQTICLSGTGMKSVTGTPYWMSPEVISGQGYG
RKADIWSVACTVVEMLTEKPPWAEFEAMAAIFKIATQPTNPKLPP
HVSDYTRDFLKRIFVEAKLRPSADELLRHMFVHYH.
(SEQ ID No. 1).

Molecular Biology

Full-length human SMYD3 isoform 1 (BAB86333) was inserted into a modified pET21b plasmid containing a His6 tag and TEV and SUMO cleavage sites. Because two common variants of SMYD3 exist in the population, site directed mutagenesis was subsequently performed to change amino acid 13 from an asparagine to a lysine, resulting in plasmid pEPZ533. A lysine at position 13 conforms to the more commonly occurring sequence (NP_001161212).

Protein Expression

*E. coli* (BL21 codonplus RIL strain, Stratagene) were transformed with plasmid pEPZ553 by mixing competent cells and plasmid DNA and incubating on ice for 30 minutes followed by heat shock at 42 °( for 1 minute and cooling on ice for 2 minutes. Transformed cells were grown and selected on LB agar with 100 μg/mL ampicillin and 17 μg/mL chloramphenicol at 37 °C overnight. A single clone was used to inoculate 200 mL of LB medium with 100 μg/mL ampicillin and 17 μg/mL chloramphenicol and incubated at 37 °C on an orbital shaker at 180 rpm. Once in log growth, the culture was diluted 1:100 into 2 L of LB medium and grown until $OD_{600}$ was about 0.3 after which the culture was incubated at 15 °C and 160 rpm. Once $OD_{600}$ reached about 0.4, IPTG was added to a final concentration of 0.1 mM and the cells were grown overnight at 15 °C and 160 rpm. Cells were harvested by centrifugation at 8000 rpm, for 4 minutes at 4 °C and stored at -80 °C for purification.

Protein Purification

Expressed full-length human His-tagged SMYD3 protein was purified from cell paste by Nickel affinity chromatography after equilibration of the resin with Buffer A (25 mM Tris, 200 mM NaCl, 5% glycerol, 5 mM β-mercaptoethanol, pH7.8). The column was washed with Buffer B (Buffer A plus 20 mM imidazole) and His-tagged SMYD3 was eluted with Buffer C (Buffer A plus 300 mM imidazole). The His tag, TEV and SUMO cleavage sites were removed generating native SMYD3 by addition of ULP1 protein at a ratio of 1:200 (ULP1:SMYD3). Imidazole was removed by dialysis overnight in Buffer A. The dialyzed solution was applied to a second Nickel column and the native SMYD3 protein was collected from the column flow-through. The flow-through was dialyzed in Buffer D (25 mM Tris, 5% glycerol, 5 mM β-mercaptoethanol, 50 mM NaCl, pH7.8) and ULP1 was removed using a Q sepharose fast flow column. SMYD3 was eluted in Buffer A and further purified using an S200 size-exclusion column equilibrated with Buffer A. SMYD3 was concentrated to 2 mg/mL with a final purity of 89%.

Predicted Translation:

SMYD3 (Q9H7B4)

MEPLKVEKFATAKRGNGLRAVTPLRPGELLFRSDPLAYTVCKGSR
GVVCDRCLLGKEKLMRCSQCRVAKYCSAKCQKKAWPDHKRECK
CLKSCKPRYPPDSVRLLGRVVFKLMDGAPSESEKLYSFYDLESNIN
KLTEDKKEGLRQLVMTFQHFMREEIQDASQLPPAFDLFEAFAKVIC
NSFTICNAEMQEVGVGLYPSISLLNHSCDPNCSIVFNGPHLLLRAV
RDIEVGEELTICYLDMLMTSEERRKQLRDQYCFECDCFRCQTQDK
DADMLTGDEQVWKEVQESLKKIEELKAHWKWEQVLAMCQAIISS
NSERLPDINIYQLKVLDCAMDACINLGLLEEALFYGTRTMEPYRIFF
PGSHPVRGVQVMKVGKLQLHQGMFPQAMKNLRLAFDIMRVTHG
REHSLIEDLILLLEECDANIRAS. (SEQ ID No. 2).

General Procedure for SMYD3 Enzyme Assays on MEKK2 protein substrate

The assays were all performed in a buffer consisting of 25 mM Tris-Cl pH 8.0, 1 mM TCEP, 0.005% BSG, and 0.005% Tween 20, prepared on the day of use. Compounds in 100% DMSO (lul) were spotted into a 384-well white opaque OptiPlate using a Bravo automated liquid handling platform outfitted with a 384-channel head (Agilent Technologies). DMSO (lul) was added to Columns 11, 12, 23, 24, rows A-H for the maximum signal control and 1ul of SAH, a known product and inhibitor of SMYD3, was added to columns 11, 12, 23, 24, rows I-P for the minimum signal control. A cocktail (40ul) containing the SMYD3 enzyme was added by Multidrop Combi (Thermo-Fisher). The compounds were allowed to incubate with SMYD3 for 30 min at room temperature, then a cocktail (10ul) containing SAM and MEKK2 was added to initiate the reaction (final volume = 51ul). The final concentrations of the components were as follows: SMYD3 was 0.4 nM, $^3$H-SAM was 8 nM, MEKK2 was 12 nM, SAH in the minimum signal control wells was 1 mM, and the DMSO concentration was 2%. The assays were stopped by the addition of non-radiolabeled SAM (10ul) to a final concentration of 100 uM, which dilutes the $^3$H-SAM to a level where its incorporation into MEKK2 is no longer detectable. Radiolabeled MEKK2 was detected using a scintillation proximity assay (SPA). 10 uL of a 10 mg/mL solution of SPA beads in 0.5 M citric acid was added and the plates centrifuged at 600 rpm for 1 min to precipitate the radiolabeled MEKK2 onto the SPA beads. The plates were then read in a PerkinElmer TopCount plate reader to measure the quantity of $^3$H-labeled MEKK2 as disintegrations per minute (dpm) or alternatively, referred to as counts per minute (cpm).
% inhibition calculation

$$\% \, inh = 100 - \left(\frac{dpm_{cmpd} - dpm_{min}}{dpm_{max} - dpm_{min}}\right) \times 100$$

Where dpm = disintegrations per minute, cmpd = signal in assay well, and min and max are the respective minimum and maximum signal controls.
Four-parameter IC50 fit

$$Y = Bottom + \frac{(Top - Bottom)}{(1 + (\frac{X}{IC_{50}})^{Hill\ Coefficient}}$$

Where top and bottom are the normally allowed to float, but may be fixed at 100 or 0 respectively in a 3-parameter fit. The Hill Coefficient normally allowed to float but may also be fixed at 1 in a 3-parameter fit. Y is the % inhibition and X is the compound concentration.

SMYD3 biochemical assay data for representative Compounds of the Disclosure are presented in Table 1 in the column titled "SMYD3 Biochem IC50 (μM)."

EXAMPLE 9

SMYD3 Cell Assay

Trimethyl-MEKK2-In-Cell Western Assay

293T/17 adherent cells were purchased from ATCC (American Type Culture Collection), Manassas, VA, USA. MEM/Glutamax medium, Optimem Reduced Serum medium, penicillin-streptomycin, 0.05% trypsin and 1x D-PBS were purchased from Life Technologies, Grand Island, NY, USA. PBS-10X was purchased from Ambion, Life Technologies, Grand Island, New York, USA. PBS with Tween 20 (PBST (10x)) was purchased from KPL, Gaithersburg, Maryland,USA. Tet System FBS- approved FBS US Source was purchased from Clontech, Mountain View, California, USA. Odyssey blocking buffer, 800CW goat anti-rabbit IgG (H+L) antibody, 680CW Goat anti-mouse IgG (H+L) and Licor Odyssey infrared scanner were purchased from Licor Biosciences, Lincoln, NE, USA. Tri-methyl-Lysine [A260]-MEKK2 antibody, MEKK2 and SMYD3 plasmids were made at Epizyme. Anti-flag monoclonal mouse antibody was purchased from Sigma, St. Louis, MO, USA. Methanol was purchased from VWR, Franklin, MA, USA. 10% Tween 20 was purchased from KPL, Inc., Gaithersburg, Maryland, USA. Fugene was purchased from Promega, Madison, WI, USA. The Biotek ELx405 was purchased from BioTek, Winooski, Vermont, USA. The multidrop combi was purchased from Thermo Scientific, Waltham, Massachusetts, USA.

293T/17 adherent cells were maintained in growth medium (MEM/Glutamax medium supplemented with 10% v/v Tet System FBS and cultured at 37 °C under 5% $CO_2$.

Cell treatment, In Cell Western (ICW) for detection of trimethyl-lysine-MEKK2 and MEKK2.

293T/17 cells were seeded in assay medium at a concentration of 33,333 cells per $cm^2$ in 30 mL medium per T150 flask and incubated at 37 °C under 5% $CO_2$. Plasmids were prepared for delivery to cells by first mixing 1350 μL Opti-MEM with Fugene (81 μL) in a sterile Eppendorf and incubated for five minutes at room temperature (RT). MEKK2-flag (13.6 ug/T150) MEKK2 p3XFlag-CMV-14 with C-3XFlag and SMYD3 (0.151 ug/T150) SMYD3 p3XFlag-CMV-14 without C-3XFlag plasmids were aliquotted to a 1.7 mL sterile microfuge tube. The gene ID for MEKK2 and SMYD3 is NM_006609.3 and Q9H7B4, respectively. Entire volume of Opti-MEM/Fugene mixture was then added to a microfuge tube containing DNA plasmid, mixed and then incubated x 15 minutes at RT. The medium on the 293T/17 cells was refreshed, and the DNA/Fugene complex is added aseptically to each flask, rocked gently, and incubated at 37 C for 5 hours. Medium was then removed, and cells were washed once with PBS in the flask. Trypsin 0.05% (3mL) was added and cells incubated for three minutes. Room temperature MEM+10% Tet system FBS was added and cells were mixed gently, and counted using the Vi-cell. Cells were seeded at 100,000 cells/mL in 50 μL MEM/10%Tet FBS/Pen/Strep to a 384 well black/clear poly-D-lysine coated plate containing test agent diluted in DMSO. The final top concentration of test compound was 40 μM. The total concentration of DMSO did not exceed 0.2% (v/v). Plates were incubated x 30 minutes at RT in low-airflow area, followed by incubation at 37 °C under 5% $CO_2$ for 24 hours. Medium was aspirated from all wells of assay plates prior to fixation and permeabilization with ice cold (-20 °C) methanol (90 μL/well) for ten minutes. Plates were rinsed with PBS three times on BioTek ELx405. PBS was removed with a final aspiration, and Odyssey blocking buffer (50 μL/well) was added to each well and incubated for one hour at RT. Primary antibody solution was prepared (anti-trimethyl-MEKK2 at 1:600 dilution plus mouse anti-flag antibody at 1:10,000 dilution in diluent (Odyssey Blocking buffer + 0.1% Tween 20)) and 20 μL per well was dispensed using the Multidrop Combi. Assay plates were then sealed with foil, and incubated overnight at 4° C. Plates were washed five times with PBS-Tween (IX) on Biotek ELx405 and blotted on paper towel to remove excess reagent. Detection antibody solution (IRDye 800 CW goat anti-rabbit IgG diluted 1:400 in diluent (Odyssey Blocking buffer + 0.1% Tween 20), plus IRDye 680CW goat anti-mouse IgG at 1:500 in diluent (Odyssey Blocking buffer + 0.1% Tween 20) was added (20 μL/well) and incubated in dark for one hour at RT. Plates were then washed four times with PBS-T (IX) on ELx405. A final rinse with water was performed (115 μL/well x three washes on the ELx405). Plates were then centrifuged upside down, on paper towel, at 200 x g to remove excess reagent. Plates were left to dry in dark for one hour. The Odyssey Imager was used to measure the integrated intensity of 700 and 800 wavelengths at resolution of 84 μm, medium quality, focus offset 4.0, 700 channel intensity = 3.5 to measure the MEKK2-flag signal, 800 channel intensity = 5 to measure the Trimethyl-MEKK2 signal of each well.

Calculations:

First, the ratio for each well was determined by:

$$\left(\frac{Trimethyl\ MEKK2\ 800nm\ value}{flag\ tagged\ MEKK2\ 700nm\ value}\right)$$

Each plate included fourteen control wells of DMSO only treatment (Minimum Inhibition) as well as fourteen control wells for maximum inhibition (Background). The average of the ratio values for each control type was calculated and used to determine the percent inhibition for each test well in the plate. Reference compound was serially diluted two-fold in DMSO for a total of nine test concentrations, beginning at 40 $\mu$M. Percent inhibition was calculated (below).

$$\text{Percent Inhibition} = 100\text{-}\left(\left(\frac{(\text{Individual Test Sample Ratio})-(\text{Background Avg Ratio})}{(\text{Minimum Inhibition Ratio})-(\text{Background Average Ratio})}\right)*100\right)$$

Non-linear regression curves were generated to calculate the $IC_{50}$ and dose-response relationship using triplicate wells per concentration of compound.

EXAMPLE 10

SMYD2 Biochemical Assay

General Materials

S-adenosylmethionine (SAM), S-adenosylhomocysteine (SAH), bicine, Tween20, dimethylsulfoxide (DMSO), bovine skin gelatin (BSG), and Tris(2-carboxyethyl)phosphine hydrochloride (TCEP) were purchased from Sigma-Aldrich at the highest level of purity possible. $^3$H-SAM was purchase from American Radiolabeled Chemicals with a specific activity of 80 Ci/mmol. 384-well streptavidin Flashplates were purchased from PerkinElmer.

Substrates

Peptide was synthesized with a N-terminal linker-affinity tag motif and a C-terminal amide cap by 21$^{st}$ Century Biochemicals. The peptide was high highperfomance liquid chromatography (HPLC) purified to greater than 95% purity and confirmed by liquid chromatography mass spectrometry (LC-MS). The sequence was ARTKQTARKSTGGKAPRKQLATKAARKSA(K-Biot)-amide. (SEQ ID No: 3)

Production of Recombinant SMYD2 Enzymes for Biochemical Enzyme Activity Assays

Full length SMYD2 (NP_064582.2) was cloned into a pFastbac-Htb-lic vector with an N-terminal His6 *tag* and *FLAG tag,* preceded by a *TEV* protease *cleavage* site. The protein was expressed in Sf9 insect cells. Cells were resuspended in lysis buffer (25 mM HEPES-NaOH, pH 7.5, 200 mM NaCl, 5% glycerol, and 5 mM $\beta$-ME) and lysed by sonication. The protein was purified by Ni-NTA (Qiagen), followed by TEV cleavage to remove the His6 tag, subtractive Ni-NTA (Qiagen), and gel filtration chromatography using an S200 column (GE Healthcare). Purified protein was stored in 20 mM Tris-HCl, pH 8.0, 100 mM NaCl, and 1 mM TCEP.

General Procedure for SMYD2 Enzyme Assays on Peptide Substrates

The assays were all performed in a buffer consisting of 20mM Bicine (pH=7.6), ImM TCEP, 0.005% Bovine Skin Gelatin, and 0.002% Tween20, prepared on the day of use. Compounds in 100% DMSO (lul) were spotted into a polypropylene 384-well V-bottom plates (Greiner) using a Platemate Plus outfitted with a 384-channel head (Thermo Scientific). DMSO (lul) was added to Columns 11, 12, 23, 24, rows A-H for the maximum signal control and 1ul of SAH, a known product and inhibitor of SMYD2, was added to columns 11, 12, 23, 24, rows I-P for the minimum signal control. A cocktail (40ul) containing the SMYD2 enzyme was added by Multidrop Combi (Thermo-Fisher). The compounds were allowed to incubate with SMYD2 for 30 min at room temperature, then a cocktail (10ul) containing $^3$H-SAM and peptide was added to initiate the reaction (final volume = 51ul). The final concentrations of the components were as follows: SMYD2 was 1.5nM, $^3$H-SAM was 10nM, and peptide was 60nM, SAH in the minimum signal control wells was 1000uM, and the DMSO concentration was 2%. The assays were stopped by the addition of non-radioactive SAM (10ul) to a final concentration of 600uM, which dilutes the $^3$H-SAM to a level where its incorporation into the peptide substrate is no longer detectable. 50ul of the reaction in the 384-well polypropylene plate was then transferred to a 384-well Flashplate and the biotinylated peptides were allowed to bind to the streptavidin surface for at least 1 hour before being washed three times with 0.1%Tween20 in a Biotek ELx405 plate washer. The plates were then read in a PerkinElmer TopCount plate reader to measure the quantity of $^3$H-labeled peptide bound to the Flashplate surface, measured as disintegrations per minute (dpm) or alternatively, referred to as counts per minute (cpm).
% inhibition calculation

$$\%\,inh = 100 - \left(\frac{dpm_{cmpd} - dpm_{min}}{dpm_{max} - dpm_{min}}\right) \times 100$$

Where dpm = disintegrations per minute, cmpd = signal in assay well, and min and max are the respective minimum and maximum signal controls.
Four-parameter IC50 fit

$$\%\,inhibition = Bottom + \frac{Top - Bottom}{\left(1 + \left(IC_{50}/[I]\right)^{Hill\,coefficient}\right)}$$

Where top and bottom are the normally allowed to float, but may be fixed at 100 or 0 respectively in a 3-parameter fit. The Hill Coefficient normally allowed to float but may also be fixed at 1 in a 3-parameter fit. *I* is the compound concentration.
SMYD2 biochemical assay data for representative Compounds of the Disclosure are presented in Tables 1-3 in the column titled "SMYD2 Biochem $IC_{50}$ (μM)."
Having now fully described this invention, it will be understood by those of ordinary skill in the art that the same can be performed within a wide and equivalent range of conditions, formulations, and other parameters without affecting the scope of the invention or any embodiment thereof.
Other embodiments of the invention will be apparent to those skilled in the art from consideration of the specification and practice of the invention disclosed herein. It is intended that the specification and examples be considered as exemplary only, with a scope of the invention being indicated by the following claims.

## Claims

1. A compound for use in treating cancer, the compound having Formula I:

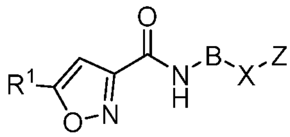

I

or a pharmaceutically acceptable salt or hydrate thereof, wherein:

B is:

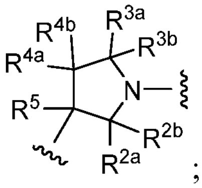

;

X is selected from the group consisting of $-S(=O)_2-$, $-S(=O)_2N(R^6)-$, $-S(=O)_2C(R^7)(H)-$, -C(=O)-, $-C(=O)N(R^6)-$, -C(=O)O-, and $-C(=O)C(R^7)(H)-$; or X is absent;

Z is selected from the group consisting of hydrogen, optionally substituted $C_{1-6}$ alkyl, haloalkyl, (cycloalkyl)alkyl, hydroxyalkyl, (amino)alkyl, (alkylamino)alkyl, (dialkylamino)alkyl, (cycloalkylamino)alkyl, (heterocyclo)alkyl, (amino)(hydroxy)alkyl, (amino)(aryl)alkyl, (hydroxy)(aryl)alkyl, (aralkylamino)alkyl, alkoxyalkyl, optionally substituted $C_{6-14}$ aryl, optionally substituted 4- to 14-membered heterocyclo, optionally substituted $C_{5-14}$ heteroaryl, optionally substituted $C_{3-12}$ cycloalkyl, aralkyl, heteroaralkyl, and $-CH_2N(H)C(=O)R^{8c}$;;

$R^1$ is selected from the group consisting of hydrogen, cyano, $C_{1-6}$ alkyl, haloalkyl, optionally substituted 4- to 14-membered heterocyclo, alkynyl, and $C_{3-6}$ cycloalkyl;

$R^{2a}$, $R^{2b}$, $R^{3a}$, $R^{3b}$, $R^{4a}$, and $R^{4b}$ are each independently selected from the group consisting of hydrogen, amino, alkylamino, dialkylamino, cycloalkylamino, halo, hydroxy, $C_{1-6}$ alkyl, alkoxy, haloalkyl, hydroxyalkyl, (amino)alkyl, (alkylamino)alkyl, (dialkylamino)alkyl, (cycloalkylamino)alkyl, (heterocyclo)alkyl, optionally substituted $C_{3-12}$ cycloalkyl, optionally substituted $C_{6-14}$ aryl, optionally substituted 4- to 14-membered heterocyclo, optionally substituted $C_{5-14}$ heteroaryl, alkoxyalkyl, aralkyl, alkoxycarbonyl, sulfonamido, carboxamido, $-N(H)C(=O)R^{8a}$; and $-CH2N(H)C(=O)R^{8b}$; or

$R^{2a}$ and $R^{2b}$ taken together with the carbon atom to which they are attached form a carbonyl; and $R^{3a}$, $R^{3b}$, $R^{4a}$, and $R^{4b}$ are each independently selected from the group consisting of hydrogen, hydroxy, $C_{1-6}$ alkyl, alkoxy, hydroxyalkyl, $C_{3-12}$ cycloalkyl, optionally substituted $C_{6-14}$ aryl, alkoxyalkyl, aralkyl, $-N(H)C(=O)R^{8a}$; and $-CH2N(H)C(=O)R^{8b}$;or

$R^{3a}$ and $R^{3b}$ taken together with the carbon atom to which they are attached form a carbonyl; and $R^{2a}$, $R^{2b}$, $R^{4a}$, and $R^{4b}$ are each independently selected from the group consisting of hydrogen, hydroxy, $C_{1-6}$ alkyl, alkoxy, hydroxyalkyl, $C_{3-12}$ cycloalkyl, optionally substituted $C_{6-14}$ aryl, alkoxyalkyl, $-N(H)C(=O)R^{8a}$; and $-CH2N(H)C(=O)R^{8b}$; or

$R^{4a}$ and $R^{4b}$ taken together with the carbon atom to which they are attached form a carbonyl; and $R^{2a}$, $R^{2b}$, $R^{3a}$, and $R^{3b}$ are each independently selected from the group consisting of hydrogen, hydroxy, $C_{1-6}$ alkyl, alkoxy, hydroxyalkyl, $C_{3-12}$ cycloalkyl, optionally substituted $C_{6-14}$ aryl, alkoxyalkyl, $-N(H)C(=O)R^{8a}$; and $-CH2N(H)C(=O)R^{8b}$; or

$R^{2a}$ and $R^{2b}$ taken together with the carbon atom to which they are attached form a $C_{3-6}$ cycloalkyl or $C_{3-6}$ heterocyclo; and $R^{3a}$, $R^{3b}$, $R^{4a}$, and $R^{4b}$ are each independently selected from the group consisting of hydrogen, hydroxy, $C_{1-6}$ alkyl, alkoxy, hydroxyalkyl, $C_{3-12}$ cycloalkyl, optionally substituted $C_{6-14}$ aryl, alkoxyalkyl, aralkyl, - $N(H)C(=O)R^{8a}$; and $-CH2N(H)C(=O)R^{8b}$;or

$R^{3a}$ and $R^{3b}$ taken together with the carbon atom to which they are attached form a $C_{3-6}$ cycloalkyl or $C_{3-6}$ heterocyclo; and $R^{2a}$, $R^{2b}$, $R^{4a}$, and $R^{4b}$ are each independently selected from the group consisting of hydrogen, hydroxy, $C_{1-6}$ alkyl, alkoxy, hydroxyalkyl, $C_{3-12}$ cycloalkyl, optionally substituted $C_{6-14}$ aryl, alkoxyalkyl, $-N(H)C(=O)R^{8a}$; and $-CH2N(H)C(=O)R^{8b}$;or

$R^{4a}$ and $R^{4b}$ taken together with the carbon atom to which they are attached form a $C_{3-6}$ cycloalkyl or $C_{3-6}$ heterocyclo; and $R^{2a}$, $R^{2b}$, $R^{3a}$, and $R^{3b}$ are each independently selected from the group consisting of hydrogen, hydroxy, $C_{1-6}$ alkyl, alkoxy, hydroxyalkyl, $C_{3-12}$ cycloalkyl, optionally substituted $C_{6-14}$ aryl, alkoxyalkyl, -N(H)C(=O)$R^{8a}$; and -CH2N(H)C(=O)$R^{8b}$;

$R^5$ is selected from the group consisting of hydrogen and $C_{1-4}$ alkyl;

$R^6$ is selected from the group consisting of hydrogen and $C_{1-4}$ alkyl;

$R^7$ is selected from the group consisting of hydrogen, $C_{1-4}$ alkyl, amino, alkylamino, dialkylamino, (amino)alkyl, (alkylamino)alkyl, (dialkylamino)alkyl, and hydroxyalkyl;

$R^{8a}$ is selected from the group consisting of $C_{1-6}$ alkyl, haloalkyl, hydroxyalkyl, (amino)alkyl, (alkylamino)alkyl, (dialkylamino)alkyl, (cycloalkylamino)alkyl, (heterocyclo)alkyl, optionally substituted $C_{6-14}$ aryl, optionally substituted 4- to 14-membered heterocyclo, optionally substituted 5- to 14-membered heteroaryl, optionally substituted $C_{3-12}$ cycloalkyl, aralkyl, and heteroaralkyl;

$R^{8b}$ is selected from the group consisting of $C_{1-6}$ alkyl, haloalkyl, hydroxyalkyl, (amino)alkyl, (alkylamino)alkyl, (dialkylamino)alkyl, (cycloalkylamino)alkyl, (heterocyclo)alkyl, optionally substituted $C_{6-14}$ aryl, optionally substituted 4- to 14-membered heterocyclo, optionally substituted 5- to 14-membered heteroaryl, optionally substituted $C_{3-12}$ cycloalkyl, aralkyl, and heteroaralkyl; and

$R^{8c}$ is selected from the group consisting of $C_{1-6}$ alkyl, haloalkyl, hydroxyalkyl, (amino)alkyl, (alkylamino)alkyl, (dialkylamino)alkyl, (cycloalkylamino)alkyl, (heterocyclo)alkyl, optionally substituted $C_{6-14}$ aryl, optionally substituted 4- to 14-membered heterocyclo, optionally substituted 5- to 14-membered heteroaryl, optionally substituted $C_{3-12}$ cycloalkyl, aralkyl, and heteroaralkyl.

**2.** The compound for use of claim 1, or a pharmaceutically acceptable salt or hydrate thereof, wherein B is selected from the group consisting of:

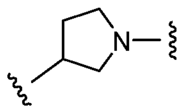 , 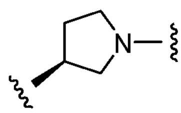 and 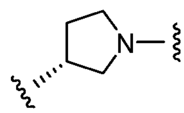 .

**3.** The compound for use of claim 1, or a pharmaceutically acceptable salt or hydrate thereof, wherein B is selected from the group consisting of:

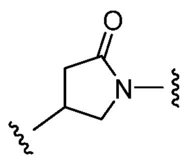 , 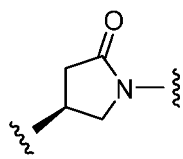 and 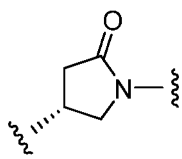 .

**4.** The compound for use of claim 1, or a pharmaceutically acceptable salt or hydrate thereof, wherein B is selected from the group consisting of:

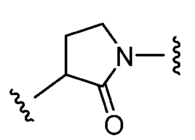 , 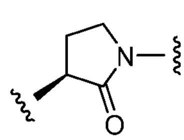 and 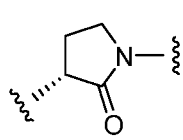 .

**5.** The compound for use of claim 1, or a pharmaceutically acceptable salt or hydrate thereof, wherein B is selected from:

(a) the group consisting of:

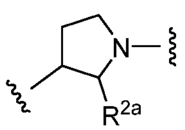 , 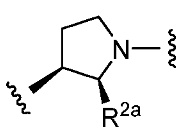 , 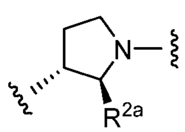 ,

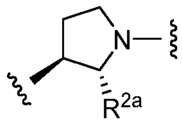 and 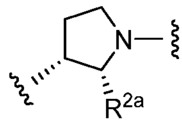 ;

and

$R^{2a}$ is selected from the group consisting of $C_{1-6}$ alkyl, $C_{3-12}$ cycloalkyl, and optionally substituted $C_{6-14}$ aryl, optionally wherein $R^{2a}$ is selected from the group consisting of:

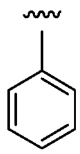 , 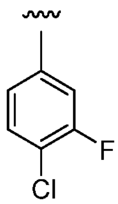 , 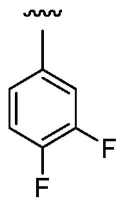 , 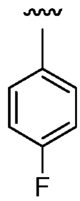 and 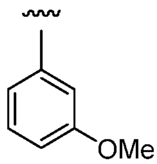 ;

or

(b) the group consisting of:

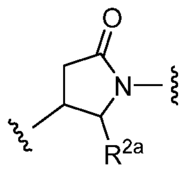 , 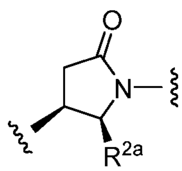 , 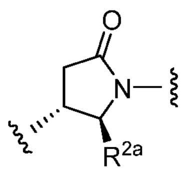 ,

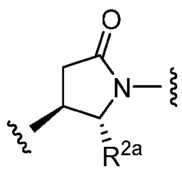 and 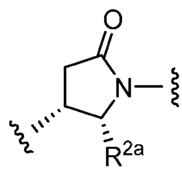 ;

and

$R^{2a}$ is selected from the group consisting of $C_{1-6}$ alkyl, $C_{3-12}$ cycloalkyl, and optionally substituted $C_{6-14}$ aryl, optionally wherein $R^{2a}$ is selected from the group consisting of:

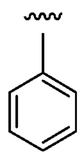 , 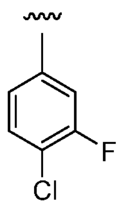 , 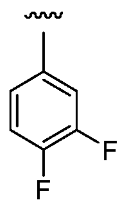 , 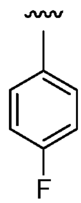 and 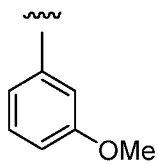 .

**6.** The compound for use of claim 1, or a pharmaceutically acceptable salt or hydrate thereof, wherein B is selected from:

(a) the group consisting of:

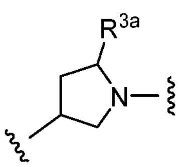 , 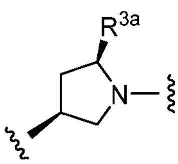 , 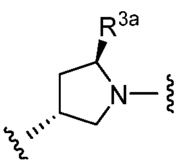 ,

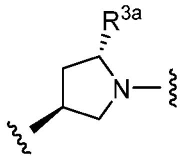 and 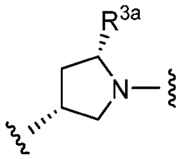 ;

and

$R^{3a}$ is selected from the group consisting of $C_{1-6}$ alkyl, hydroxyalkyl, $C_{3-12}$ cycloalkyl, optionally substituted $C_{6-14}$ aryl, alkoxyalkyl, and $-CH_2N(H)C(=O)R^{8b}$, optionally wherein $R^{3a}$ is selected from the group consisting of:

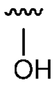 , 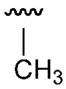 , 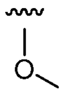 , 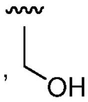 , 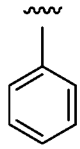 , 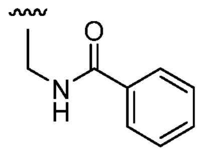 ,

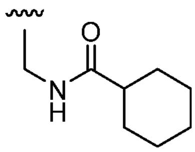 , 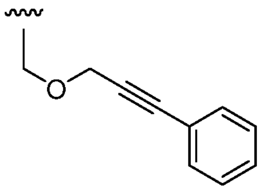 and 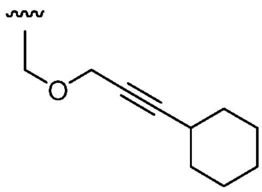 ;

or

(b) the group consisting of:

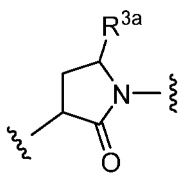 , 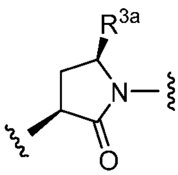 , 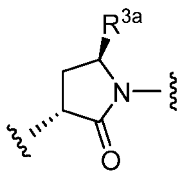 ,

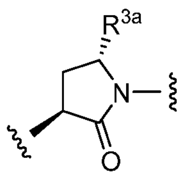 and 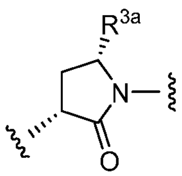 ;

and

$R^{3a}$ is selected from the group consisting of $C_{1-6}$ alkyl, hydroxyalkyl, $C_{3-12}$ cycloalkyl, optionally substituted $C_{6-14}$ aryl, alkoxyalkyl, and $-CH_2N(H)C(=O)R^{8b}$, optionally wherein $R^{3a}$ is selected from the group consisting of:

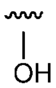 , 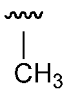 , 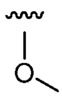 , 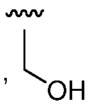 , 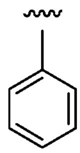 , 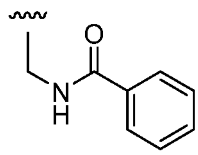 ,

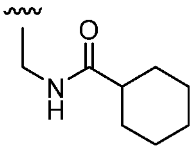, 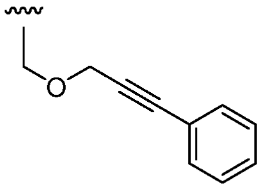 and 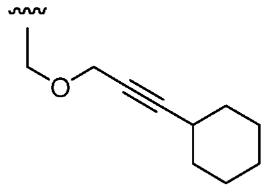;

or

(c) the group consisting of:

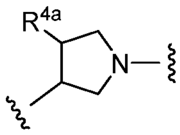, 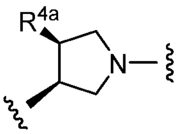, 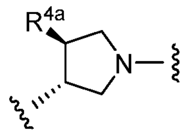,

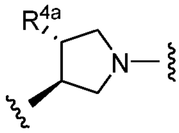 and 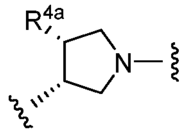;

and

$R^{4a}$ is selected from the group consisting of hydroxy, $C_{1-6}$ alkyl, alkoxy, hydroxyalkyl, $C_{3-12}$ cycloalkyl, optionally substituted $C_{6-14}$ aryl, alkoxyalkyl, aralkyl, - N(H)C(=O)$R^{8a}$, and -$CH_2$N(H)C(=O)$R^{8b}$, optionally wherein $R^{4a}$ is selected from the group consisting of:

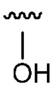, 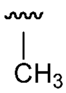, 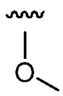, 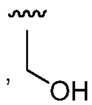, 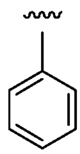, 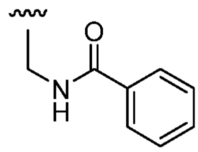,

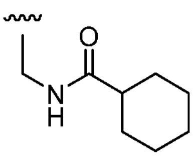, 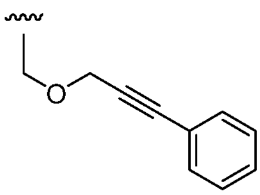 and 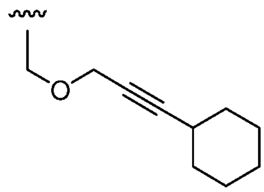;

or

(d) the group consisting of:

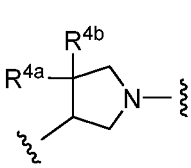, 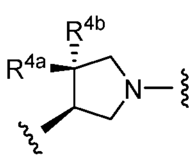, 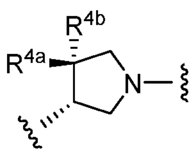,

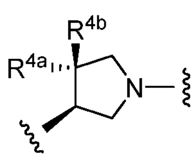 and 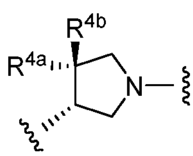;

$R^{4a}$ is $C_{1-4}$ alkyl; and $R^{4b}$ is hydroxy.

**7.** The compound for use of any one of claims 1-6, or a pharmaceutically acceptable salt or hydrate thereof, wherein X is:

(a) selected from the group consisting of $-S(=O)_2-$ and -C(=O)-; or X is absent; or
(b) $-S(=O)_2-$; or
(c) -C(=O)-; or
(d) absent.

**8.** The compound for use of any one of claims 1-7, or a pharmaceutically acceptable salt or solvate thereof, wherein Z is selected from the group consisting of optionally substituted $C_{1-6}$ alkyl, hydroxyalkyl, (amino)alkyl, (alkylamino)alkyl, (dialkylamino)alkyl, (cycloalkylamino)alkyl, (heterocyclo)alkyl, optionally substituted $C_{6-14}$ aryl, optionally substituted 4- to 14-membered heterocyclo, optionally substituted 5-to 14-membered heteroaryl, optionally substituted $C_{3-12}$ cycloalkyl, aralkyl, and heteroaralkyl, and optionally wherein Z is selected from the group consisting of optionally substituted $C_{1-6}$ alkyl, (amino)alkyl, (alkylamino)alkyl, (heterocyclo)alkyl, optionally substituted $C_{6-14}$ aryl, optionally substituted 4- to 14-membered heterocyclo, optionally substituted 5- to 14-membered heteroaryl, optionally substituted $C_{3-12}$ cycloalkyl, aralkyl, and heteroaralkyl.

**9.** The compound for use of claim 1, or a pharmaceutically acceptable salt or solvate thereof, wherein $R^1$ is ethyl or cyclopropyl; optionally wherein X is selected from the group consisting of $-S(=O)_2-$, $-S(=O)_2N(R^6)-$, $-S(=O)_2C(R^7)(H)-$, -C(=O)-, $-C(=O)N(R^6)-$, -C(=O)O-, and $-C(=O)C(R^7)(H)$.

**10.** The compound for use of claim 1, wherein the compound is selected from Table 1:

| Cpd. No. | Structure |
|---|---|
| **1** | 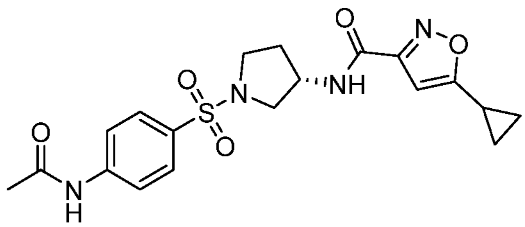 |
| **2** | 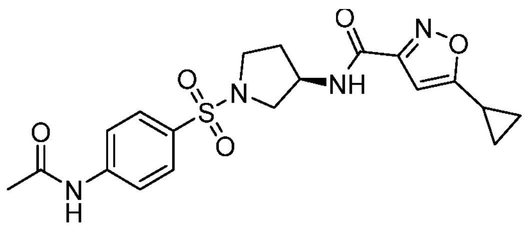 |
| **3** | 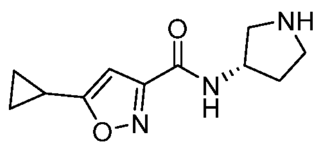 |
| **4** | 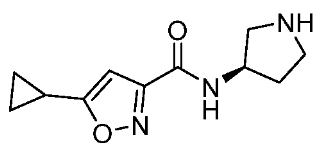 |

(continued)

| Cpd. No. | Structure |
|---|---|
| **5** | 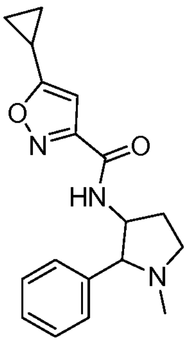 |
| **6** | 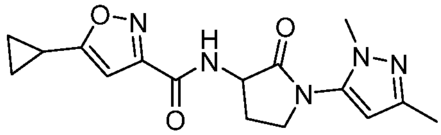 |
| **7** | 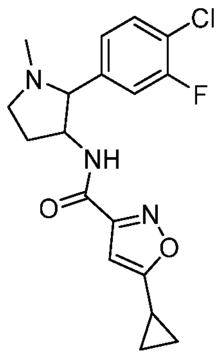 |
| **8** | 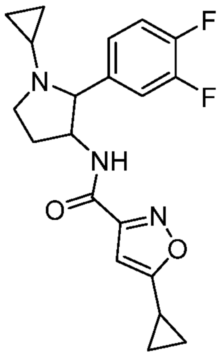 |
| **9** | 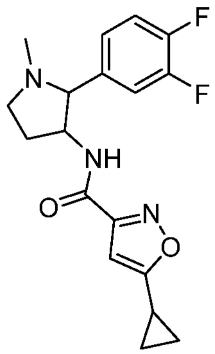 |

(continued)

| Cpd. No. | Structure |
|---|---|
| **10** | 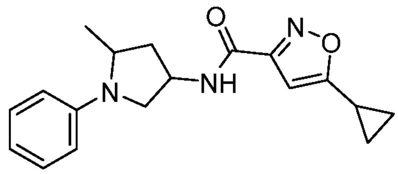 |
| **11** | 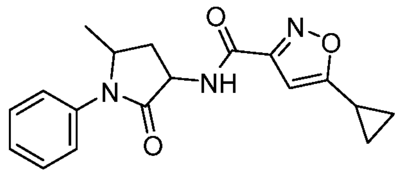 |
| **12** | 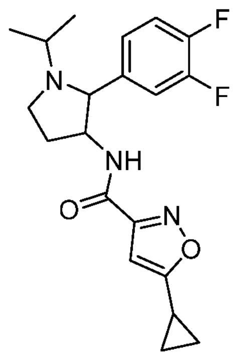 |
| **13** | 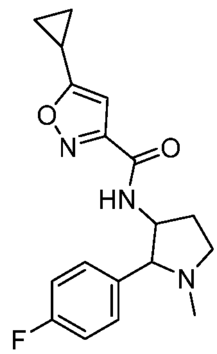 |
| **14** | 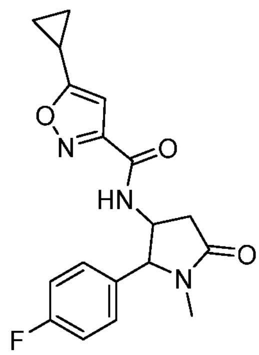 |
| **15** | 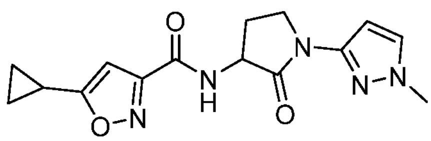 |

(continued)

| Cpd. No. | Structure |
|---|---|
| **16** | 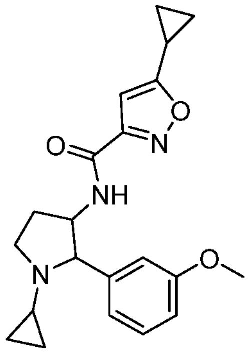 |
| **17** | 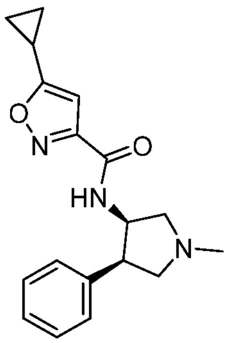 |
| **18** | 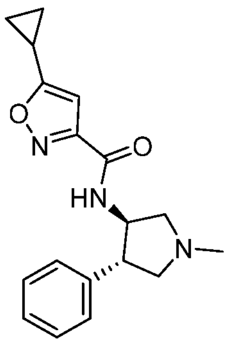 |
| **19** | 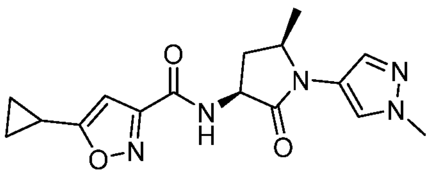 |
| **20** | 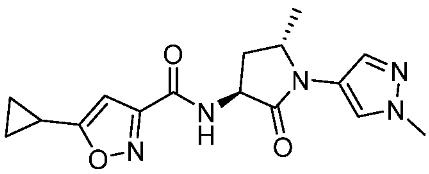 |
| **21** | 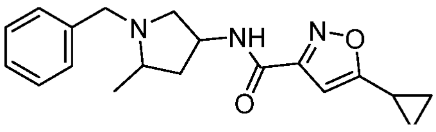 |

(continued)

| Cpd. No. | Structure |
|---|---|
| **22** | 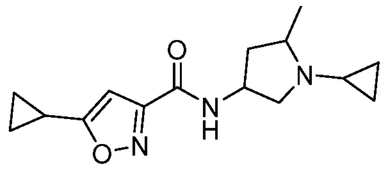 |
| **23** | 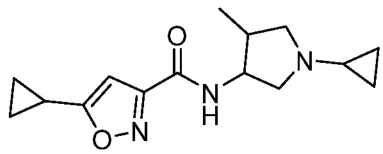 |
| **24** | 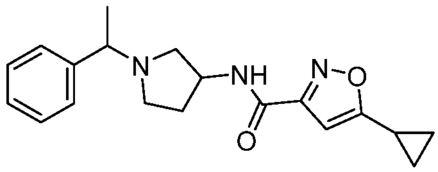 |
| **25** | 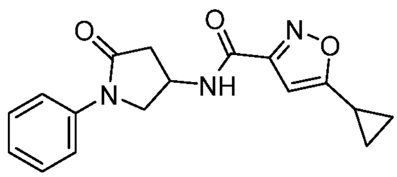 |
| **26** | 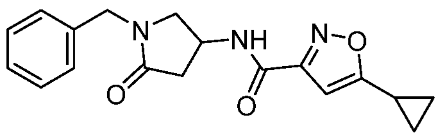 |
| **27** | 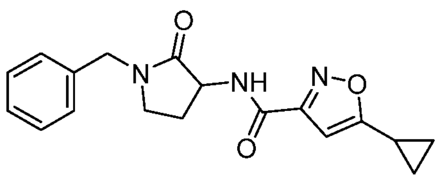 |
| **28** | 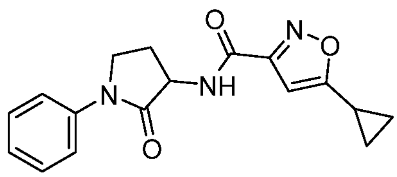 |
| **29** | 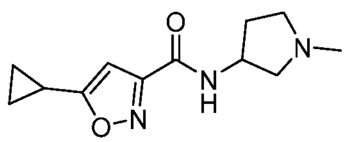 |
| **30** | 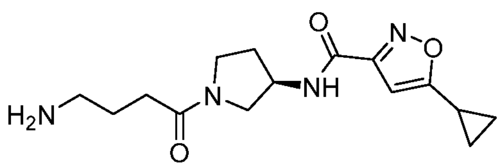 |

(continued)

| Cpd. No. | Structure |
|---|---|
| **31** | 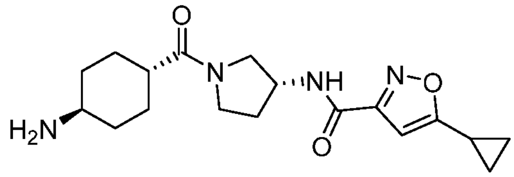 |
| **32** | 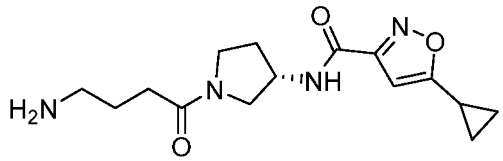 |
| **33** | 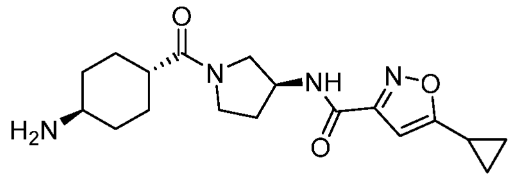 |
| **34** | 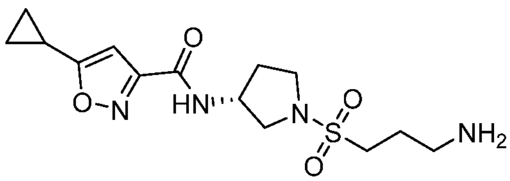 |
| **35** | 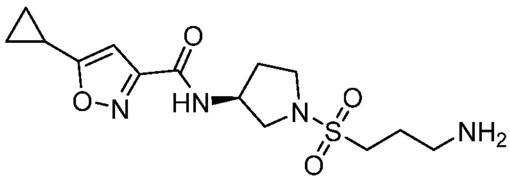 |
| **36** | 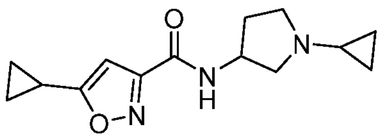 |
| **37** | 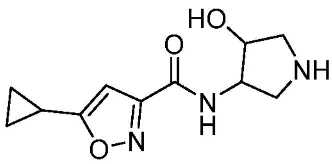 |
| **38** | 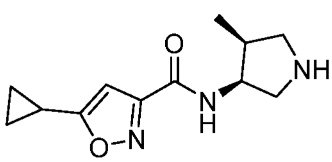 |
| **39** | 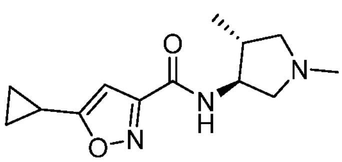 |

(continued)

| Cpd. No. | Structure |
|---|---|
| **40** | 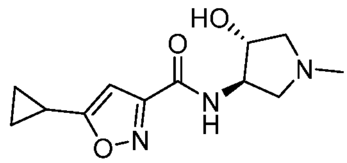 |
| **41** | 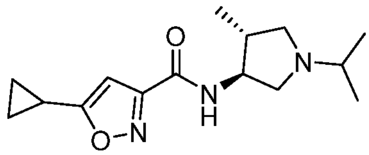 |
| **42** | 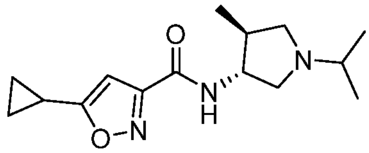 |
| **43** | 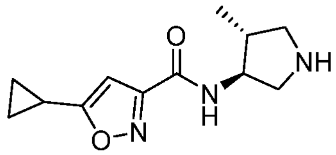 |
| **44** | 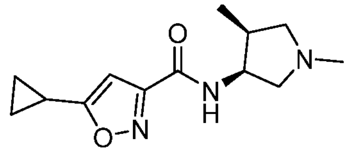 |
| **45** | 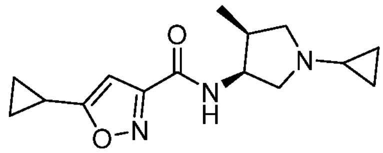 |
| **46** | 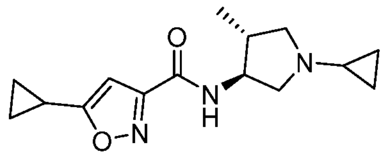 |
| **47** | 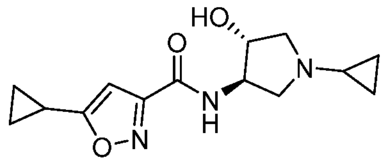 |
| **48** | 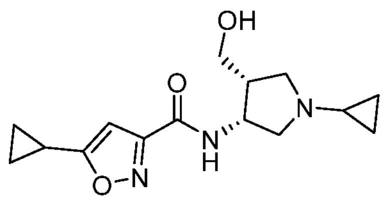 |

(continued)

| Cpd. No. | Structure |
|---|---|
| 49 | 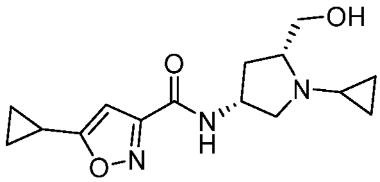 |
| 50 | 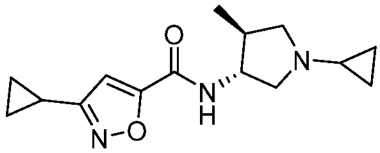 |
| 51 | 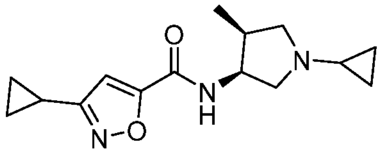 |
| 52 | 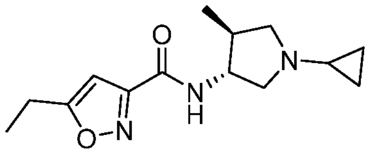 |
| 53 | 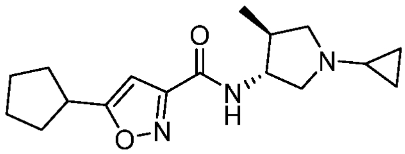 |
| 54 | 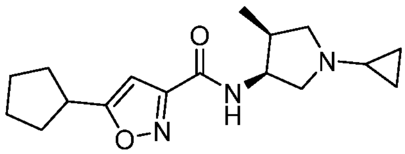 |
| 112 | 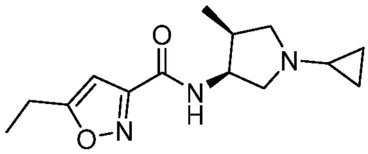 |

or Table 2:

| Cpd. No. | Structure |
| --- | --- |
| **55** | 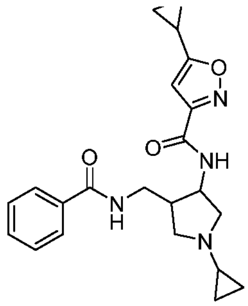 |
| **56** | 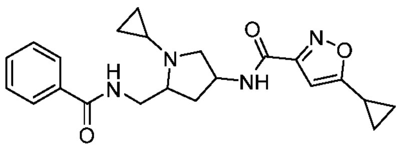 |
| **57** | 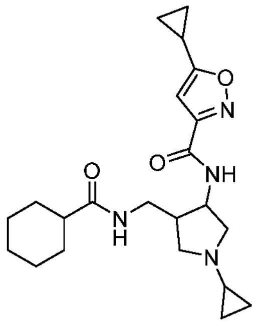 |
| **58** | 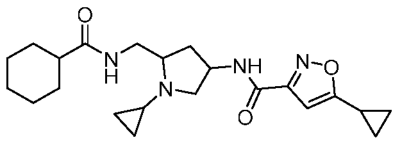 |
| **59** | 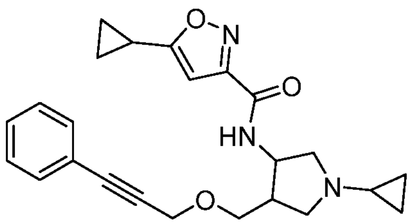 |
| **60** | 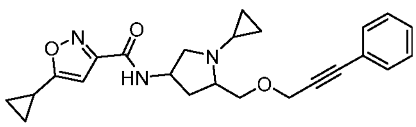 |
| **61** | 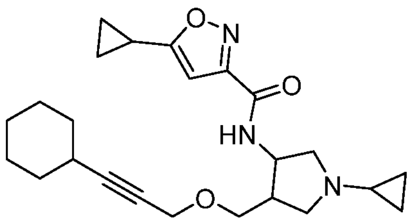 |

(continued)

| Cpd. No. | Structure |
|---|---|
| **62** | 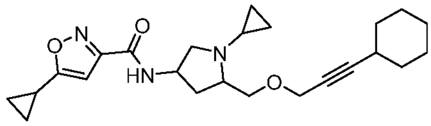 |
| **63** | 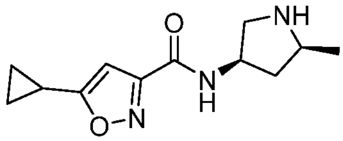 |
| **64** | 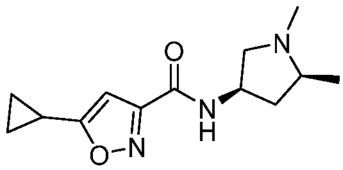 |
| **65** | 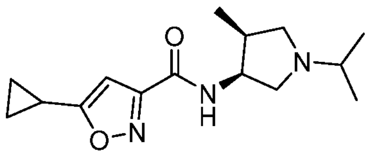 |
| **66** | 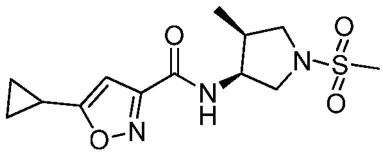 |
| **67** | 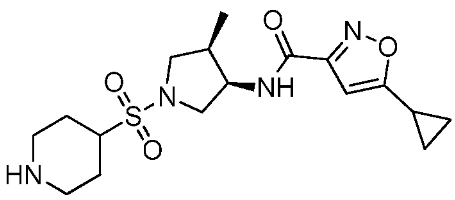 |
| **68** | 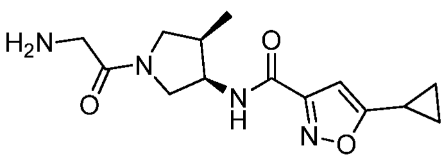 |
| **69** | 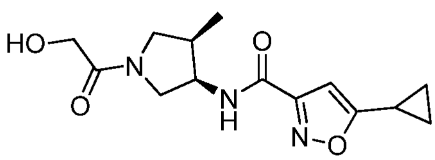 |
| **70** | 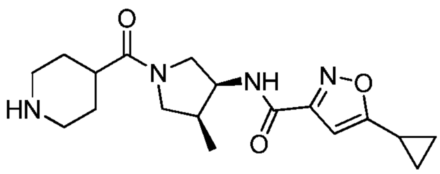 |

(continued)

| Cpd. No. | Structure |
|---|---|
| **71** | 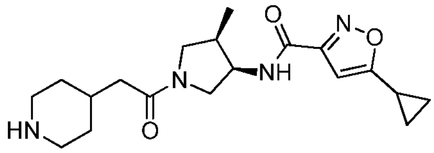 |
| **72** | 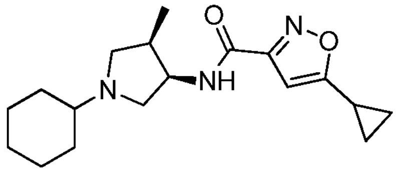 |
| **73** | 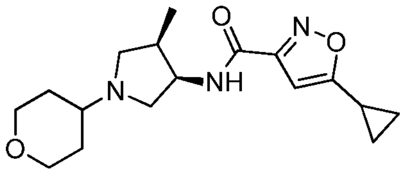 |
| **74** | 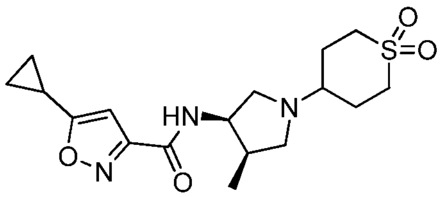 |
| **75** | 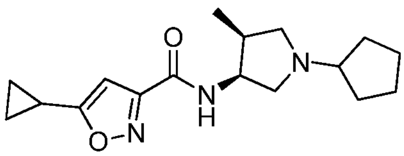 |
| **76** | 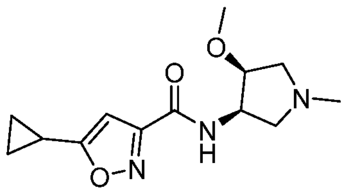 |
| **77** | 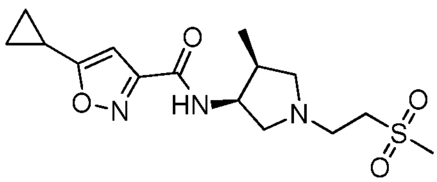 |
| **78** | 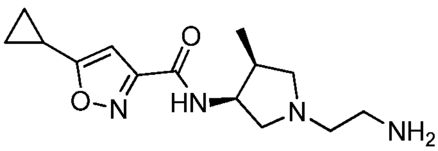 |

(continued)

| Cpd. No. | Structure |
|---|---|
| **79** | 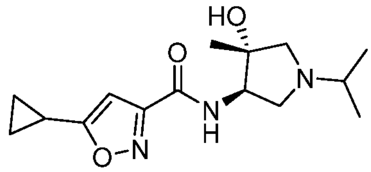 |
| **80** | 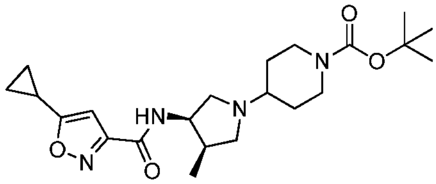 |
| **81** | 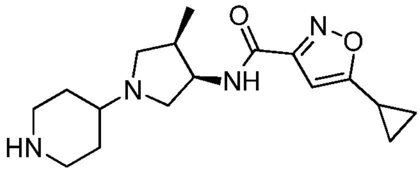 |
| **82** | 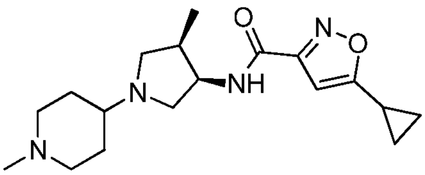 |
| **83** | 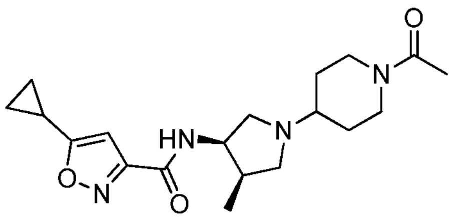 |
| **84** | 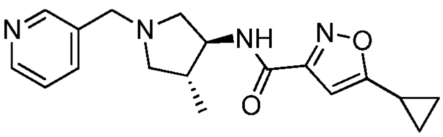 |
| **85** | 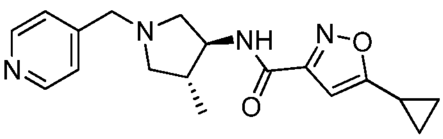 |
| **86** | 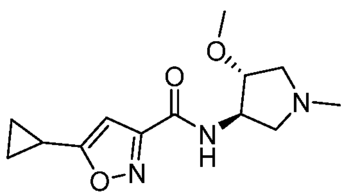 |

(continued)

| Cpd. No. | Structure |
|---|---|
| **87** | 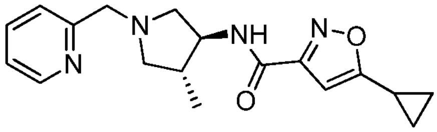 |
| **88** | 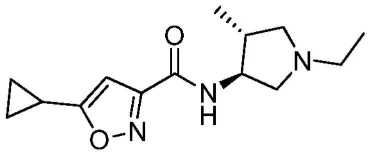 |
| **89** | 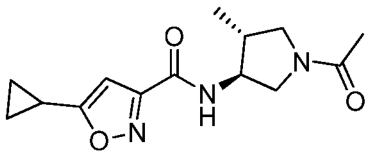 |
| **90** | 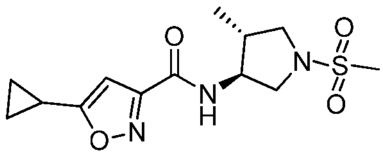 |
| **91** | 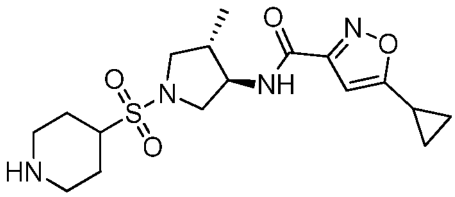 |
| **92** | 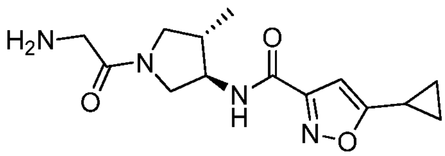 |
| **93** | 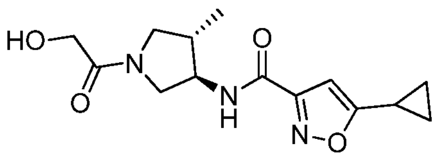 |
| **94** | 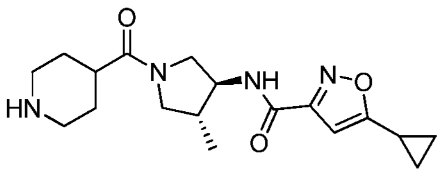 |
| **95** | 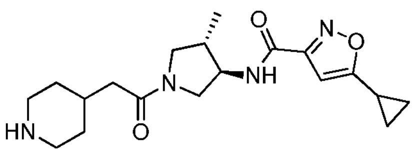 |

(continued)

| Cpd. No. | Structure |
|---|---|
| **96** | 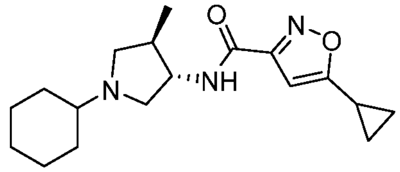 |
| **97** | 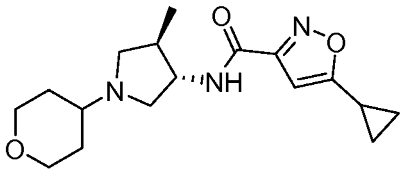 |
| **98** | 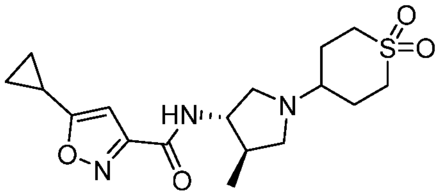 |
| **99** | 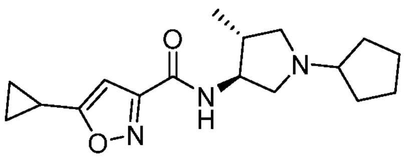 |
| **100** | 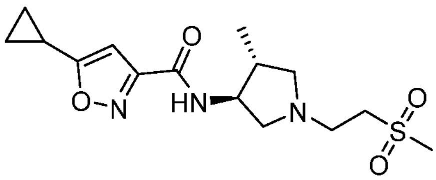 |
| **101** | 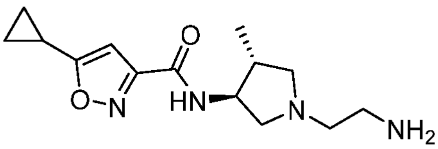 |
| **102** | 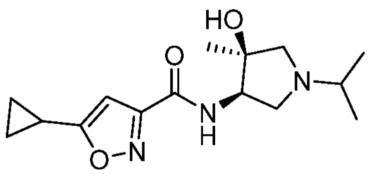 |
| **103** | 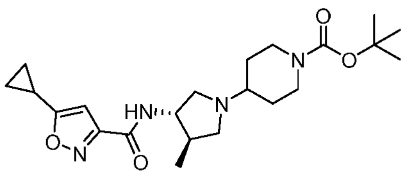 |

(continued)

| Cpd. No. | Structure |
|---|---|
| **104** | 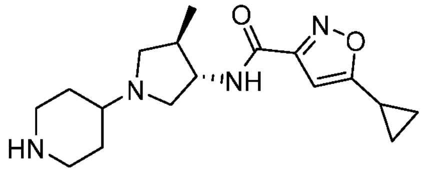 |
| **105** | 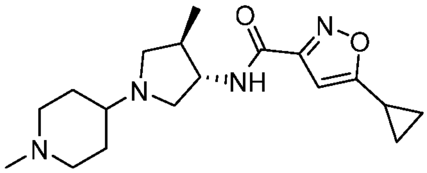 |
| **106** | 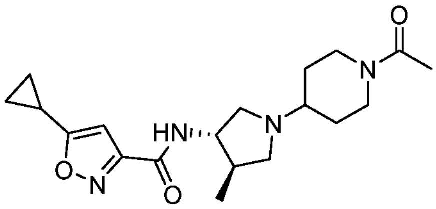 |
| **107** | 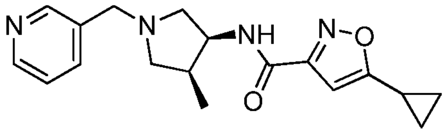 |
| **108** | 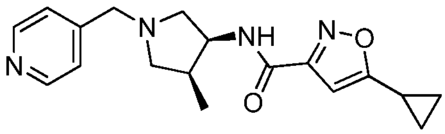 |
| **109** | 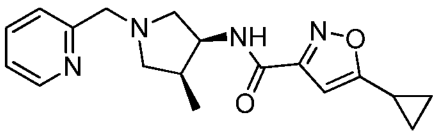 |
| **110** | 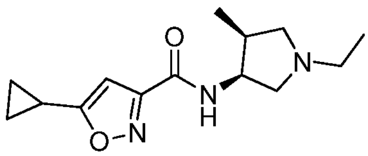 |
| **111** | 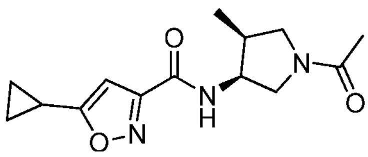 |

or Table 3:

| Cpd. No. | Structure |
|---|---|
| **113** | 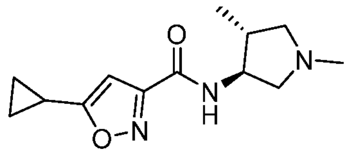 |
| **114** | 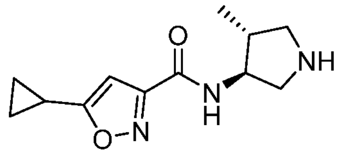 |
| **115** | 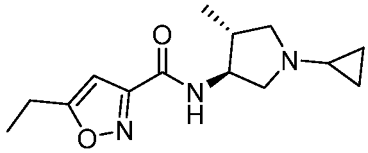 |
| **116** | 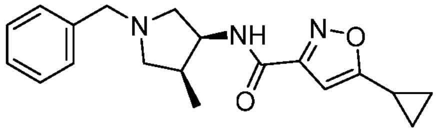 |
| **117** | 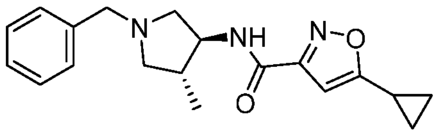 |
| **118** | 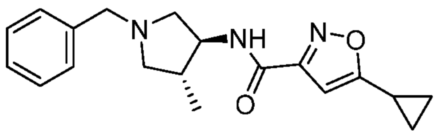 |
| **119** | 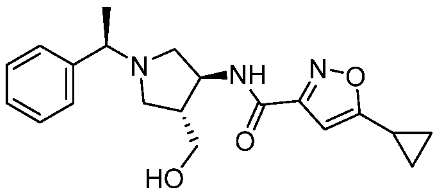 |
| **120** | 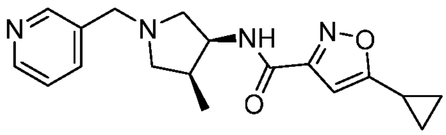 |
| **121** | 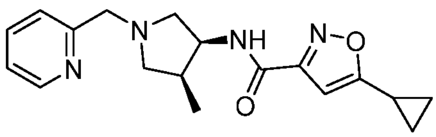 |
| **122** | 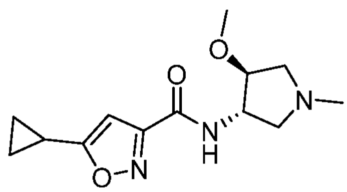 |

(continued)

| Cpd. No. | Structure |
|---|---|
| 123 | 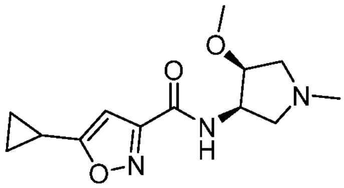 |
| 124 | 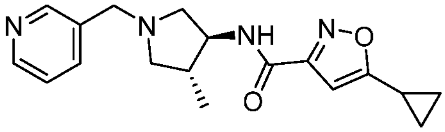 |
| 125 | 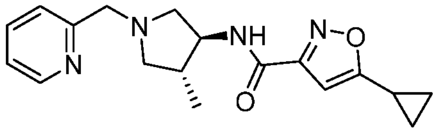 |
| 126 | 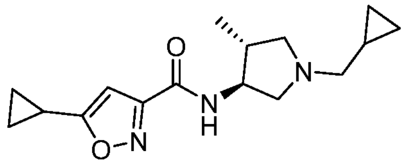 |
| 127 | 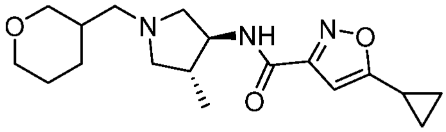 |
| 128 | 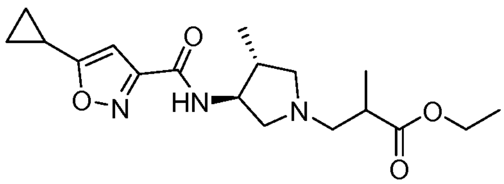 |
| 129 | 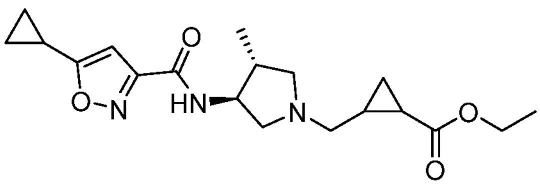 |
| 130 | 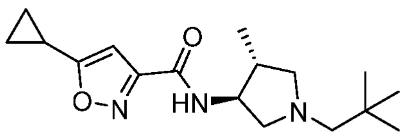 |
| 131 | 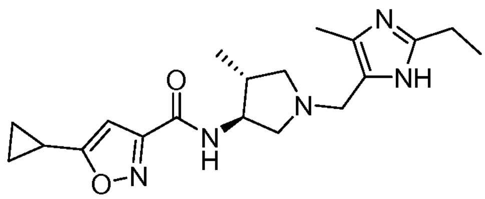 |

(continued)

| Cpd. No. | Structure |
|---|---|
| **132** | 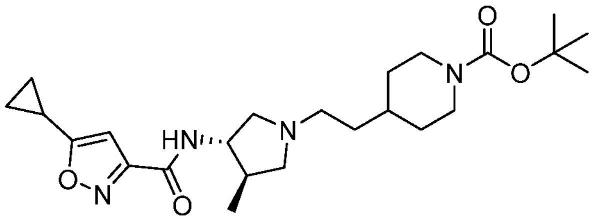 |
| **133** | 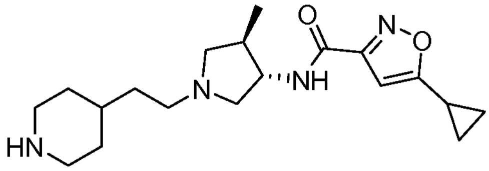 |
| **134** | 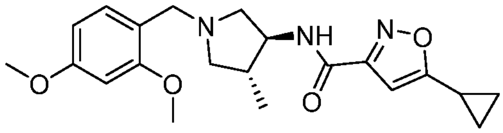 |
| **135** | 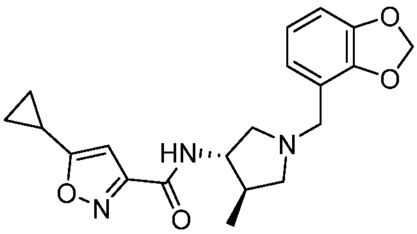 |
| **136** | 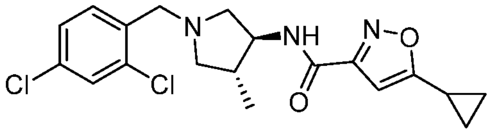 |
| 137 | 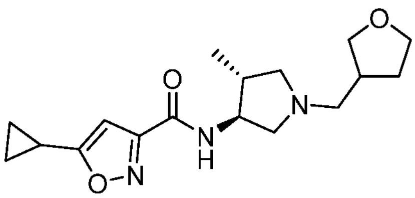 |
| 138 | 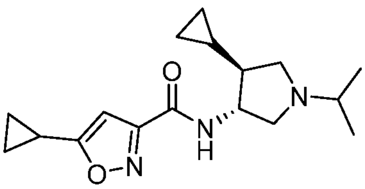 |
| 139 | 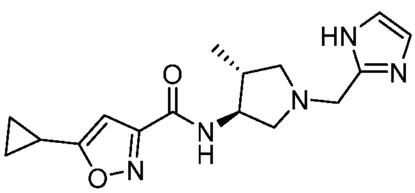 |

(continued)

| Cpd. No. | Structure |
|---|---|
| 140 | 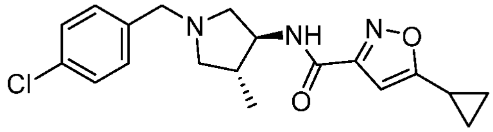 |
| 141 | 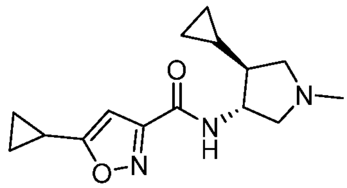 |
| 142 | 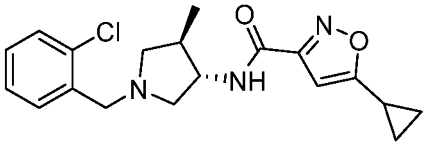 |
| 143 | 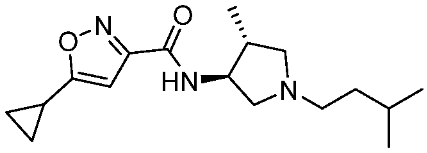 |
| 144 | 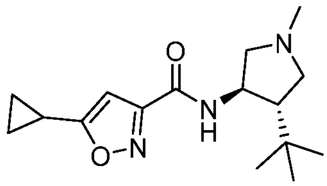 |
| 146 | 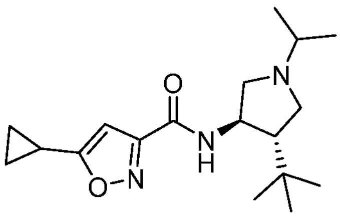 |
| 148 | 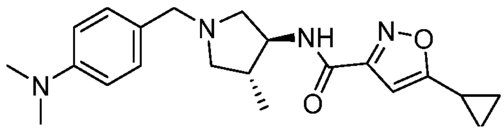 |
| **149** | 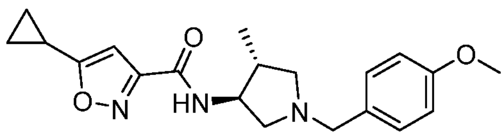 |
| **150** | 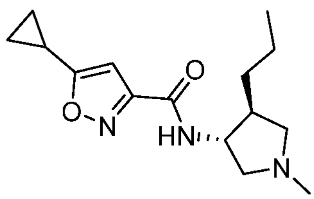 |

(continued)

| Cpd. No. | Structure |
|---|---|
| **151** | 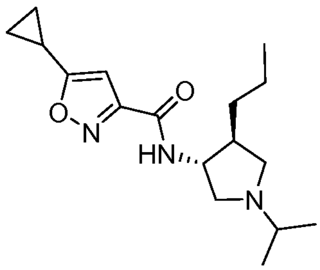 |
| **152** | 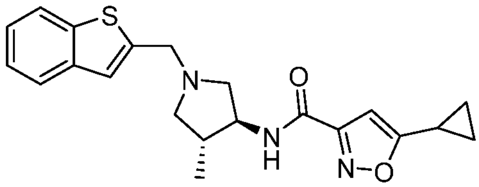 |
| **153** | 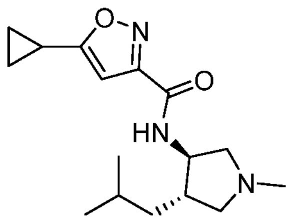 |
| **154** | 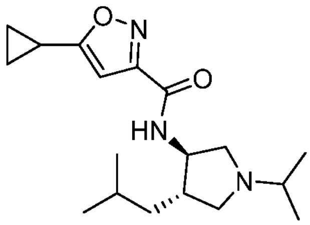 |
| **155** | 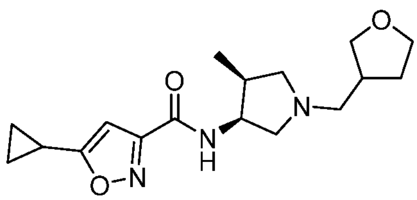 |
| **156** | 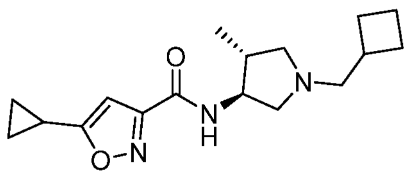 |
| **157** | 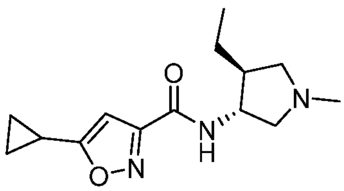 |

(continued)

| Cpd. No. | Structure |
|---|---|
| **158** | 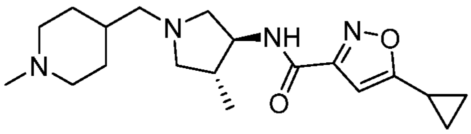 |
| **159** | 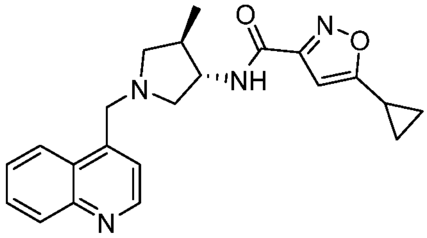 |
| **160** | 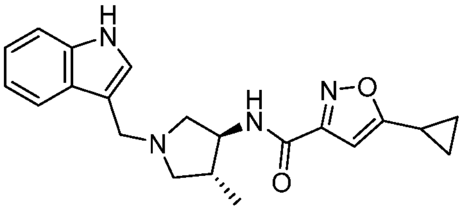 |
| **161** | 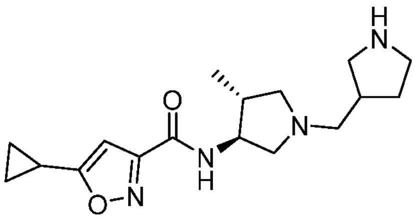 |
| **162** | 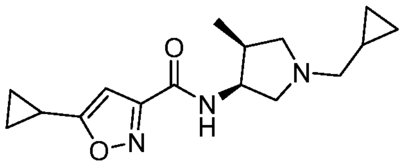 |
| **163** | 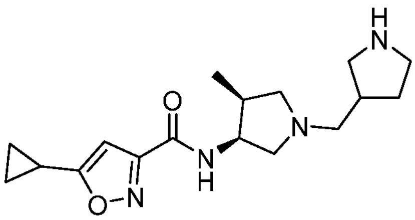 |
| **164** | 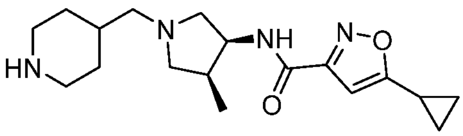 |
| **165** | 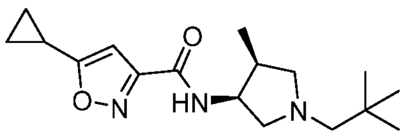 |

(continued)

| Cpd. No. | Structure |
|---|---|
| **166** | 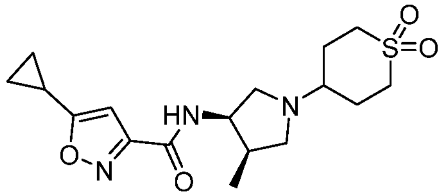 |
| **168** | 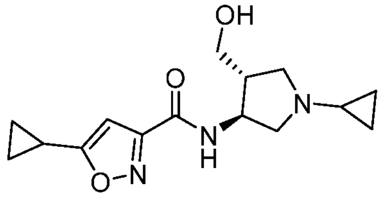 |
| **169** | 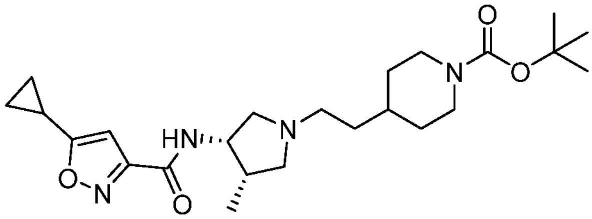 |
| **170** | 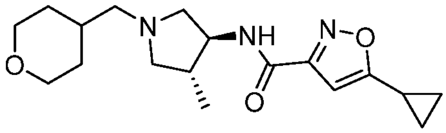 |
| **171** | 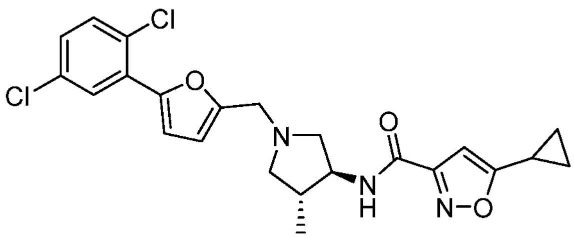 |
| **172** | 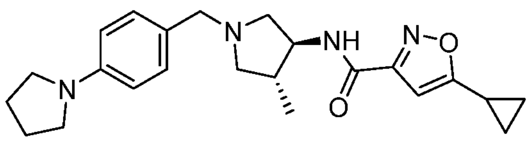 |
| **173** | 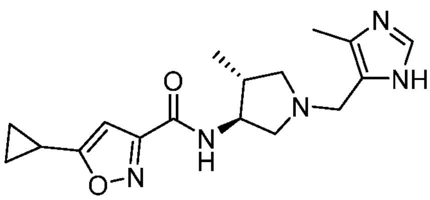 |
| **174** | 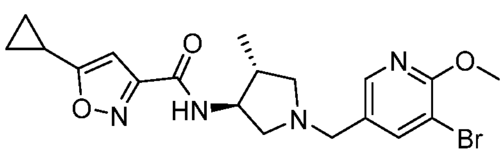 |

(continued)

| Cpd. No. | Structure |
|---|---|
| **175** | 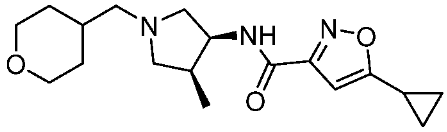 |
| **176** | 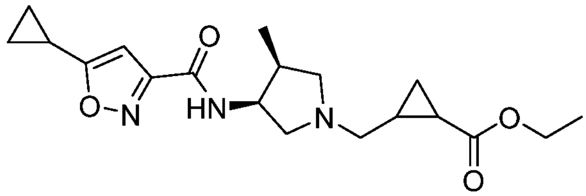 |
| **177** | 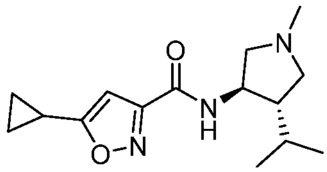 |
| **178** | 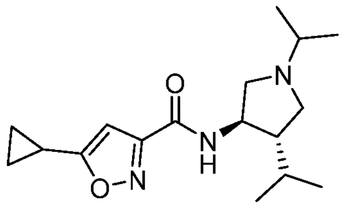 |
| **179** | 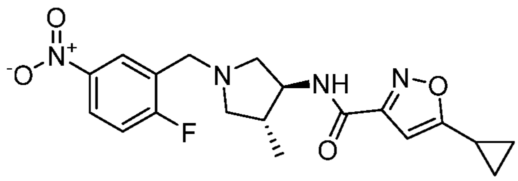 |
| **180** | 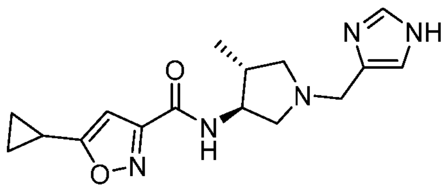 |
| **181** | 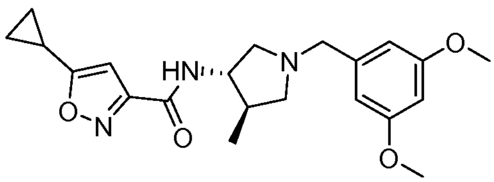 |
| **182** | 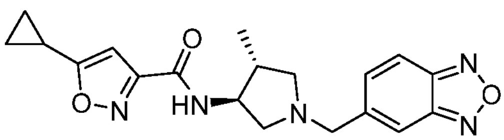 |

(continued)

| Cpd. No. | Structure |
|---|---|
| **183** | 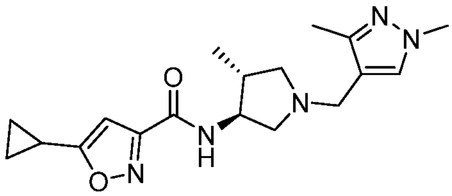 |
| **184** | 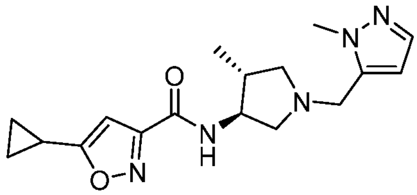 |
| **185** | 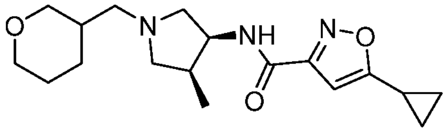 |
| **186** | 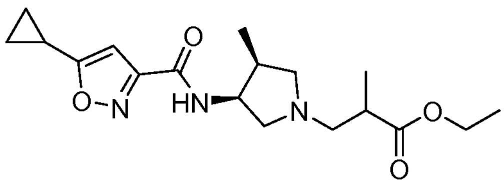 |
| **187** | 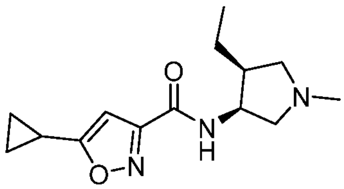 |
| **188** | 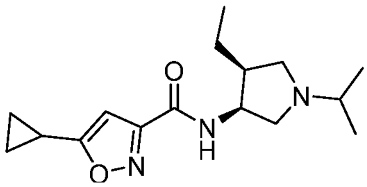 |
| **189** | 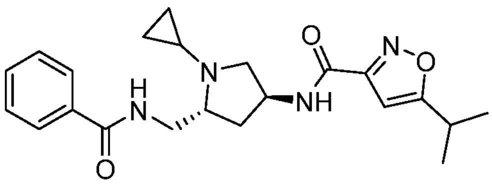 |
| **190** | 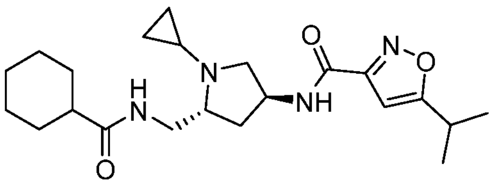 |

(continued)

| Cpd. No. | Structure |
|---|---|
| **191** | 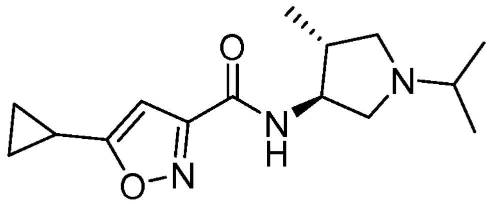 |
| **192** | 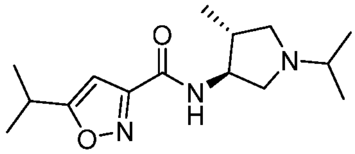 |
| **193** | 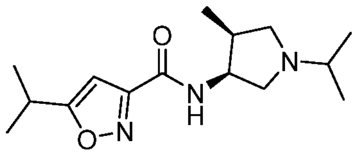 |
| **194** | 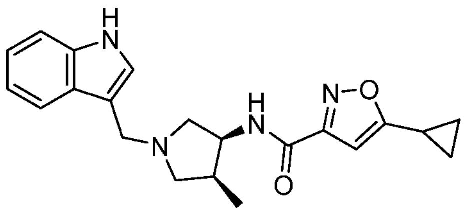 |
| **195** | 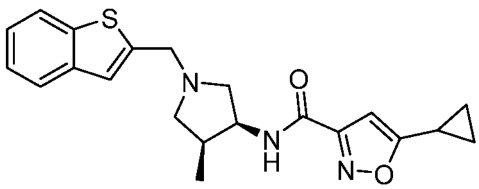 |
| **196** | 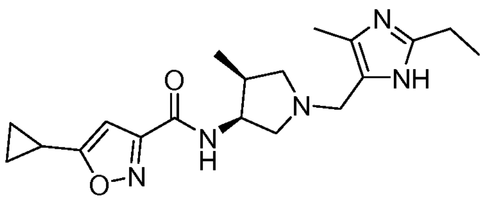 |
| **197** | 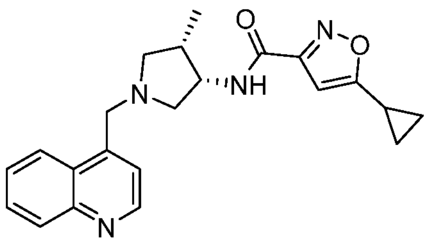 |

(continued)

| Cpd. No. | Structure |
|---|---|
| **198** | 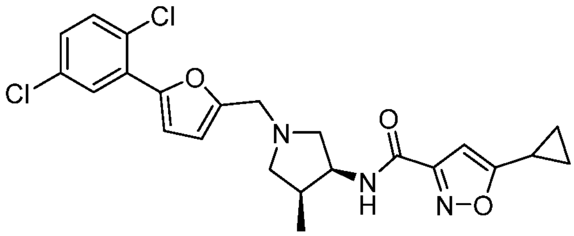 |
| **199** | 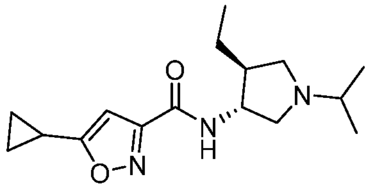 |
| **200** | 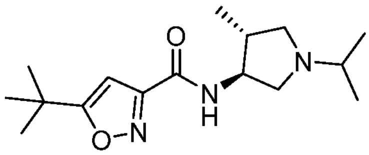 |
| **201** | 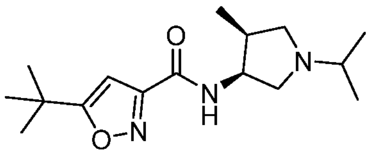 |
| **202** | 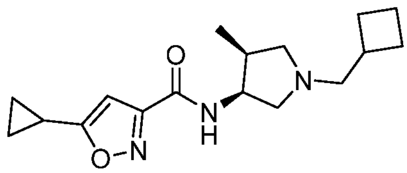 |
| **203** | 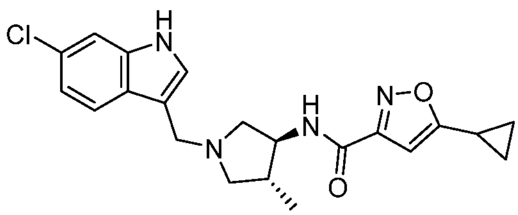 |
| **204** | 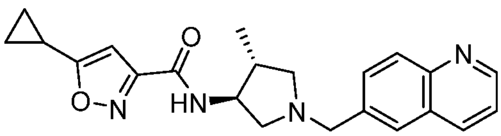 |
| **205** | 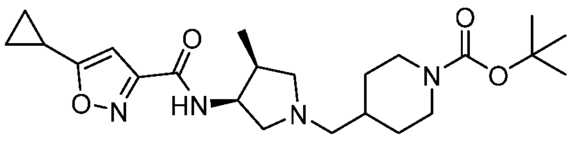 |

(continued)

| Cpd. No. | Structure |
|---|---|
| **206** | 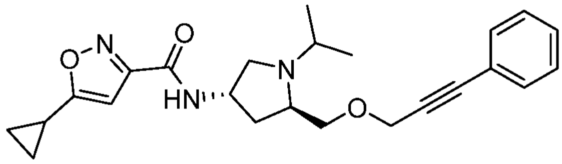 |
| **207** | 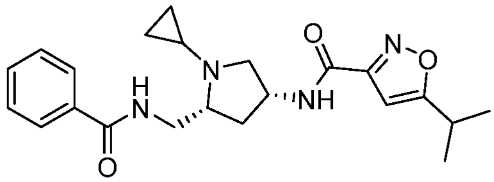 |
| **208** | 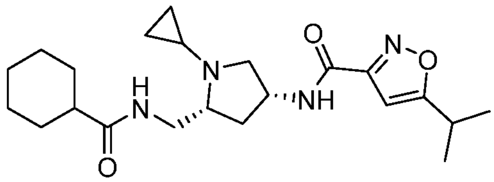 |
| **209** | 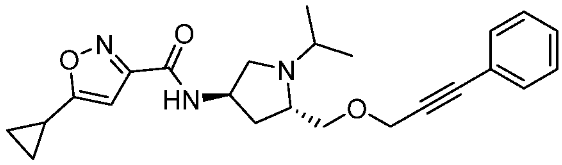 |
| **210** | 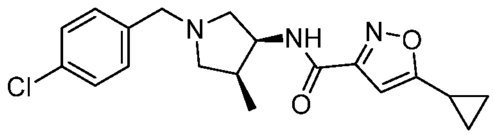 |
| **211** | 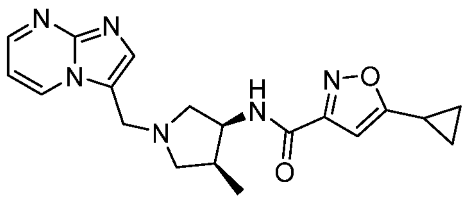 |
| **212** | 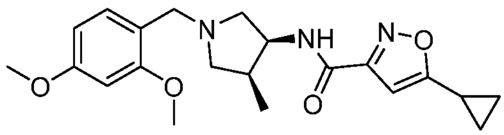 |
| **213** | 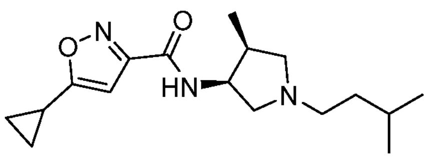 |

(continued)

| Cpd. No. | Structure |
|---|---|
| 214 | 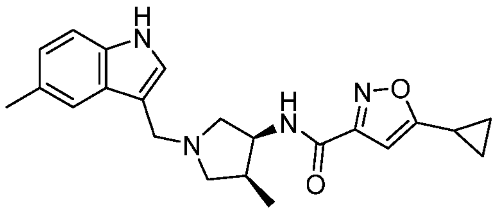 |
| 215 | 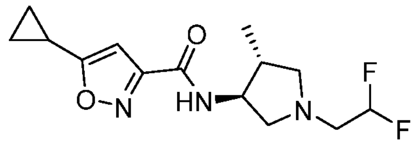 |
| 216 | 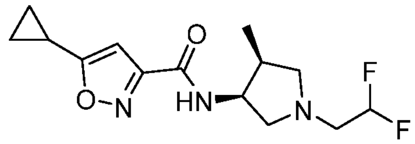 |
| 217 | 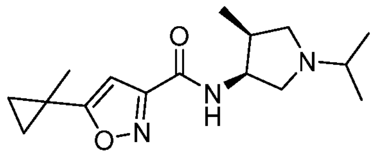 |
| 218 | 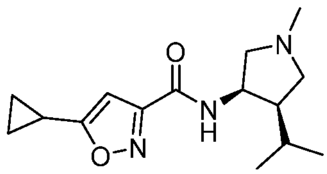 |
| 219 | 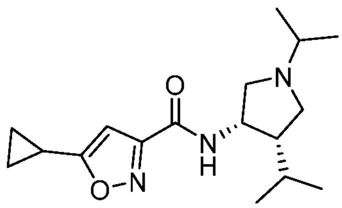 |
| 220 | 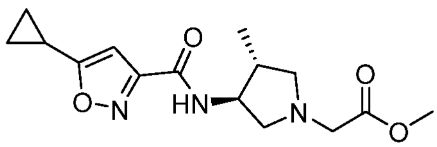 |
| 221 | 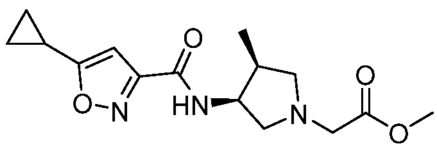 |
| 222 | 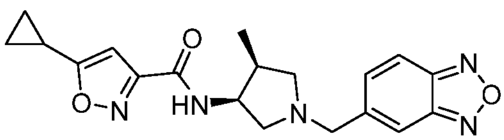 |

(continued)

| Cpd. No. | Structure |
|---|---|
| **223** | 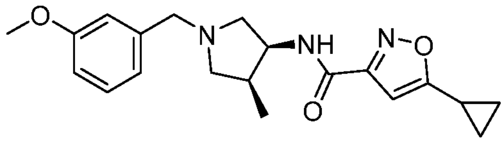 |
| **224** | 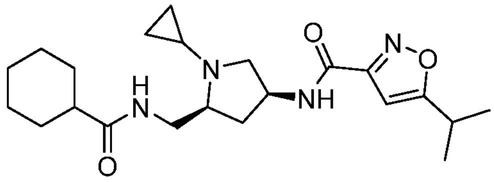 |
| **225** | 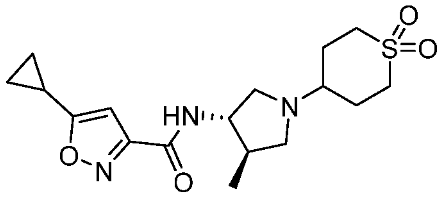 |
| **226** | 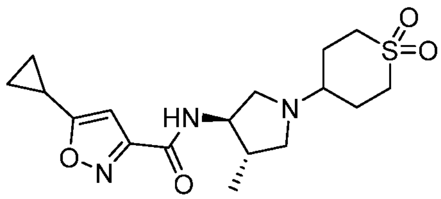 |
| **227** | 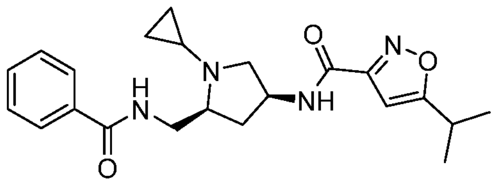 |
| **228** | 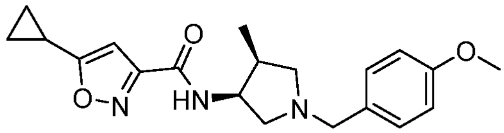 |
| **229** | 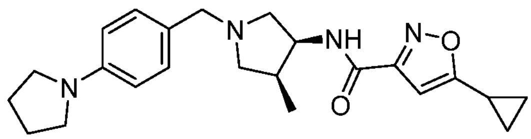 |
| **230** | 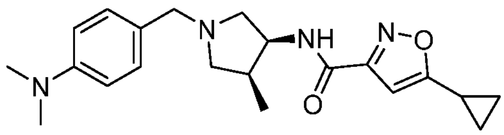 |
| **231** | 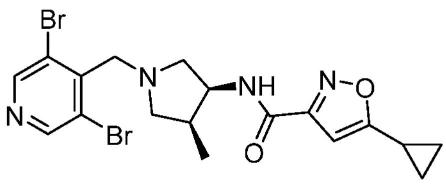 |

(continued)

| Cpd. No. | Structure |
|---|---|
| **232** | 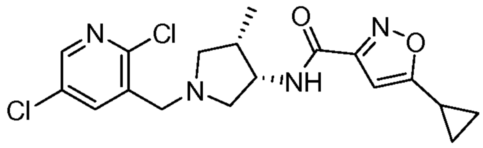 |
| **233** | 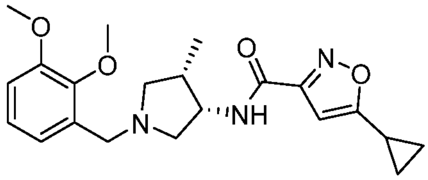 |
| **234** | 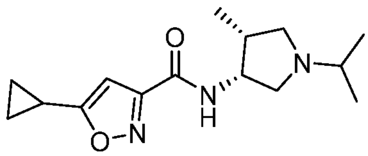 |
| **235** | 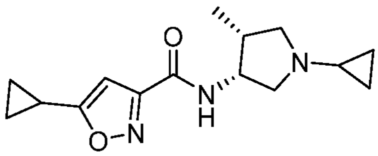 |
| **236** | 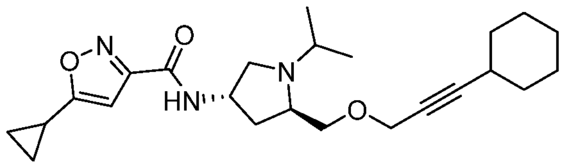 |
| **237** | 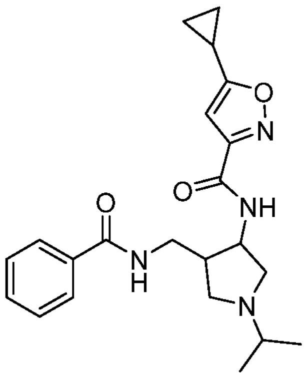 |
| **238** | 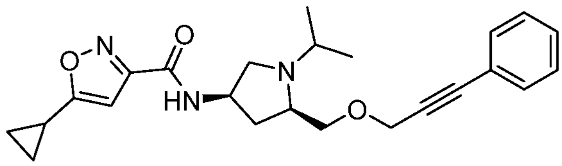 |
| **239** | 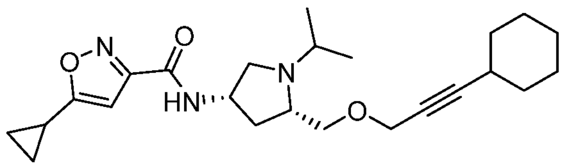 |

(continued)

| Cpd. No. | Structure |
|---|---|
| **240** | 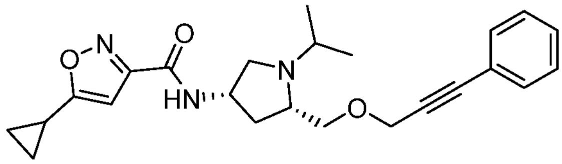 |
| **241** | 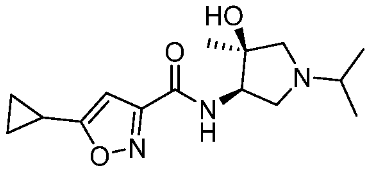 |
| **242** | 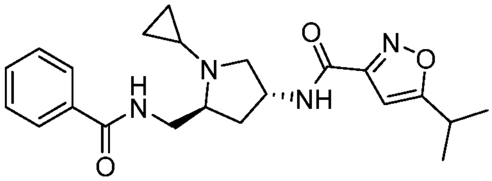 |
| **243** | 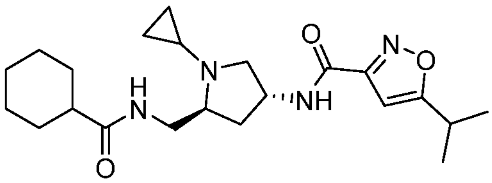 |
| **244** | 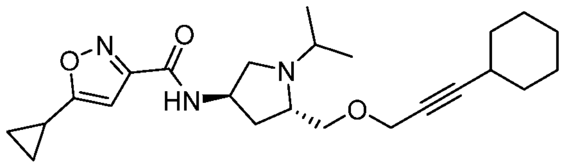 |
| **245** | 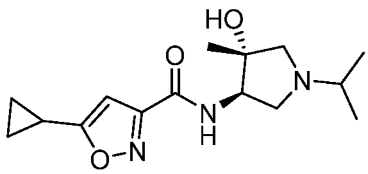 |
| **246** | 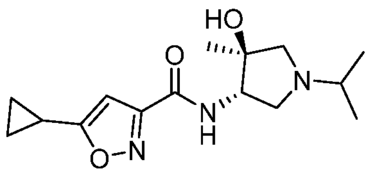 |
| **247** | 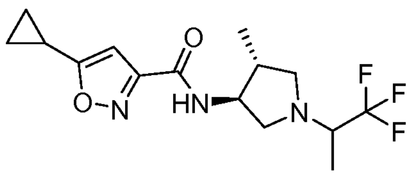 |

(continued)

| Cpd. No. | Structure |
|---|---|
| **248** | 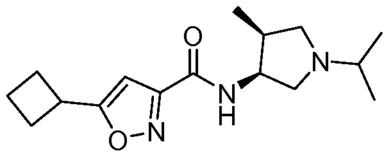 |
| **249** | 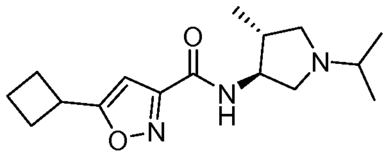 |
| **250** | 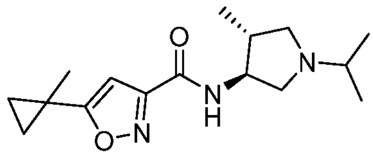 |
| **251** | 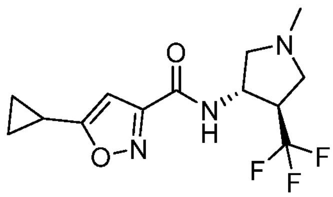 |
| **252** | 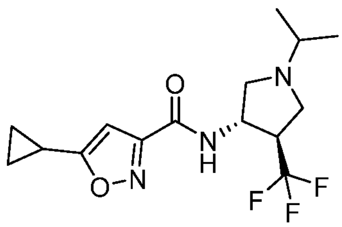 |
| **253** | 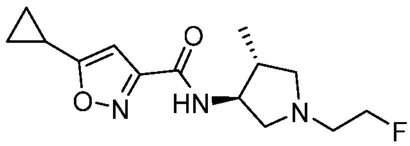 |
| **254** | 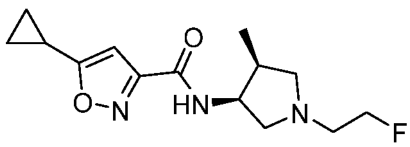 |
| **255** | 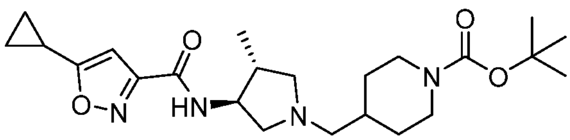 |
| **256** | 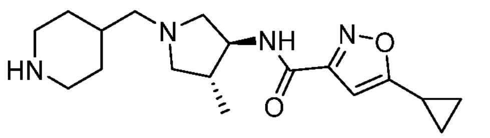 |

(continued)

| Cpd. No. | Structure |
|---|---|
| **257** | 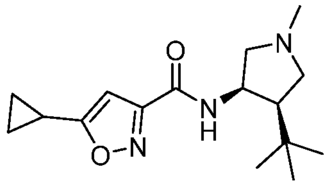 |
| **258** | 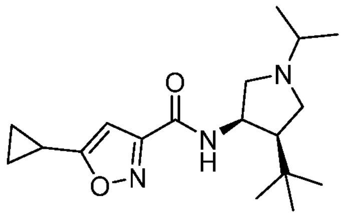 |
| **259** | 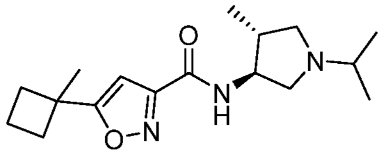 |
| **260** | 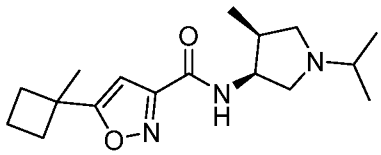 |
| **261** | 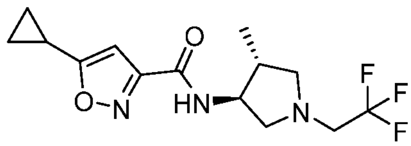 |
| **262** | 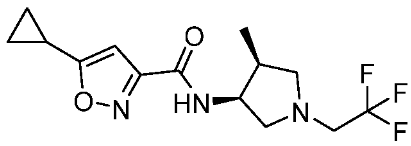 |
| **263** | 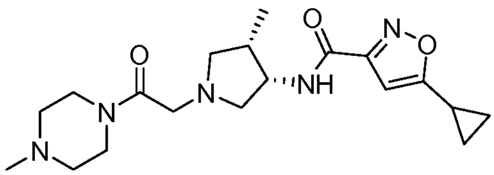 |
| **264** | 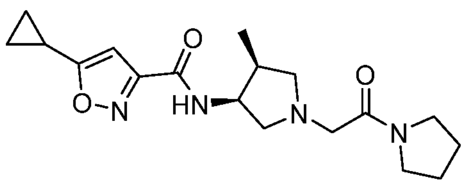 |

(continued)

| Cpd. No. | Structure |
|---|---|
| **265** | 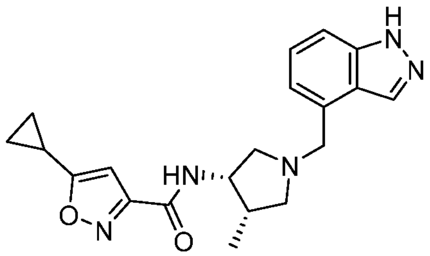 |
| **266** | 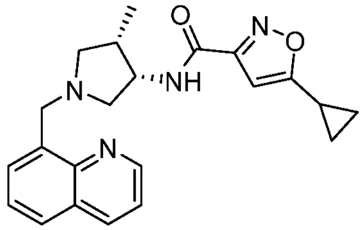 |
| **267** | 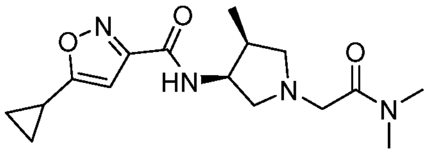 |
| **268** | 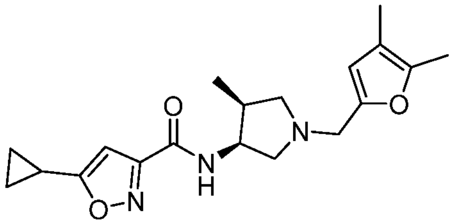 |
| **269** | 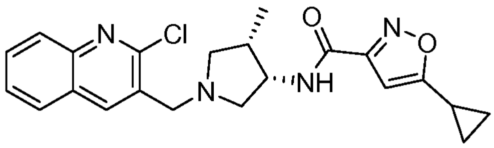 |
| **270** | 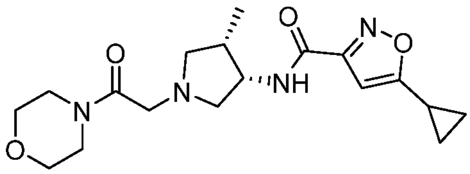 |
| **271** | 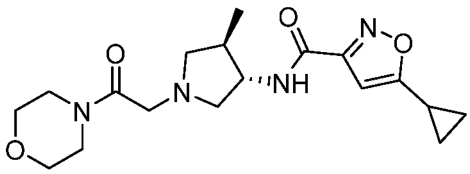 |

(continued)

| Cpd. No. | Structure |
|---|---|
| **272** | 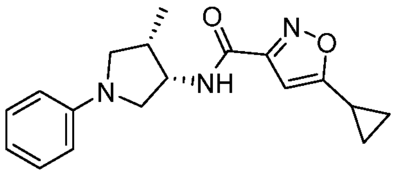 |
| **273** | 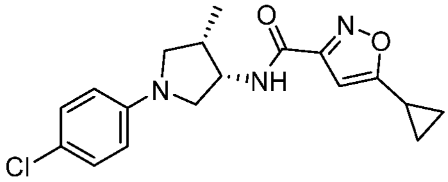 |
| **274** | 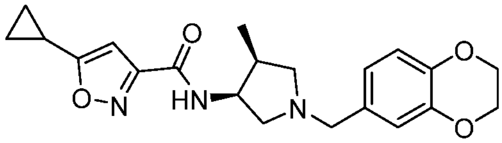 |
| **275** | 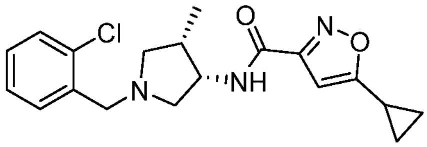 |
| **276** | 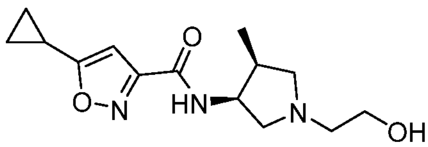 |
| **277** | 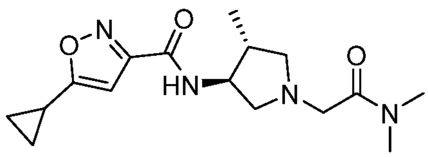 |
| **278** | 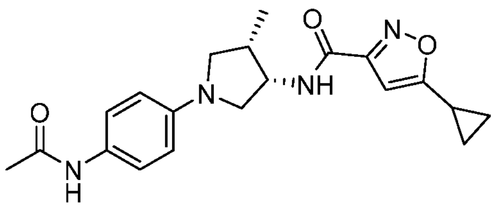 |
| **279** | 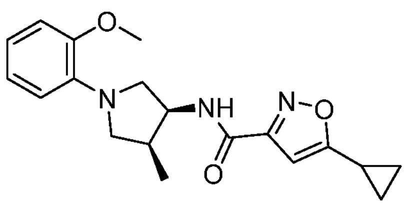 |

(continued)

| Cpd. No. | Structure |
|---|---|
| **280** | 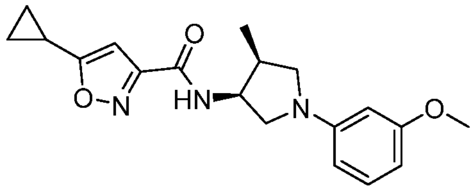 |
| **281** | 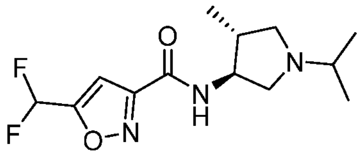 |
| **282** | 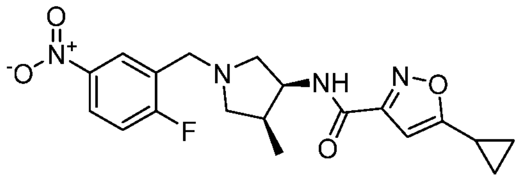 |
| **283** | 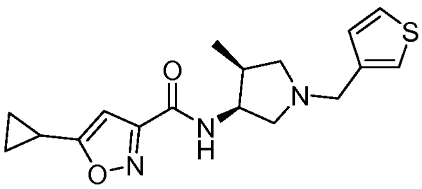 |
| **284** | 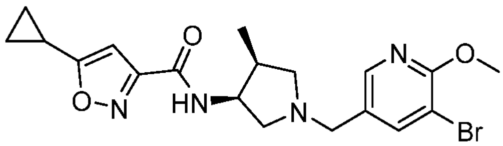 |
| **285** | 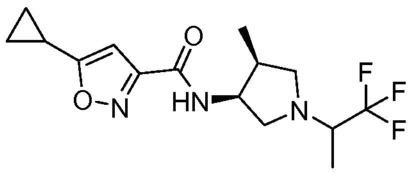 |
| **286** | 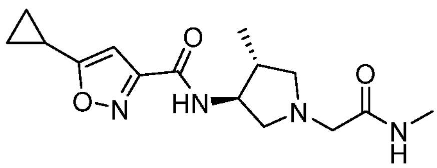 |
| **287** | 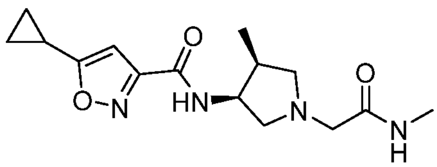 |

(continued)

| Cpd. No. | Structure |
|---|---|
| **288** | 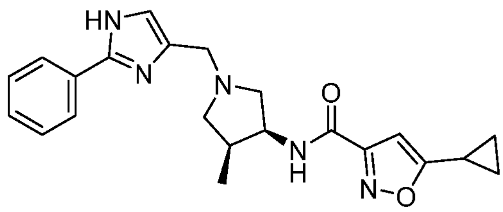 |
| **289** | 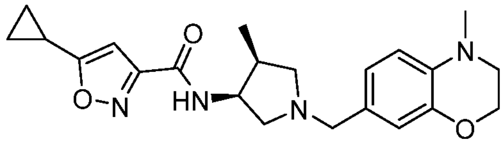 |
| **290** | 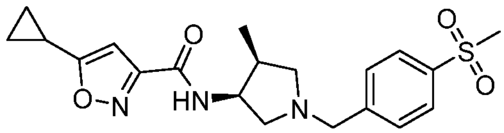 |
| **291** | 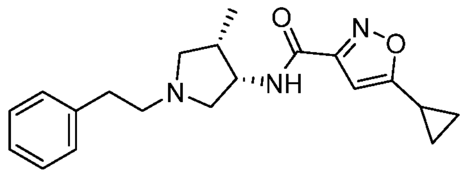 |
| **292** | 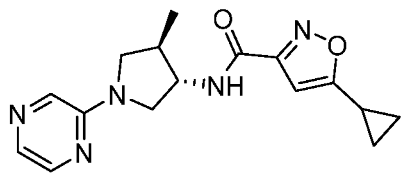 |
| **293** | 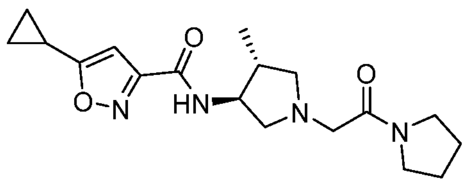 |
| **294** | 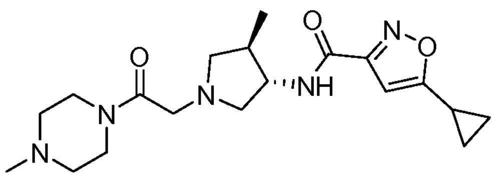 |
| **295** | 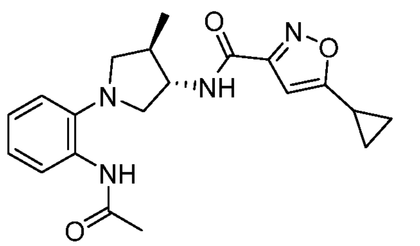 |

(continued)

| Cpd. No. | Structure |
|---|---|
| **296** | 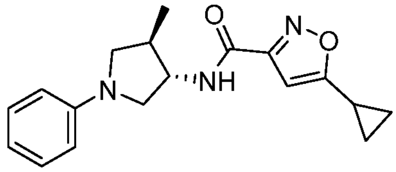 |
| **297** | 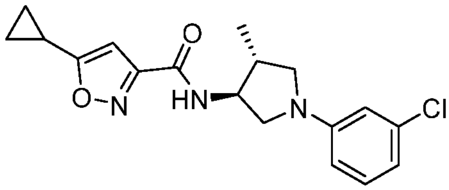 |
| **298** | 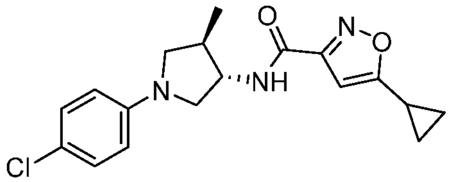 |
| **299** | 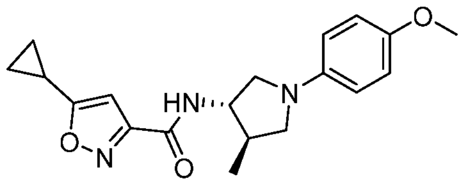 |
| **300** | 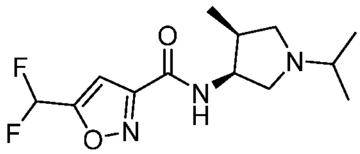 |
| **301** | 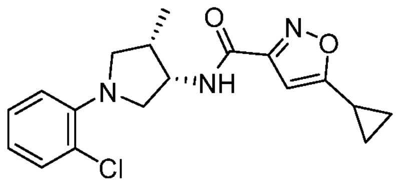 |
| **302** | 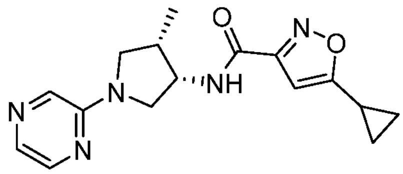 |
| **303** | 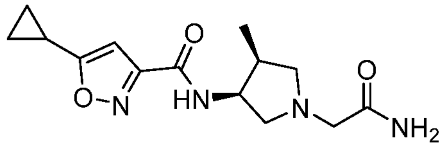 |

(continued)

| Cpd. No. | Structure |
|---|---|
| **304** | 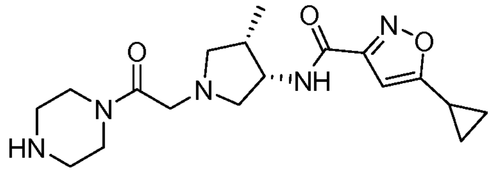 |
| **305** | 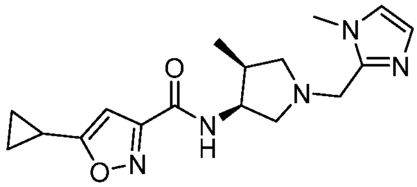 |
| **306** | 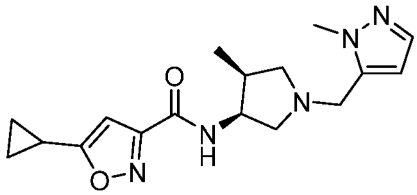 |
| **307** | 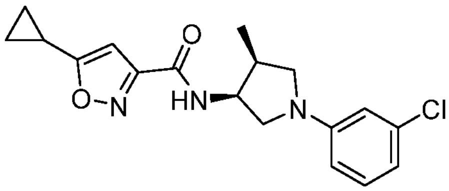 |
| **308** | 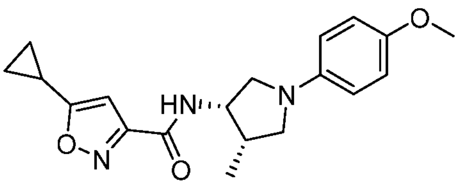 |
| **309** | 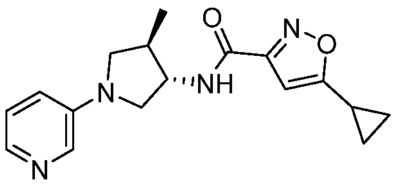 |
| **310** | 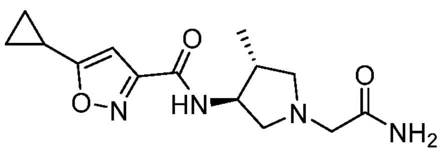 |
| **311** | 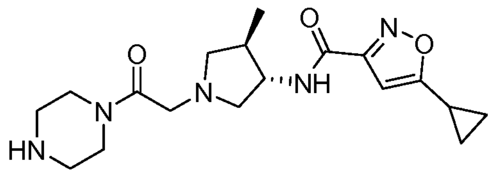 |

(continued)

| Cpd. No. | Structure |
|---|---|
| **312** | 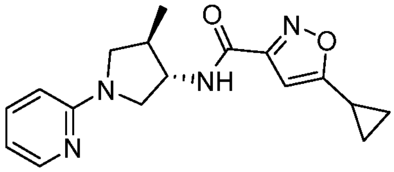 |
| **313** | 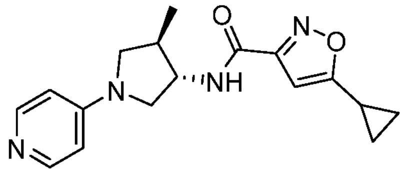 |
| **314** | 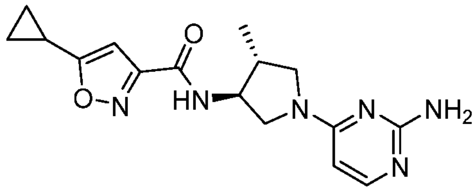 |
| **315** | 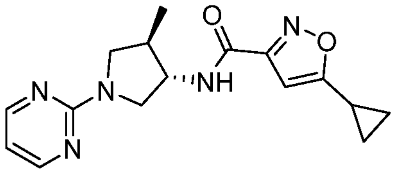 |
| **316** | 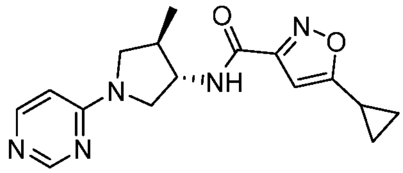 |
| **317** | 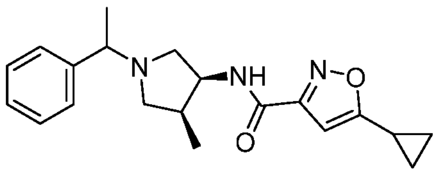 |
| **318** | 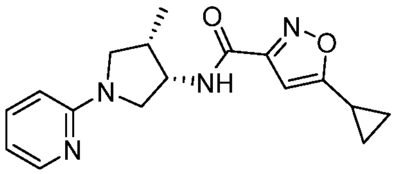 |
| **319** | 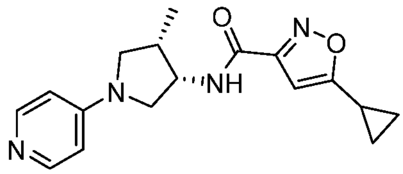 |

(continued)

| Cpd. No. | Structure |
|---|---|
| **320** | 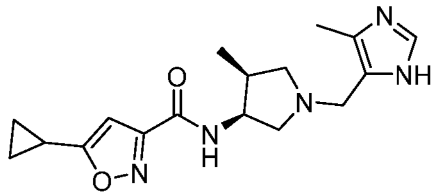 |
| **321** | 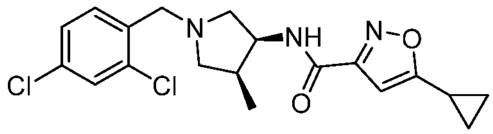 |
| **322** | 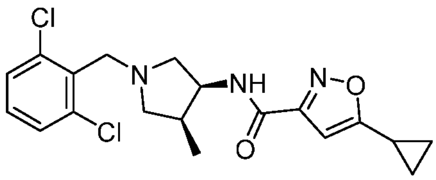 |
| **323** | 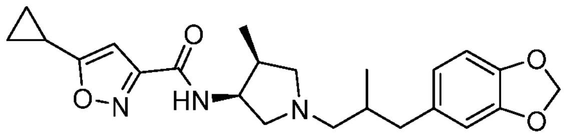 |
| **324** | 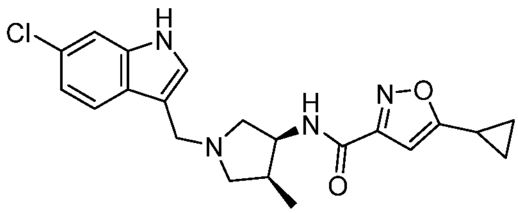 |
| **325** | 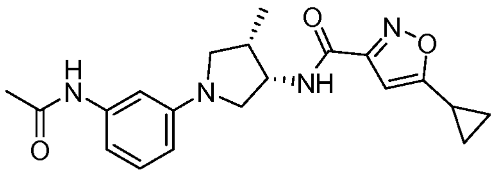 |
| **326** | 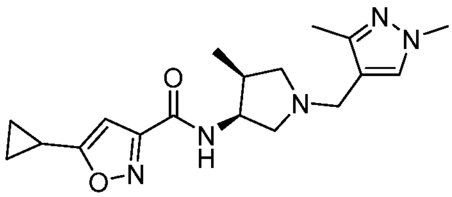 |
| **327** | 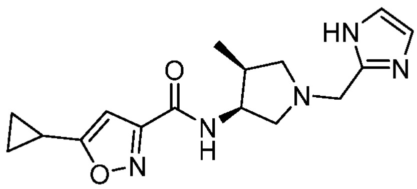 |

(continued)

| Cpd. No. | Structure |
|---|---|
| 328 | 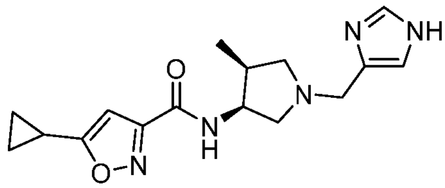 |
| 329 | 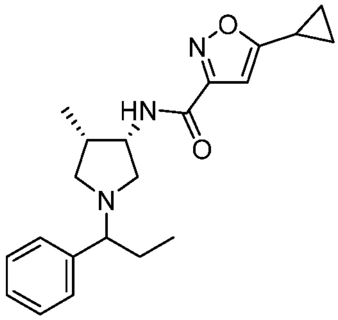 |
| 330 | 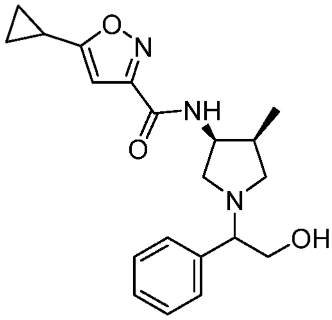 |
| 331 | 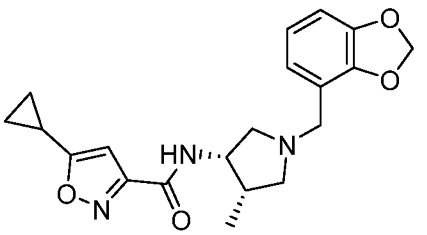 |
| 332 | 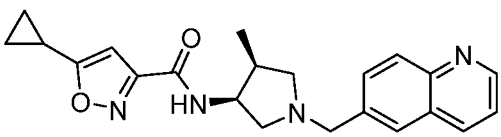 |
| 333 | 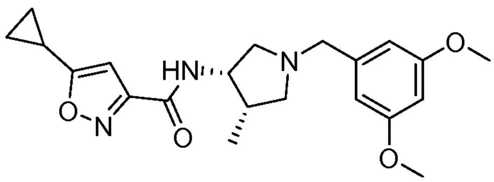 |
| 334 | 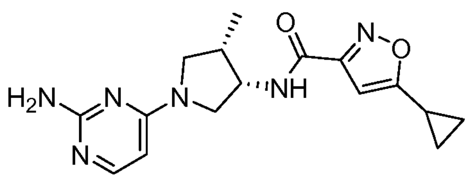 |

(continued)

| Cpd. No. | Structure |
|---|---|
| **335** | 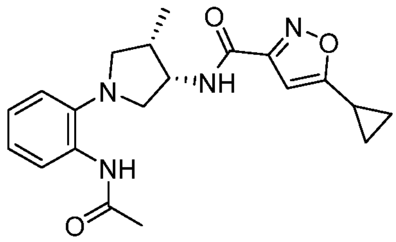 |
| **336** | 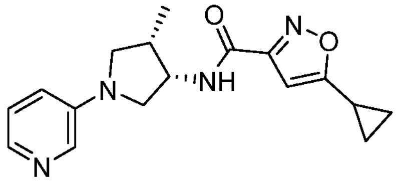 |
| **337** | 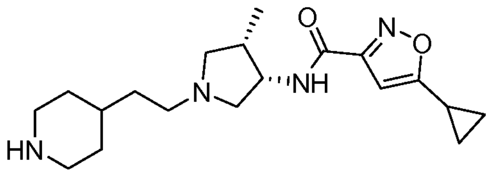 |
| **338** | 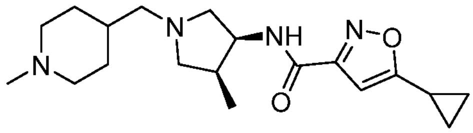 |
| **339** | 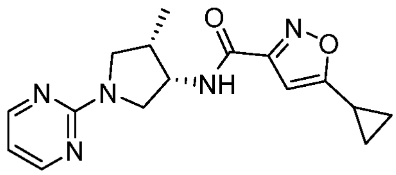 |
| **340** | 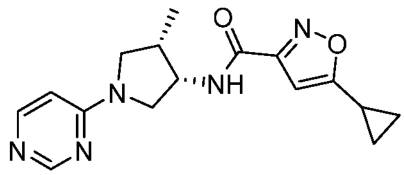 |
| **341** | 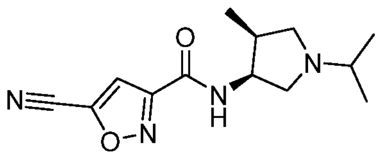 |
| **342** | 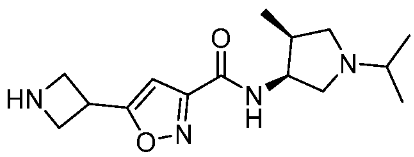 |

(continued)

| Cpd. No. | Structure |
|---|---|
| **343** | 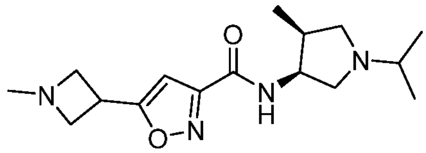 |
| **344** | 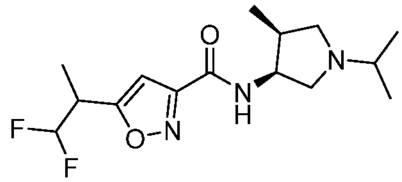 |
| **345** | 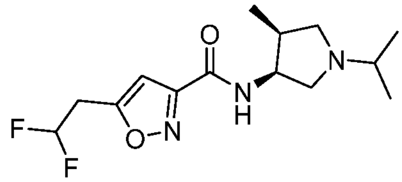 |
| **346** | 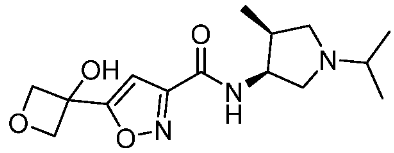 |
| **347** | 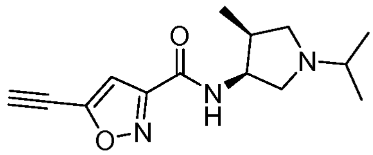 |
| **348** | 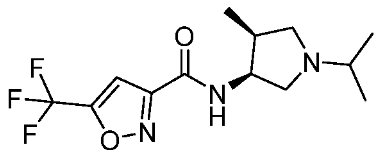 |
| **349** | 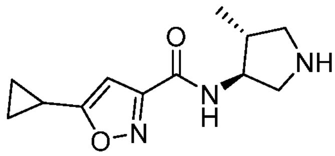 |
| **350** | 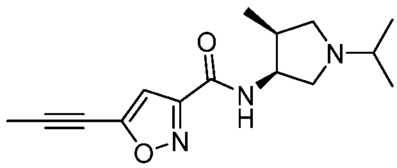 |
| **351** | 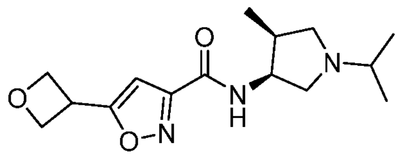 |

(continued)

| Cpd. No. | Structure |
|---|---|
| **352** | 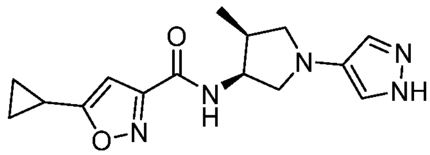 |

or a pharmaceutically acceptable salt or hydrate thereof.

**11.** A pharmaceutical composition for use in treating cancer, the pharmaceutical composition comprising the compound of any one of claims 1-10, or a pharmaceutically acceptable salt or hydrate thereof, and a pharmaceutically acceptable carrier.

**12.** The pharmaceutical composition for use of claim 11, wherein the cancer is selected from the group consisting of adrenal cancer, acinic cell carcinoma, acoustic neuroma, acral lentigious melanoma, acrospiroma, acute eosinophilic leukemia, acute erythroid leukemia, acute lymphoblastic leukemia, acute megakaryoblastic leukemia, acute monocytic leukemia, acute promyelocytic leukemia, adenocarcinoma, adenoid cystic carcinoma, adenoma, adenomatoid odontogenic tumor, adenosquamous carcinoma, adipose tissue neoplasm, adrenocortical carcinoma, adult T-cell leukemia/lymphoma, aggressive NK-cell leukemia, AIDS-related lymphoma, alveolar rhabdomyosarcoma, alveolar soft part sarcoma, ameloblastic fibroma, anaplastic large cell lymphoma, anaplastic thyroid cancer, angioimmunoblastic T-cell lymphoma, angiomyolipoma, angiosarcoma, astrocytoma, atypical teratoid rhabdoid tumor, B-cell chronic lymphocytic leukemia, B-cell prolymphocytic leukemia, B-cell lymphoma, basal cell carcinoma, biliary tract cancer, bladder cancer, blastoma, bone cancer, Brenner tumor, Brown tumor, Burkitt's lymphoma, breast cancer, brain cancer, carcinoma, carcinoma in situ, carcinosarcoma, cartilage tumor, cementoma, myeloid sarcoma, chondroma, chordoma, choriocarcinoma, choroid plexus papilloma, clear-cell sarcoma of the kidney, craniopharyngioma, cutaneous T-cell lymphoma, cervical cancer, colorectal cancer, Degos disease, desmoplastic small round cell tumor, diffuse large B-cell lymphoma, dysembryoplastic neuroepithelial tumor, dysgerminoma, embryonal carcinoma, endocrine gland neoplasm, endodermal sinus tumor, enteropathy-associated T-cell lymphoma, esophageal cancer, fetus in fetu, fibroma, fibrosarcoma, follicular lymphoma, follicular thyroid cancer, ganglioneuroma, gastrointestinal cancer, germ cell tumor, gestational choriocarcinoma, giant cell fibroblastoma, giant cell tumor of the bone, glial tumor, glioblastoma multiforme, glioma, gliomatosis cerebri, glucagonoma, gonadoblastoma, granulosa cell tumor, gynandroblastoma, gallbladder cancer, gastric cancer, hairy cell leukemia, hemangioblastoma, head and neck cancer, hemangiopericytoma, hematological malignancy, hepatoblastoma, hepatosplenic T-cell lymphoma, Hodgkin's lymphoma, non-Hodgkin's lymphoma, invasive lobular carcinoma, intestinal cancer, kidney cancer, laryngeal cancer, lentigo maligna, lethal midline carcinoma, leukemia, leydig cell tumor, liposarcoma, lung cancer, lymphangioma, lymphangiosarcoma, lymphoepithelioma, lymphoma, acute lymphocytic leukemia, acute myelogeous leukemia, chronic lymphocytic leukemia, liver cancer, small cell lung cancer, non-small cell lung cancer, MALT lymphoma, malignant fibrous histiocytoma, malignant peripheral nerve sheath tumor, malignant triton tumor, mantle cell lymphoma, marginal zone B-cell lymphoma, mast cell leukemia, mediastinal germ cell tumor, medullary carcinoma of the breast, medullary thyroid cancer, medulloblastoma, melanoma, meningioma, merkel cell cancer, mesothelioma, metastatic urothelial carcinoma, mixed Mullerian tumor, mucinous tumor, multiple myeloma, muscle tissue neoplasm, mycosis fungoides, myxoid liposarcoma, myxoma, myxosarcoma, nasopharyngeal carcinoma, neurinoma, neuroblastoma, neurofibroma, neuroma, nodular melanoma, ocular cancer, oligoastrocytoma, oligodendroglioma, oncocytoma, optic nerve sheath meningioma, optic nerve tumor, oral cancer, osteosarcoma, ovarian cancer, Pancoast tumor, papillary thyroid cancer, paraganglioma, pinealoblastoma, pineocytoma, pituicytoma, pituitary adenoma, pituitary tumor, plasmacytoma, polyembryoma, precursor T-lymphoblastic lymphoma, primary central nervous system lymphoma, primary effusion lymphoma, preimary peritoneal cancer, prostate cancer, pancreatic cancer, pharyngeal cancer, pseudomyxoma periotonei, renal cell carcinoma, renal medullary carcinoma, retinoblastoma, rhabdomyoma, rhabdomyosarcoma, Richter's transformation, rectal cancer, sarcoma, Schwannomatosis, seminoma, Sertoli cell tumor, sex cord-gonadal stromal tumor, signet ring cell carcinoma, skin cancer, small blue round cell tumors, small cell carcinoma, soft tissue sarcoma, somatostatinoma, soot wart, spinal tumor, splenic marginal zone lymphoma, squamous cell carcinoma, synovial sarcoma, Sezary's disease, small intestine cancer, squamous carcinoma, stomach cancer, T-cell lymphoma, testicular cancer, thecoma, thyroid cancer, transitional cell carcinoma, throat cancer, urachal cancer, urogenital cancer, urothelial carcinoma, uveal melanoma, uterine cancer, verrucous carcinoma, visual pathway glioma, vulvar cancer, vaginal cancer, Waldenstrom's macroglobulinemia, Warthin's

tumor, and Wilms' tumor.

13. A compound for use of any one of claims 1-10, or a pharmaceutically acceptable salt or hydrate thereof, wherein the cancer is selected from the group consisting of adrenal cancer, acinic cell carcinoma, acoustic neuroma, acral lentigious melanoma, acrospiroma, acute eosinophilic leukemia, acute erythroid leukemia, acute lymphoblastic leukemia, acute megakaryoblastic leukemia, acute monocytic leukemia, acute promyelocytic leukemia, adenocarcinoma, adenoid cystic carcinoma, adenoma, adenomatoid odontogenic tumor, adenosquamous carcinoma, adipose tissue neoplasm, adrenocortical carcinoma, adult T-cell leukemia/lymphoma, aggressive NK-cell leukemia, AIDS-related lymphoma, alveolar rhabdomyosarcoma, alveolar soft part sarcoma, ameloblastic fibroma, anaplastic large cell lymphoma, anaplastic thyroid cancer, angioimmunoblastic T-cell lymphoma, angiomyolipoma, angiosarcoma, astrocytoma, atypical teratoid rhabdoid tumor, B-cell chronic lymphocytic leukemia, B-cell prolymphocytic leukemia, B-cell lymphoma, basal cell carcinoma, biliary tract cancer, bladder cancer, blastoma, bone cancer, Brenner tumor, Brown tumor, Burkitt's lymphoma, breast cancer, brain cancer, carcinoma, carcinoma in situ, carcinosarcoma, cartilage tumor, cementoma, myeloid sarcoma, chondroma, chordoma, choriocarcinoma, choroid plexus papilloma, clear-cell sarcoma of the kidney, craniopharyngioma, cutaneous T-cell lymphoma, cervical cancer, colorectal cancer, Degos disease, desmoplastic small round cell tumor, diffuse large B-cell lymphoma, dysembryoplastic neuroepithelial tumor, dysgerminoma, embryonal carcinoma, endocrine gland neoplasm, endodermal sinus tumor, enteropathy-associated T-cell lymphoma, esophageal cancer, fetus in fetu, fibroma, fibrosarcoma, follicular lymphoma, follicular thyroid cancer, ganglioneuroma, gastrointestinal cancer, germ cell tumor, gestational choriocarcinoma, giant cell fibroblastoma, giant cell tumor of the bone, glial tumor, glioblastoma multiforme, glioma, gliomatosis cerebri, glucagonoma, gonadoblastoma, granulosa cell tumor, gynandroblastoma, gallbladder cancer, gastric cancer, hairy cell leukemia, hemangioblastoma, head and neck cancer, hemangiopericytoma, hematological malignancy, hepatoblastoma, hepatosplenic T-cell lymphoma, Hodgkin's lymphoma, non-Hodgkin's lymphoma, invasive lobular carcinoma, intestinal cancer, kidney cancer, laryngeal cancer, lentigo maligna, lethal midline carcinoma, leukemia, leydig cell tumor, liposarcoma, lung cancer, lymphangioma, lymphangiosarcoma, lymphoepithelioma, lymphoma, acute lymphocytic leukemia, acute myelogeous leukemia, chronic lymphocytic leukemia, liver cancer, small cell lung cancer, non-small cell lung cancer, MALT lymphoma, malignant fibrous histiocytoma, malignant peripheral nerve sheath tumor, malignant triton tumor, mantle cell lymphoma, marginal zone B-cell lymphoma, mast cell leukemia, mediastinal germ cell tumor, medullary carcinoma of the breast, medullary thyroid cancer, medulloblastoma, melanoma, meningioma, merkel cell cancer, mesothelioma, metastatic urothelial carcinoma, mixed Mullerian tumor, mucinous tumor, multiple myeloma, muscle tissue neoplasm, mycosis fungoides, myxoid liposarcoma, myxoma, myxosarcoma, nasopharyngeal carcinoma, neurinoma, neuroblastoma, neurofibroma, neuroma, nodular melanoma, ocular cancer, oligoastrocytoma, oligodendroglioma, oncocytoma, optic nerve sheath meningioma, optic nerve tumor, oral cancer, osteosarcoma, ovarian cancer, Pancoast tumor, papillary thyroid cancer, paraganglioma, pinealoblastoma, pineocytoma, pituicytoma, pituitary adenoma, pituitary tumor, plasmacytoma, polyembryoma, precursor T-lymphoblastic lymphoma, primary central nervous system lymphoma, primary effusion lymphoma, preimary peritoneal cancer, prostate cancer, pancreatic cancer, pharyngeal cancer, pseudomyxoma periotonei, renal cell carcinoma, renal medullary carcinoma, retinoblastoma, rhabdomyoma, rhabdomyosarcoma, Richter's transformation, rectal cancer, sarcoma, Schwannomatosis, seminoma, Sertoli cell tumor, sex cord-gonadal stromal tumor, signet ring cell carcinoma, skin cancer, small blue round cell tumors, small cell carcinoma, soft tissue sarcoma, somatostatinoma, soot wart, spinal tumor, splenic marginal zone lymphoma, squamous cell carcinoma, synovial sarcoma, Sezary's disease, small intestine cancer, squamous carcinoma, stomach cancer, T-cell lymphoma, testicular cancer, thecoma, thyroid cancer, transitional cell carcinoma, throat cancer, urachal cancer, urogenital cancer, urothelial carcinoma, uveal melanoma, uterine cancer, verrucous carcinoma, visual pathway glioma, vulvar cancer, vaginal cancer, Waldenstrom's macroglobulinemia, Warthin's tumor, and Wilms' tumor.

**Patentansprüche**

1. Verbindung zur Verwendung bei der Behandlung von Krebs, wobei die Verbindung der Formel I aufweist:

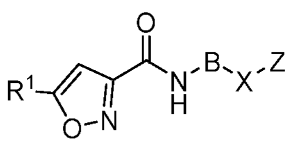

I

oder ein pharmazeutisch unbedenkliches Salz oder Hydrat davon,

wobei:

B für:

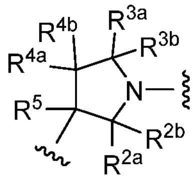

steht;

X aus der Gruppe bestehend aus $-S(=O)_2-$, $-S(=O)_2N(R^6)-$, $-S(=O)_2C(R^7)(H)-$, -C(=O)-, $-C(=O)N(R^6)-$, -C(=O)O- und $-C(=O)C(R^7)(H)-$ ausgewählt ist oder X fehlt;

Z aus der Gruppe bestehend aus Wasserstoff, gegebenenfalls substituiertem $C_{1-6}$-Alkyl, Halogenalkyl, (Cycloalkyl)alkyl, Hydroxyalkyl, (Amino)alkyl, (Alkylamino)alkyl, (Dialkylamino)alkyl, (Cycloalkylamino)-alkyl, (Heterocyclo)alkyl, (Amino)(hydroxy)alkyl, (Amino)(aryl)alkyl, (Hydroxy)(aryl)alkyl, (Aralkylamino)alkyl, Alkoxyalkyl, gegebenenfalls substituiertem $C_{6-14}$-Aryl, gegebenenfalls substituiertem 4- bis 14-gliedrigem Heterocyclo, gegebenenfalls substituiertem $C_{5-14}$-Heteroaryl, gegebenenfalls substituiertem $C_{3-12}$-Cycloalkyl, Aralkyl, Heteroaralkyl und $-CH_2N(H)C(=O)R^{8c}$ ausgewählt ist;

$R^1$ aus der Gruppe bestehend aus Wasserstoff, Cyano, $C_{1-6}$-Alkyl, Halogenalkyl, gegebenenfalls substituiertem 4-bis 14-gliedrigem Heterocyclo, Alkinyl und $C_{3-6}$-Cycloalkyl ausgewählt ist;

$R^{2a}$, $R^{2b}$, $R^{3a}$, $R^{3b}$, $R^{4a}$, and $R^{4b}$ jeweils unabhängig aus der Gruppe bestehend aus Wasserstoff, Amino, Alkylamino, Dialkylamino, Cycloalkylamino, Halogen, Hydroxy, $C_{1-6}$-Alkyl, Alkoxy, Halogenalkyl, Hydroxyalkyl, (Amino)-alkyl, (Alkylamino)alkyl, (Dialkylamino)alkyl, (Cycloalkylamino)alkyl, (Heterocyclo)alkyl, gegebenenfalls substituiertem $C_{3-12}$-Cycloalkyl, gegebenenfalls substituiertem $C_{6-14}$-Aryl, gegebenenfalls substituiertem 4- bis 14-gliedrigem Heterocyclo, gegebenenfalls substituiertem $C_{5-14}$-Heteroaryl, Alkoxyalkyl, Aralkyl, Alkoxycarbonyl, Sulfonamido, Carboxamido, $-N(H)C(=O)R^{8a}$ und $-CH_2N(H)C(=O)R^{8b}$ ausgewählt sind; oder

$R^{2a}$ und $R^{2b}$ zusammen mit dem Kohlenstoffatom, an das sie gebunden sind, ein Carbonyl bilden und $R^{3a}$, $R^{3b}$, $R^{4a}$ und $R^{4b}$ jeweils unabhängig aus der Gruppe bestehend aus Wasserstoff, Hydroxy, $C_{1-6}$-Alkyl, Alkoxy, Hydroxyalkyl, $C_{3-12}$-Cycloalkyl, gegebenenfalls substituiertem $C_{6-14}$-Aryl, Alkoxyalkyl, Aralkyl, $-N(H)C(=O)R^{8a}$ und $-CH_2N(H)C(=O)R^{8b}$ ausgewählt sind; oder

$R^{3a}$ und $R^{3b}$ zusammen mit dem Kohlenstoffatom, an das sie gebunden sind, ein Carbonyl bilden und $R^{2a}$, $R^{2b}$, $R^{4a}$ und $R^{4b}$ jeweils unabhängig aus der Gruppe bestehend aus Wasserstoff, Hydroxy, $C_{1-6}$-Alkyl, Alkoxy, Hydroxyalkyl, $C_{3-12}$-Cycloalkyl, gegebenenfalls substituiertem $C_{6-14}$-Aryl, Alkoxyalkyl, $-N(H)C(=O)R^{8a}$ und $-CH_2N(H)C(=O)R^{8b}$ ausgewählt sind; oder

$R^{4a}$ und $R^{4b}$ zusammen mit dem Kohlenstoffatom, an das sie gebunden sind, ein Carbonyl bilden und $R^{2a}$, $R^{2b}$, $R^{3a}$ und $R^{3b}$ jeweils unabhängig aus der Gruppe bestehend aus Wasserstoff, Hydroxy, $C_{1-6}$-Alkyl, Alkoxy, Hydroxyalkyl, $C_{3-12}$-Cycloalkyl, gegebenenfalls substituiertem $C_{6-14}$-Aryl, Alkoxyalkyl, $-N(H)C(=O)R^{8a}$ und $-CH_2N(H)C(=O)R^{8b}$ ausgewählt sind; oder

$R^{2a}$ und $R^{2b}$ zusammen mit dem Kohlenstoffatom, an das sie gebunden sind, ein $C_{3-6}$-Cycloalkyl oder $C_{3-6}$ Heterocyclo bilden und $R^{3a}$, $R^{3b}$, $R^{4a}$ und $R^{4b}$ jeweils unabhängig aus der Gruppe bestehend aus Wasserstoff, Hydroxy, $C_{1-6}$-Alkyl, Alkoxy, Hydroxyalkyl, $C_{3-12}$-Cycloalkyl, gegebenenfalls substituiertem $C_{6-14}$-Aryl, Alkoxyalkyl, Aralkyl, $-N(H)C(=O)R^{8a}$ und $-CH_2N(H)C(=O)R^{8b}$ ausgewählt sind; oder

$R^{3a}$ und $R^{3b}$ zusammen mit dem Kohlenstoffatom, an das sie gebunden sind, ein $C_{3-6}$-Cycloalkyl oder $C_{3-6}$ Heterocyclo bilden und $R^{2a}$, $R^{2b}$, $R^{4a}$ und $R^{4b}$ jeweils unabhängig aus der Gruppe bestehend aus Wasserstoff, Hydroxy, $C_{1-6}$-Alkyl, Alkoxy, Hydroxyalkyl, $C_{3-12}$-Cycloalkyl, gegebenenfalls substituiertem $C_{6-14}$-Aryl, Alkoxyalkyl, $-N(H)C(=O)R^{8a}$ und $-CH_2N(H)C(=O)R^{8b}$ ausgewählt sind; oder $R^{4a}$ und $R^{4b}$ zusammen mit dem Kohlenstoffatom, an das sie gebunden sind, ein $C_{3-6}$-Cycloalkyl oder $C_{3-6}$-Heterocyclo bilden und $R^{2a}$, $R^{2b}$, $R^{3a}$ und $R^{3b}$ jeweils unabhängig aus der Gruppe bestehend aus Wasserstoff, Hydroxy, $C_{1-6}$-Alkyl, Alkoxy, Hydroxyalkyl, $C_{3-12}$-Cycloalkyl, gegebenenfalls substituiertem $C_{6-14}$-Aryl, Alkoxyalkyl, $-N(H)C(=O)R^{8a}$ und $-CH_2N(H)C(=O)R^{8b}$ ausgewählt sind;

$R^5$ aus der Gruppe bestehend aus Wasserstoff and $C_{1-4}$-Alkyl ausgewählt ist;

$R^6$ aus der Gruppe bestehend aus Wasserstoff and $C_{1-4}$-Alkyl ausgewählt ist;

$R^7$ aus der Gruppe bestehend aus Wasserstoff, $C_{1-4}$-Alkyl, Amino, Alkylamino, Dialkylamino, (Amino)alkyl, (Alkylamino)alkyl, (Dialkylamino)alkyl und Hydroxyalkyl ausgewählt ist;

$R^{8a}$ aus der Gruppe bestehend aus $C_{1-6}$-Alkyl, Halogenalkyl, Hydroxyalkyl, (Amino)alkyl, (Alkylamino)alkyl, (Dialkylamino)alkyl, (Cycloalkylamino)alkyl, (Heterocyclo)alkyl, gegebenenfalls substituiertem $C_{6-14}$-Aryl, ge-

gebenenfalls substituiertem 4- bis 14-gliedrigem Heterocyclo, gegebenenfalls substituiertem 5- bis 14-gliedrigem Heteroaryl, gegebenenfalls substituiertem $C_{3-12}$-Cycloalkyl, Aralkyl and Heteroaralkyl ausgewählt ist; $R^{8b}$ aus der Gruppe bestehend aus $C_{1-6}$-Alkyl, Halogenalkyl, Hydroxyalkyl, (Amino)alkyl, (Alkylamino)alkyl, (Dialkylamino)alkyl, (Cycloalkylamino)alkyl, (Heterocyclo)alkyl, gegebenenfalls substituiertem $C_{6-14}$-Aryl, gegebenenfalls substituiertem 4- bis 14-gliedrigem Heterocyclo, gegebenenfalls substituiertem 5- bis 14-gliedrigem Heteroaryl, gegebenenfalls substituiertem $C_{3-12}$-Cycloalkyl, Aralkyl and Heteroaralkyl ausgewählt ist und $R^{8c}$ aus der Gruppe bestehend aus $C_{1-6}$-Alkyl, Halogenalkyl, Hydroxyalkyl, (Amino)alkyl, (Alkylamino)alkyl, (Dialkylamino)alkyl, (Cycloalkylamino)alkyl, (Heterocyclo)alkyl, gegebenenfalls substituiertem $C_{6-14}$-Aryl, gegebenenfalls substituiertem 4- bis 14-gliedrigem Heterocyclo, gegebenenfalls substituiertem 5- bis 14-gliedrigem Heteroaryl, gegebenenfalls substituiertem $C_{3-12}$-Cycloalkyl, Aralkyl and Heteroaralkyl ausgewählt ist.

**2.** Verbindung zur Verwendung nach Anspruch 1 oder ein pharmazeutisch unbedenkliches Salz oder Hydrat davon, wobei B aus der Gruppe bestehend aus:

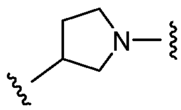 , 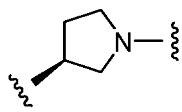 und 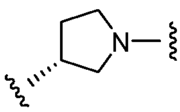

ausgewählt ist.

**3.** Verbindung zur Verwendung nach Anspruch 1 oder ein pharmazeutisch unbedenkliches Salz oder Hydrat davon, wobei B aus der Gruppe bestehend aus:

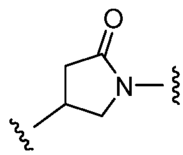 , 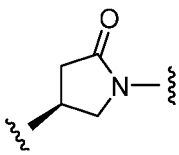 und 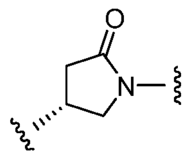

ausgewählt ist.

**4.** Verbindung zur Verwendung nach Anspruch 1 oder ein pharmazeutisch unbedenkliches Salz oder Hydrat davon, wobei B aus der Gruppe bestehend aus:

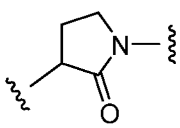 , 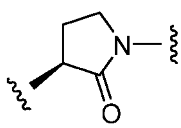 und 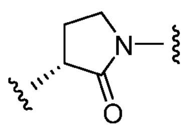

ausgewählt ist.

**5.** Verbindung zur Verwendung nach Anspruch 1 oder ein pharmazeutisch unbedenkliches Salz oder Hydrat davon, wobei B aus:

(a) der Gruppe bestehend aus:

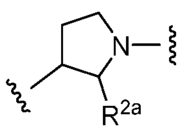 , 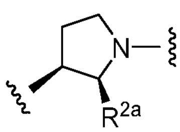 , 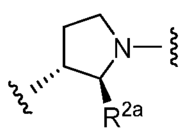 ,

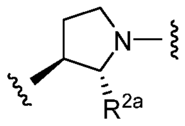 und 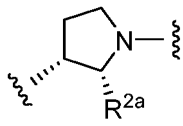

ausgewählt ist und

$R^{2a}$ aus der Gruppe bestehend aus $C_{1-6}$-Alkyl, $C_{3-12}$-Cycloalkyl and gegebenenfalls substituiertem $C_{6-14}$-Aryl ausgewählt ist, gegebenenfalls wobei $R^{2a}$ aus der Gruppe bestehend aus:

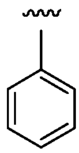, 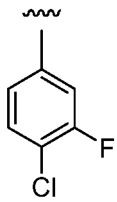, 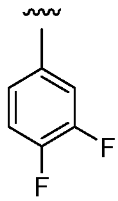, 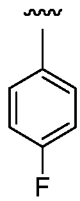 und 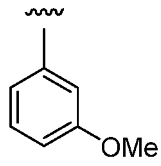

ausgewählt ist; oder

(b) aus der Gruppe bestehend aus:

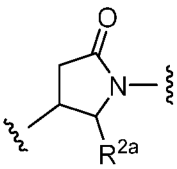, 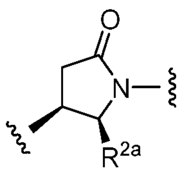, 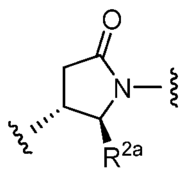,

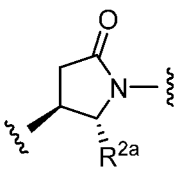 und 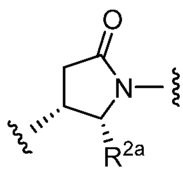

ausgewählt ist und

$R^{2a}$ aus der Gruppe bestehend aus $C_{1-6}$-Alkyl, $C_{3-12}$-Cycloalkyl und gegebenenfalls substituiertem $C_{6-14}$-Aryl ausgewählt ist, gegebenenfalls wobei $R^{2a}$ aus der Gruppe bestehend aus:

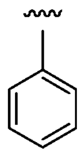, 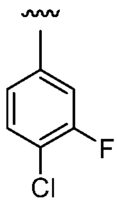, 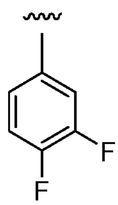, 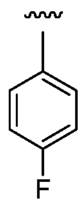 und 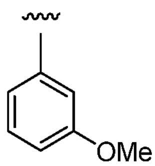

ausgewählt ist.

**6.** Verbindung zur Verwendung nach Anspruch 1 oder ein pharmazeutisch unbedenkliches Salz oder Hydrat davon, wobei B aus:

(a) der Gruppe bestehend aus:

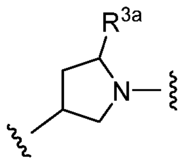 , 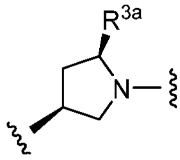 , 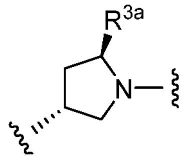 ,

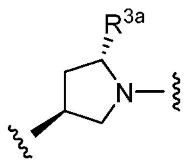 und 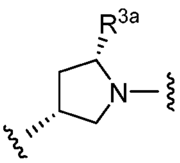

ausgewählt ist und

$R^{3a}$ aus der Gruppe bestehend aus $C_{1-6}$-Alkyl, Hydroxyalkyl, $C_{3-12}$-Cycloalkyl, gegebenenfalls substituiertem $C_{6-14}$-Aryl, Alkoxyalkyl und $-CH_2N$ (H) C (=O)$R^{8b}$ ausgewählt ist, gegebenenfalls wobei $R^{3a}$ aus der Gruppe bestehend aus:

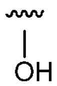 , 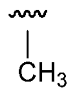 , 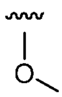 , 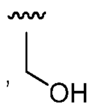 , 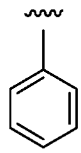 , 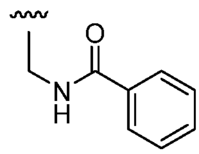 ,

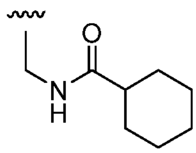 , 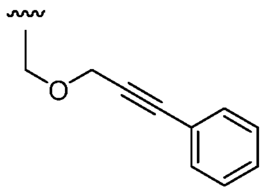 und 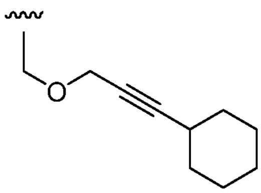

ausgewählt ist; oder

(b) der Gruppe bestehend aus:

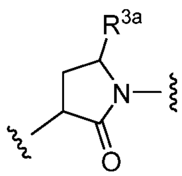 , 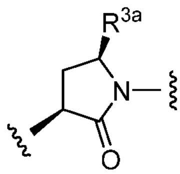 , 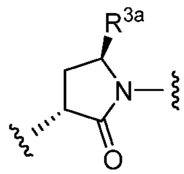 ,

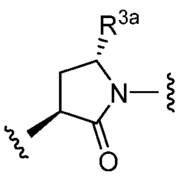 and 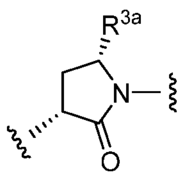 ;

and

$R^{3a}$ aus der Gruppe bestehend aus $C_{1-6}$-Alkyl, Hydroxyalkyl, $C_{3-12}$-Cycloalkyl, gegebenenfalls substituiertem $C_{6-14}$-Aryl, Alkoxyalkyl und $-CH_2N$ (H) C(=O)$R^{8b}$ ausgewählt ist, gegebenenfalls wobei $R^{3a}$ aus der Gruppe bestehend aus:

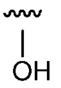 , 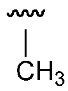 , 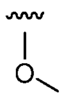 , 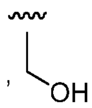 , 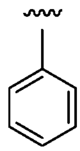 , 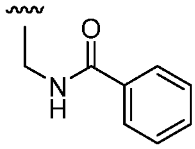 ,

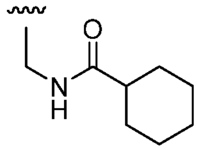 , 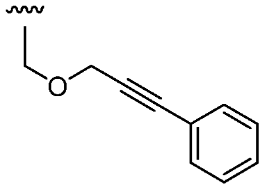 und 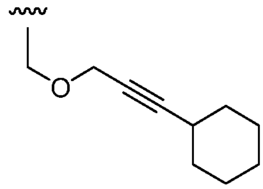

ausgewählt ist; oder

(c) der Gruppe bestehend aus

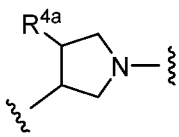 , 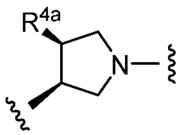 , 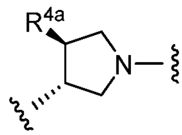 ,

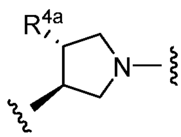 und 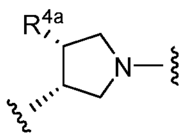

ausgewählt ist und

$R^{4a}$ aus der Gruppe bestehend aus Hydroxy, $C_{1-6}$-Alkyl, Alkoxy, Hydroxyalkyl, $C_{3-12}$-Cycloalkyl, gegebenenfalls substituiertem $C_{6-14}$-Aryl, Alkoxyalkyl, Aralkyl, -N(H)C(=O)$R^{8a}$ and -$CH_2$N (H) C(=O)$R^{8b}$ ausgewählt ist, gegebenenfalls wobei $R^{4a}$ aus der Gruppe bestehend aus:

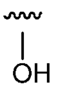 , 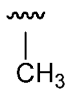 , 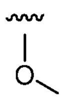 , 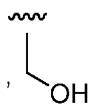 , 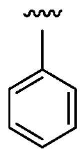 , 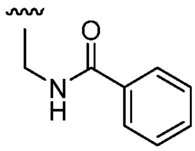 ,

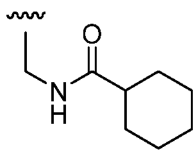 , 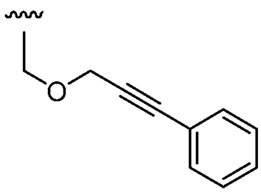 und 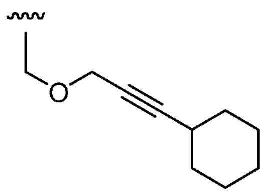

ausgewählt ist; oder

(d) der Gruppe bestehend aus:

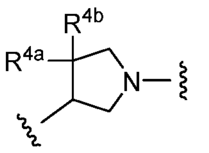 , 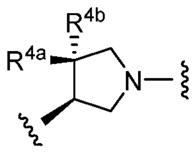 , 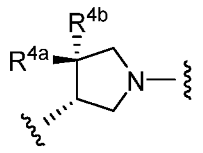 ,

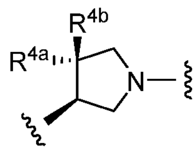 und 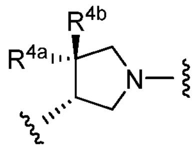

ausgewählt ist;
$R^{4a}$ für $C_{1-4}$-Alkyl steht und $R^{4b}$ für Hydroxy steht.

**7.** Verbindung zur Verwendung nach einem der Ansprüche 1-6 oder ein pharmazeutisch unbedenkliches Salz oder Hydrat davon, wobei X:

(a) aus der Gruppe bestehend aus -S(=O)$_2$- und -C(=O)-ausgewählt ist oder X fehlt; oder
(b) für -S(=O)$_2$- steht; oder
(c) für -C(=O)- steht; oder
(d) fehlt.

**8.** Verbindung zur Verwendung nach einem der Ansprüche 1-7 oder ein pharmazeutisch unbedenkliches Salz oder Hydrat davon, wobei Z aus der Gruppe bestehend aus gegebenenfalls substituiertem $C_{1-6}$-Alkyl, Hydroxyalkyl, (Amino)alkyl, (Alkylamino)alkyl, (Dialkylamino)alkyl, (Cycloalkylamino)alkyl, (Heterocyclo)alkyl, gegebenenfalls substituiertem $C_{6-14}$-Aryl, gegebenenfalls substituiertem 4- bis 14-gliedrigem Heterocyclo, gegebenenfalls substituiertem 5- bis 14-gliedrigem Heteroaryl, gegebenenfalls substituiertem $C_{3-12}$-Cycloalkyl, Aralkyl und Heteroaralkyl ausgewählt ist und gegebenenfalls wobei Z aus der Gruppe bestehend aus gegebenenfalls substituiertem $C_{1-6}$-Alkyl, (Amino)alkyl, (Alkylamino)alkyl, (Heterocyclo)alkyl, gegebenenfalls substituiertem $C_{6-14}$-Aryl, gegebenenfalls substituiertem 4- bis 14-gliedrigem Heterocyclo, gegebenenfalls substituiertem 5- bis 14-gliedrigem Heteroaryl, gegebenenfalls substituiertem $C_{3-12}$-Cycloalkyl, Aralkyl und Heteroaralkyl ausgewählt ist.

**9.** Verbindung zur Verwendung nach Anspruch 1 oder ein pharmazeutisch unbedenkliches Salz oder Hydrat davon, wobei $R^1$ für Ethyl oder Cyclopropyl steht; gegebenenfalls wobei X aus der Gruppe bestehend aus -S(=O)$_2$-, -S(=O)$_2$N($R^6$)-, -S(=O)$_2$C($R^7$)(H)-, -C(=O)-, -C(=O)N($R^6$)-, -C(=O)O- und -C(=O)C($R^7$)(H) ausgewählt ist.

**10.** Verbindung zur Verwendung nach Anspruch 1, wobei die Verbindung aus Tabelle 1:

| Verb. Nr. | Struktur |
|---|---|
| 1 | 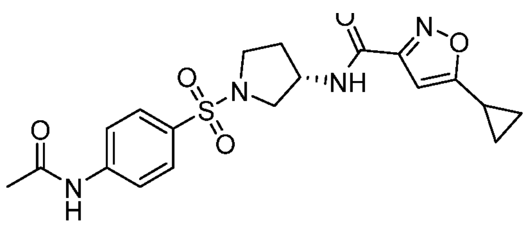 |
| 2 | 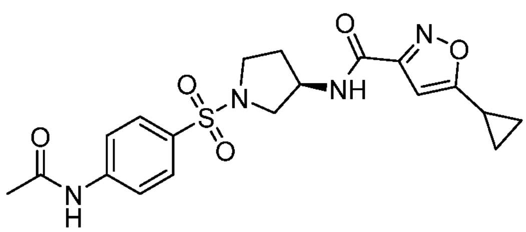 |

(fortgesetzt)

| Verb. Nr. | Struktur |
|---|---|
| 3 | 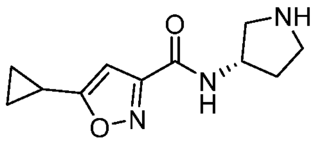 |
| 4 | 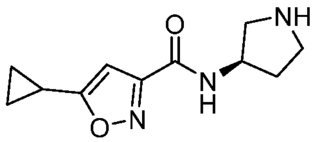 |
| 5 | 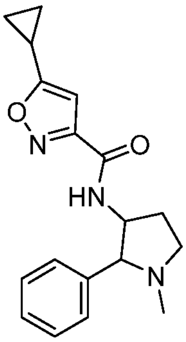 |
| 6 | 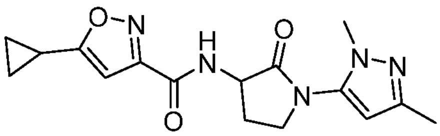 |
| 7 | 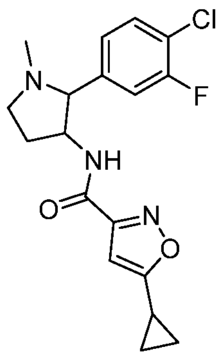 |
| 8 | 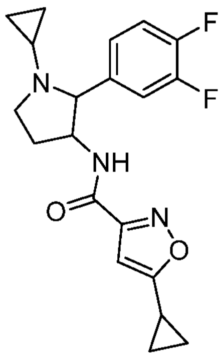 |

(fortgesetzt)

| Verb. Nr. | Struktur |
|---|---|
| 9 | 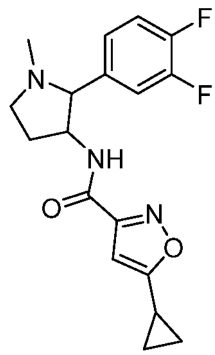 |
| 10 | 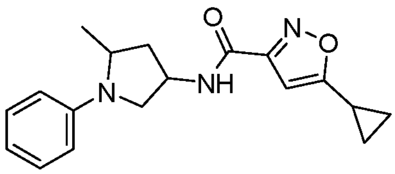 |
| 11 | 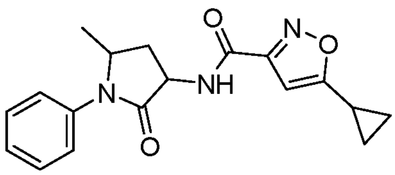 |
| 12 | 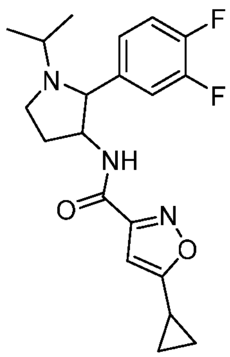 |
| 13 | 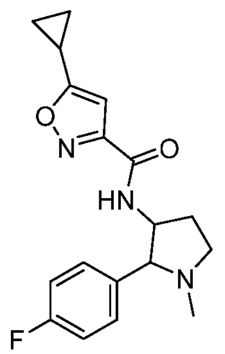 |

(fortgesetzt)

| Verb. Nr. | Struktur |
|---|---|
| 14 | 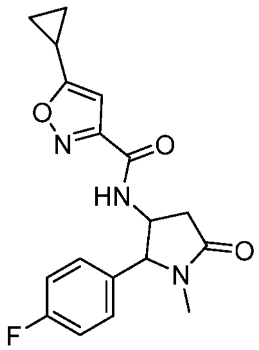 |
| 15 | 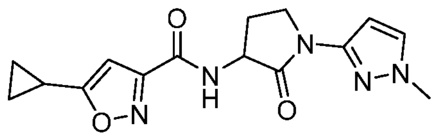 |
| 16 | 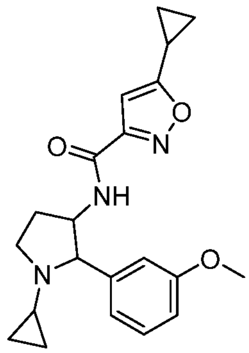 |
| 17 | 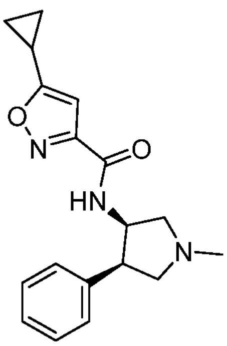 |
| 18 | 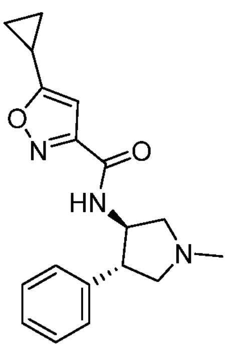 |

(fortgesetzt)

| Verb. Nr. | Struktur |
|---|---|
| 19 | 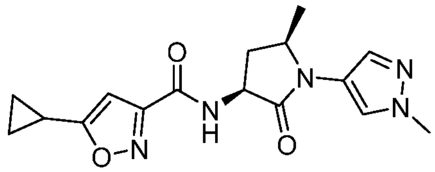 |
| 20 | 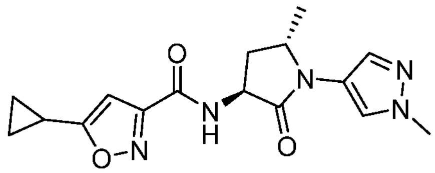 |
| 21 | 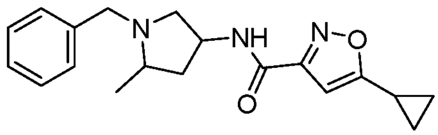 |
| 22 | 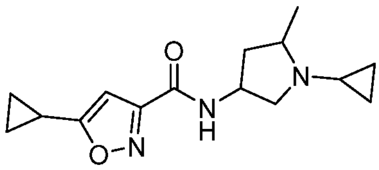 |
| 23 | 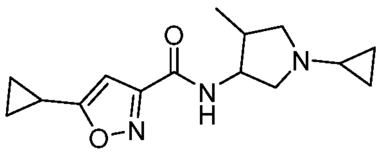 |
| 24 | 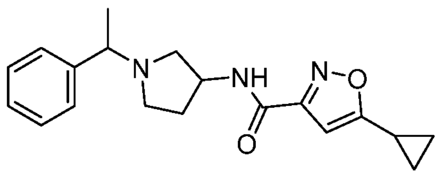 |
| 25 | 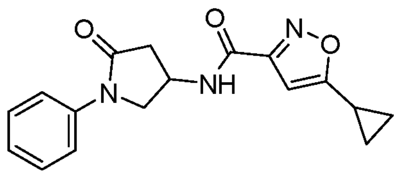 |
| 26 | 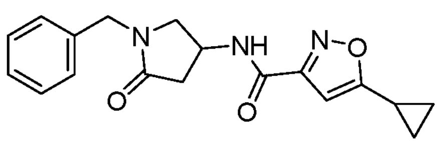 |
| 27 | 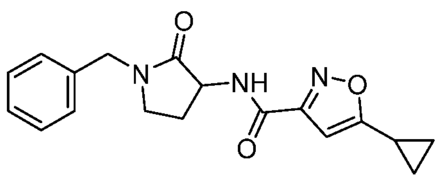 |

(fortgesetzt)

| Verb. Nr. | Struktur |
|---|---|
| 28 | 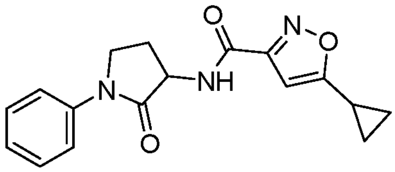 |
| 29 | 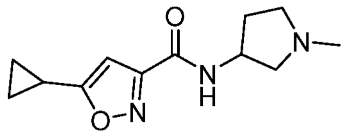 |
| 30 | 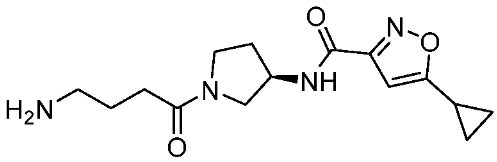 |
| 31 | 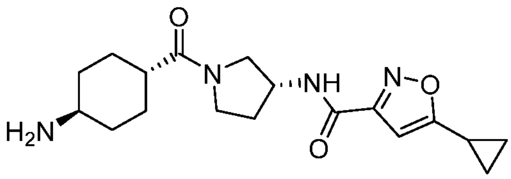 |
| 32 | 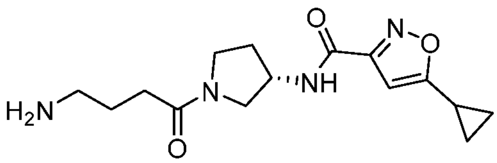 |
| 33 | 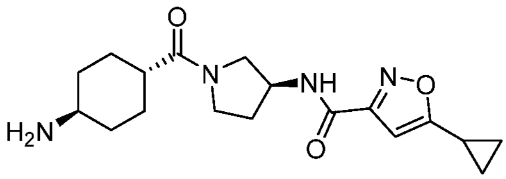 |
| 34 | 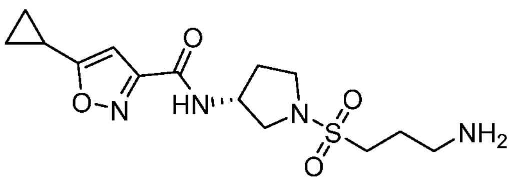 |
| 35 | 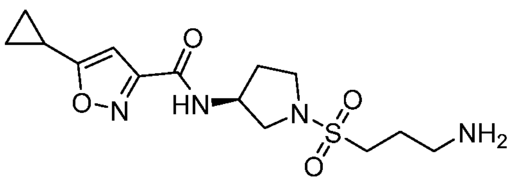 |
| 36 | 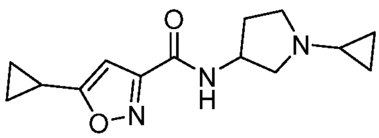 |

(fortgesetzt)

| Verb. Nr. | Struktur |
|---|---|
| 37 | 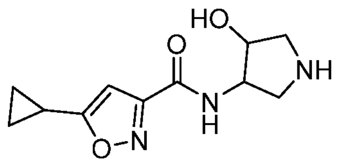 |
| 38 | 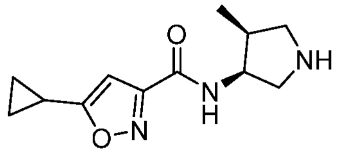 |
| 39 | 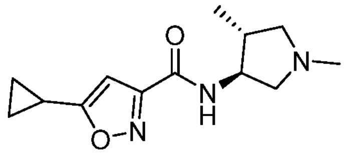 |
| 40 | 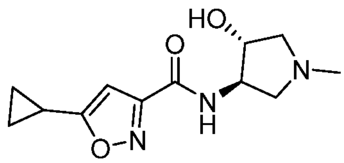 |
| 41 | 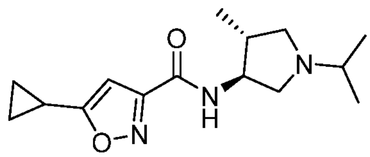 |
| 42 | 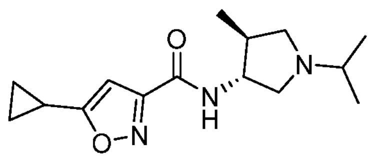 |
| 43 | 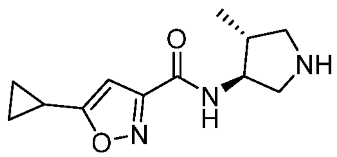 |
| 44 | 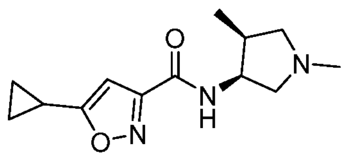 |
| 45 | 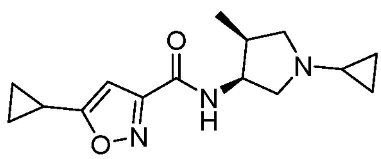 |

(fortgesetzt)

| Verb. Nr. | Struktur |
|---|---|
| 46 | 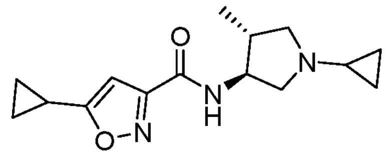 |
| 47 | 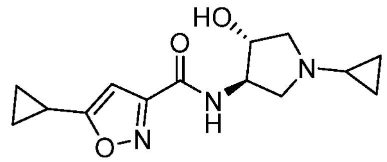 |
| 48 | 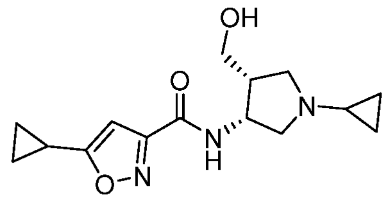 |
| 49 | 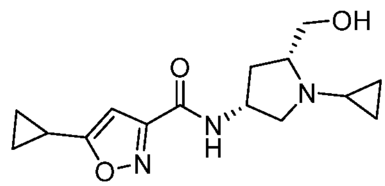 |
| 50 | 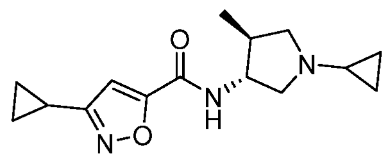 |
| 51 | 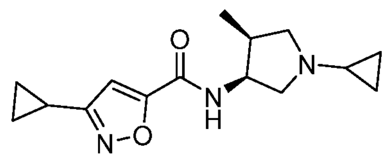 |
| 52 | 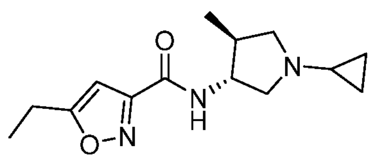 |
| 53 | 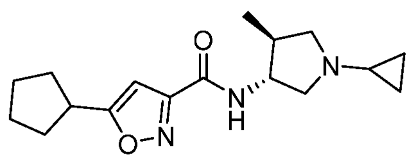 |
| 54 | 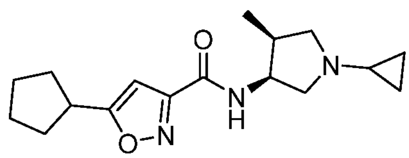 |

(fortgesetzt)

| Verb. Nr. | Struktur |
|---|---|
| 112 | 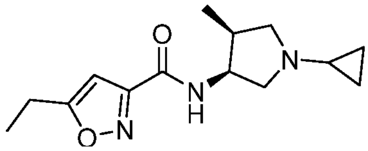 |

oder Tabelle 2:

| Verb. Nr. | Struktur |
|---|---|
| 55 | 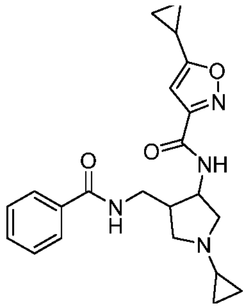 |
| 56 | 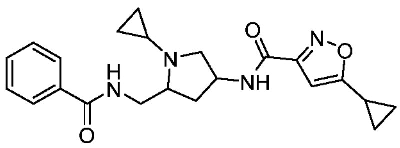 |
| 57 | 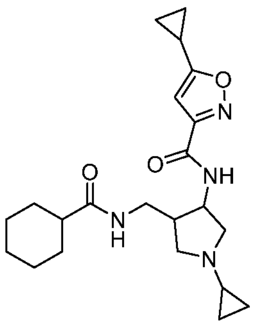 |
| 58 | 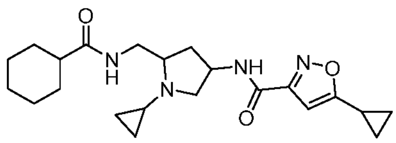 |
| 59 | 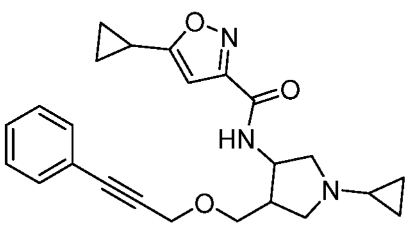 |
| 60 | 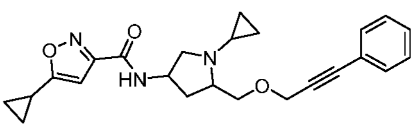 |

(fortgesetzt)

| Verb. Nr. | Struktur |
|---|---|
| 61 | 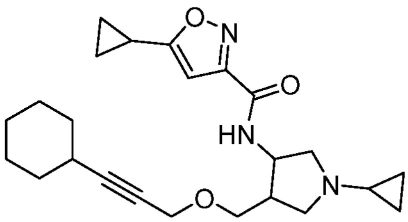 |
| 62 | 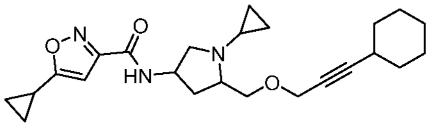 |
| 63 | 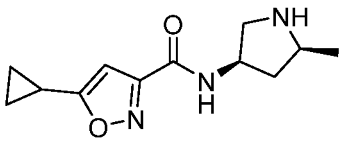 |
| 64 | 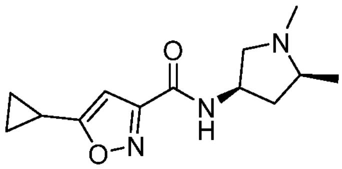 |
| 65 | 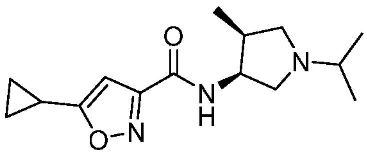 |
| 66 | 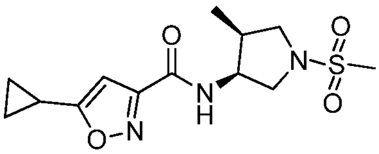 |
| 67 | 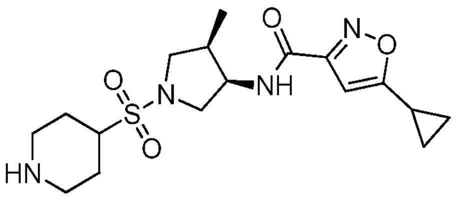 |
| 68 | 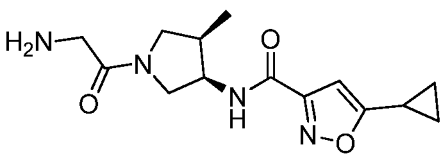 |
| 69 | 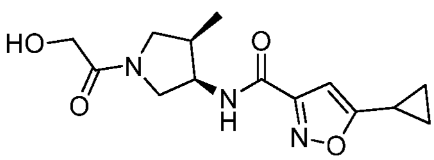 |

(fortgesetzt)

| Verb. Nr. | Struktur |
| --- | --- |
| 70 | 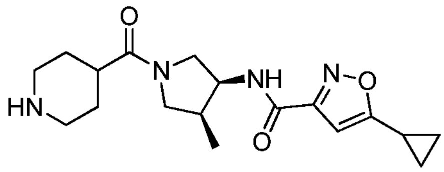 |
| 71 | 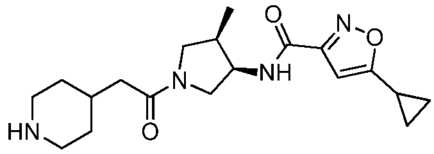 |
| 72 | 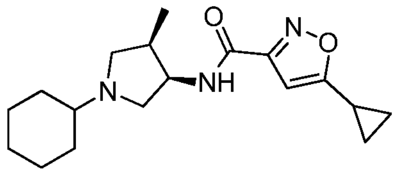 |
| 73 | 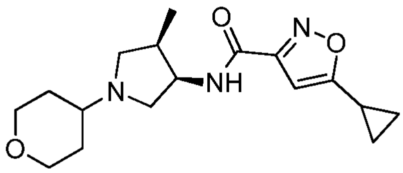 |
| 74 | 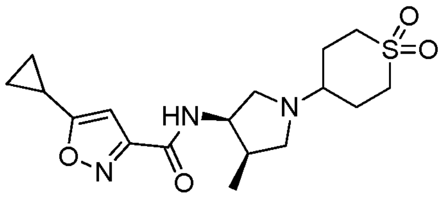 |
| 75 | 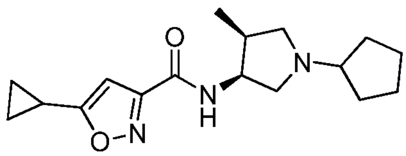 |
| 76 | 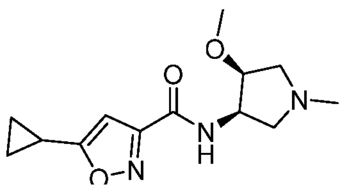 |
| 77 | 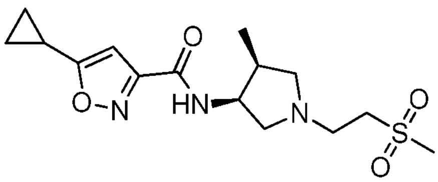 |

(fortgesetzt)

| Verb. Nr. | Struktur |
|---|---|
| 78 | 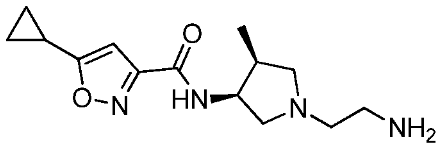 |
| 79 | 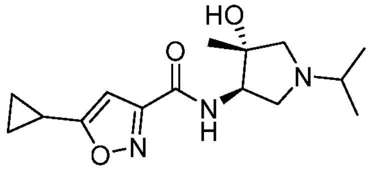 |
| 80 | 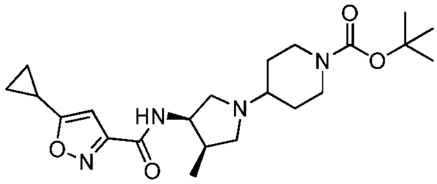 |
| 81 | 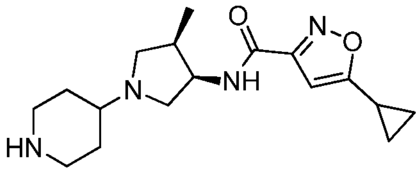 |
| 82 | 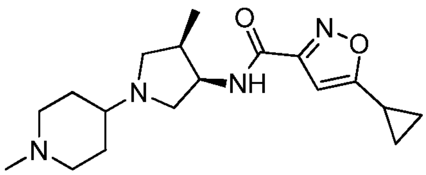 |
| 83 | 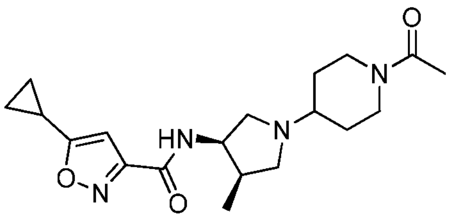 |
| 84 | 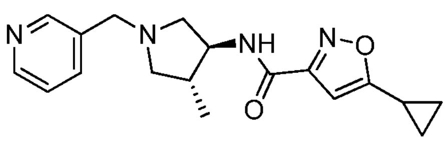 |
| 85 | 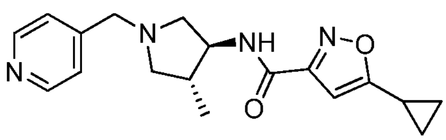 |

(fortgesetzt)

| Verb. Nr. | Struktur |
|---|---|
| 86 | 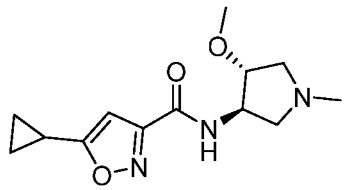 |
| 87 | 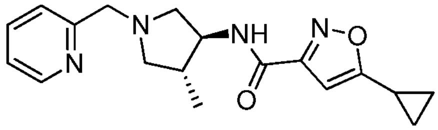 |
| 88 | 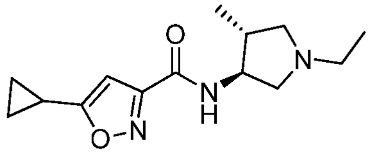 |
| 89 | 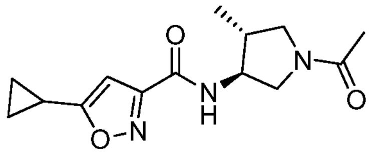 |
| 90 | 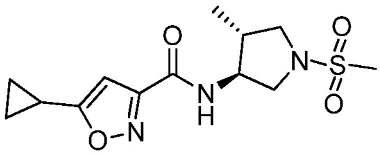 |
| 91 | 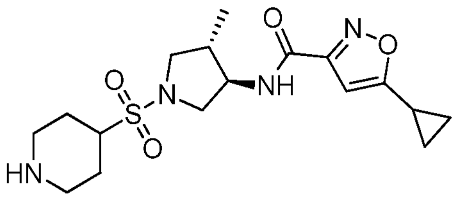 |
| 92 | 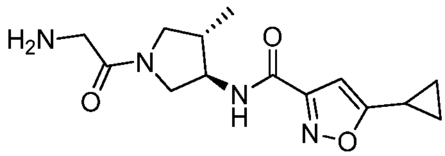 |
| 93 | 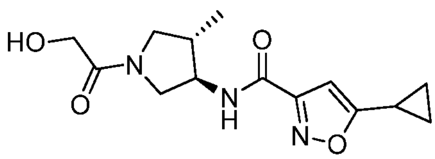 |
| 94 | 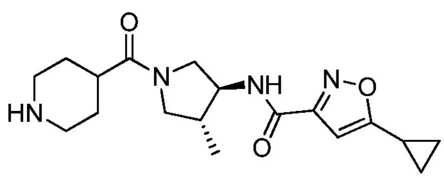 |

(fortgesetzt)

| Verb. Nr. | Struktur |
|---|---|
| 95 | 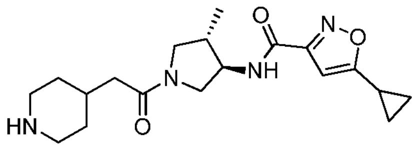 |
| 96 | 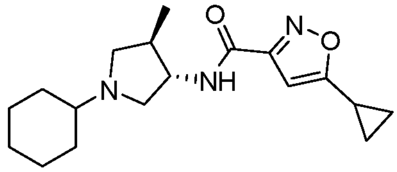 |
| 97 | 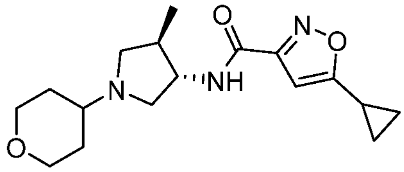 |
| 98 | 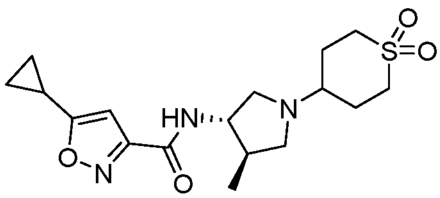 |
| 99 | 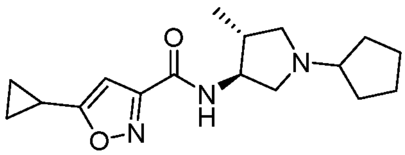 |
| 100 | 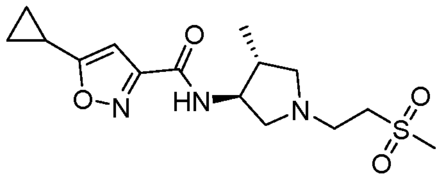 |
| 101 | 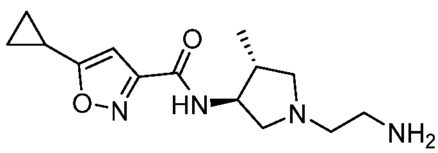 |
| 102 | 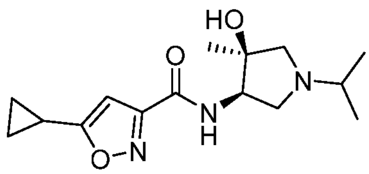 |

(fortgesetzt)

| Verb. Nr. | Struktur |
|---|---|
| 103 | 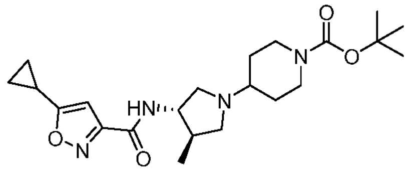 |
| 104 | 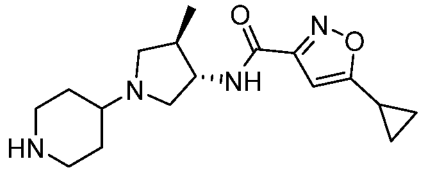 |
| 105 | 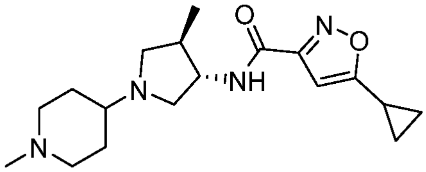 |
| 106 | 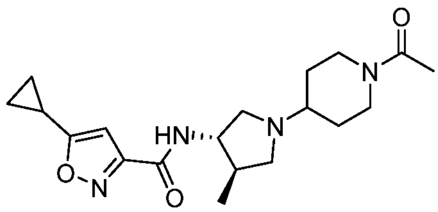 |
| 107 | 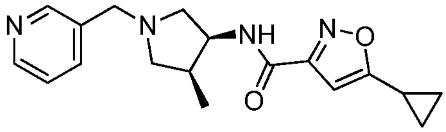 |
| 108 | 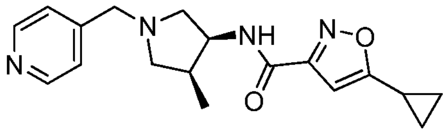 |
| 109 | 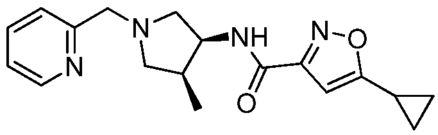 |
| 110 | 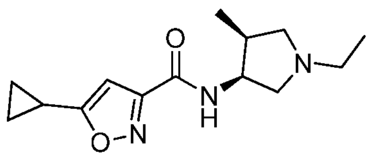 |
| 111 | 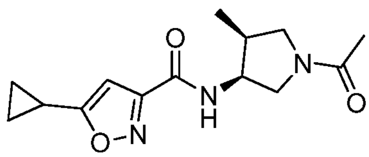 |

oder Tabelle 3:

| Verb. Nr. | Struktur |
|---|---|
| 113 | 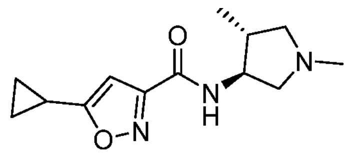 |
| 114 | 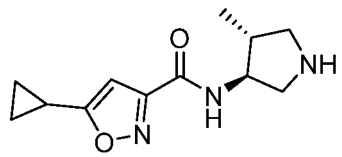 |
| 115 | 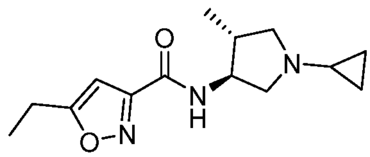 |
| 116 | 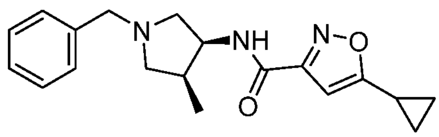 |
| 117 | 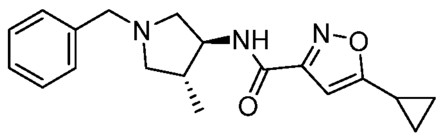 |
| 118 | 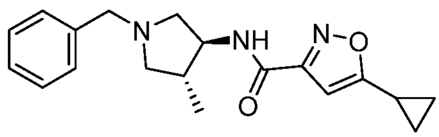 |
| 119 | 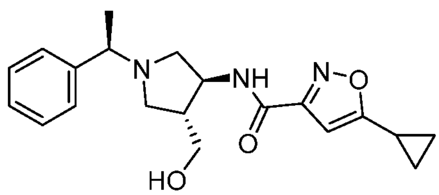 |
| 120 | 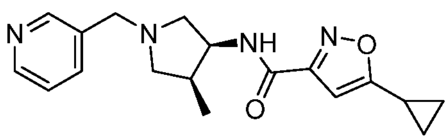 |
| 121 | 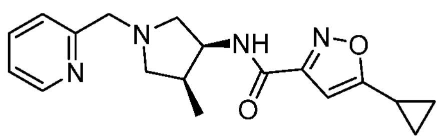 |

(fortgesetzt)

| Verb. Nr. | Struktur |
|---|---|
| 122 | 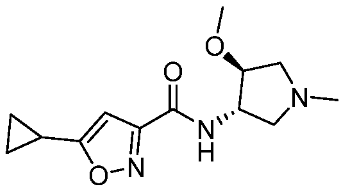 |
| 123 | 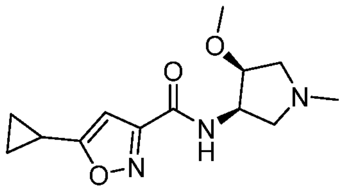 |
| 124 | 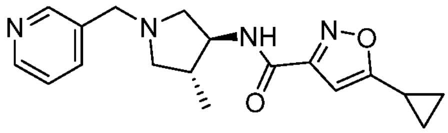 |
| 125 | 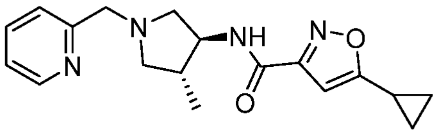 |
| 126 | 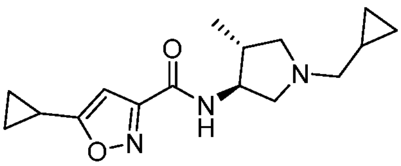 |
| 127 | 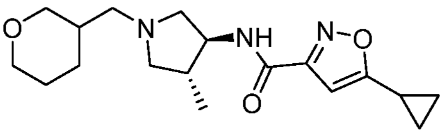 |
| 128 | 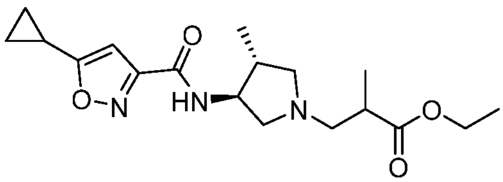 |
| 129 | 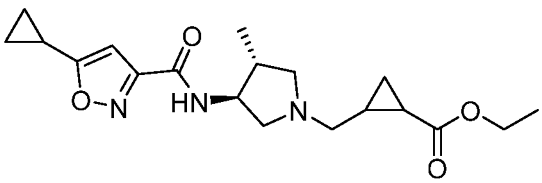 |
| 130 | 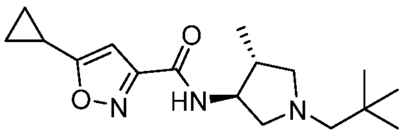 |

(fortgesetzt)

| Verb. Nr. | Struktur |
|---|---|
| 131 | 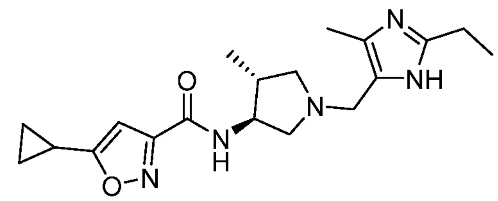 |
| 132 | 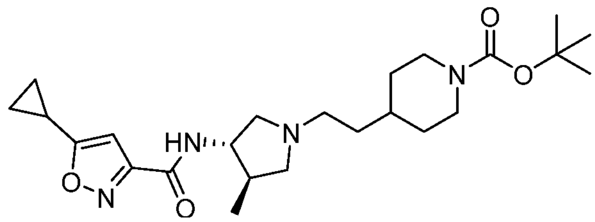 |
| 133 | 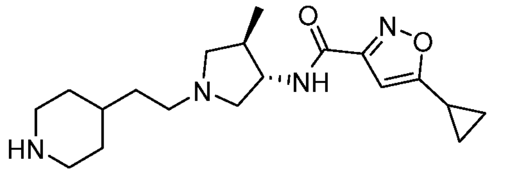 |
| 134 | 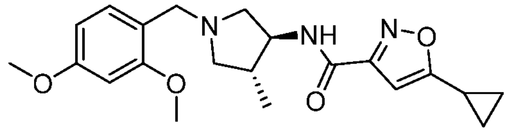 |
| 135 | 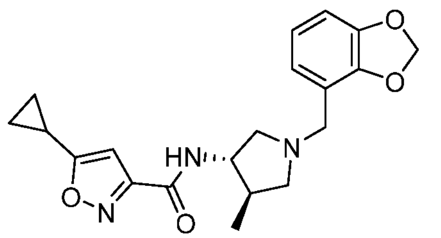 |
| 136 | 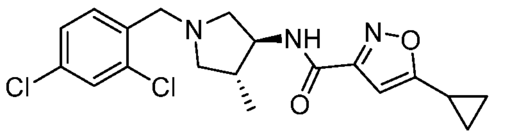 |
| 137 | 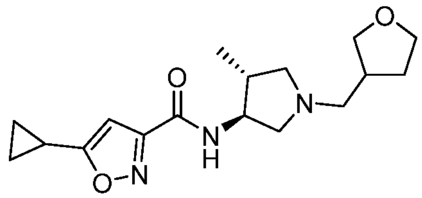 |
| 138 | 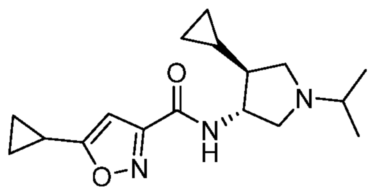 |

(fortgesetzt)

| Verb. Nr. | Struktur |
|---|---|
| 139 | 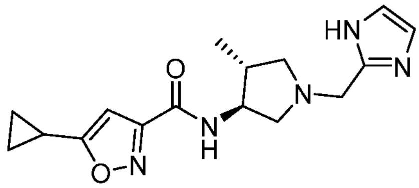 |
| 140 | 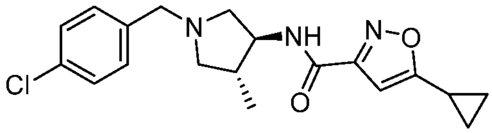 |
| 141 | 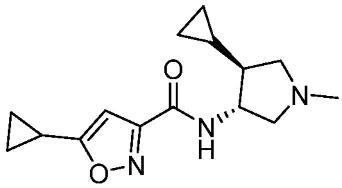 |
| 142 | 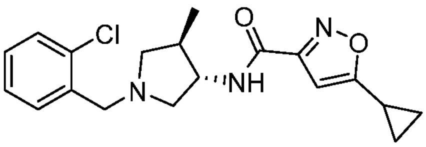 |
| 143 | 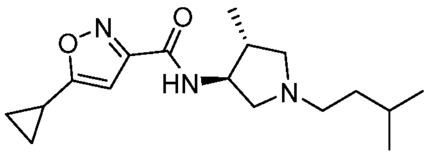 |
| 144 | 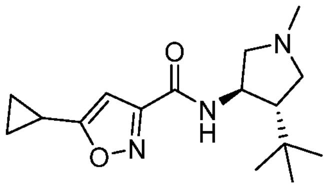 |
| 146 | 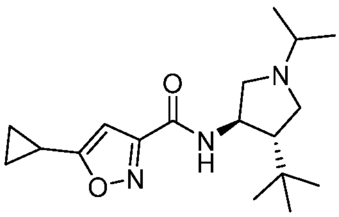 |
| 148 | 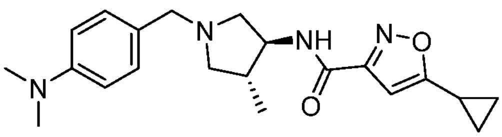 |
| 149 | 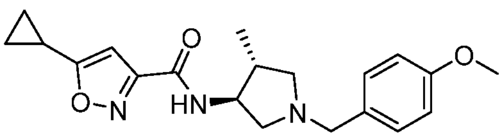 |

(fortgesetzt)

| Verb. Nr. | Struktur |
|---|---|
| 150 | 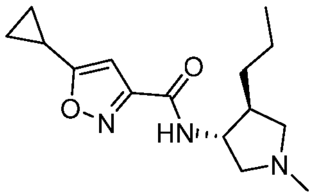 |
| 151 | 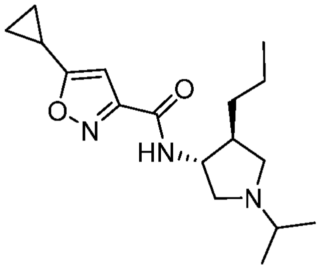 |
| 152 | 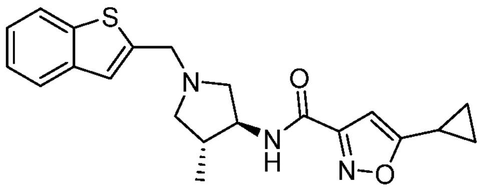 |
| 153 | 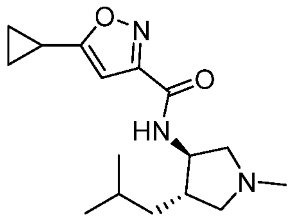 |
| 154 | 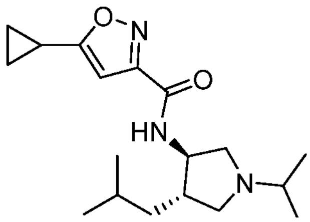 |
| 155 | 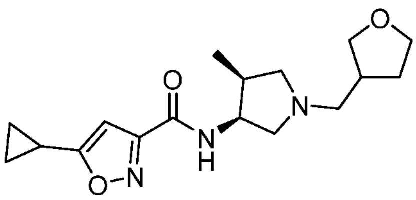 |
| 156 | 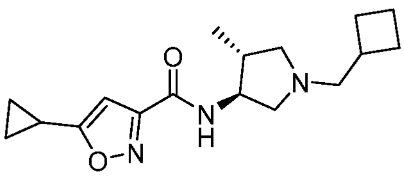 |

(fortgesetzt)

| Verb. Nr. | Struktur |
|---|---|
| 157 | 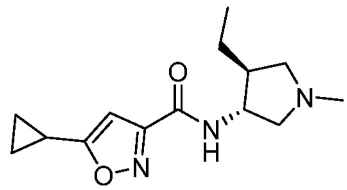 |
| 158 | 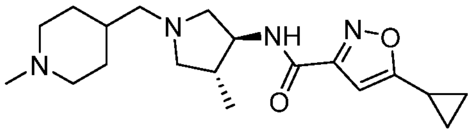 |
| 159 | 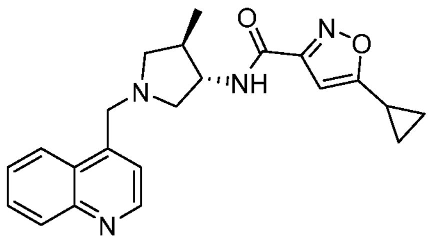 |
| 160 | 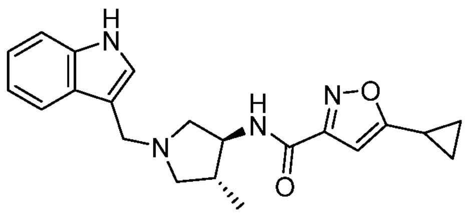 |
| 161 | 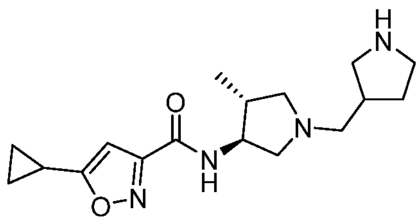 |
| 162 | 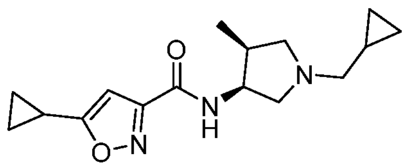 |
| 163 | 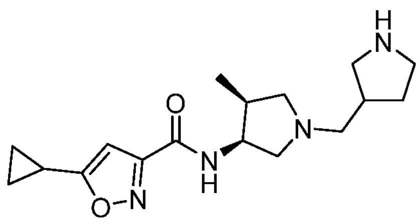 |
| 164 | 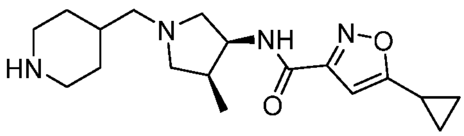 |

(fortgesetzt)

| Verb. Nr. | Struktur |
|---|---|
| 165 | 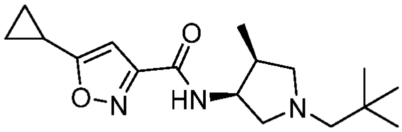 |
| 166 | 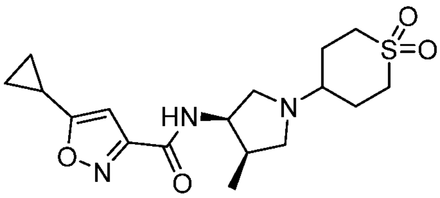 |
| 168 | 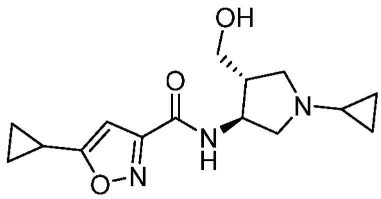 |
| 169 | 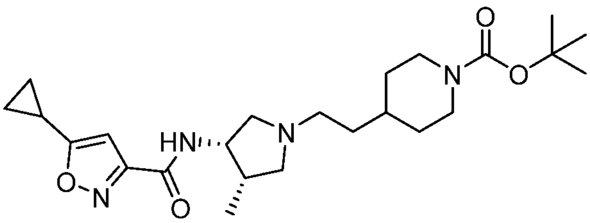 |
| 170 | 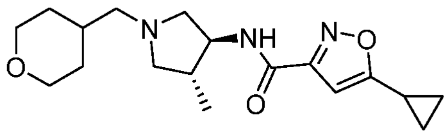 |
| 171 | 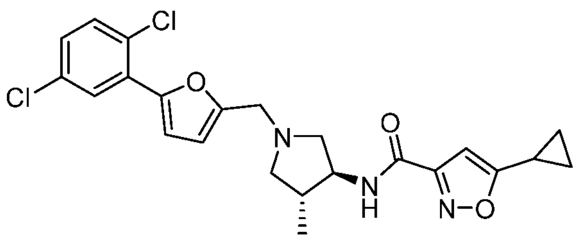 |
| 172 | 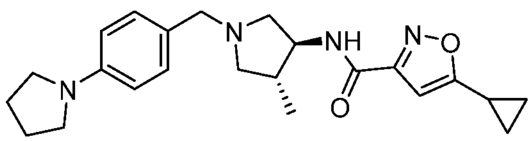 |
| 173 | 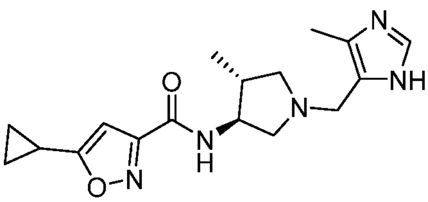 |

(fortgesetzt)

| Verb. Nr. | Struktur |
|---|---|
| 174 | 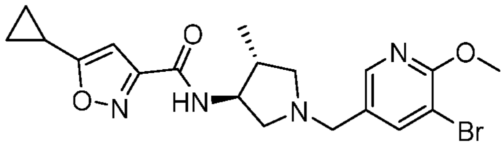 |
| 175 | 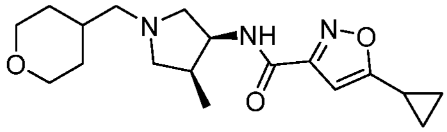 |
| 176 | 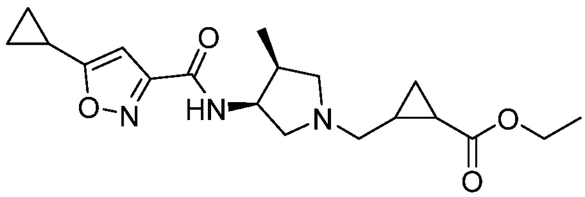 |
| 177 | 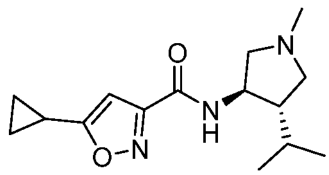 |
| 178 | 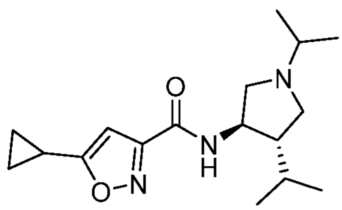 |
| 179 | 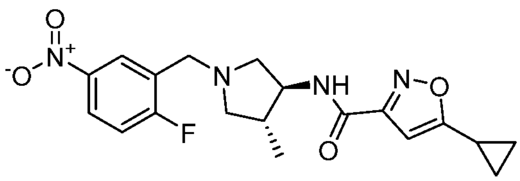 |
| 180 | 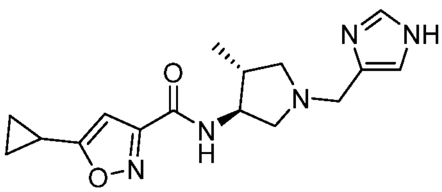 |
| 181 | 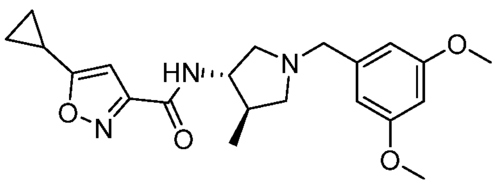 |

(fortgesetzt)

| Verb. Nr. | Struktur |
|---|---|
| 182 | 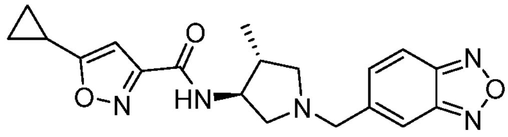 |
| 183 | 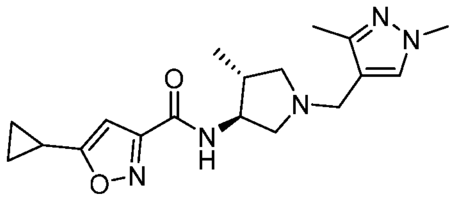 |
| 184 | 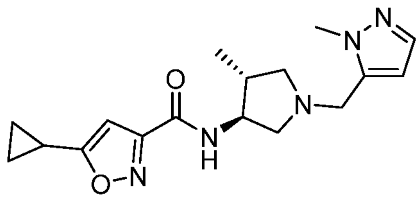 |
| 185 | 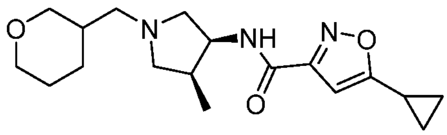 |
| 186 | 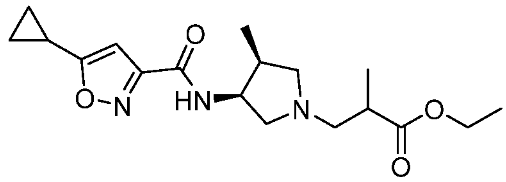 |
| 187 | 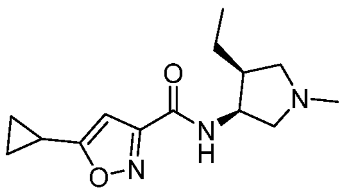 |
| 188 | 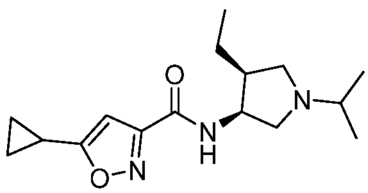 |
| 189 | 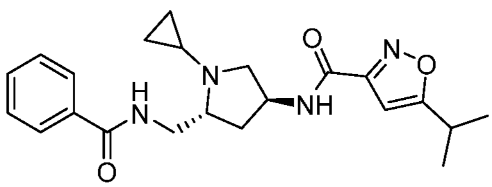 |

(fortgesetzt)

| Verb. Nr. | Struktur |
|---|---|
| 190 | 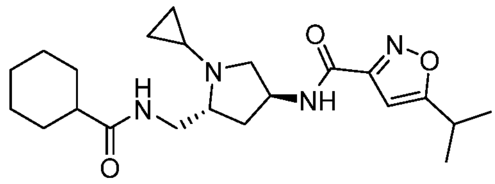 |
| 191 | 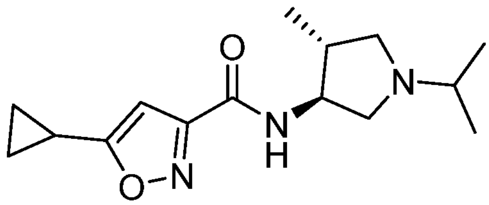 |
| 192 | 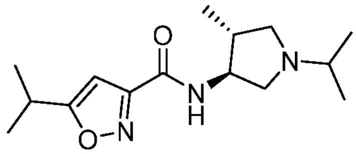 |
| 193 | 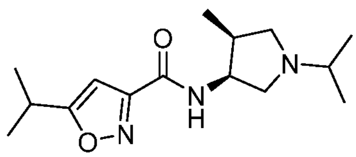 |
| 194 | 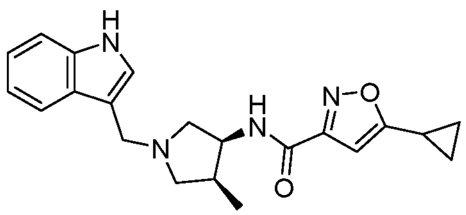 |
| 195 | 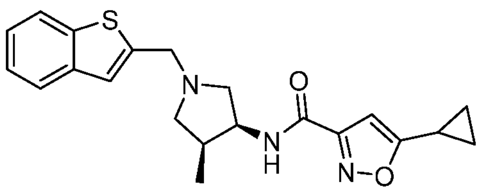 |
| 196 | 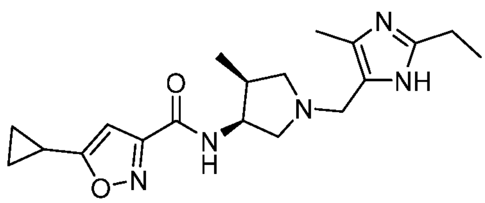 |
| 197 | 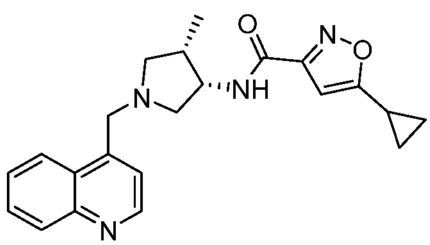 |

(fortgesetzt)

| Verb. Nr. | Struktur |
|---|---|
| 198 | 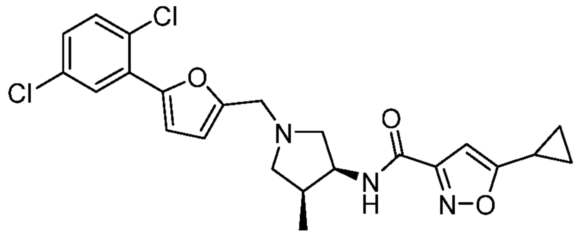 |
| 199 | 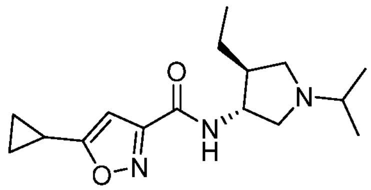 |
| 200 | 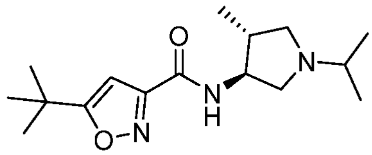 |
| 201 | 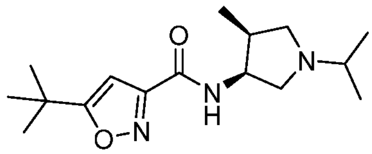 |
| 202 | 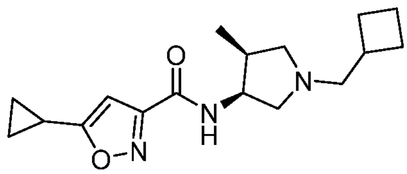 |
| 203 | 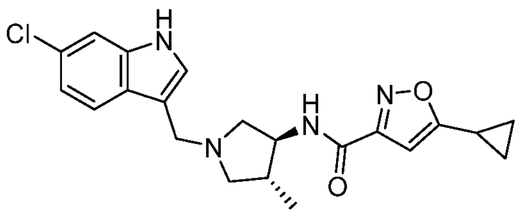 |
| 204 | 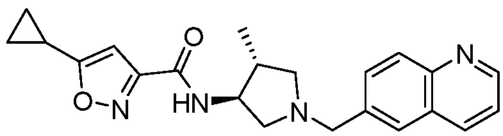 |
| 205 | 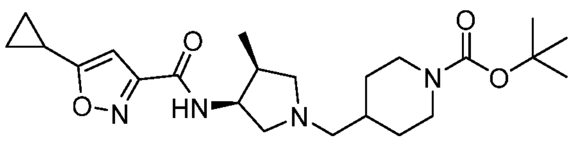 |

(fortgesetzt)

| Verb. Nr. | Struktur |
|---|---|
| 206 | 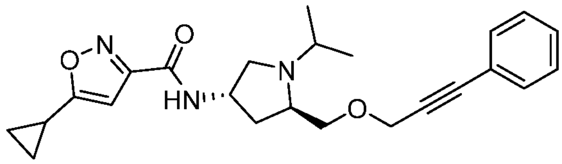 |
| 207 | 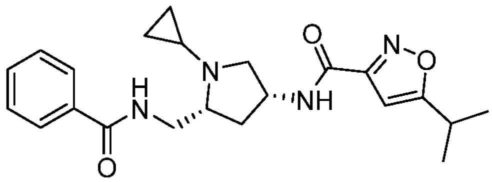 |
| 208 | 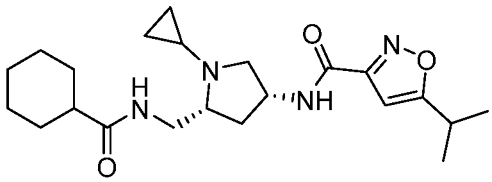 |
| 209 | 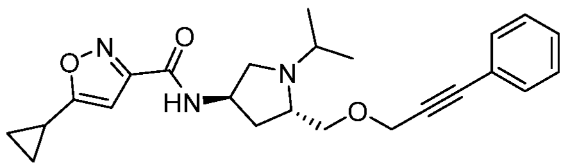 |
| 210 | 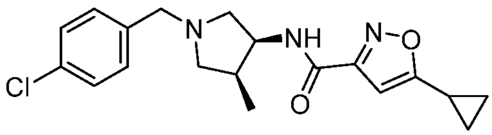 |
| 211 | 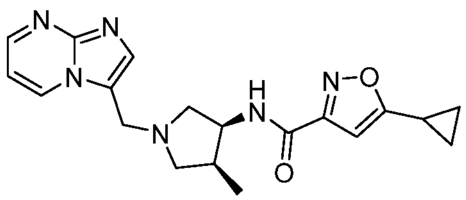 |
| 212 | 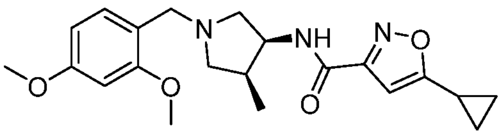 |
| 213 | 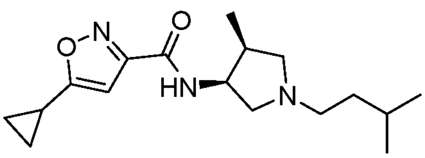 |

(fortgesetzt)

| Verb. Nr. | Struktur |
|---|---|
| 214 | 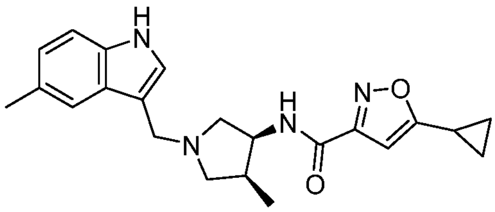 |
| 215 | 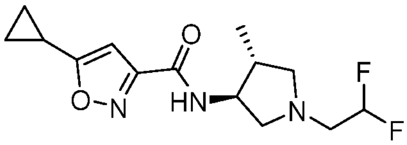 |
| 216 | 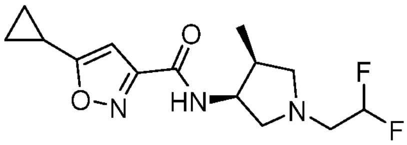 |
| 217 | 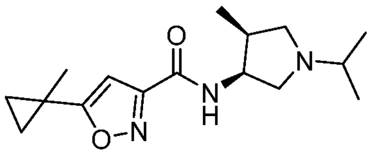 |
| 218 | 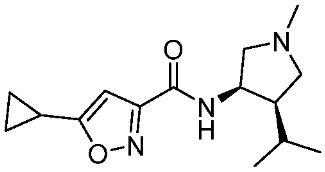 |
| 219 | 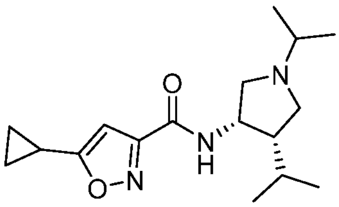 |
| 220 | 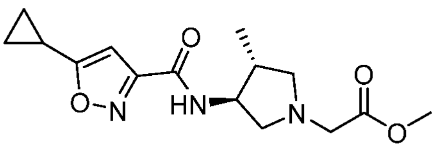 |
| 221 | 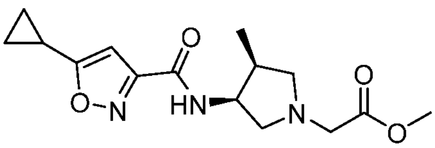 |
| 222 | 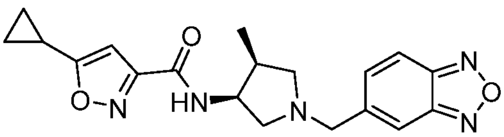 |

(fortgesetzt)

| Verb. Nr. | Struktur |
|---|---|
| 223 | 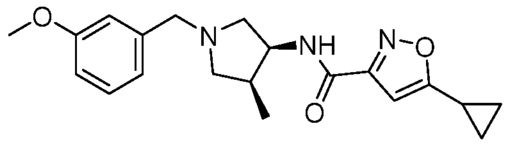 |
| 224 | 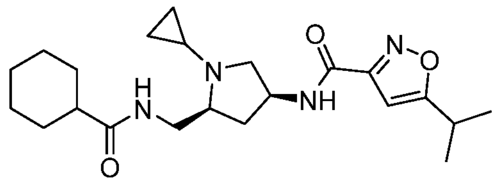 |
| 225 | 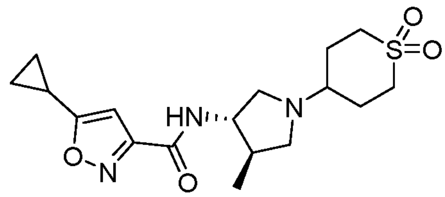 |
| 226 | 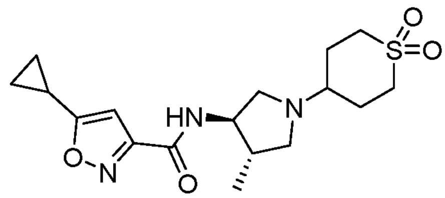 |
| 227 | 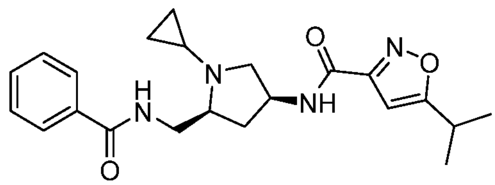 |
| 228 | 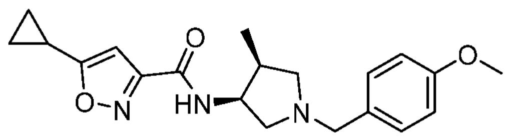 |
| 229 | 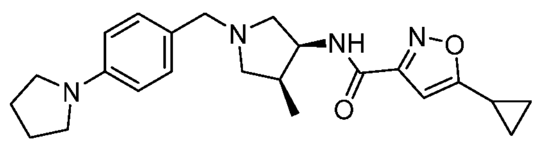 |
| 230 | 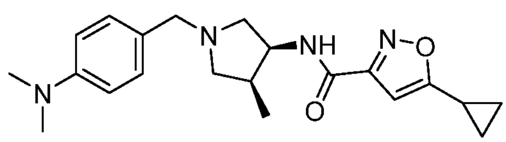 |
| 231 | 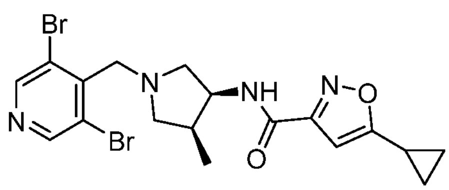 |

(fortgesetzt)

| Verb. Nr. | Struktur |
|---|---|
| 232 | 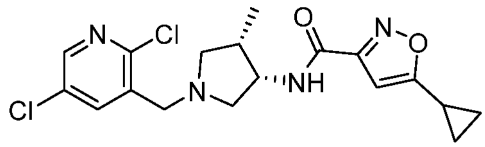 |
| 233 | 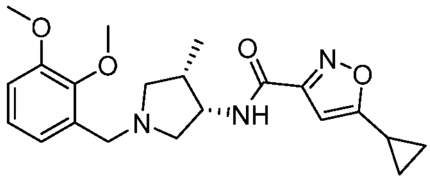 |
| 234 | 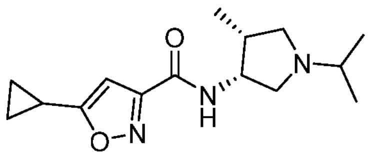 |
| 235 | 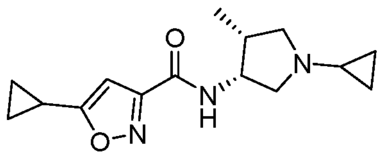 |
| 236 | 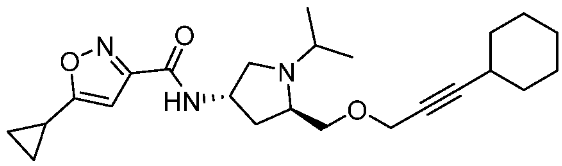 |
| 237 | 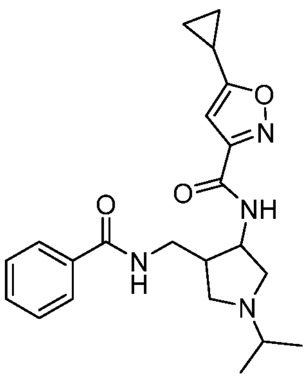 |
| 238 | 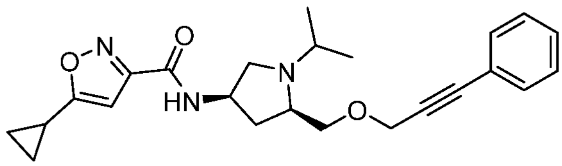 |
| 239 | 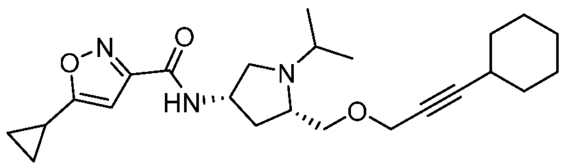 |

(fortgesetzt)

| Verb. Nr. | Struktur |
|---|---|
| 240 | 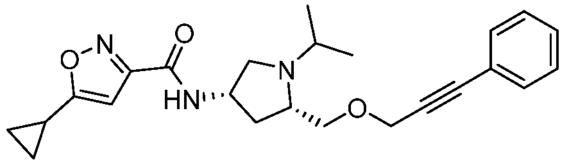 |
| 241 | 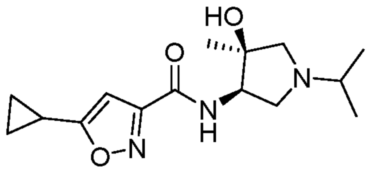 |
| 242 | 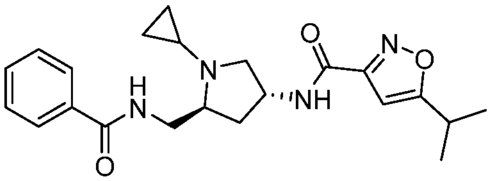 |
| 243 | 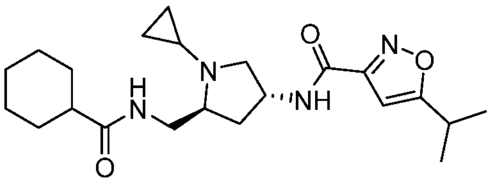 |
| 244 | 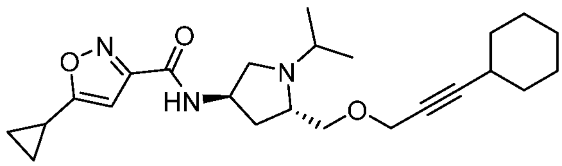 |
| 245 | 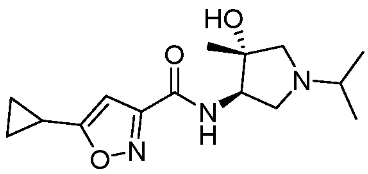 |
| 246 | 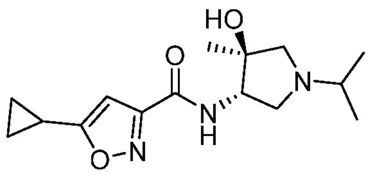 |
| 247 | 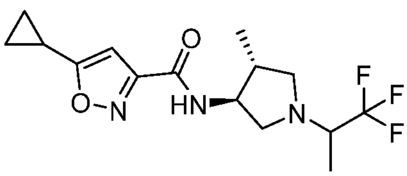 |

(fortgesetzt)

| Verb. Nr. | Struktur |
|---|---|
| 248 | 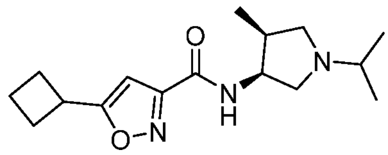 |
| 249 | 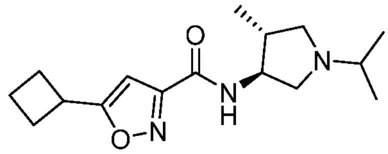 |
| 250 | 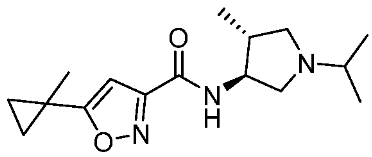 |
| 251 | 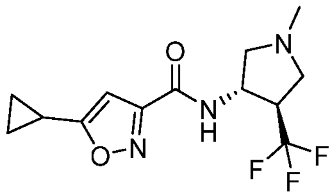 |
| 252 | 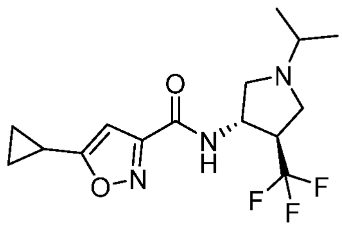 |
| 253 | 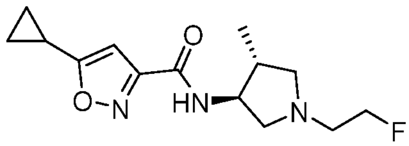 |
| 254 | 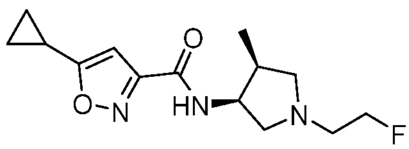 |
| 255 | 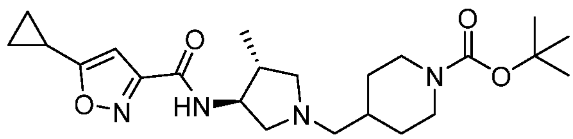 |
| 256 | 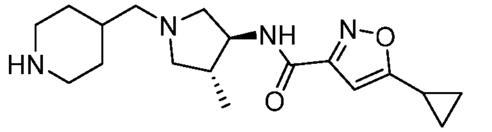 |

(fortgesetzt)

| Verb. Nr. | Struktur |
|---|---|
| 257 | 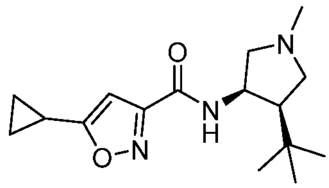 |
| 258 | 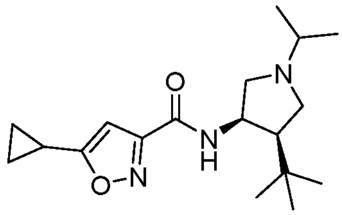 |
| 259 | 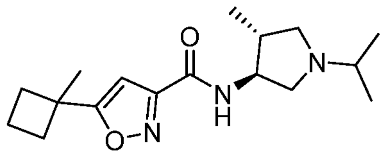 |
| 260 | 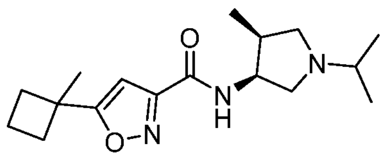 |
| 261 | 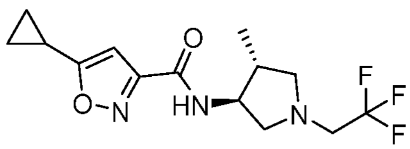 |
| 262 | 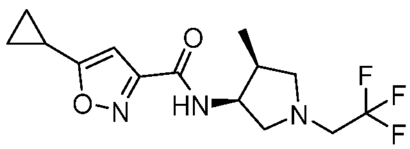 |
| 263 | 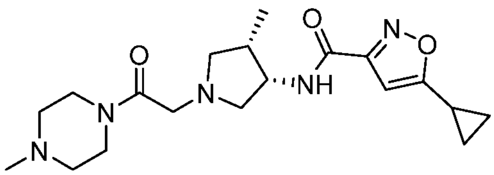 |
| 264 | 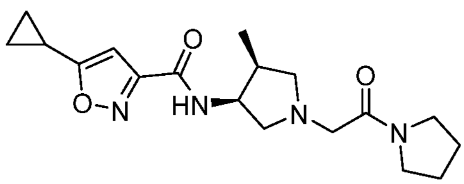 |

(fortgesetzt)

| Verb. Nr. | Struktur |
|---|---|
| 265 | 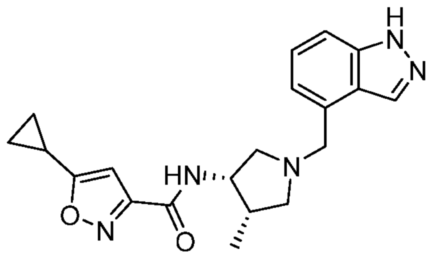 |
| 266 | 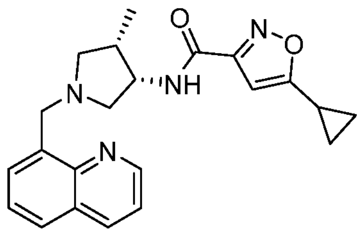 |
| 267 | 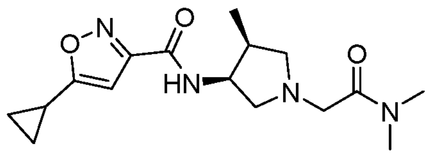 |
| 268 | 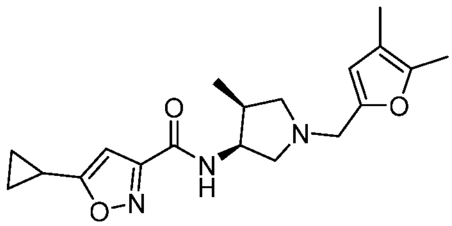 |
| 269 | 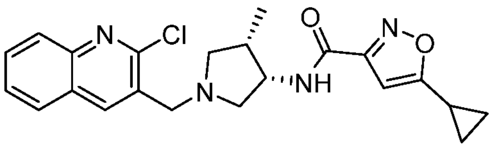 |
| 270 | 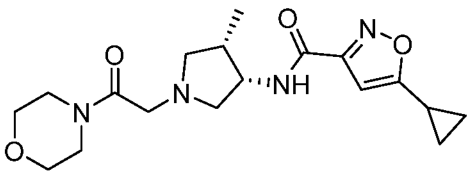 |
| 271 | 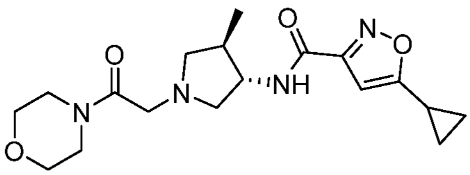 |
| 272 | 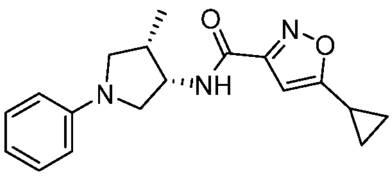 |

(fortgesetzt)

| Verb. Nr. | Struktur |
|---|---|
| 273 | 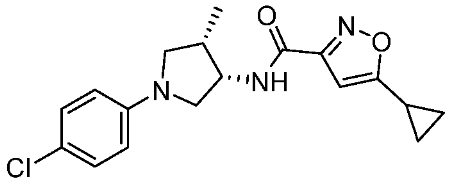 |
| 274 | 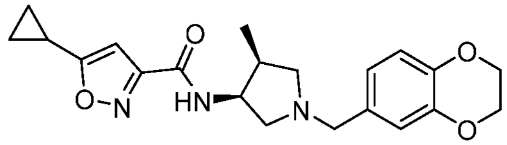 |
| 275 | 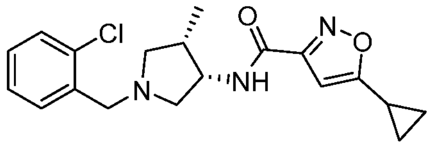 |
| 276 | 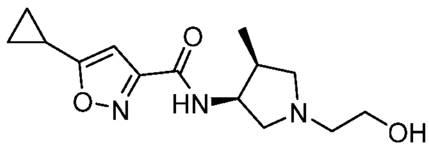 |
| 277 | 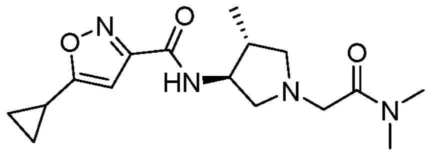 |
| 278 | 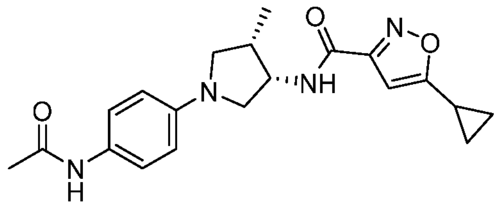 |
| 279 | 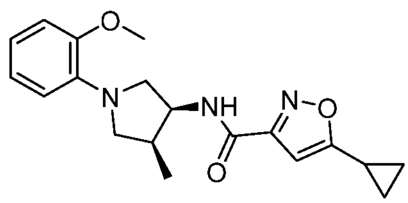 |
| 280 | 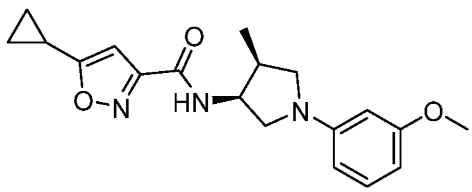 |

(fortgesetzt)

| Verb. Nr. | Struktur |
|---|---|
| 281 | 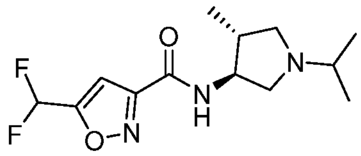 |
| 282 | 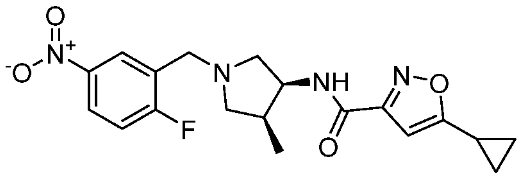 |
| 283 | 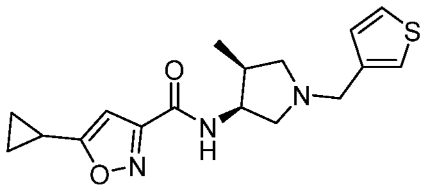 |
| 284 | 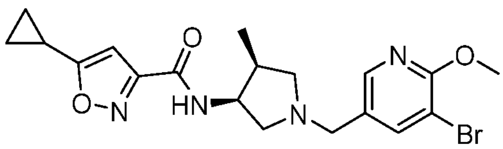 |
| 285 | 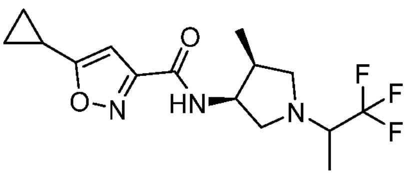 |
| 286 | 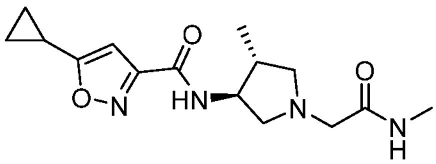 |
| 287 | 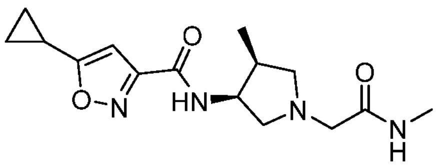 |
| 288 | 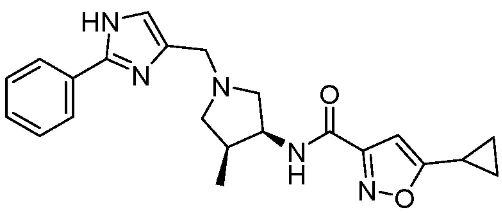 |

(fortgesetzt)

| Verb. Nr. | Struktur |
|---|---|
| 289 | 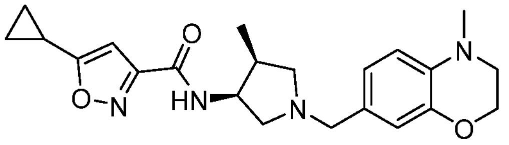 |
| 290 | 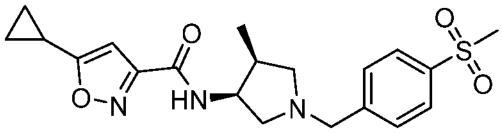 |
| 291 | 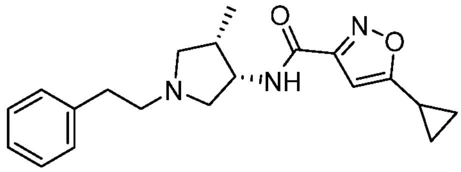 |
| 292 | 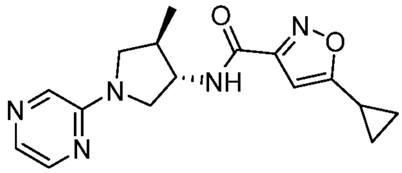 |
| 293 | 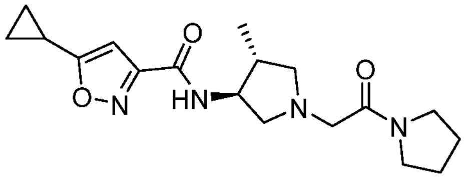 |
| 294 | 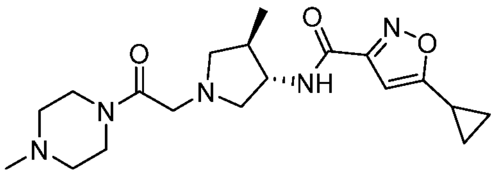 |
| 295 | 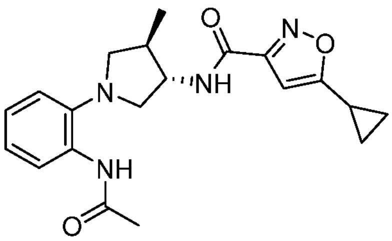 |
| 296 | 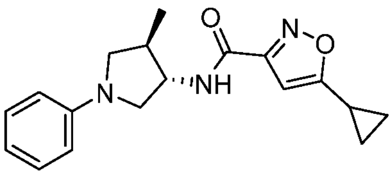 |

(fortgesetzt)

| Verb. Nr. | Struktur |
|---|---|
| 297 | 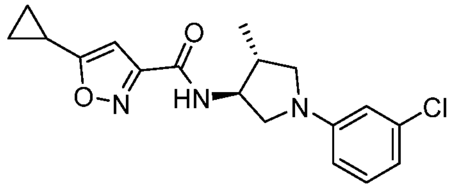 |
| 298 | 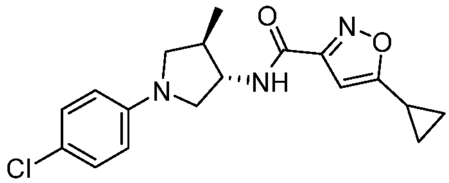 |
| 299 | 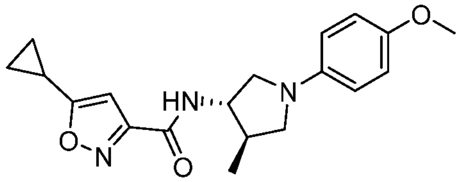 |
| 300 | 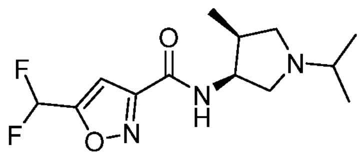 |
| 301 | 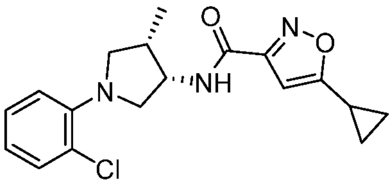 |
| 302 | 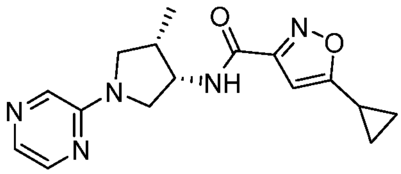 |
| 303 | 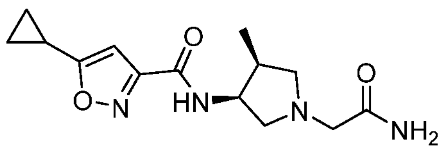 |
| 304 | 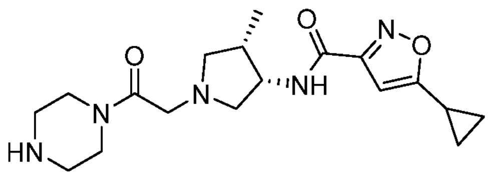 |

(fortgesetzt)

| Verb. Nr. | Struktur |
|---|---|
| 305 | 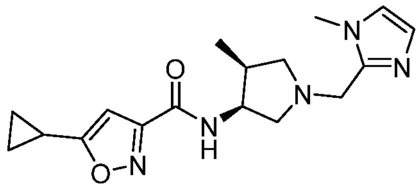 |
| 306 | 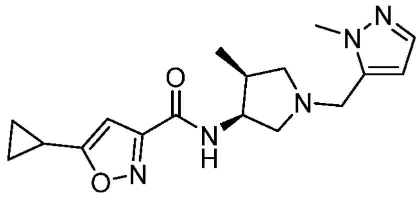 |
| 307 | 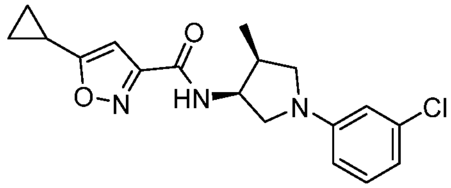 |
| 308 | 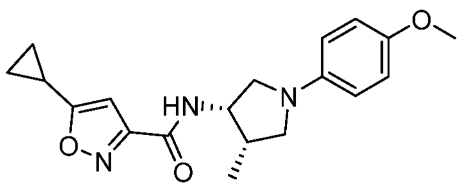 |
| 309 | 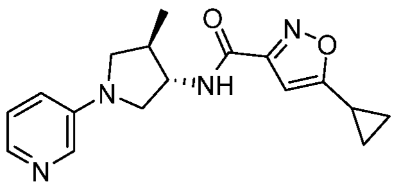 |
| 310 | 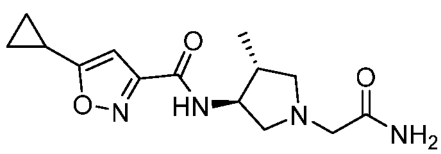 |
| 311 | 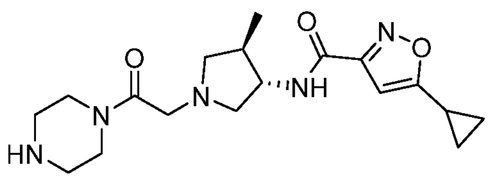 |
| 312 | 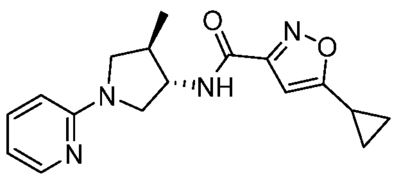 |

(fortgesetzt)

| Verb. Nr. | Struktur |
|---|---|
| 313 | 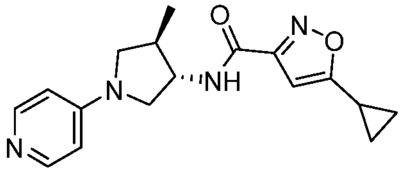 |
| 314 | 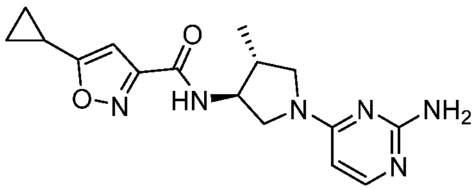 |
| 315 | 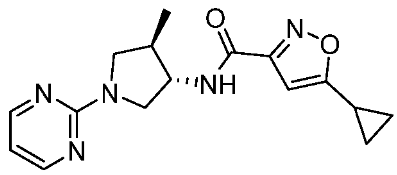 |
| 316 | 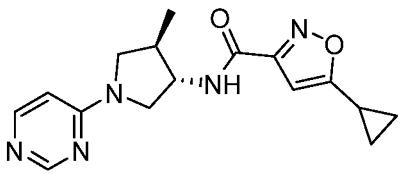 |
| 317 | 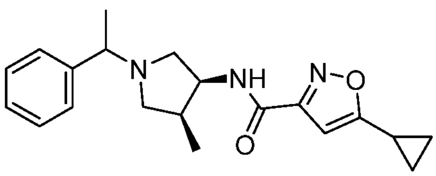 |
| 318 | 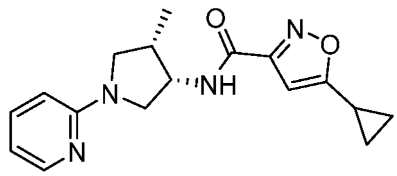 |
| 319 | 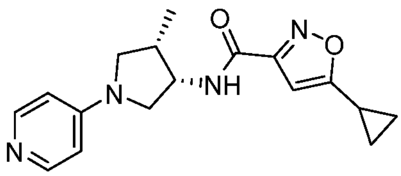 |
| 320 | 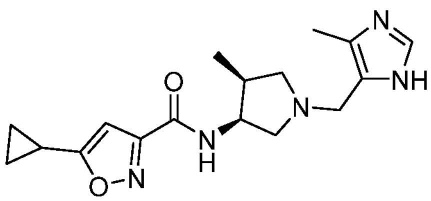 |

(fortgesetzt)

| Verb. Nr. | Struktur |
|---|---|
| 321 | 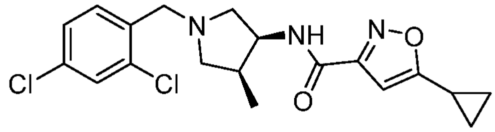 |
| 322 | 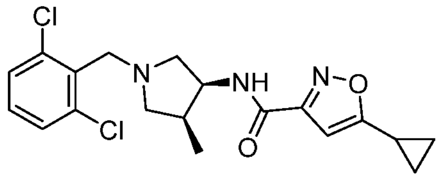 |
| 323 | 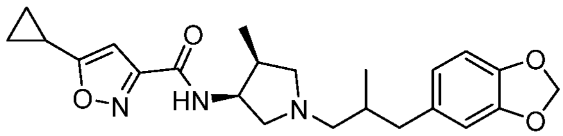 |
| 324 | 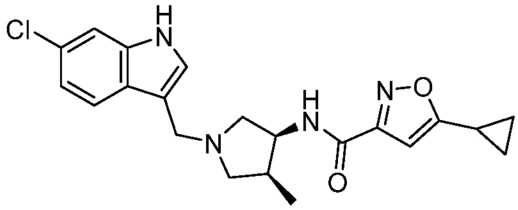 |
| 325 | 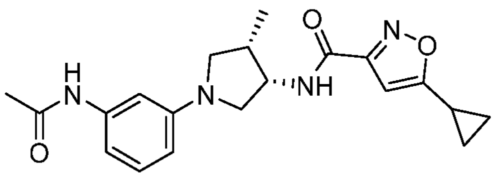 |
| 326 | 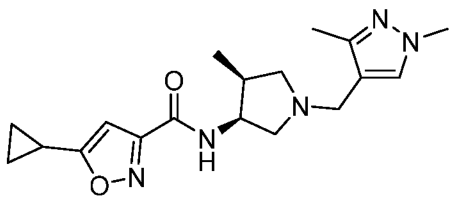 |
| 327 | 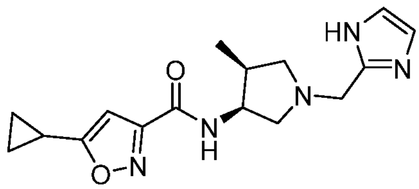 |
| 328 | 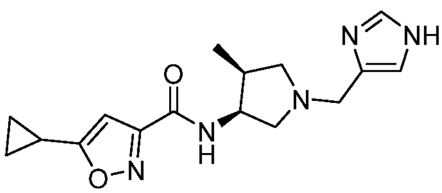 |

(fortgesetzt)

| Verb. Nr. | Struktur |
|---|---|
| 329 | 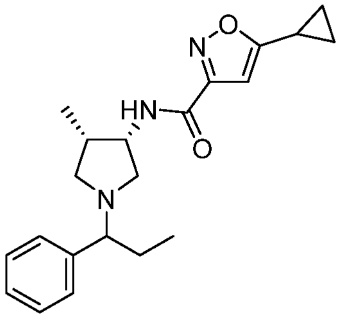 |
| 330 | 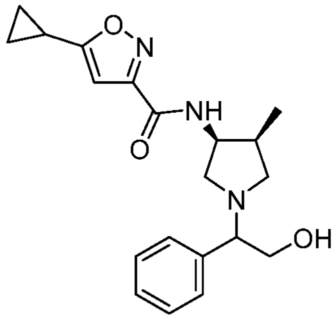 |
| 331 | 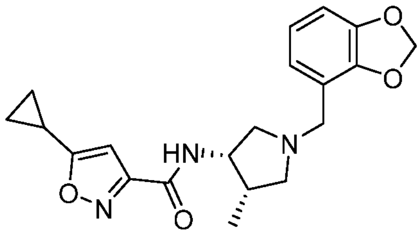 |
| 332 | 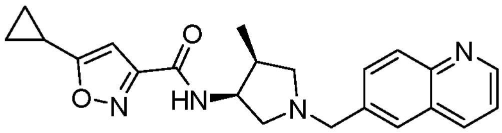 |
| 333 | 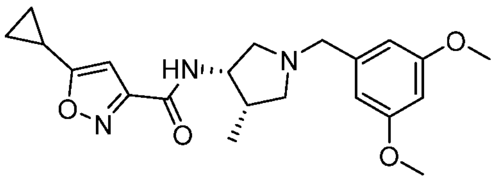 |
| 334 | 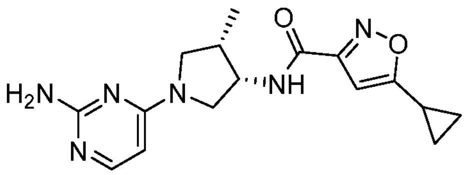 |

(fortgesetzt)

| Verb. Nr. | Struktur |
|---|---|
| 335 | 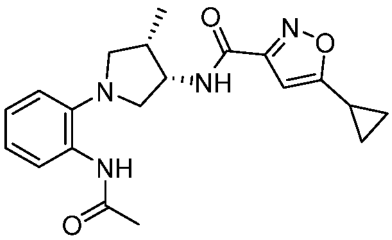 |
| 336 | 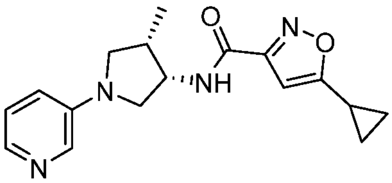 |
| 337 | 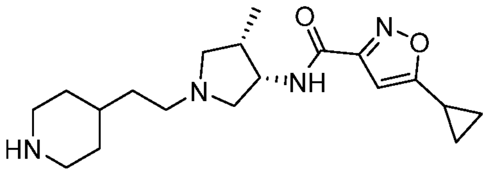 |
| 338 | 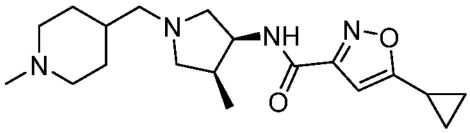 |
| 339 | 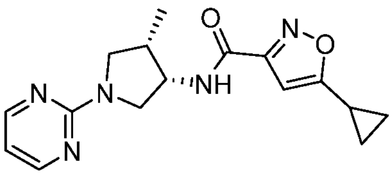 |
| 340 | 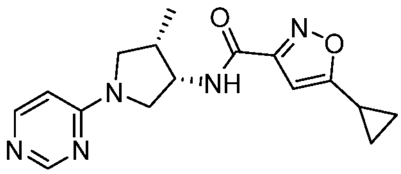 |
| 341 | 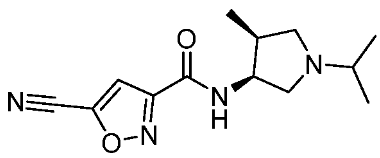 |
| 342 | 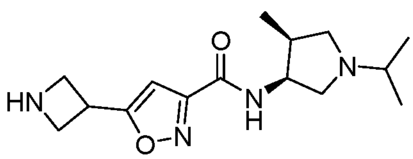 |

(fortgesetzt)

| Verb. Nr. | Struktur |
|---|---|
| 343 | 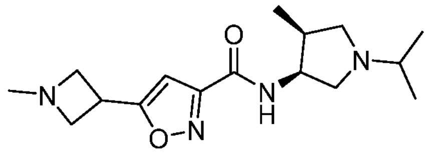 |
| 344 | 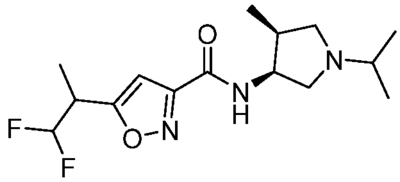 |
| 345 | 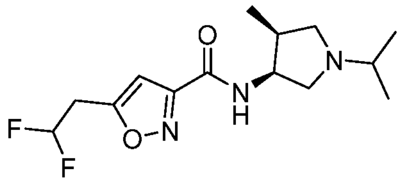 |
| 346 | 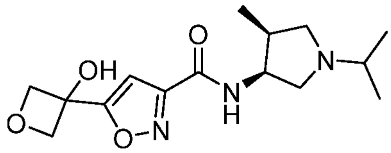 |
| 347 | 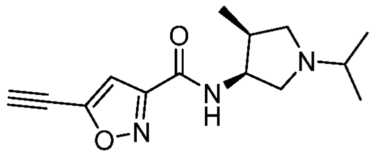 |
| 348 | 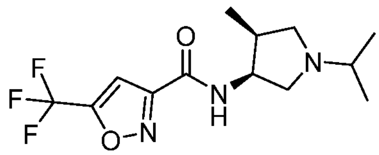 |
| 349 | 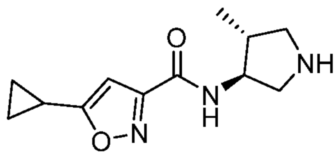 |
| 350 | 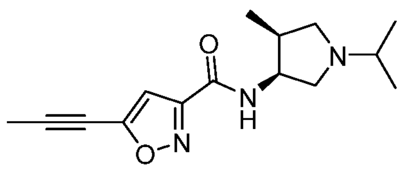 |
| 351 | 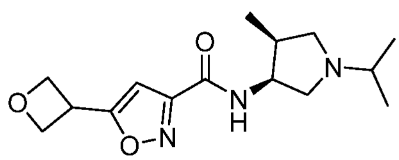 |

(fortgesetzt)

| Verb. Nr. | Struktur |
|---|---|
| 352 | 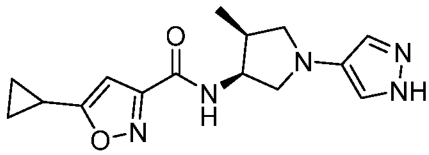 |

ausgewählt ist, oder ein pharmazeutisch unbedenkliches Salz oder Hydrat davon.

**11.** Pharmazeutische Zusammensetzung zur Verwendung bei der Behandlung von Krebs, wobei die pharmazeutische Zusammensetzung die Verbindung nach einem der Ansprüche 1-10 oder ein pharmazeutisch unbedenkliches Salz oder Hydrat davon und einen pharmazeutisch unbedenklichen Träger umfasst.

**12.** Pharmazeutische Zusammensetzung zur Verwendung nach Anspruch 11, wobei der Krebs aus der Gruppe bestehend aus Nebennierenkrebs, Azinuszellkarzinom, Akustikusneurinom, akrolentiginösem Melanom, Akrospirom, akuter Eosinophilenleukämie, akuter Erythroleukämie, akuter lymphoblastischer Leukämie, akuter Megakaryoblastenleukämie, akuter Monozytenleukämie, akuter Promyelozytenleukämie, Adenokarzinom, adenoid-zystischem Karzinom, Adenom, adenomatoid odontogenem Tumor, adenosquamösem Karzinom, Fettgewebeneoplasie, Nebennierenrindenkarzinom, adulter/adultem T-Zell-Leukämie/Lymphom, aggressiver NK-Zell-Leukämie, AIDS-assoziiertem Lymphom, alveolärem Rhabdomyosarkom, alveolärem Weichteilsarkom, ameloblastischem Fibrom, anaplastisch-großzelligem Lymphom, anaplastischem Schilddrüsenkrebs, angioimmunoblastischem T-Zell-Lymphom, Angiomyolipom, Angiosarkom, Astrozytom, atypischem teratoidem rhabdoidem Tumor, chronischer lymphatischer B-Zell-Leukämie, B-Zell-Prolymphozytenleukämie, B-Zell-Lymphom, Basalzellkarzinom, Gallengangkrebs, Blasenkrebs, Blastom, Knochenkrebs, Brenner-Tumor, braunem Tumor, Burkitt-Lymphom, Brustkrebs, Hirntumor, Karzinom, Karzinom in situ, Karzinosarkom, Knorpeltumor, Zementom, myeloischem Sarkom, Chondrom, Chordom, Chorionkarzinom, Choroidalplexuspapillom, Klarzellsarkom der Niere, Kraniopharyngeom, kutanem T-Zell-Lymphom, Gebärmutterhalskrebs, Dickdarmkrebs, Degos-Syndrom, desmoplastischem klein- und rundzelligem Tumor, diffusem großzelligem B-Zell-Lymphom, dysembryoplastischem neuroepithelialem Tumor, Dysgerminom, embryonalem Karzinom, endokriner Neoplasie, endodermalem Sinuszelltumor, Enteropathieassoziiertem T-Zell-Lymphom, Speiseröhrenkrebs, Fetus in fetu, Fibrom, Fibrosarkom, follikulärem Lymphom, follikulärem Schilddrüsenkrebs, Ganglioneurom, gastrointestinalem Krebs, Keimzelltumor, gestationalem Chorionkarzinom, Riesenzellfibroblastom, Riesenzelltumor der Knochen, glialem Tumor, Glioblastoma multiforme, Gliom, Gliomatosis cerebri, Glukagonom, Gonadoblastom, Granulosazelltumor, Gynandroblastom, Gallenblasenkrebs, Magenkarzinom, Haarzellleukämie, Hämangioblastom, Kopf-Hals-Karzinom, Hämangioperizytom, hämatologischem Tumor, Hepatoblastom, hepatosplenischem T-Zell-Lymphom, Hodgkin-Lymphom, Non-Hodgkin-Lymphom, invasivem lobulärem Karzinom, Darmkrebs, Nierenkrebs, Kehlkopfkrebs, Lentigo maligna, letalem Midline-Karzinom, Leukämie, Leydigzelltumor, Liposarkom, Lungenkrebs, Lymphangiom, Lymphangiosarkom, Lymphoepitheliom, Lymphom, akuter lymphatischer Leukämie, akuter myelogener Leukämie, chronischer lymphatischer Leukämie, Leberkrebs, kleinzelligem Lungenkrebs, nichtkleinzelligem Lungenkrebs, MALT-Lymphom, malignem fibrösem Histiozytom, malignem peripherem Nervenscheidentumor, malignem Tritontumor, Mantelzell-Lymphom, Marginalzonen-B-Zell-Lymphom, Mastzellenleukämie, mediastinalem Keimzelltumor, medullärem Karzinom der Brust, medullärem Schilddrüsenkrebs, Medulloblastom, Melanom, Meningiom, Merkelzellkarzinom, Mesotheliom, metastasierendem Urothelkarzinom, Müllerschem Mischtumor, muzinösem Tumor, multiplem Myelom, Muskelgewebeneoplasie, Mycosis fungoides, myxoidem Liposarkom, Myxom, Myxosarkom, Nasopharynxkarzinom, Neurinom, Neuroblastom, Neurofibrom, Neurom, nodulärem Melanom, Augenkrebs, Oligoastrozytom, Oligodendrogliom, Onkozytom, Sehnervenscheidenmeningiom, Sehnervtumor, Mundkrebs, Osteosarkom, Eierstockkrebs, Pancoast-Tumor, papillärem Schilddrüsenkrebs, Paragangliom, Pinealoblastom, Pineozytom, Pituizytom, Hypophysenadenom, Hypophysentumor, Plasmazytom, Polyembryom, Vorläufer-T-lymphoblastischem Lymphom, primärem Zentralnervensystem-Lymphom, primärem Effusionslymphom, primärem Peritonealkrebs, Prostatakrebs, Bauchspeicheldrüsenkrebs, Pharynxkarzinom, Pseudomyxoma periotonei, Nierenzellkarzinom, medullärem Nierenkarzinom, Retinoblastom, Rhabdomyom, Rhabdomyosarkom, Richter-Transformation, Enddarmkrebs, Sarkom, Schwannomatose, Seminom, Sertoli-Zell-Tumor, Gonadenstromatumor, Siegelringzellkarzinom, Hautkrebs, klein-, blau- und rundzelligen Tumoren, kleinzelligem Karzinom, Weichgewebesarkom, Somatostatinom, Schornsteinfegerkrebs, Rückenmarktumor, splenischem Marginalzonenlymphom, Plattenepithelkarzinom, Synovialsarkom, Sezary-Syndrom, Dünndarmkrebs, Plattenepithelkrebs, Magenkrebs, T-Zell-Lymphom, Hodenkrebs, Thekom, Schilddrüsenkrebs, Transitionalzellkarzinom, Rachenkrebs,

Urachuskarzinom, Urogenitalkrebs, Urothelkarzinom, Aderhautmelanom, Uteruskarzinom, verrukösem Karzinom, Sehbahngliom, Vulvakrebs, Vaginalkrebs, Morbus Waldenström, Warthin-Tumor und Wilms-Tumor ausgewählt ist.

**13.** Verbindung zur Verwendung nach einem der Ansprüche 1-10 oder ein pharmazeutisch unbedenkliches Salz oder Hydrat davon, wobei der Krebs aus der Gruppe bestehend aus Nebennierenkrebs, Azinuszellkarzinom, Akustikusneurinom, akrolentiginösem Melanom, Akrospirom, akuter Eosinophilenleukämie, akuter Erythroleukämie, akuter lymphoblastischer Leukämie, akuter Megakaryoblastenleukämie, akuter Monozytenleukämie, akuter Promyelozytenleukämie, Adenokarzinom, adenoid-zystischem Karzinom, Adenom, adenomatoid odontogenem Tumor, adenosquamösem Karzinom, Fettgewebeneoplasie, Nebennierenrindenkarzinom, adulter/adultem T-Zell-Leukämie/Lymphom, aggressiver NK-Zell-Leukämie, AIDS-assoziiertem Lymphom, alveolärem Rhabdomyosarkom, alveolärem Weichteilsarkom, ameloblastischem Fibrom, anaplastisch-großzelligem Lymphom, anaplastischem Schilddrüsenkrebs, angioimmunoblastischem T-Zell-Lymphom, Angiomyolipom, Angiosarkom, Astrozytom, atypischem teratoidem rhabdoidem Tumor, chronischer lymphatischer B-Zell-Leukämie, B-Zell-Prolymphozytenleukämie, B-Zell-Lymphom, Basalzellkarzinom, Gallengangkrebs, Blasenkrebs, Blastom, Knochenkrebs, Brenner-Tumor, braunem Tumor, Burkitt-Lymphom, Brustkrebs, Hirntumor, Karzinom, Karzinom in situ, Karzinosarkom, Knorpeltumor, Zementom, myeloischem Sarkom, Chondrom, Chordom, Chorionkarzinom, Choroidalplexuspapillom, Klarzellsarkom der Niere, Kraniopharyngeom, kutanem T-Zell-Lymphom, Gebärmutterhalskrebs, Dickdarmkrebs, Degos-Syndrom, desmoplastischem klein- und rundzelligem Tumor, diffusem großzelligem B-Zell-Lymphom, dysembryoplastischem neuroepithelialem Tumor, Dysgerminom, embryonalem Karzinom, endokriner Neoplasie, endodermalem Sinuszelltumor, Enteropathieassoziiertem T-Zell-Lymphom, Speiseröhrenkrebs, Fetus in fetu, Fibrom, Fibrosarkom, follikulärem Lymphom, follikulärem Schilddrüsenkrebs, Ganglioneurom, gastrointestinalem Krebs, Keimzelltumor, gestationalem Chorionkarzinom, Riesenzellfibroblastom, Riesenzelltumor der Knochen, glialem Tumor, Glioblastoma multiforme, Gliom, Gliomatosis cerebri, Glukagonom, Gonadoblastom, Granulosazelltumor, Gynandroblastom, Gallenblasenkrebs, Magenkarzinom, Haarzellleukämie, Hämangioblastom, Kopf-Hals-Karzinom, Hämangioperizytom, hämatologischem Tumor, Hepatoblastom, hepatosplenischem T-Zell-Lymphom, Hodgkin-Lymphom, Non-Hodgkin-Lymphom, invasivem lobulärem Karzinom, Darmkrebs, Nierenkrebs, Kehlkopfkrebs, Lentigo maligna, letalem Midline-Karzinom, Leukämie, Leydigzelltumor, Liposarkom, Lungenkrebs, Lymphangiom, Lymphangiosarkom, Lymphoepitheliom, Lymphom, akuter lymphatischer Leukämie, akuter myelogener Leukämie, chronischer lymphatischer Leukämie, Leberkrebs, kleinzelligem Lungenkrebs, nichtkleinzelligem Lungenkrebs, MALT-Lymphom, malignem fibrösem Histiozytom, malignem peripherem Nervenscheidentumor, malignem Tritontumor, Mantelzell-Lymphom, Marginalzonen-B-Zell-Lymphom, Mastzellenleukämie, mediastinalem Keimzelltumor, medullärem Karzinom der Brust, medullärem Schilddrüsenkrebs, Medulloblastom, Melanom, Meningiom, Merkelzellkarzinom, Mesotheliom, metastasierendem Urothelkarzinom, Müllerschem Mischtumor, muzinösem Tumor, multiplem Myelom, Muskelgewebeneoplasie, Mycosis fungoides, myxoidem Liposarkom, Myxom, Myxosarkom, Nasopharynxkarzinom, Neurinom, Neuroblastom, Neurofibrom, Neurom, nodulärem Melanom, Augenkrebs, Oligoastrozytom, Oligodendrogliom, Onkozytom, Sehnervenscheidenmeningiom, Sehnervtumor, Mundkrebs, Osteosarkom, Eierstockkrebs, Pancoast-Tumor, papillärem Schilddrüsenkrebs, Paragangliom, Pinealoblastom, Pineozytom, Pituizytom, Hypophysenadenom, Hypophysentumor, Plasmazytom, Polyembryom, Vorläufer-T-lymphoblastischem Lymphom, primärem Zentralnervensystem-Lymphom, primärem Effusionslymphom, primärem Peritonealkrebs, Prostatakrebs, Bauchspeicheldrüsenkrebs, Pharynxkarzinom, Pseudomyxoma periotonei, Nierenzellkarzinom, medullärem Nierenkarzinom, Retinoblastom, Rhabdomyom, Rhabdomyosarkom, Richter-Transformation, Enddarmkrebs, Sarkom, Schwannomatose, Seminom, Sertoli-Zell-Tumor, Gonadenstromatumor, Siegelringzellkarzinom, Hautkrebs, klein-, blau- und rundzelligen Tumoren, kleinzelligem Karzinom, Weichgewebesarkom, Somatostatinom, Schornsteinfegerkrebs, Rückenmarktumor, splenischem Marginalzonenlymphom, Plattenepithelkarzinom, Synovialsarkom, Sezary-Syndrom, Dünndarmkrebs, Plattenepithelkrebs, Magenkrebs, T-Zell-Lymphom, Hodenkrebs, Thekom, Schilddrüsenkrebs, Transitionalzellkarzinom, Rachenkrebs, Urachuskarzinom, Urogenitalkrebs, Urothelkarzinom, Aderhautmelanom, Uteruskarzinom, verrukösem Karzinom, Sehbahngliom, Vulvakrebs, Vaginalkrebs, Morbus Waldenström, Warthin-Tumor und Wilms-Tumor ausgewählt ist.

## Revendications

**1.** Composé pour une utilisation dans le traitement d'un cancer, le composé possédant la formule I :

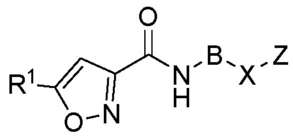

I

ou sel ou hydrate pharmaceutiquement acceptable correspondant,

B étant :

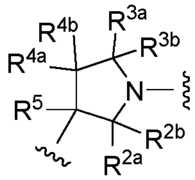

;

X étant choisi dans le groupe constitué par $-S(=O)_2-$, $-S(=O)_2N(R^6)-$, $-S(=O)_2C(R^7)(H)-$, $-C(=O)-$, $-C(=O)N(R^6)-$, $-C(=O)O-$, et $-C(=O)C(R^7)(H)-$ ; ou X étant absent ;

Z étant choisi dans le groupe constitué par hydrogène, $C_{1-6}$ alkyle éventuellement substitué, halogénoalkyle, (cycloalkyl)alkyle, hydroxyalkyle, (amino)alkyle, (alkylamino)alkyle, (dialkylamino)alkyle, (cycloalkylamino)alkyle, (hétérocyclo)alkyle, (amino)(hydroxy)alkyle, (amino)(aryl)alkyle, (hydroxy)(aryl)alkyle, (aralkylamino)alkyle, alcoxyalkyle, $C_{6-14}$ aryle éventuellement substitué, hétérocyclo à 4 à 14 chaînons éventuellement substitué, $C_{5-14}$ hétéroaryle éventuellement substitué, $C_{3-12}$ cycloalkyle éventuellement substitué, aralkyle, hétéroaralkyle, et $-CH_2N(H)C(=O)R^{8c}$ ;

$R^1$ étant choisi dans le groupe constitué par hydrogène, cyano, $C_{1-6}$ alkyle, halogénoalkyle, hétérocyclo à 4 à 14 chaînons éventuellement substitué, alcynyle, et $C_{3-6}$ cycloalkyle ;

$R^{2a}$, $R^{2b}$, $R^{3a}$, $R^{3b}$, $R^{4a}$, et $R^{4b}$ étant chacun indépendamment choisis dans le groupe constitué par hydrogène, amino, alkylamino, dialkylamino, cycloalkylamino, halogéno, hydroxy, $C_{1-6}$ alkyle, alcoxy, halogénoalkyle, hydroxyalkyle, (amino)alkyle, (alkylamino)alkyle, (dialkylamino)alkyle, (cycloalkylamino)alkyle, (hétérocyclo)alkyle, $C_{3-12}$ cycloalkyle éventuellement substitué, $C_{6-14}$ aryle éventuellement substitué, hétérocyclo à 4 à 14 chaînons éventuellement substitué, $C_{5-14}$ hétéroaryle éventuellement substitué, alcoxyalkyle, aralkyle, alcoxycarbonyle, sulfonamido, carboxamido, $-N(H)C(=O)R^{8a}$ ; et $-CH_2N(H)C(=O)R^{8b}$ ; ou

$R^{2a}$ et $R^{2b}$ pris ensemble avec l'atome de carbone auquel ils sont fixés formant un carbonyle ; et $R^{3a}$, $R^{3b}$, $R^{4a}$, et $R^{4b}$ étant chacun indépendamment choisis dans le groupe constitué par hydrogène, hydroxy, $C_{1-6}$ alkyle, alcoxy, hydroxyalkyle, $C_{3-12}$ cycloalkyle, $C_{6-14}$ aryle éventuellement substitué, alcoxyalkyle, aralkyle, $-N(H)C(=O)R^{8a}$ ; et $-CH_2N(H)C(=O)R^{8b}$ ; ou

$R^{3a}$ et $R^{3b}$ pris ensemble avec l'atome de carbone auquel ils sont fixés formant un carbonyle ; et $R^{2a}$, $R^{2b}$, $R^{4a}$, et $R^{4b}$ étant chacun indépendamment choisis dans le groupe constitué par hydrogène, hydroxy, $C_{1-6}$ alkyle, alcoxy, hydroxyalkyle, $C_{3-12}$ cycloalkyle, $C_{6-14}$ aryle éventuellement substitué, alcoxyalkyle, $-N(H)C(=O)R^{8a}$; et $-CH_2N(H)C(=O)R^{8b}$ ; ou

$R^{4a}$ et $R^{4b}$ pris ensemble avec l'atome de carbone auquel ils sont fixés formant un carbonyle ; et $R^{2a}$, $R^{2b}$, $R^{3a}$, et $R^{3b}$ étant chacun indépendamment choisis dans le groupe constitué par hydrogène, hydroxy, $C_{1-6}$ alkyle, alcoxy, hydroxyalkyle, $C_{3-12}$ cycloalkyle, $C_{6-14}$ aryle éventuellement substitué, alcoxyalkyle, $-N(H)C(=O)R^{8a}$; et $-CH_2N(H)C(=O)R^{8b}$ ; ou

$R^{2a}$ et $R^{2b}$ pris ensemble avec l'atome de carbone auquel ils sont fixés formant un $C_{3-6}$ cycloalkyle ou $C_{3-6}$ hétérocyclo ; et $R^{3a}$, $R^{3b}$, $R^{4a}$, et $R^{4b}$ étant chacun indépendamment choisis dans le groupe constitué par hydrogène, hydroxy, $C_{1-6}$ alkyle, alcoxy, hydroxyalkyle, $C_{3-12}$ cycloalkyle, $C_{6-14}$ aryle éventuellement substitué, alcoxyalkyle, aralkyle, $-N(H)C(=O)R^{8a}$ ; et $-CH_2N(H)C(=O)R^{8b}$ ; ou

$R^{3a}$ et $R^{3b}$ pris ensemble avec l'atome de carbone auquel ils sont fixés formant un $C_{3-6}$ cycloalkyle ou $C_{3-6}$ hétérocyclo ; et $R^{2a}$, $R^{2b}$, $R^{4a}$, et $R^{4b}$ étant chacun indépendamment choisis dans le groupe constitué par hydrogène, hydroxy, $C_{1-6}$ alkyle, alcoxy, hydroxyalkyle, $C_{3-12}$ cycloalkyle, $C_{6-14}$ aryle éventuellement substitué, alcoxyalkyle, $-N(H)C(=O)R^{8a}$ ; et $-CH_2N(H)C(=O)R^{8b}$ ; ou

$R^{4a}$ et $R^{4b}$ pris ensemble avec l'atome de carbone auquel ils sont fixés formant un $C_{3-6}$ cycloalkyle ou $C_{3-6}$ hétérocyclo ; et $R^{2a}$, $R^{2b}$, $R^{3a}$, et $R^{3b}$ étant chacun indépendamment choisis dans le groupe constitué par hydrogène, hydroxy, $C_{1-6}$ alkyle, alcoxy, hydroxyalkyle, $C_{3-12}$ cycloalkyle, $C_{6-14}$ aryle éventuellement substitué, alcoxyalkyle, $-N(H)C(=O)R^{8a}$ ; et $-CH_2N(H)C(=O)R^{8b}$ ;

$R^5$ étant choisi dans le groupe constitué par hydrogène et $C_{1-4}$ alkyle ;
$R^6$ étant choisi dans le groupe constitué par hydrogène et $C_{1-4}$ alkyle ;
$R^7$ étant choisi dans le groupe constitué par hydrogène, $C_{1-4}$ alkyle, amino, alkylamino, dialkylamino, (amino)alkyle, (alkylamino)alkyle, (dialkylamino)alkyle, et hydroxyalkyle ;
$R^{8a}$ étant choisi dans le groupe constitué par $C_{1-6}$ alkyle, halogénoalkyle, hydroxyalkyle, (amino)alkyle, (alkylamino)alkyle, (dialkylamino)alkyle, (cycloalkylamino)alkyle, (hétérocyclo)alkyle, $C_{6-14}$ aryle éventuellement substitué, hétérocyclo à 4 à 14 chaînons éventuellement substitué, hétéroaryle à 5 à 14 chaînons éventuellement substitué, $C_{3-12}$ cycloalkyle éventuellement substitué, aralkyle, et hétéroaralkyle ;
$R^{8b}$ étant choisi dans le groupe constitué par $C_{1-6}$ alkyle, halogénoalkyle, hydroxyalkyle, (amino)alkyle, (alkylamino)alkyle, (dialkylamino)alkyle, (cycloalkylamino)alkyle, (hétérocyclo)alkyle, $C_{6-14}$ aryle éventuellement substitué, hétérocyclo à 4 à 14 chaînons éventuellement substitué, hétéroaryle à 5 à 14 chaînons éventuellement substitué, $C_{3-12}$ cycloalkyle éventuellement substitué, aralkyle, et hétéroaralkyle ; and
$R^{8c}$ étant choisi dans le groupe constitué par $C_{1-6}$ alkyle, halogénoalkyle, hydroxyalkyle, (amino)alkyle, (alkylamino)alkyle, (dialkylamino)alkyle, (cycloalkylamino)alkyle, (hétérocyclo)alkyle, $C_{6-14}$ aryle éventuellement substitué, hétérocyclo à 4 à 14 chaînons éventuellement substitué, hétéroaryle à 5 à 14 chaînons éventuellement substitué, $C_{3-12}$ cycloalkyle éventuellement substitué, aralkyle, et hétéroaralkyle.

**2.** Composé pour une utilisation selon la revendication 1, ou sel ou hydrate pharmaceutiquement acceptable correspondant, B étant choisi dans le groupe constitué par :

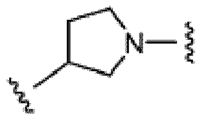 , 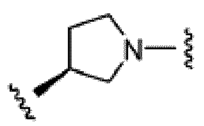 et 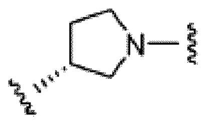 .

**3.** Composé pour une utilisation selon la revendication 1, ou sel ou hydrate pharmaceutiquement acceptable correspondant, B étant choisi dans le groupe constitué par :

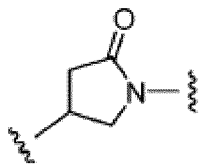 , 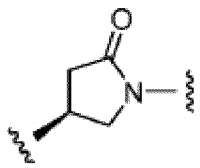 et 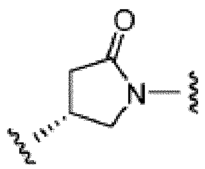 .

**4.** Composé pour une utilisation selon la revendication 1, ou sel ou hydrate pharmaceutiquement acceptable correspondant, B étant choisi dans le groupe constitué par :

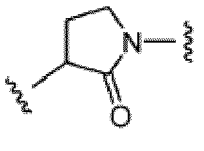 , 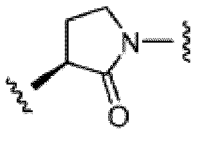 et 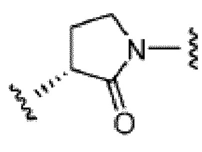 .

**5.** Composé pour une utilisation selon la revendication 1, ou sel ou hydrate pharmaceutiquement acceptable correspondant, B étant choisi parmi :

(a) le groupe constitué par :

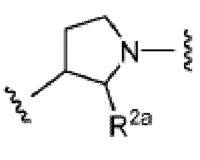 , 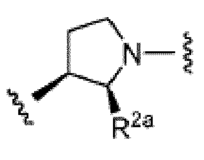 , 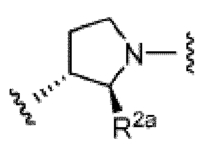 ,

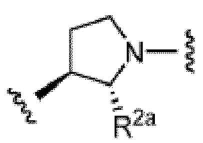 et 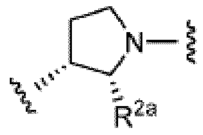 ;

et

$R^{2a}$ étant choisi dans le groupe constitué par $C_{1-6}$ alkyle, $C_{3-12}$ cycloalkyle, et $C_{6-14}$ aryle éventuellement substitué, éventuellement $R^{2a}$ étant choisi dans le groupe constitué par :

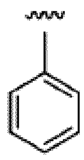 , 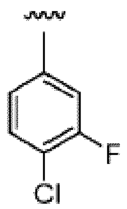 , 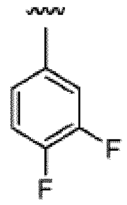 , 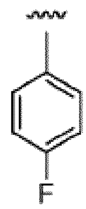 et 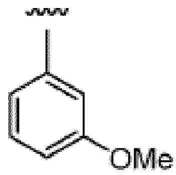 ;

et

(b) le groupe constitué par :

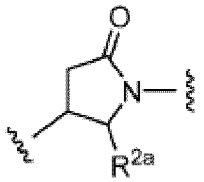 , 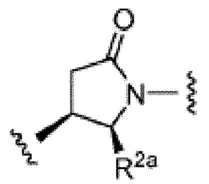 , 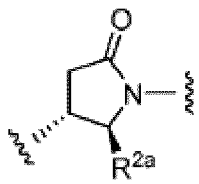 ,

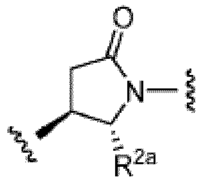 et 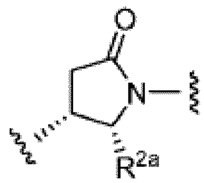 ;

et

$R^{2a}$ étant choisi dans le groupe constitué par $C_{1-6}$ alkyle, $C_{3-12}$ cycloalkyle, et $C_{6-14}$ aryle éventuellement substitué, éventuellement $R^{2a}$ étant choisi dans le groupe constitué par :

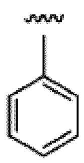 , 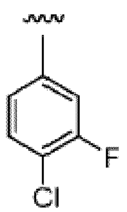 , 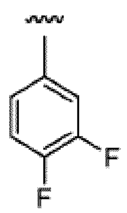 , 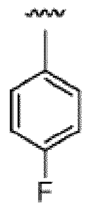 et 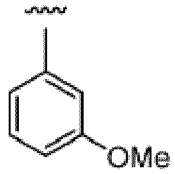 .

**6.** Composé pour une utilisation selon la revendication 1, ou sel ou hydrate pharmaceutiquement acceptable correspondant, B étant choisi parmi :

(a) le groupe constitué par :

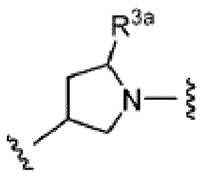 , 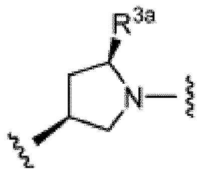 , 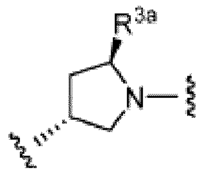 ,

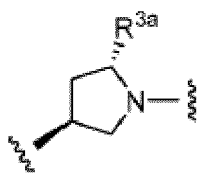 et 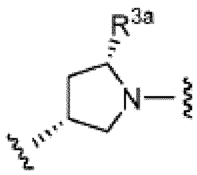 ;

et

$R^{3a}$ étant choisi dans le groupe constitué par $C_{1-6}$ alkyle, hydroxyalkyle, $C_{3-12}$ cycloalkyle, $C_{6-14}$ aryle éventuellement substitué, alcoxyalkyle, et - $CH_2N(H)C(=O)R^{8b}$, éventuellement $R^{3a}$ étant choisi dans le groupe constitué par :

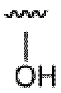 , 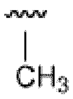 , 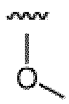 , 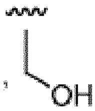 , 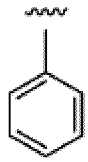 , 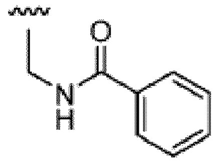 ,

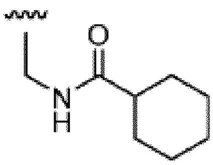 , 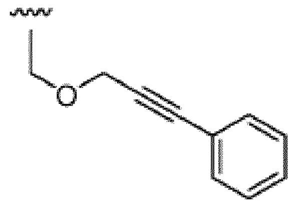 et 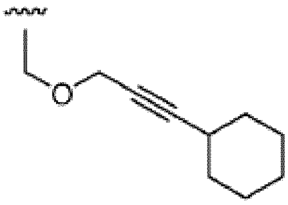 ;

ou

(b) le groupe constitué par :

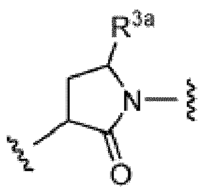 , 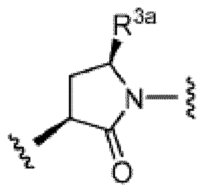 , 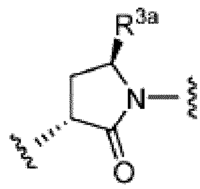 ,

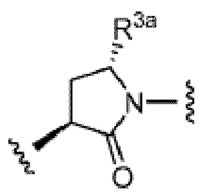 et 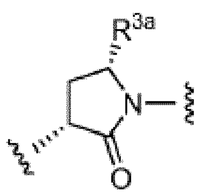 ;

et

$R^{3a}$ étant choisi dans le groupe constitué par $C_{1-6}$ alkyle, hydroxyalkyle, $C_{3-12}$ cycloalkyle, $C_{6-14}$ aryle éventuel-

lement substitué, alcoxyalkyle, et - $CH_2N(H)C(=O)R^{8b}$, éventuellement $R^{3a}$ étant choisi dans le groupe constitué par :

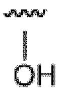 , 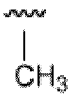 , 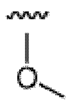 , 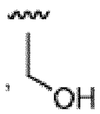 , 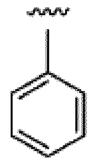 , 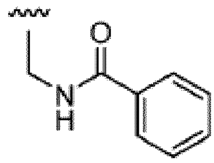 ,

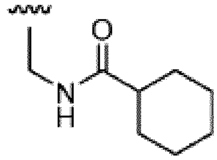 , 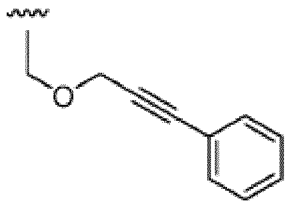 et 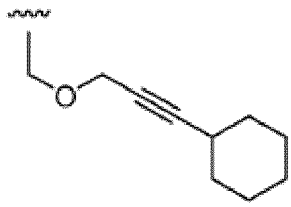 ;

ou

(c) le groupe constitué par :

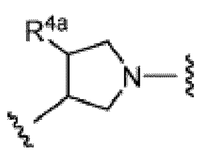 , 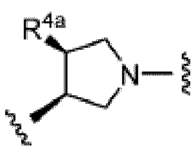 , 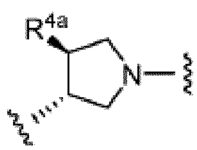 ,

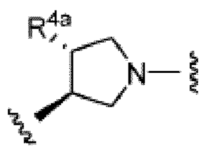 et 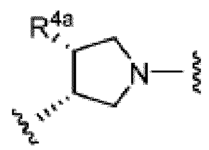 ;

et

$R^{4a}$ étant choisi dans le groupe constitué par hydroxy, $C_{1-6}$ alkyle, alcoxy, hydroxyalkyle, $C_{3-12}$ cycloalkyle, $C_{6-14}$ aryle éventuellement substitué, alcoxyalkyle, aralkyle, $-N(H)C(=O)R^{8a}$, et $-CH_2N(H)C(=O)R^{8b}$, éventuellement $R^{4a}$ étant choisi dans le groupe constitué par :

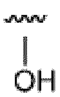 , 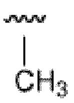 , 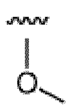 , 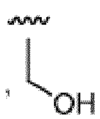 , 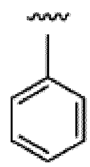 , 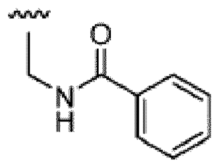 ,

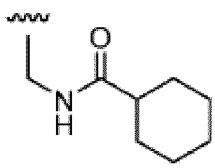 , 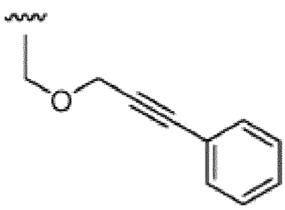 et 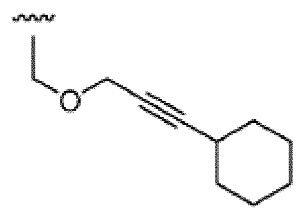 ;

ou

(d) le groupe constitué par :

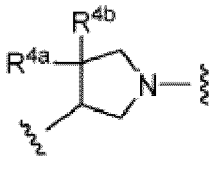 , 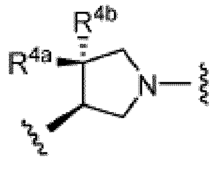 , 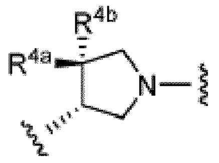 ,

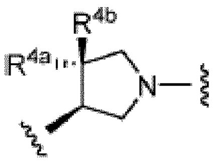 et 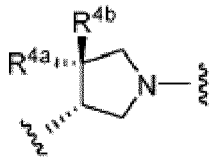 ;

$R^{4a}$ étant $C_{1-4}$ alkyle ; et $R^{4b}$ étant hydroxy.

**7.** Composé pour une utilisation selon l'une quelconque des revendications 1 à 6, ou sel ou hydrate pharmaceutiquement acceptable correspondant, X étant :

(a) choisi dans le groupe constitué par $-S(=O)_2-$ et - C(=O)- ; ou X étant absent ; ou
(b) $-S(=O)_2-$ ; ou
(c) -C(=O)- ; ou
(d) absent.

**8.** Composé pour une utilisation selon l'une quelconque des revendications 1 à 7, ou sel ou solvate pharmaceutiquement acceptable correspondant, Z étant choisi dans le groupe constitué par $C_{1-6}$ alkyle éventuellement substitué, hydroxyalkyle, (amino)alkyle, (alkylamino)alkyle, (dialkylamino)alkyle, (cycloalkylamino)alkyle, (hétérocyclo)alkyle, $C_{6-14}$ aryle éventuellement substitué, hétérocyclo à 4 à 14 chaînons éventuellement substitué, hétéroaryle à 5 à 14 chaînons éventuellement substitué, $C_{3-12}$ cycloalkyle éventuellement substitué, aralkyle, et hétéroaralkyle, et éventuellement Z étant choisi dans le groupe constitué par $C_{1-6}$ alkyle éventuellement substitué, (amino)alkyle, (alkylamino)alkyle, (hétérocyclo)alkyle, hétérocyclo à 4 à 14 chaînons éventuellement substitué, hétéroaryle à 5 à 14 chaînons éventuellement substitué, $C_{3-12}$ cycloalkyle éventuellement substitué, aralkyle, et hétéroaralkyle.

**9.** Composé pour une utilisation selon la revendication 1, ou sel ou solvate pharmaceutiquement acceptable correspondant, $R^1$ étant éthyle ou cyclopropyle ; éventuellement X étant choisi dans le groupe constitué par $-S(=O)_2-$, $-S(=O)_2N(R^6)-$, $-S(=O)_2C(R^7)(H)-$, -C(=O)-, $-C(=O)N(R^6)-$, -C(=O)O-, et $-C(=O)C(R^7)(H)$.

**10.** Composé pour une utilisation selon la revendication 1, le composé étant choisi dans le Tableau 1 :

| Composé n° | Structure |
|---|---|
| **1** | 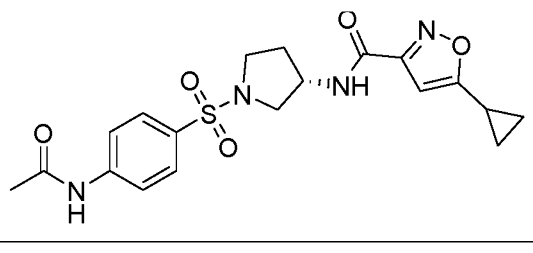 |
| **2** | 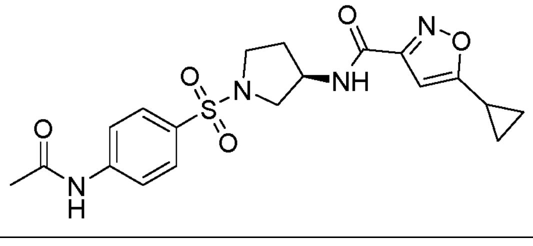 |

(suite)

| Composé n° | Structure |
|---|---|
| **3** | 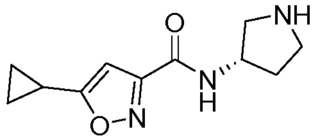 |
| **4** | 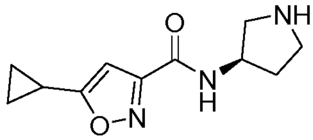 |
| **5** | 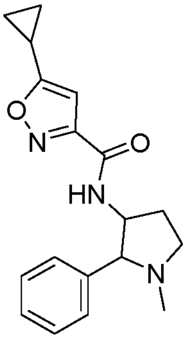 |
| **6** | 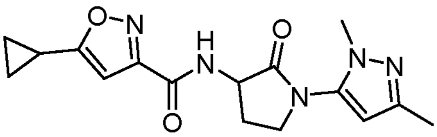 |
| **7** | 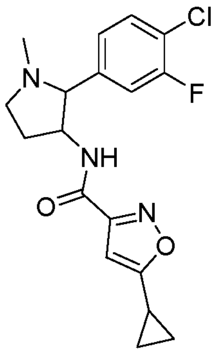 |
| **8** | 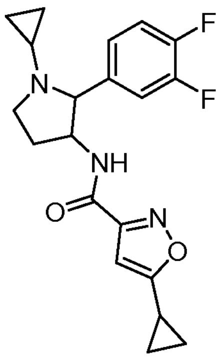 |

(suite)

| Composé n° | Structure |
|---|---|
| **9** | 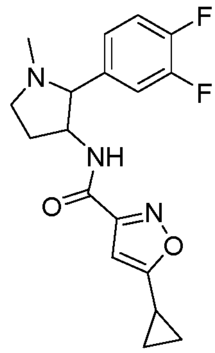 |
| **10** | 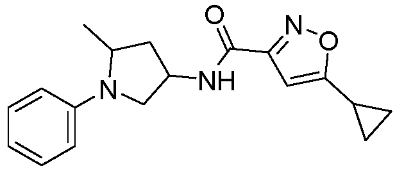 |
| **11** | 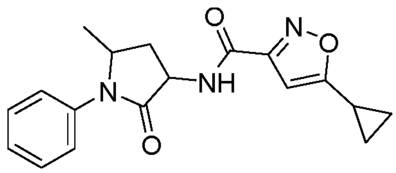 |
| **12** | 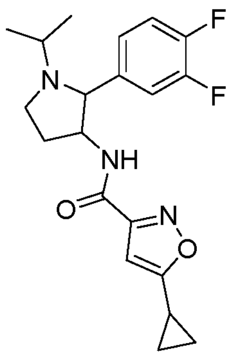 |
| **13** | 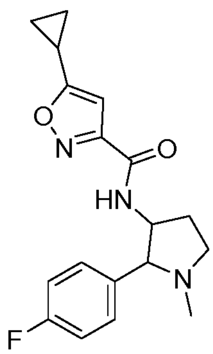 |

(suite)

| Composé n° | Structure |
|---|---|
| **14** | 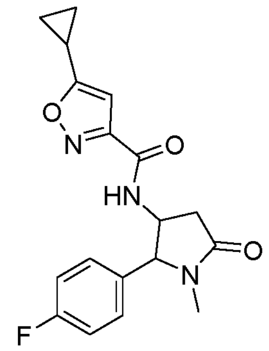 |
| **15** | 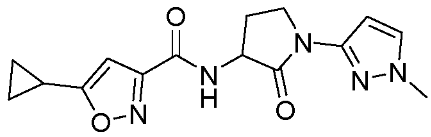 |
| **16** | 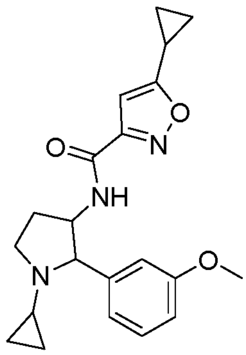 |
| **17** | 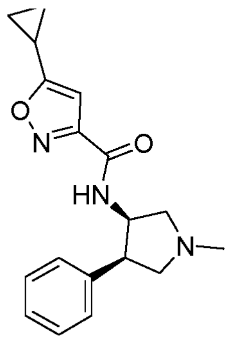 |
| **18** | 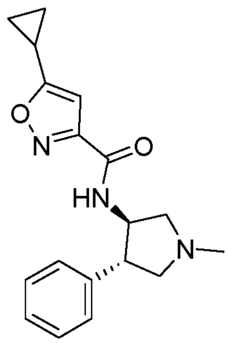 |

(suite)

| Composé n° | Structure |
|---|---|
| **19** | 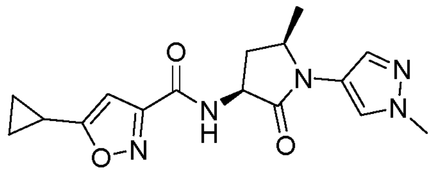 |
| **20** | 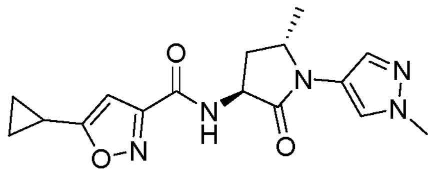 |
| **21** | 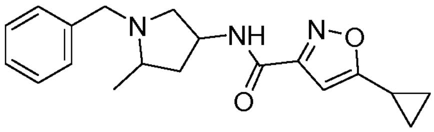 |
| **22** | 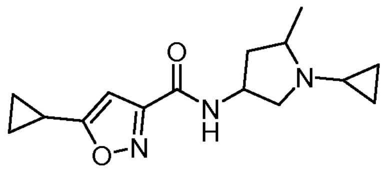 |
| **23** | 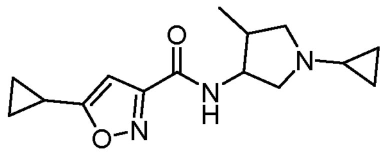 |
| **24** | 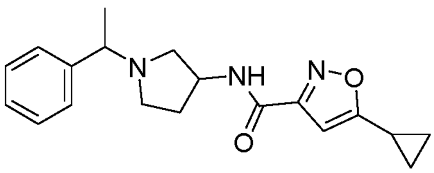 |
| **25** | 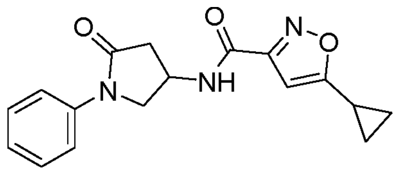 |
| **26** | 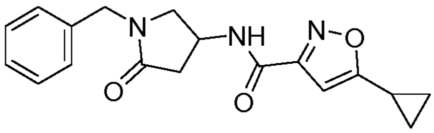 |
| **27** | 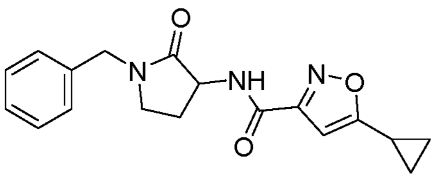 |

(suite)

| Composé n° | Structure |
|---|---|
| **28** | 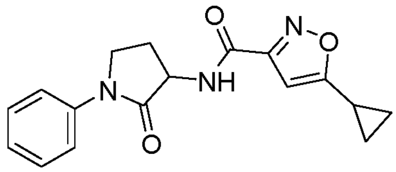 |
| **29** | 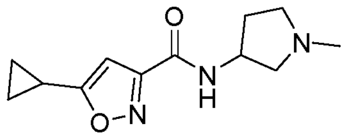 |
| **30** | 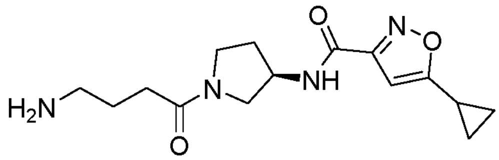 |
| **31** | 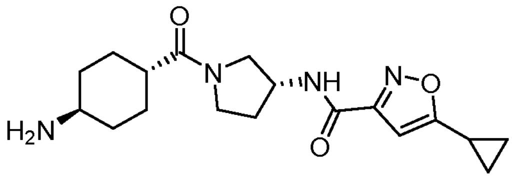 |
| **32** | 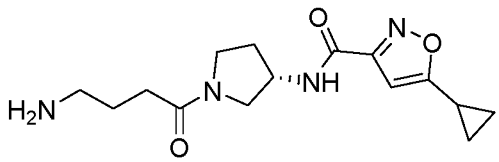 |
| **33** | 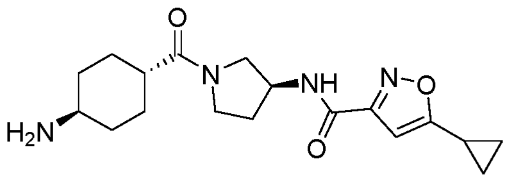 |
| **34** | 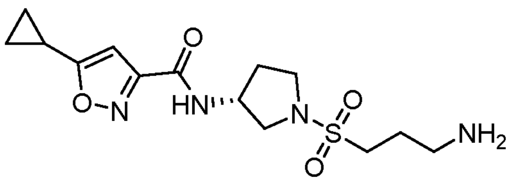 |
| **35** | 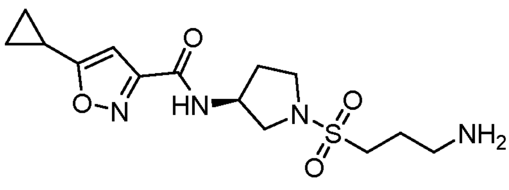 |
| **36** | 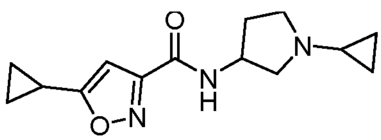 |

(suite)

| Composé n° | Structure |
|---|---|
| **37** | 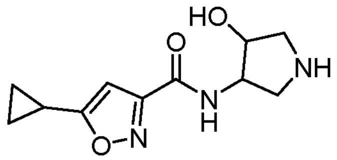 |
| **38** | 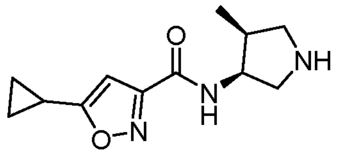 |
| **39** | 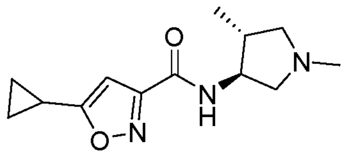 |
| **40** | 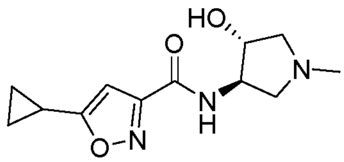 |
| **41** | 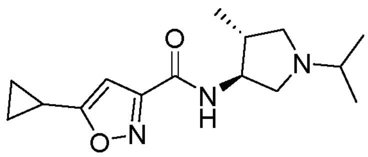 |
| **42** | 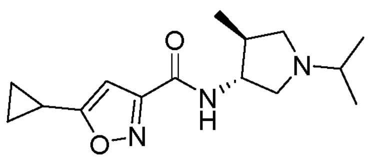 |
| **43** | 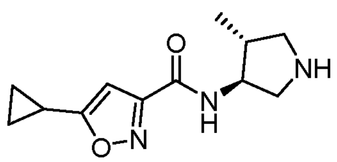 |
| **44** | 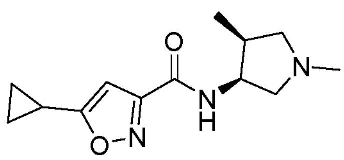 |
| **45** | 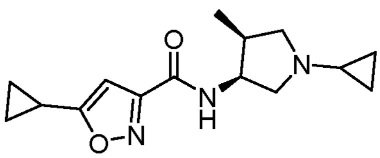 |

(suite)

| Composé n° | Structure |
|---|---|
| **46** | 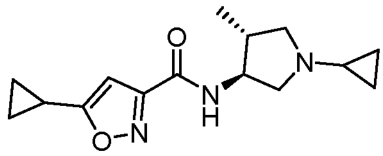 |
| **47** | 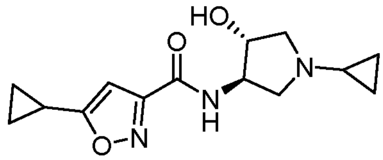 |
| **48** | 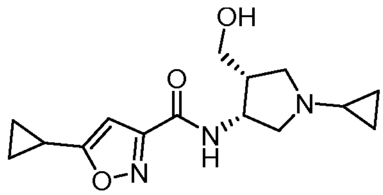 |
| **49** | 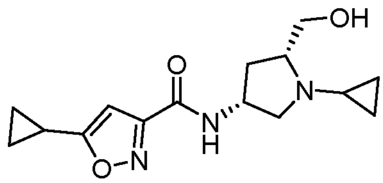 |
| **50** | 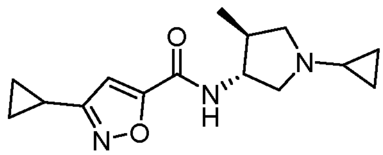 |
| **51** | 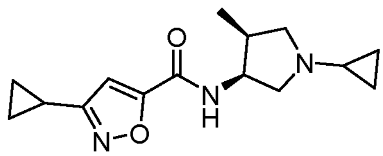 |
| **52** | 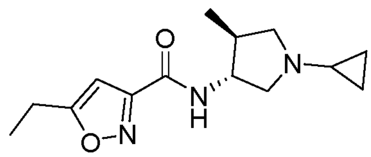 |
| **53** | 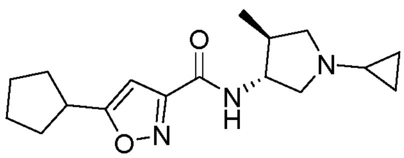 |
| **54** | 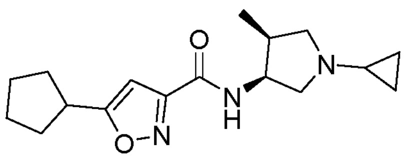 |

(suite)

| Composé n° | Structure |
|---|---|
| **112** | 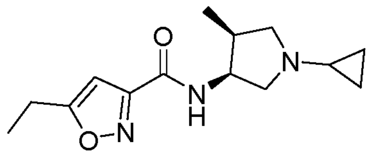 |

ou le Tableau 2 :

| Composé n° | Structure |
|---|---|
| 55 | 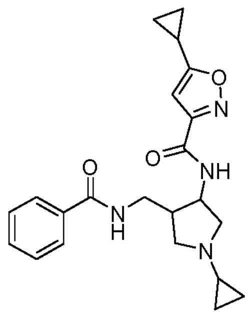 |
| 56 | 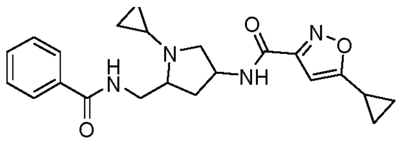 |
| 57 | 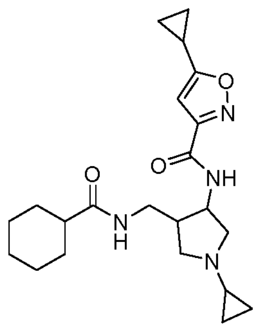 |
| 58 | 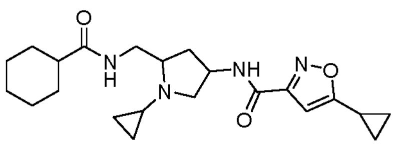 |
| 59 | 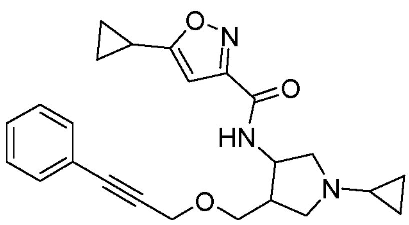 |

(suite)

| Composé n° | Structure |
|---|---|
| 60 | 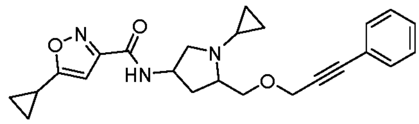 |
| 61 | 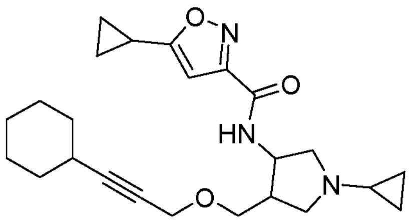 |
| 62 | 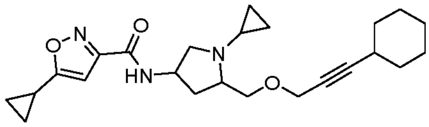 |
| 63 | 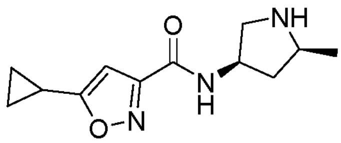 |
| 64 | 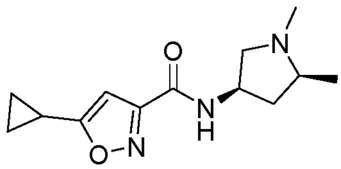 |
| 65 | 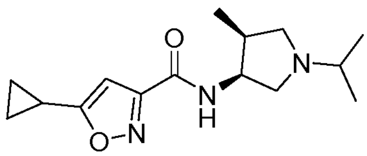 |
| 66 | 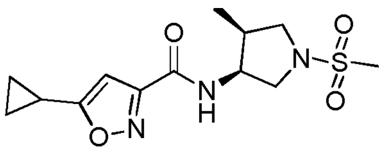 |
| 67 | 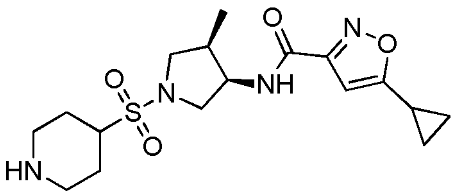 |
| 68 | 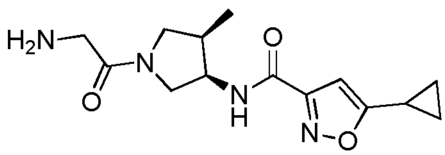 |

(suite)

| Composé n° | Structure |
|---|---|
| 69 | 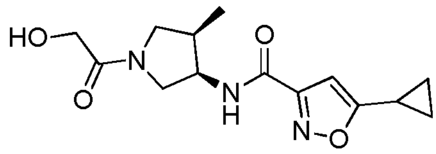 |
| 70 | 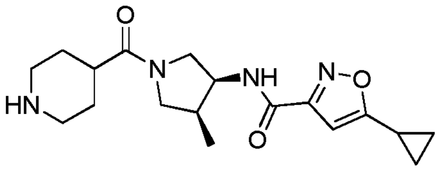 |
| 71 | 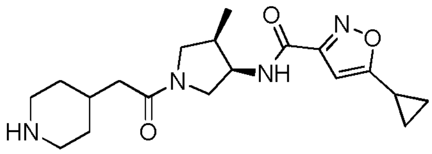 |
| 72 | 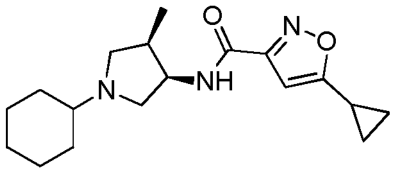 |
| 73 | 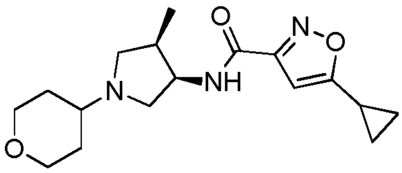 |
| 74 | 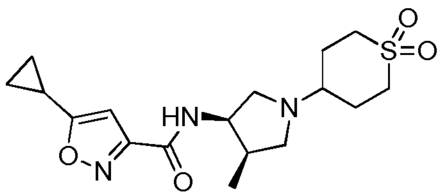 |
| 75 | 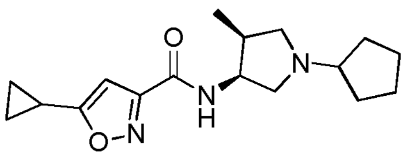 |
| 76 | 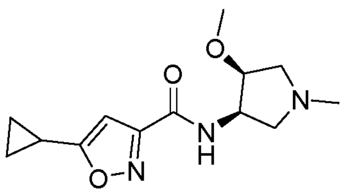 |

(suite)

| Composé n° | Structure |
|---|---|
| 77 | 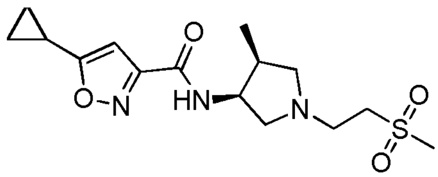 |
| 78 | 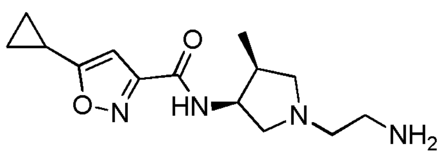 |
| 79 | 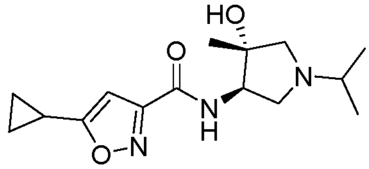 |
| 80 | 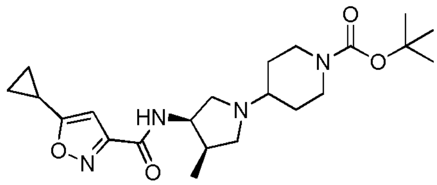 |
| 81 | 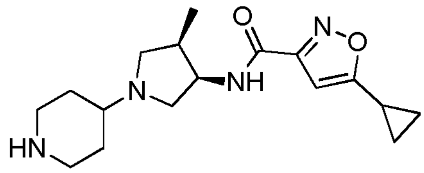 |
| 82 | 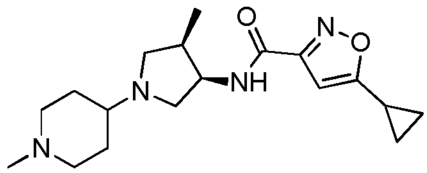 |
| 83 | 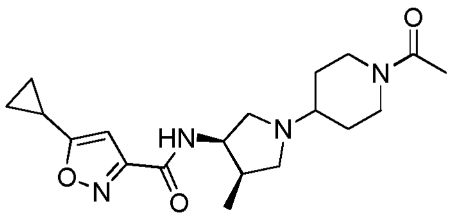 |
| 84 | 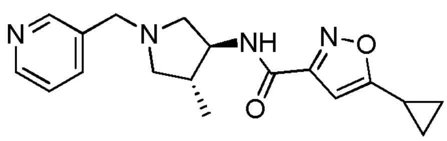 |
| 85 | 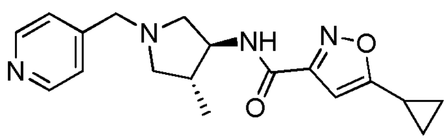 |

(suite)

| Composé n° | Structure |
|---|---|
| 86 | 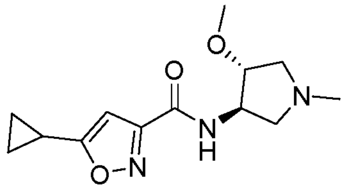 |
| 87 | 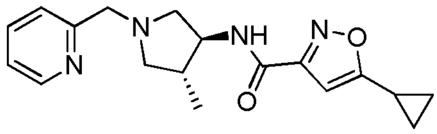 |
| 88 | 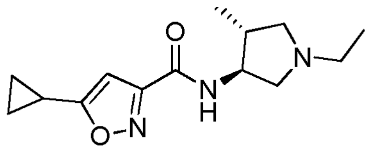 |
| 89 | 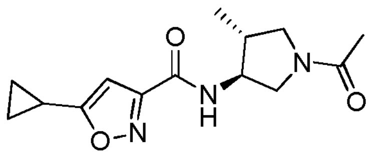 |
| 90 | 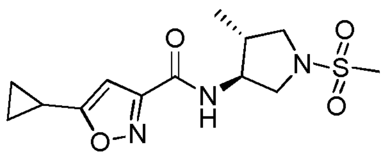 |
| 91 | 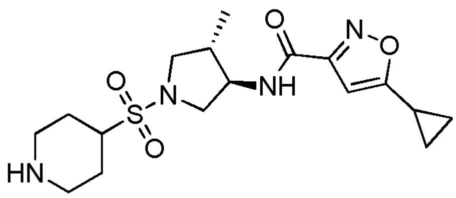 |
| 92 | 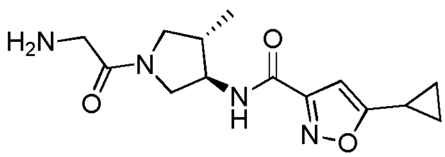 |
| 93 | 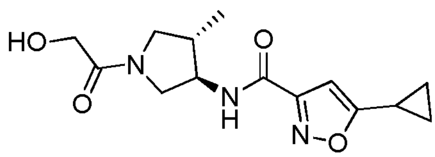 |
| 94 | 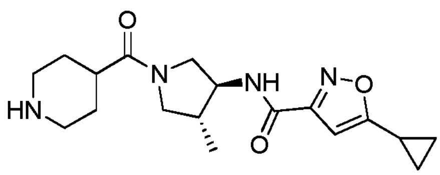 |

(suite)

| Composé n° | Structure |
|---|---|
| 95 | 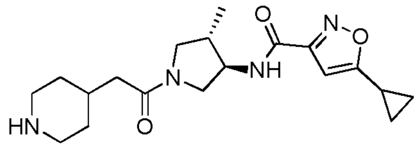 |
| 96 | 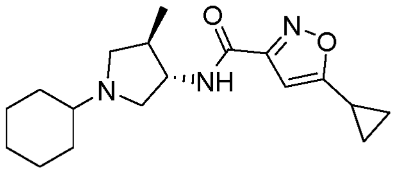 |
| 97 | 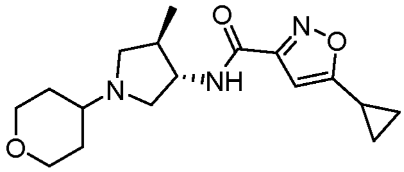 |
| 98 | 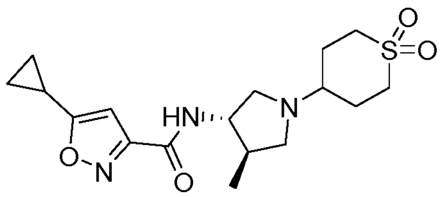 |
| 99 | 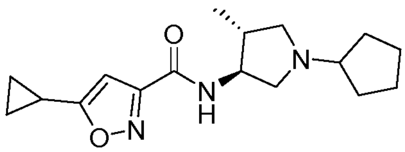 |
| 100 | 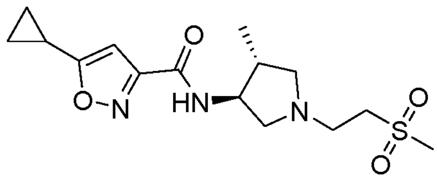 |
| 101 | 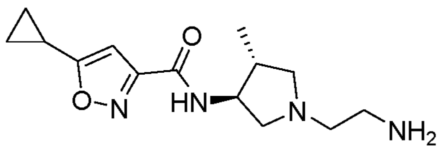 |
| 102 | 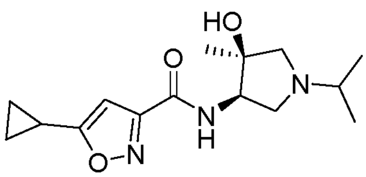 |
| 103 | 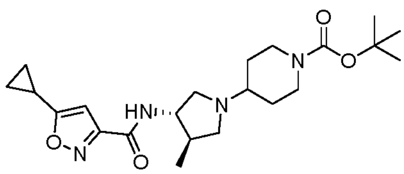 |

(suite)

| Composé n° | Structure |
|---|---|
| 104 | 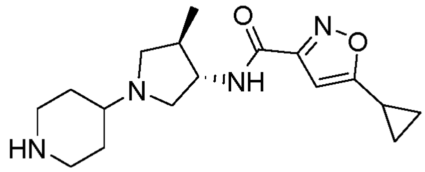 |
| 105 | 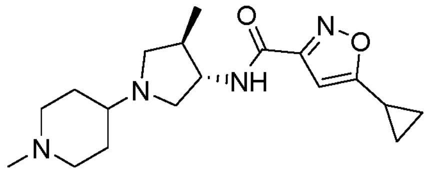 |
| 106 | 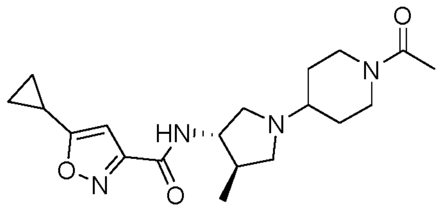 |
| 107 | 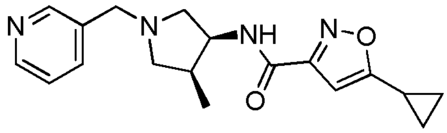 |
| 108 | 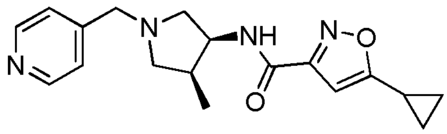 |
| 109 | 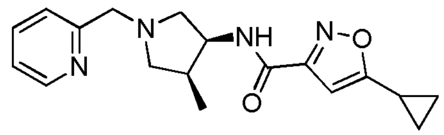 |
| 110 | 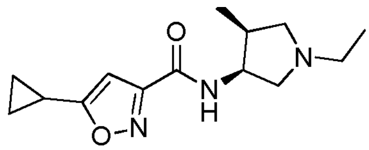 |
| 111 | 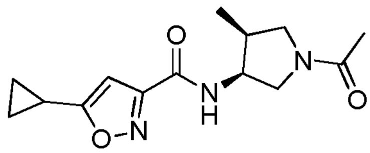 |

ou le Tableau 3 :

| Composé n° | Structure |
|---|---|
| 113 | 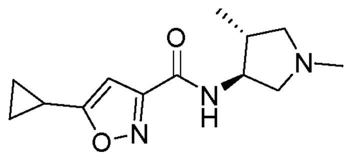 |
| 114 | 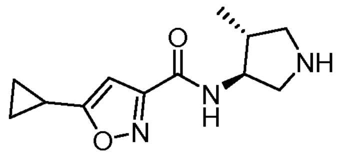 |
| 115 | 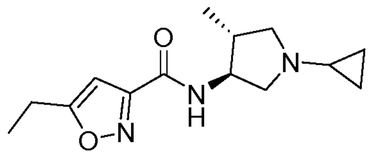 |
| 116 | 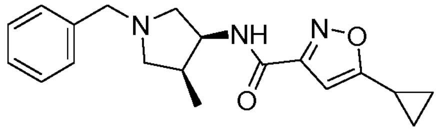 |
| 117 | 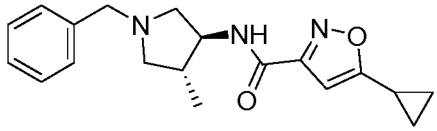 |
| 118 | 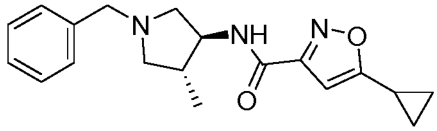 |
| 119 | 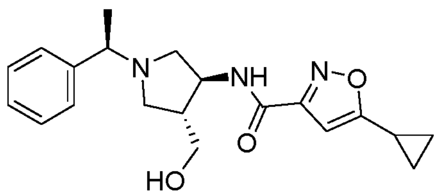 |
| 120 | 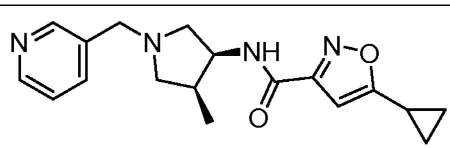 |
| 121 | 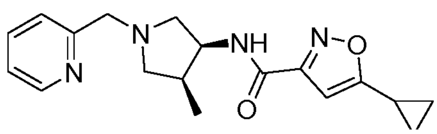 |
| 122 | 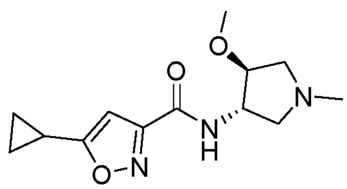 |

(suite)

| Composé n° | Structure |
|---|---|
| 123 | 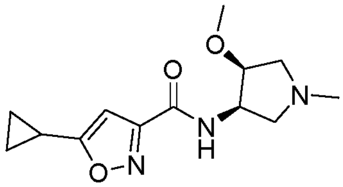 |
| 124 | 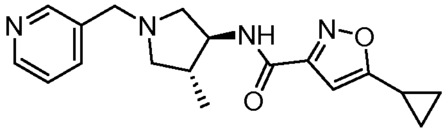 |
| 125 | 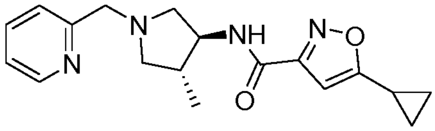 |
| 126 | 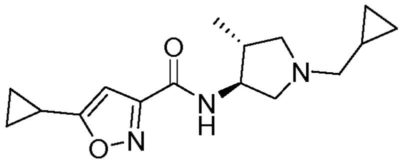 |
| 127 | 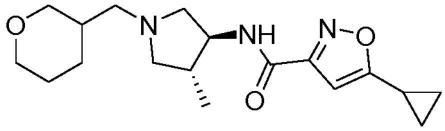 |
| 128 | 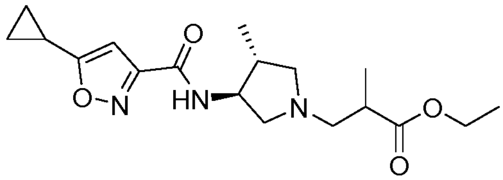 |
| 129 | 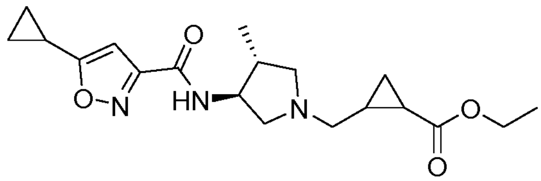 |
| 130 | 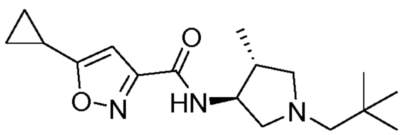 |
| 131 | 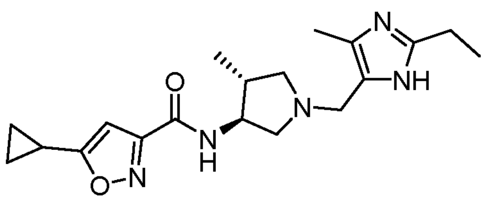 |

(suite)

| Composé n° | Structure |
|---|---|
| 132 | 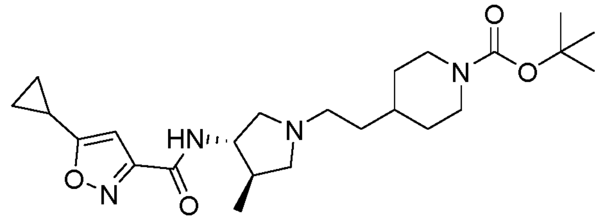 |
| 133 | 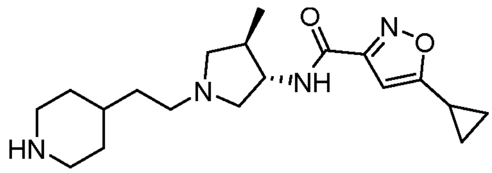 |
| 134 | 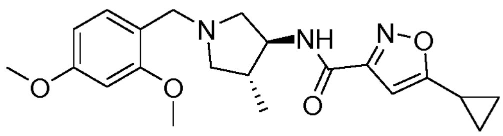 |
| 135 | 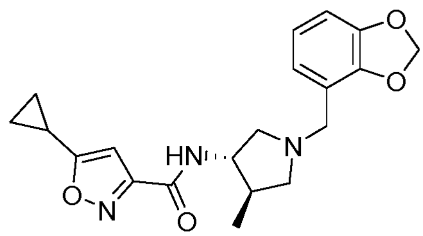 |
| 136 | 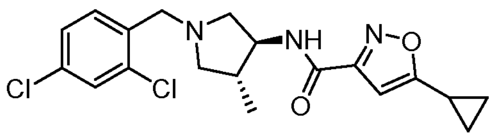 |
| 137 | 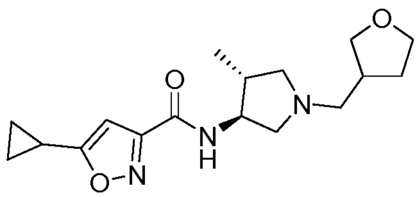 |
| 138 | 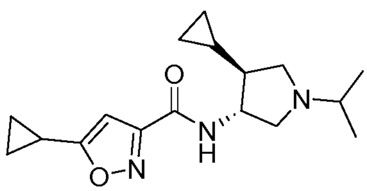 |
| 139 | 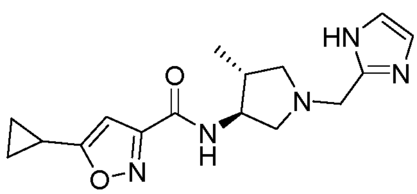 |

(suite)

| Composé n° | Structure |
|---|---|
| 140 | 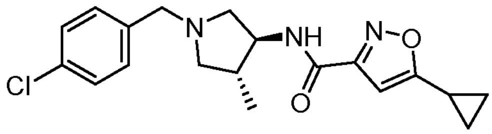 |
| 141 | 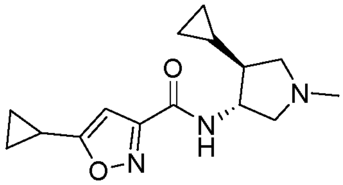 |
| 142 | 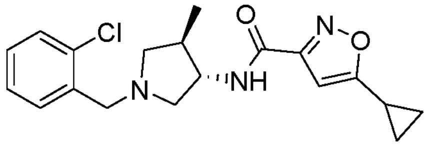 |
| 143 | 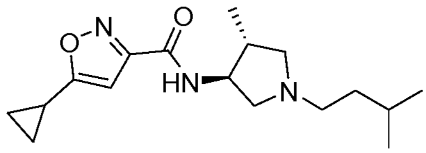 |
| 144 | 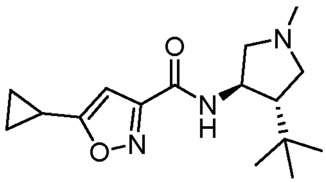 |
| 146 | 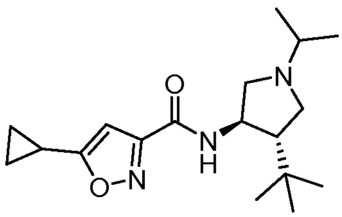 |
| 148 | 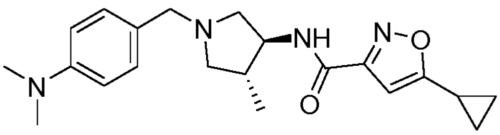 |
| 149 | 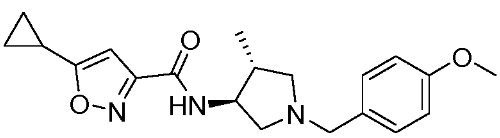 |
| 150 | 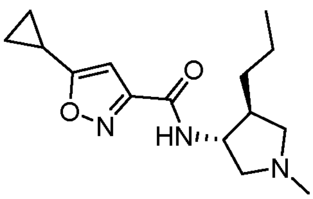 |

(suite)

| Composé n° | Structure |
|---|---|
| 151 | 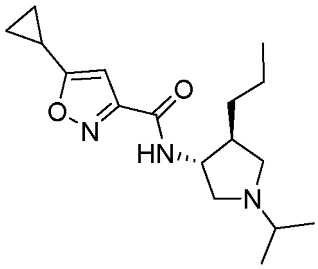 |
| 152 | 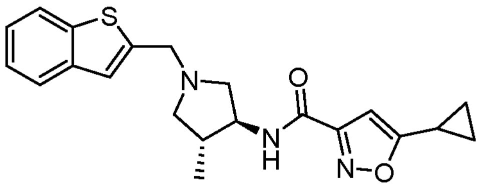 |
| 153 | 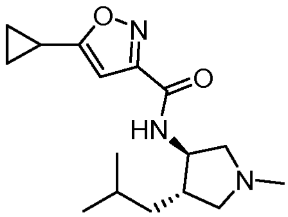 |
| 154 | 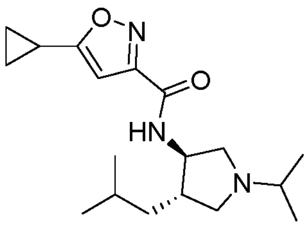 |
| 155 | 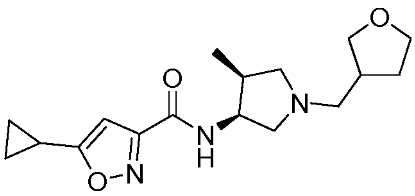 |
| 156 | 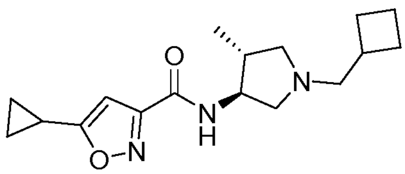 |
| 157 | 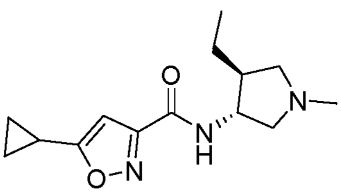 |

(suite)

| Composé n° | Structure |
|---|---|
| 158 | 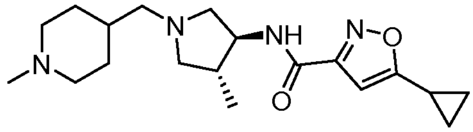 |
| 159 | 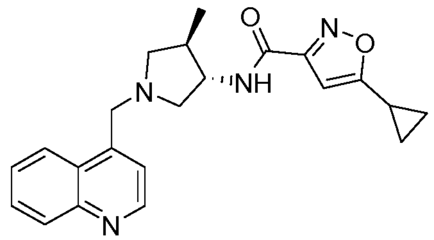 |
| 160 | 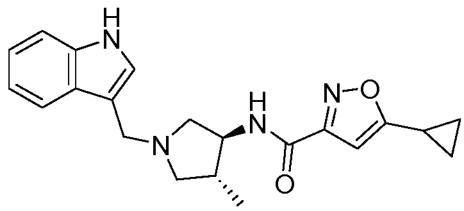 |
| 161 | 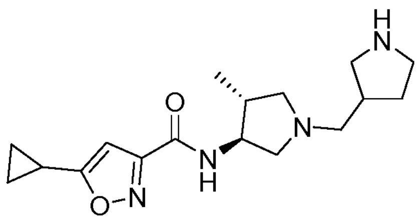 |
| 162 | 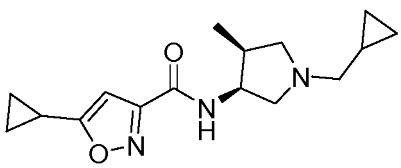 |
| 163 | 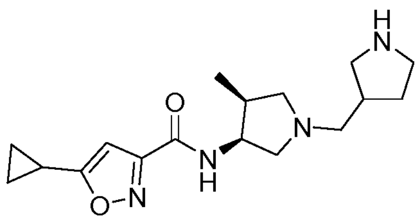 |
| 164 | 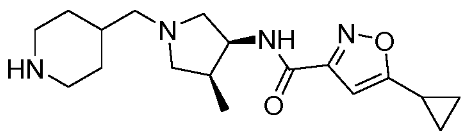 |
| 165 | 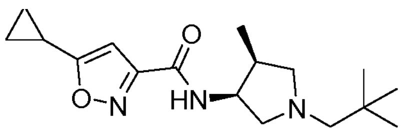 |

(suite)

| Composé n° | Structure |
|---|---|
| 166 | 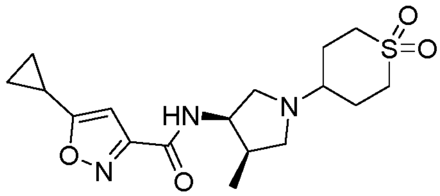 |
| 168 | 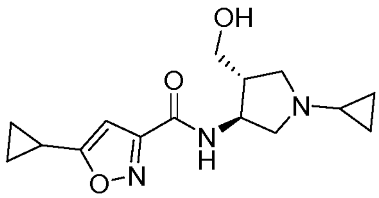 |
| 169 | 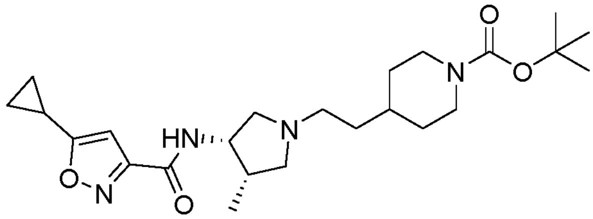 |
| 170 | 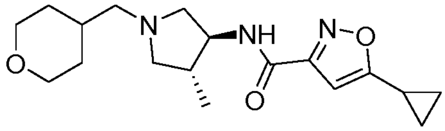 |
| 171 | 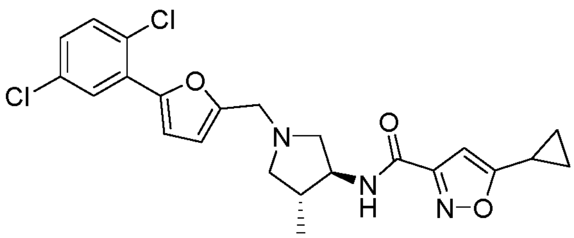 |
| 172 | 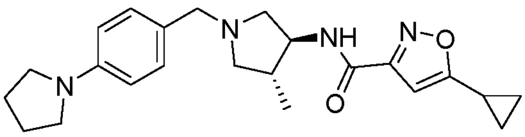 |
| 173 | 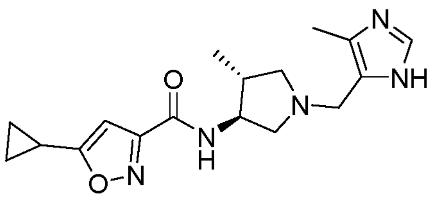 |
| 174 | 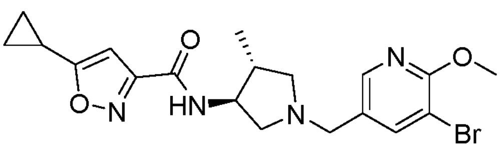 |

(suite)

| Composé n° | Structure |
|---|---|
| 175 | 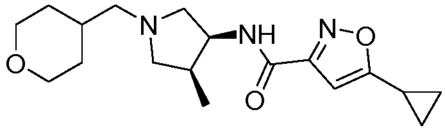 |
| 176 | 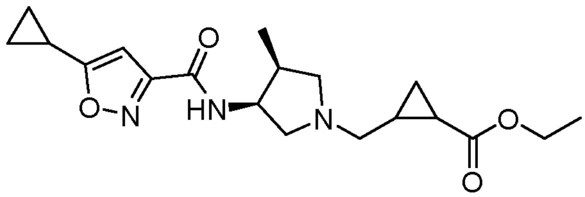 |
| 177 | 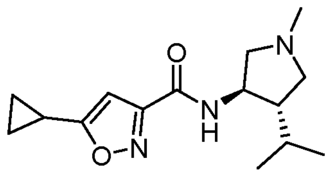 |
| 178 | 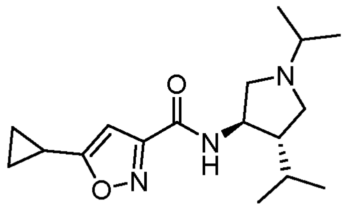 |
| 179 | 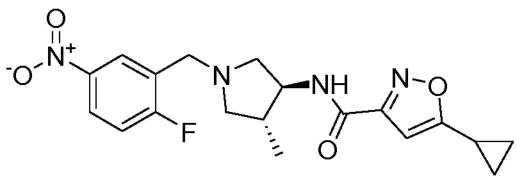 |
| 180 | 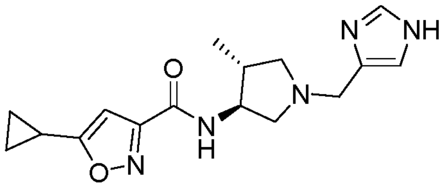 |
| 181 | 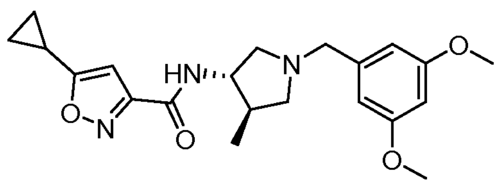 |
| 182 | 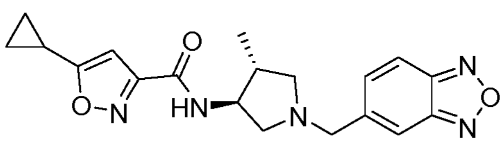 |

(suite)

| Composé n° | Structure |
|---|---|
| 183 | 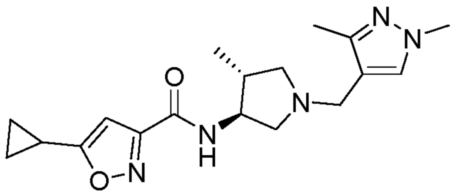 |
| 184 | 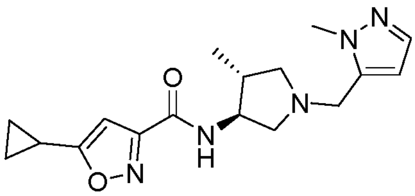 |
| 185 | 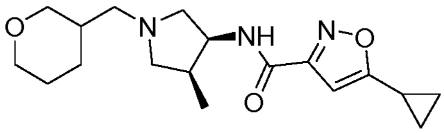 |
| 186 | 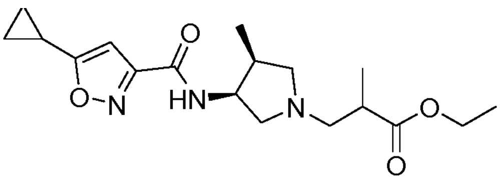 |
| 187 | 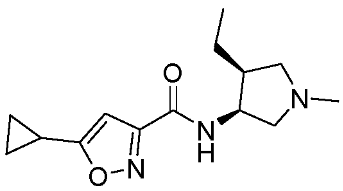 |
| 188 | 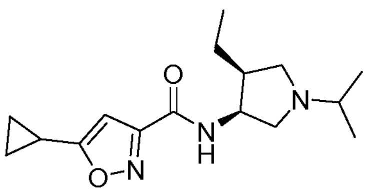 |
| 189 | 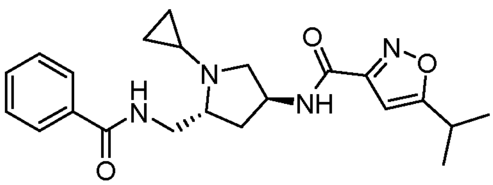 |
| 190 | 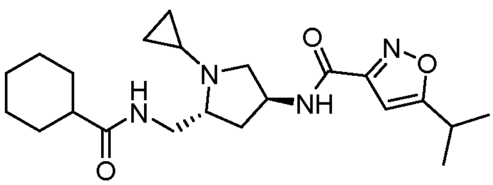 |

(suite)

| Composé n° | Structure |
|---|---|
| 191 | 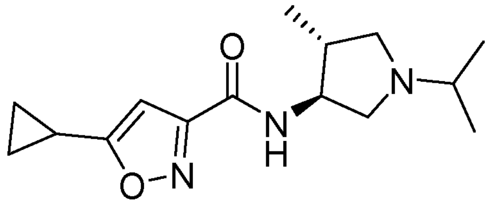 |
| 192 | 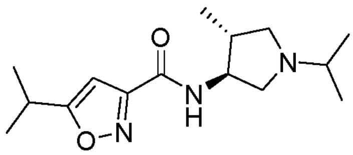 |
| 193 | 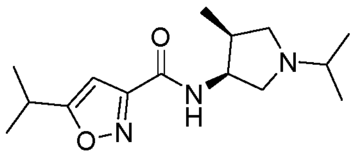 |
| 194 | 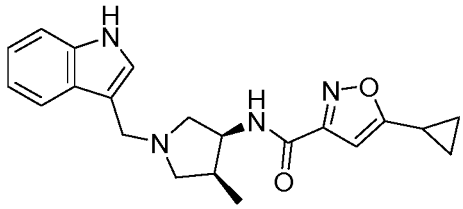 |
| 195 | 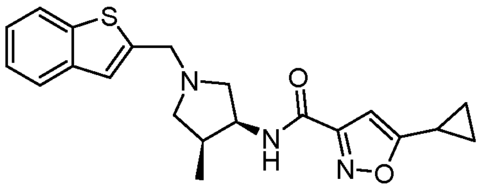 |
| 196 | 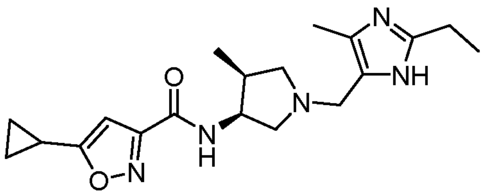 |
| 197 | 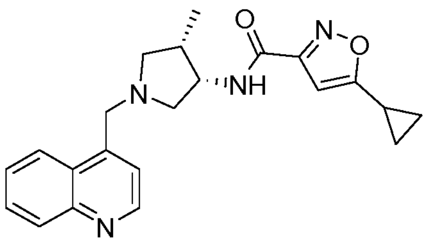 |

(suite)

| Composé n° | Structure |
|---|---|
| 198 | 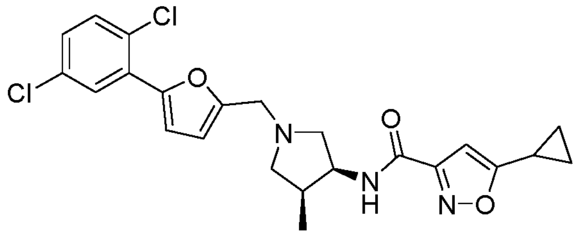 |
| 199 | 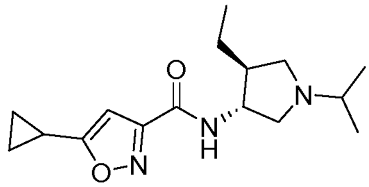 |
| 200 | 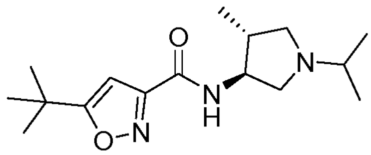 |
| 201 | 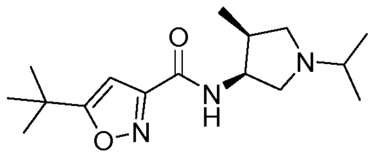 |
| 202 | 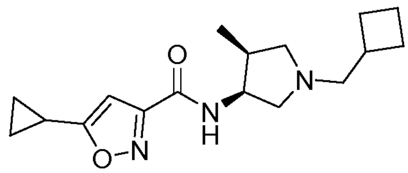 |
| 203 | 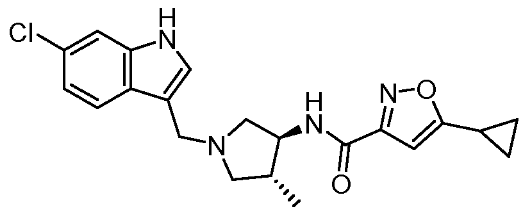 |
| 204 | 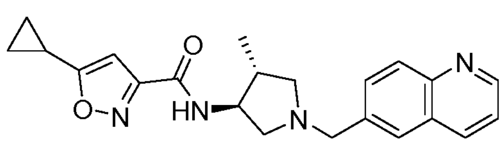 |
| 205 | 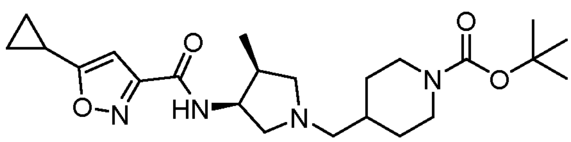 |

(suite)

| Composé n° | Structure |
|---|---|
| 206 | 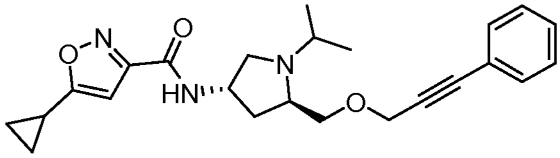 |
| 207 | 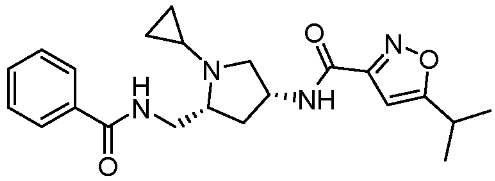 |
| 208 | 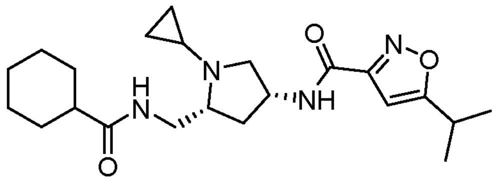 |
| 209 | 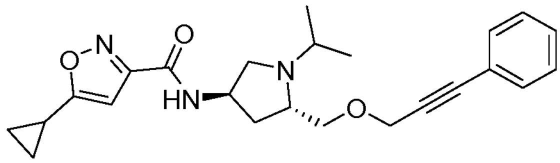 |
| 210 | 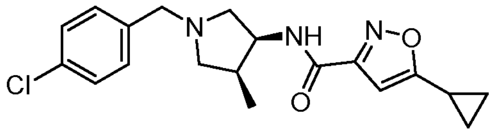 |
| 211 | 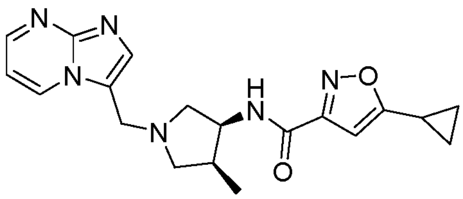 |
| 212 | 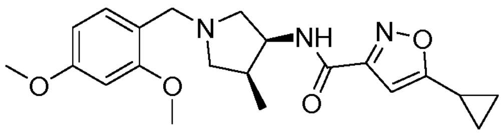 |
| 213 | 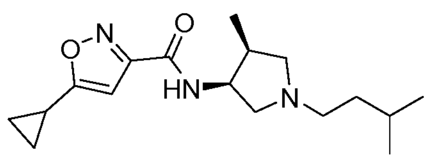 |
| 214 | 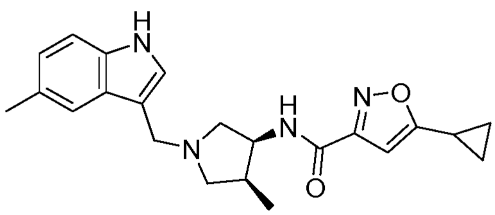 |

(suite)

| Composé n° | Structure |
|---|---|
| 215 | 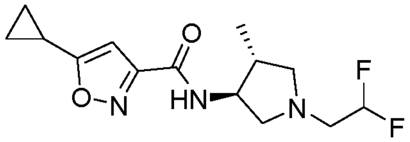 |
| 216 | 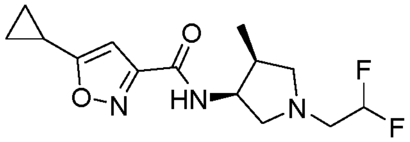 |
| 217 | 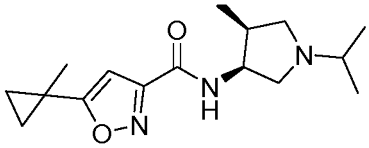 |
| 218 | 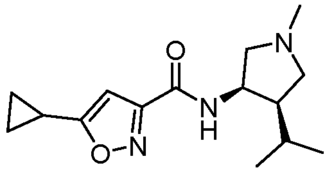 |
| 219 | 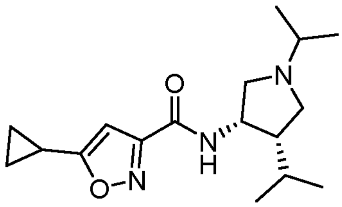 |
| 220 | 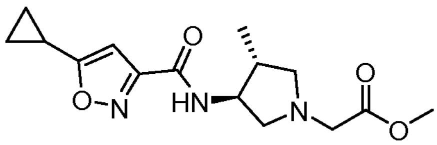 |
| 221 | 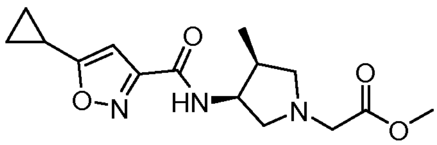 |
| 222 | 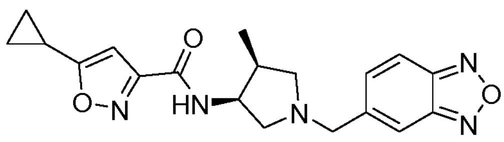 |
| 223 | 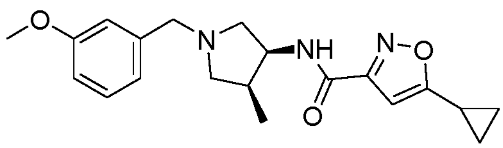 |

(suite)

| Composé n° | Structure |
|---|---|
| 224 | 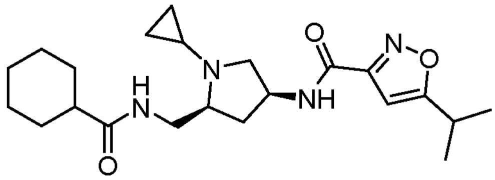 |
| 225 | 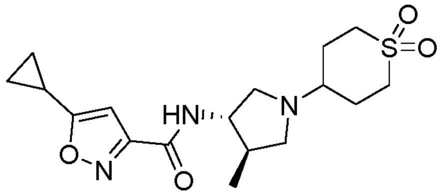 |
| 226 | 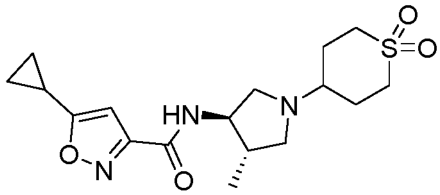 |
| 227 | 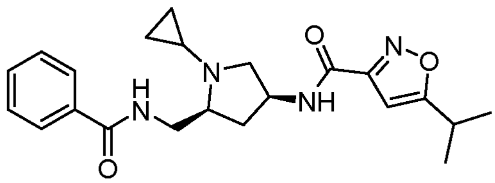 |
| 228 | 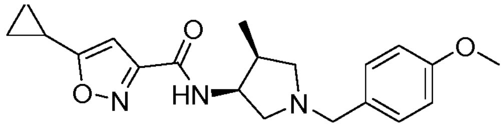 |
| 229 | 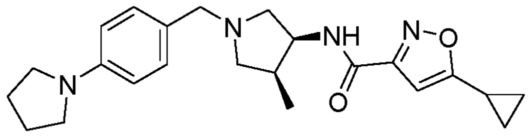 |
| 230 | 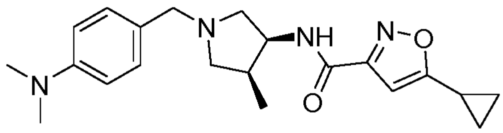 |
| 231 | 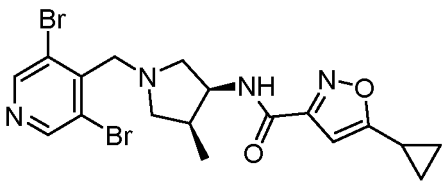 |
| 232 | 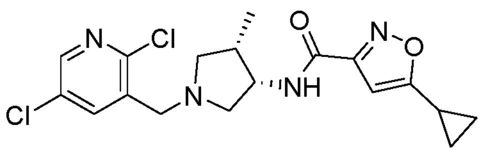 |

(suite)

| Composé n° | Structure |
|---|---|
| 233 | 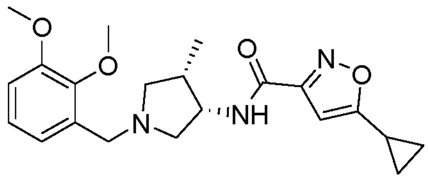 |
| 234 | 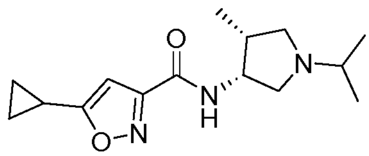 |
| 235 | 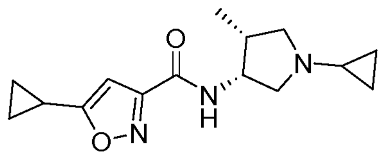 |
| 236 | 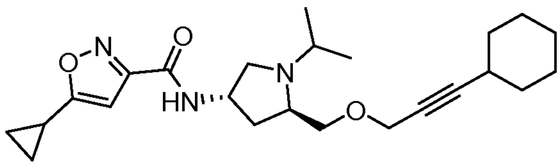 |
| 237 | 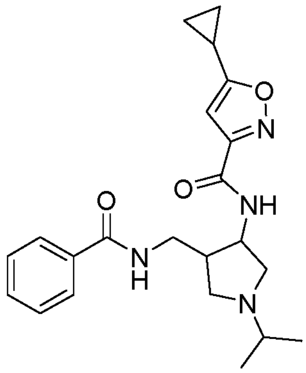 |
| 238 | 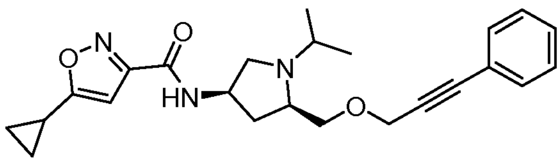 |
| 239 | 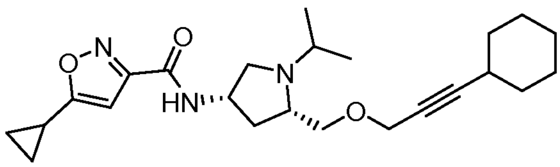 |
| 240 | 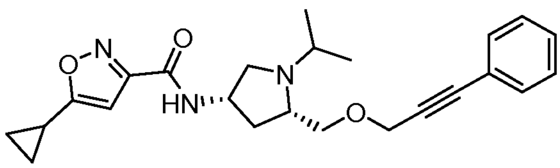 |

(suite)

| Composé n° | Structure |
|---|---|
| 241 | 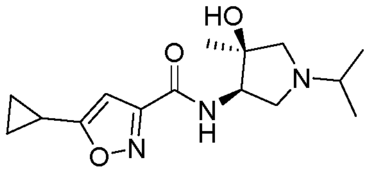 |
| 242 | 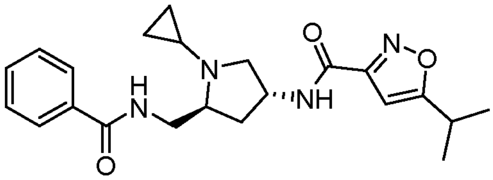 |
| 243 | 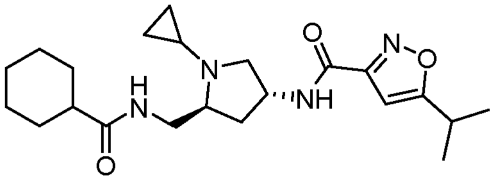 |
| 244 | 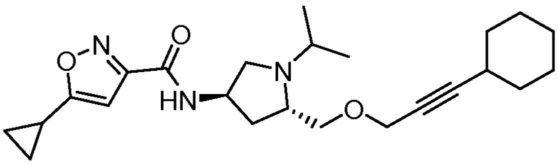 |
| 245 | 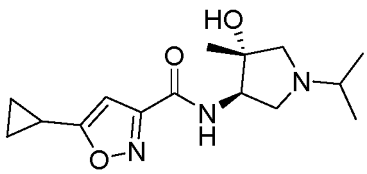 |
| 246 | 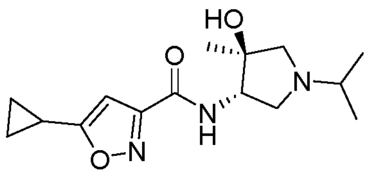 |
| 247 | 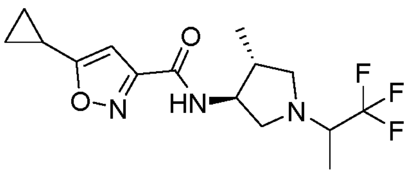 |
| 248 | 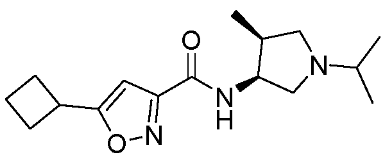 |

(suite)

| Composé n° | Structure |
|---|---|
| 249 | 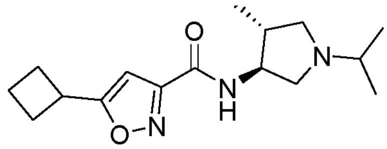 |
| 250 | 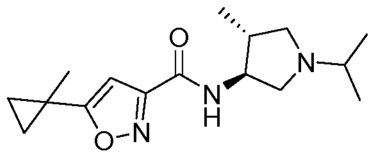 |
| 251 | 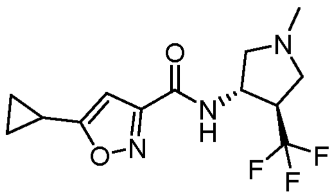 |
| 252 | 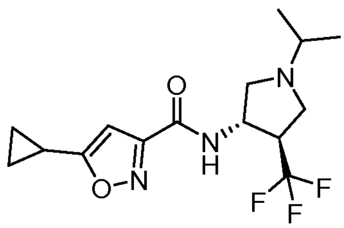 |
| 253 | 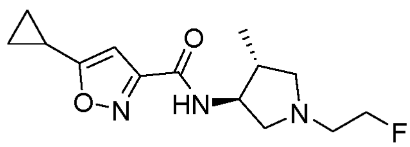 |
| 254 | 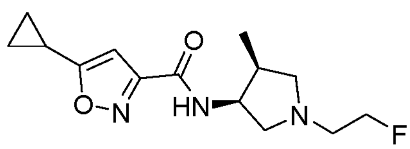 |
| 255 | 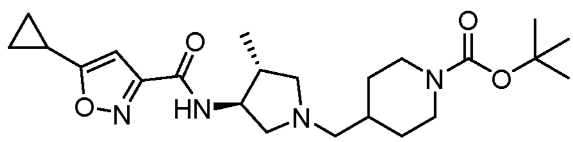 |
| 256 | 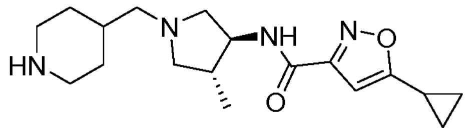 |
| 257 | 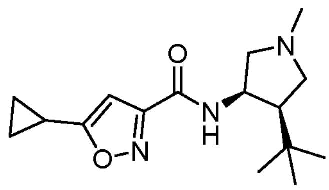 |

(suite)

| Composé n° | Structure |
|---|---|
| 258 | 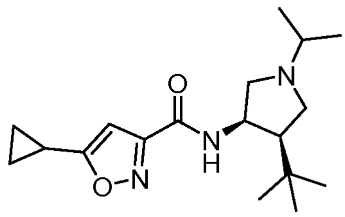 |
| 259 | 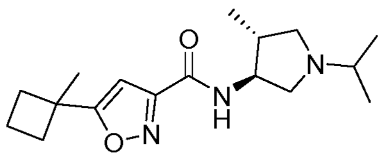 |
| 260 | 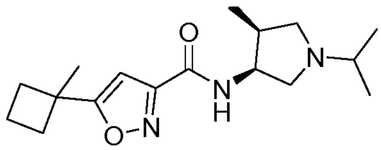 |
| 261 | 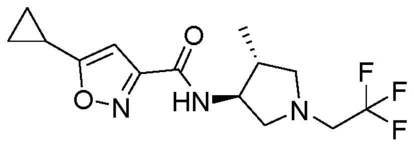 |
| 262 | 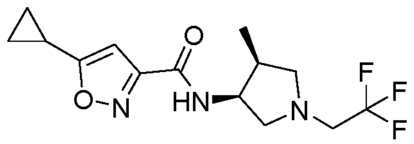 |
| 263 | 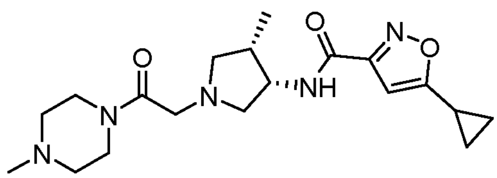 |
| 264 | 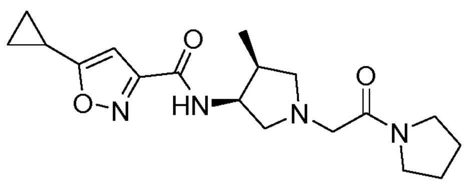 |
| 265 | 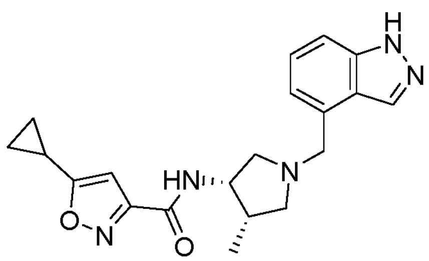 |

(suite)

| Composé n° | Structure |
|---|---|
| 266 | 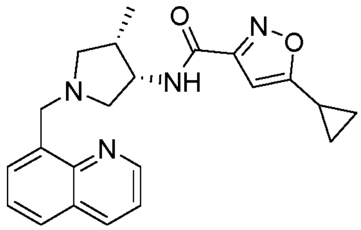 |
| 267 | 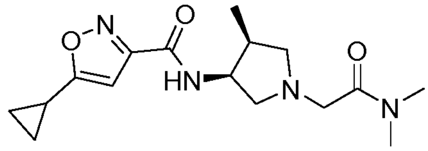 |
| 268 | 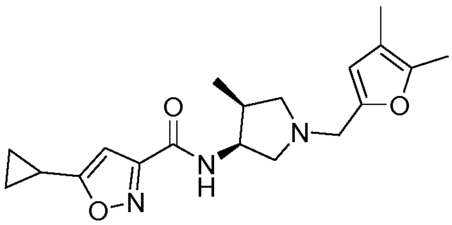 |
| 269 | 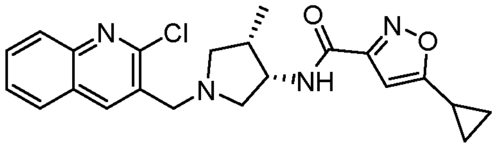 |
| 270 | 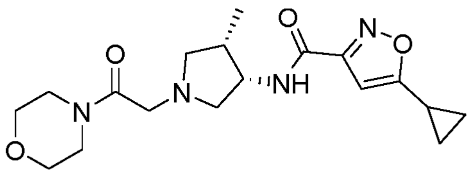 |
| 271 | 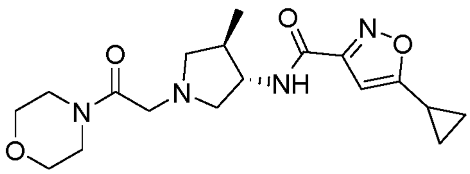 |
| 272 | 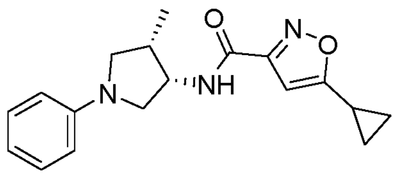 |
| 273 | 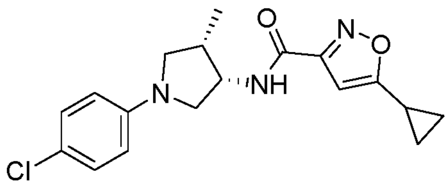 |

(suite)

| Composé n° | Structure |
|---|---|
| 274 | 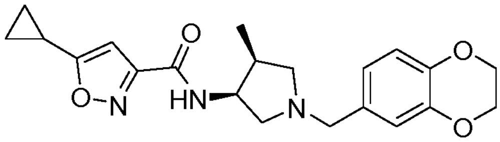 |
| 275 | 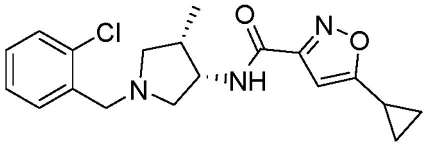 |
| 276 | 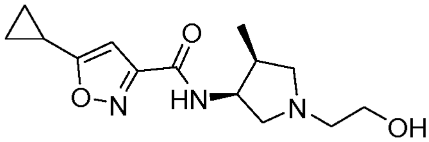 |
| 277 | 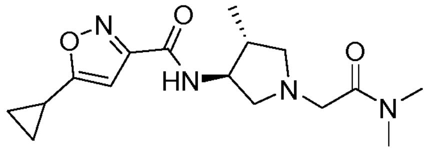 |
| 278 | 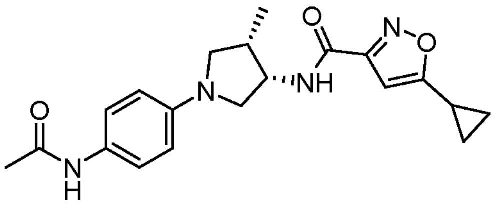 |
| 279 | 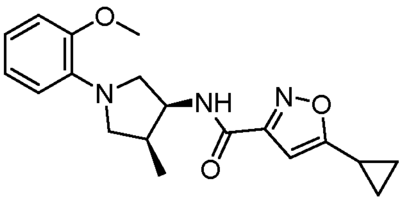 |
| 280 | 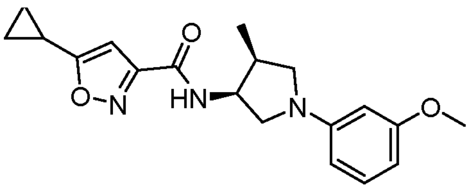 |
| 281 | 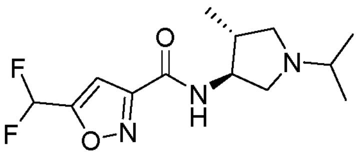 |

(suite)

| Composé n° | Structure |
|---|---|
| 282 | 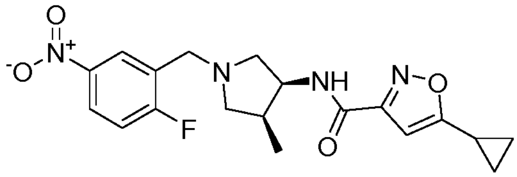 |
| 283 | 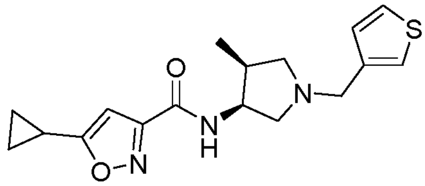 |
| 284 | 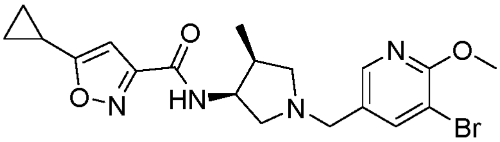 |
| 285 | 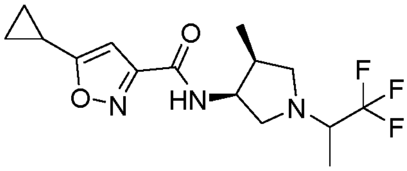 |
| 286 | 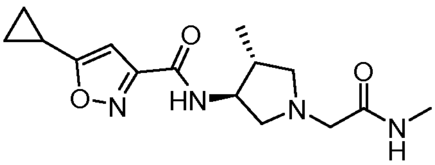 |
| 287 | 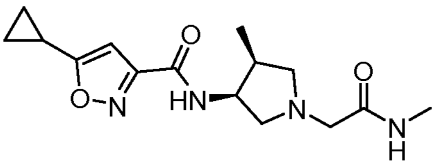 |
| 288 | 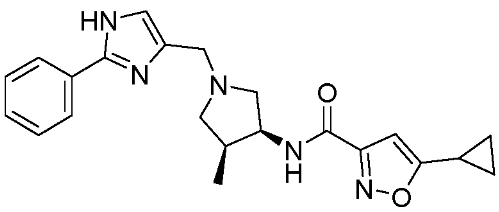 |
| 289 | 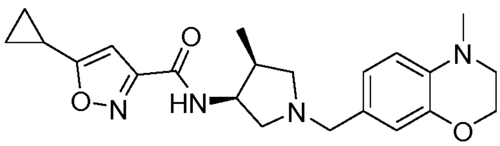 |
| 290 | 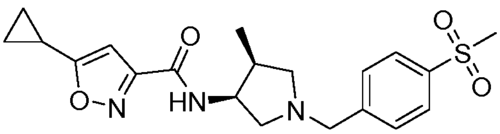 |

(suite)

| Composé n° | Structure |
|---|---|
| 291 | 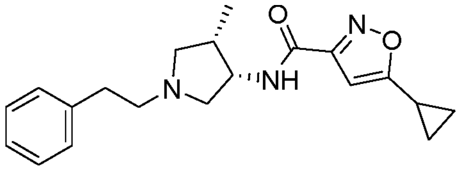 |
| 292 | 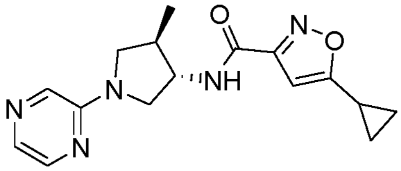 |
| 293 | 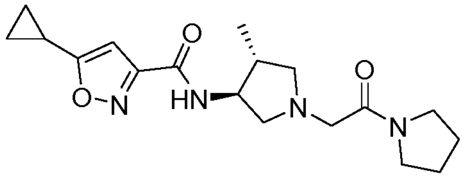 |
| 294 | 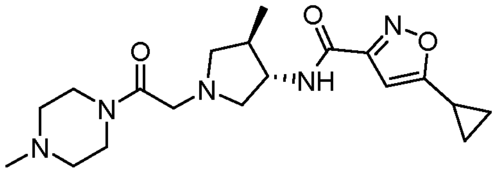 |
| 295 | 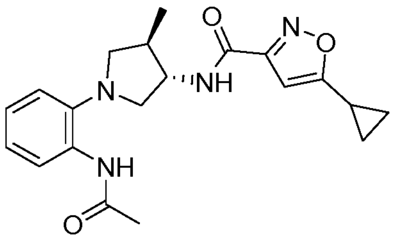 |
| 296 | 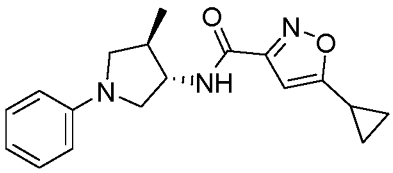 |
| 297 | 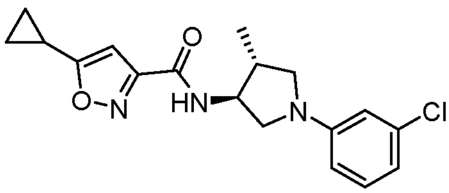 |
| 298 | 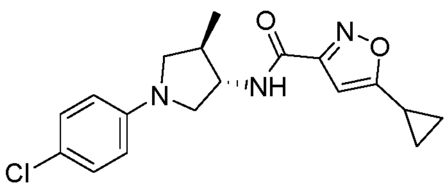 |

(suite)

| Composé n° | Structure |
|---|---|
| 299 | 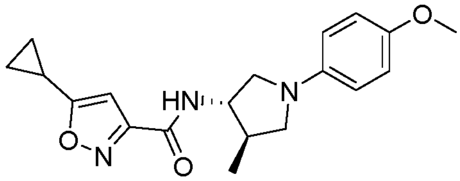 |
| 300 | 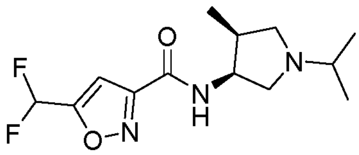 |
| 301 | 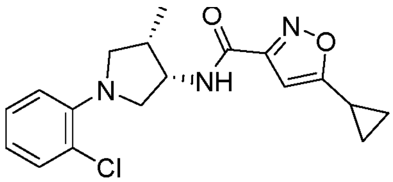 |
| 302 | 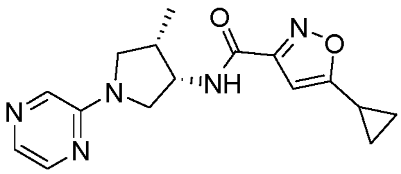 |
| 303 | 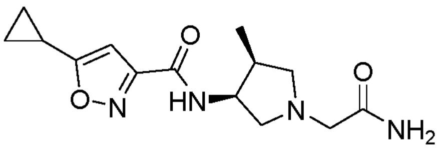 |
| 304 | 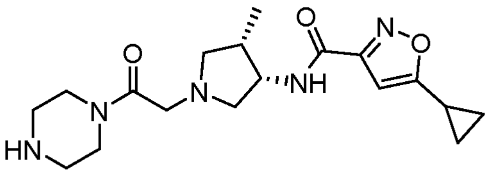 |
| 305 | 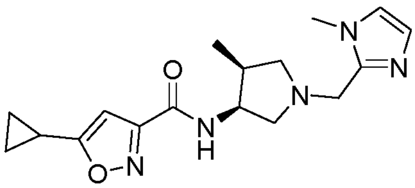 |
| 306 | 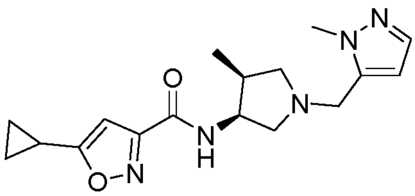 |

(suite)

| Composé n° | Structure |
|---|---|
| 307 | 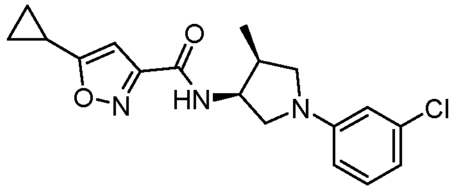 |
| 308 | 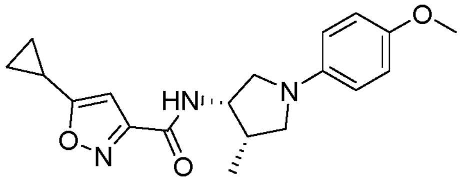 |
| 309 | 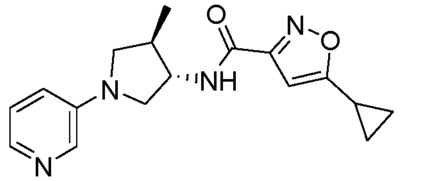 |
| 310 | 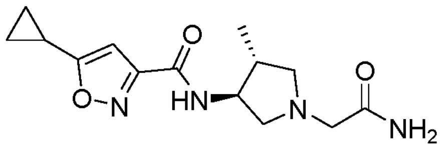 |
| 311 | 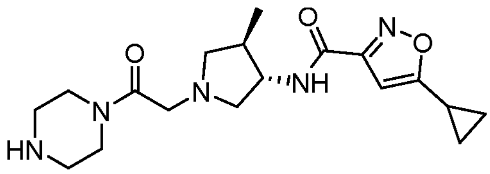 |
| 312 | 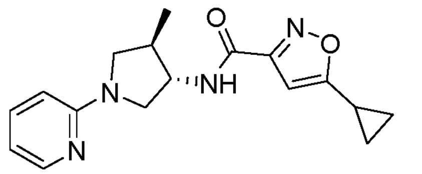 |
| 313 | 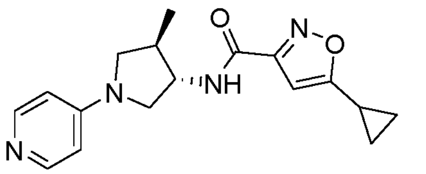 |
| 314 | 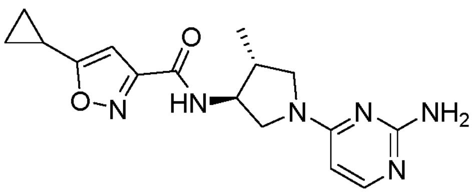 |

(suite)

| Composé n° | Structure |
|---|---|
| 315 | 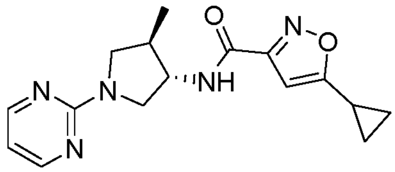 |
| 316 | 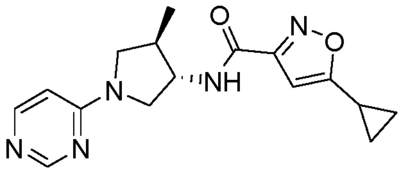 |
| 317 | 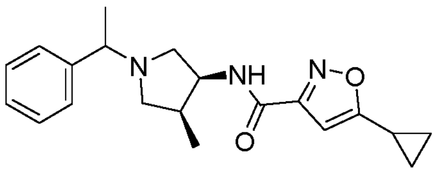 |
| 318 | 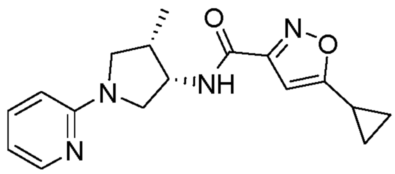 |
| 319 | 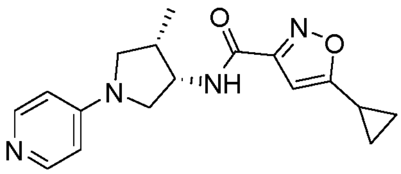 |
| 320 | 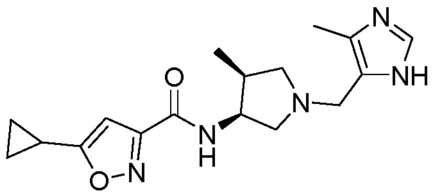 |
| 321 | 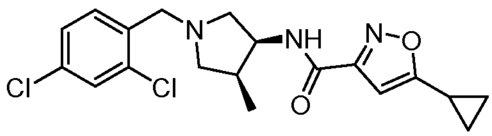 |
| 322 | 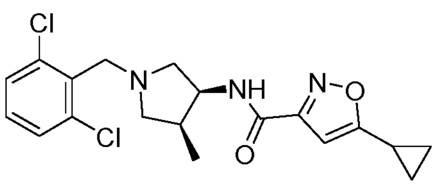 |
| 323 | 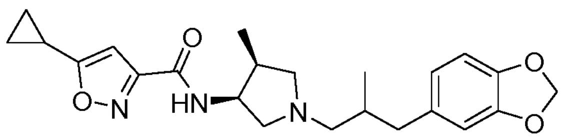 |

(suite)

| Composé n° | Structure |
|---|---|
| 324 | 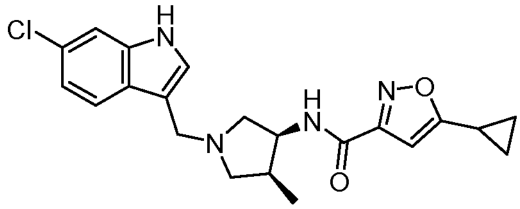 |
| 325 | 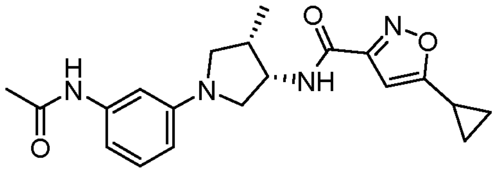 |
| 326 | 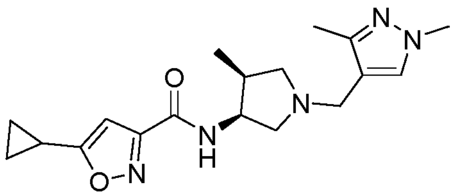 |
| 327 | 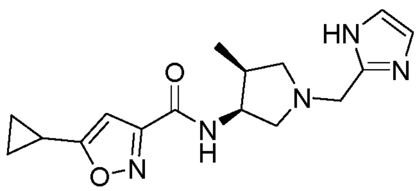 |
| 328 | 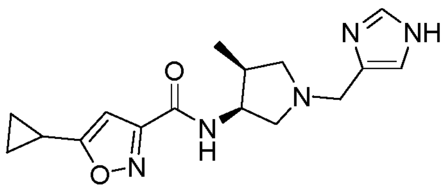 |
| 329 | 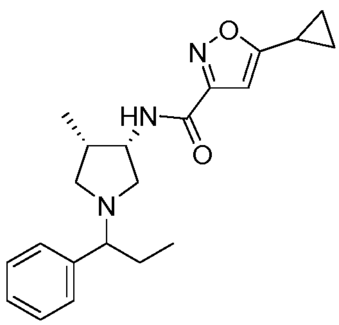 |

(suite)

| Composé n° | Structure |
|---|---|
| 330 | 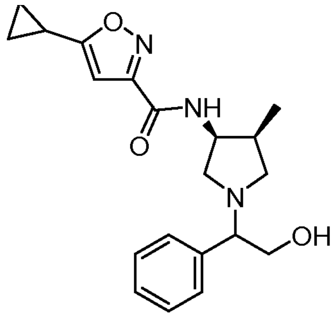 |
| 331 | 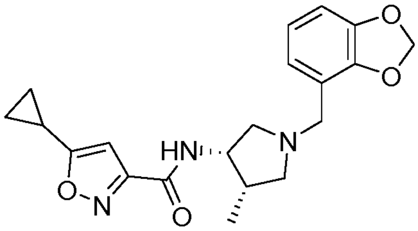 |
| 332 | 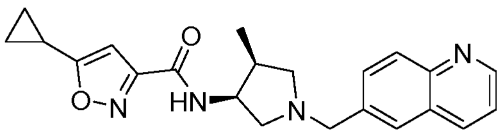 |
| 333 | 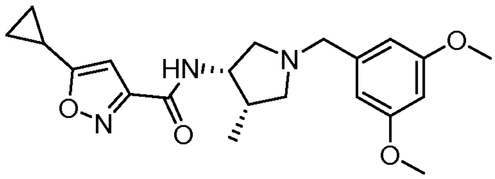 |
| 334 | 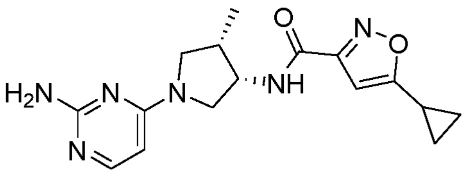 |
| 335 | 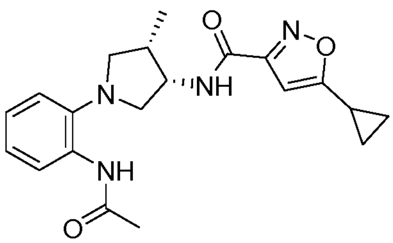 |
| 336 | 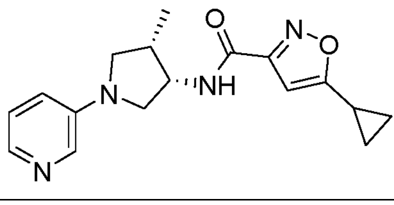 |

(suite)

| Composé n° | Structure |
|---|---|
| 337 | 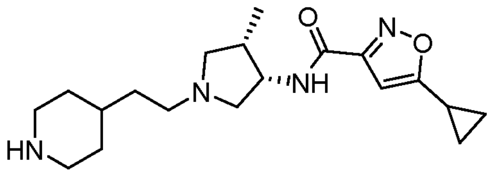 |
| 338 | 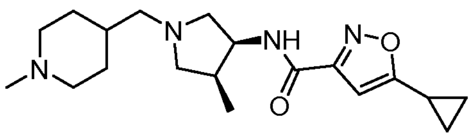 |
| 339 | 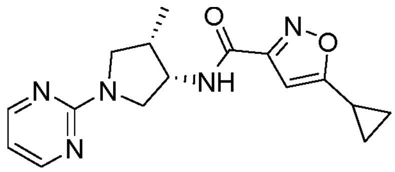 |
| 340 | 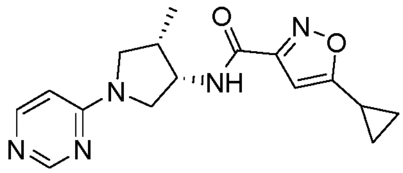 |
| 341 | 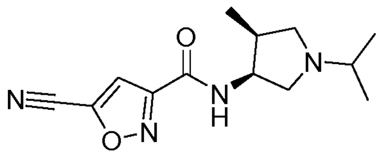 |
| 342 | 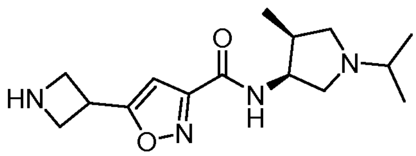 |
| 343 | 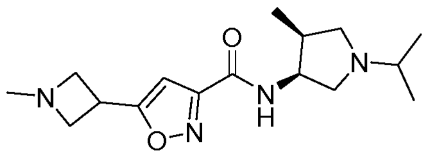 |
| 344 | 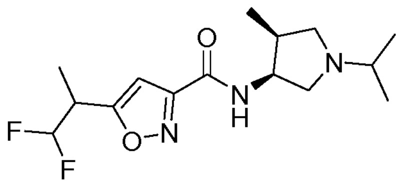 |
| 345 | 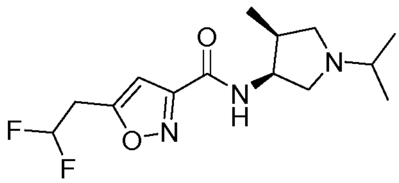 |

(suite)

| Composé n° | Structure |
|---|---|
| 346 | 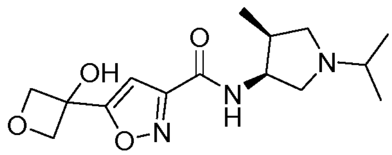 |
| 347 | 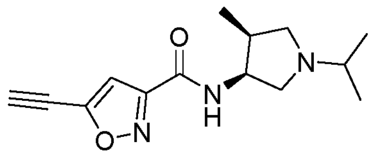 |
| 348 | 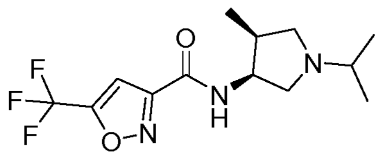 |
| 349 | 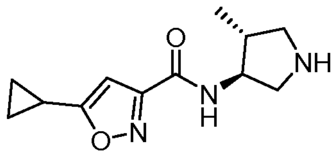 |
| 350 | 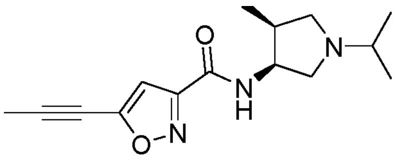 |
| 351 | 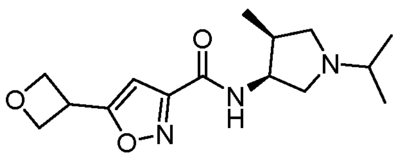 |
| 352 | 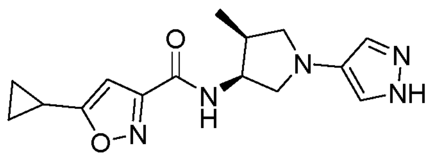 |

ou sel ou hydrate pharmaceutiquement acceptable correspondant.

**11.** Composition pharmaceutique pour une utilisation dans le traitement d'un cancer, la composition pharmaceutique comprenant le composé selon l'une quelconque des revendications 1 à 10, ou un sel ou un hydrate pharmaceutiquement acceptable correspondant, et un support pharmaceutiquement acceptable.

**12.** Composition pharmaceutique pour une utilisation selon la revendication 11, le cancer étant choisi dans le groupe constitué par un cancer de la glande surrénale, un carcinome à cellules acineuses, un névrome acoustique, un mélanome lentigineux acral , un acrospirome, une leucémie aiguë à éosinophiles, une leucémie aiguë érythroïde, une leucémie aiguë lymphoblastique, une leucémie aiguë mégacaryoblastique, une leucémie aiguë monocytaire, une leucémie aiguë promyélocytaire, un adénocarcinome, un carcinome adénoïde kystique, un adénome, une tumeur odontogène adénomatoïde, un carcinome adénosquameux, un néoplasme de tissu adipeux, un carcinome corticosurrénalien, une leucémie/un lymphome à cellules T de l'adulte, une leucémie agressive à cellules NK, un

lymphome lié au SIDA, un rhabdomyosarcome alvéolaire, un sarcome alvéolaire des parties molles, un fibrome améloblastique, un lymphome anaplasique à grandes cellules, un cancer anaplasique de la thyroïde, un lymphome T angio-immunoblastique, un angiomyolipome, un angiosarcome, un astrocytome, une tumeur rhabdoïde tératoïde atypique, une leucémie chronique lymphocytaire à cellules B, une leucémie prolymphocytaire à cellules B, un lymphome B, un carcinome basocellulaire, un cancer du tractus biliaire, un cancer de la vessie, un blastome, un cancer des os, une tumeur de Brenner, une tumeur de Brown, un lymphome de Burkitt, un cancer du sein, un cancer du cerveau, un carcinome, un carcinome in situ, un carcinosarcome, une tumeur cartilagineuse, un cémentome, un sarcome myéloïde, un chondrome, un chordome, un choriocarcinome, un papillome des plexus choroïdes, un sarcome à cellules claires du rein, un craniopharyngiome, un lymphome T cutané, un cancer du col de l'utérus, un cancer colorectal, la maladie de Degos, une tumeur desmoplasique à petites cellules rondes, un lymphome B diffus à grandes cellules, une tumeur neuroépithéliale dysembryoplasique, un dysgerminome, un carcinome embryonnaire, un néoplasme des glandes endocrines, une tumeur du sinus endodermique, un lymphome T associé à une entéropathie, un cancer de l'œsophage, un fœtus in fœtu, un fibrome, un fibrosarcome, un lymphome folliculaire, un cancer folliculaire de la thyroïde, un ganglioneurome, un cancer gastro-intestinal, une tumeur des cellules germinales, un choriocarcinome gestationnel, un fibroblastome à cellules géantes, une tumeur osseuse à cellules géantes, une tumeur gliale, un glioblastome multiforme, un gliome, une gliomatose cérébrale, un glucagonome, un gonadoblastome, une tumeur à cellules granuleuses, un gynandroblastome, un cancer de la vésicule biliaire, un cancer gastrique, une leucémie à cellules chevelues, un hémangioblastome, un cancer de la tête et du cou, un hémangiopéricytome, une malignité hématologique, un hépatoblastome, un lymphome T hépatosplénique, un lymphome hodgkinien, un lymphome non hodgkinien, un carcinome lobulaire infiltrant, un cancer intestinal, un cancer du rein, un cancer du larynx, un lentigo malin, un carcinome de la ligne médiane létal, une leucémie, une tumeur à cellules de Leydig, un liposarcome, un cancer du poumon, un lymphangiome, un lymphangiosarcome, un lymphoépithéliome, un lymphome, une leucémie aiguë lymphocytaire, une leucémie aiguë myéloïde, une leucémie chronique lymphocytaire, un cancer du foie, un cancer du poumon à petites cellules, un cancer du poumon non à petites cellules, un lymphome du MALT, un histiocytome fibreux malin, une tumeur maligne des gaines des nerfs périphériques, une tumeur triton maligne, un lymphome à cellules du manteau, un lymphome B de la zone marginale, une leucémie à mastocytes, une tumeur des cellules germinales du médiastin, un carcinome médullaire du sein, un cancer médullaire de la thyroïde, un médulloblastome, un mélanome, un méningiome, un cancer à cellules de Merkel, un mésothéliome, un carcinome urothélial métastatique, une tumeur müllérienne mixte, une tumeur mucineuse, un myélome multiple, un néoplasme de tissu musculaire, un mycosis fongoïde, un liposarcome myxoïde, un myxome, un myxosarcome, un carcinome du nasopharynx, un neurinome, un neuroblastome, un neurofibrome, un névrome, un mélanome nodulaire, un cancer oculaire, un oligoastrocytome, un oligodendrogliome, un oncocytome, un méningiome de la gaine du nerf optique, une tumeur du nerf optique, un cancer buccal, un ostéosarcome, un cancer de l'ovaire, une tumeur de Pancoast, un cancer papillaire de la thyroïde, un paragangliome, un pinéaloblastome, un pinéocytome, un pituicytome, un adénome pituitaire, une tumeur pituitaire, un plasmocytome, un polyembryome, un lymphome lymphoblastique à précurseurs T, un lymphome primitif du système nerveux central, un lymphome primitif à épanchement, un cancer péritonéal primitif, un cancer de la prostate, un cancer du pancréas, un cancer du pharynx, un pseudomyxome péritonéal, un carcinome à cellules rénales, un carcinome médullaire du rein, un rétinoblastome, un rhabdomyome, un rhabdomyosarcome, une transformation de Richter, un cancer rectal, un sarcome, une schwannomatose, un séminome, une tumeur à cellules de Sertoli, une tumeur des cordons sexuels et du stroma gonadique, un carcinome à cellules en bague à chaton, un cancer de la peau, les tumeurs à petites cellules rondes et bleues, un carcinome à petites cellules, un sarcome des tissus mous, un somatostatinome, une verrue de suie, une tumeur spinale, un lymphome de la zone marginale splénique, un carcinome à cellules squameuses, un sarcome synovial, la maladie de Sézary, un cancer de l'intestin grêle, un carcinome squameux, un cancer de l'estomac, un lymphome T, un cancer du testicule, un thécome, un cancer de la thyroïde, un carcinome à cellules transitionnelles, un cancer de la gorge, un cancer de l'ouraque, un cancer urogénital, un carcinome urothélial, un mélanome uvéal, un cancer de l'utérus, un carcinome verruqueux, un gliome des voies optiques, un cancer de la vulve, un cancer du vagin, une macroglobulinémie de Waldenström, une tumeur de Warthin et une tumeur de Wilms.

**13.** Composé pour une utilisation selon l'une quelconque des revendications 1 à 10, ou sel ou hydrate pharmaceutiquement acceptable correspondant, le cancer étant choisi dans le groupe constitué par un cancer de la glande surrénale, un carcinome à cellules acineuses, un névrome acoustique, un mélanome lentigineux acral, un acrospirome, une leucémie aiguë à éosinophiles, une leucémie aiguë érythroïde, une leucémie aiguë lymphoblastique, une leucémie aiguë mégacaryoblastique, une leucémie aiguë monocytaire, une leucémie aiguë promyélocytaire, un adénocarcinome, un carcinome adénoïde kystique, un adénome, une tumeur odontogène adénomatoïde, un carcinome adénosquameux, un néoplasme de tissu adipeux, un carcinome corticosurrénalien, une leucémie/un lymphome à cellules T de l'adulte, une leucémie agressive à cellules NK, un lymphome lié au SIDA, un rhabdomyosarcome alvéolaire, un sarcome alvéolaire des parties molles, un fibrome améloblastique, un lymphome anaplasique

à grandes cellules, un cancer anaplasique de la thyroïde, un lymphome T angio-immunoblastique, un angiomyolipome, un angiosarcome, un astrocytome, une tumeur rhabdoïde tératoïde atypique, une leucémie chronique lymphocytaire à cellules B, une leucémie prolymphocytaire à cellules B, un lymphome B, un carcinome basocellulaire, un cancer du tractus biliaire, un cancer de la vessie, un blastome, un cancer des os, une tumeur de Brenner, une tumeur de Brown, un lymphome de Burkitt, un cancer du sein, un cancer du cerveau, un carcinome, un carcinome in situ, un carcinosarcome, une tumeur cartilagineuse, un cémentome, un sarcome myéloïde, un chondrome, un chordome, un choriocarcinome, un papillome des plexus choroïdes, un sarcome à cellules claires du rein, un craniopharyngiome, un lymphome T cutané, un cancer du col de l'utérus, un cancer colorectal, la maladie de Degos, une tumeur desmoplasique à petites cellules rondes, un lymphome B diffus à grandes cellules, une tumeur neuroépithéliale dysembryoplasique, un dysgerminome, un carcinome embryonnaire, un néoplasme des glandes endocrines, une tumeur du sinus endodermique, un lymphome T associé à une entéropathie, un cancer de l'œsophage, un fœtus in fœtu, un fibrome, un fibrosarcome, un lymphome folliculaire, un cancer folliculaire de la thyroïde, un ganglioneurome, un cancer gastro-intestinal, une tumeur des cellules germinales, un choriocarcinome gestationnel, un fibroblastome à cellules géantes, une tumeur osseuse à cellules géantes, une tumeur gliale, un glioblastome multiforme, un gliome, une gliomatose cérébrale, un glucagonome, un gonadoblastome, une tumeur à cellules granuleuses, un gynandroblastome, un cancer de la vésicule biliaire, un cancer gastrique, une leucémie à cellules chevelues, un hémangioblastome, un cancer de la tête et du cou, un hémangiopéricytome, une malignité hématologique, un hépatoblastome, un lymphome T hépatosplénique, un lymphome hodgkinien, un lymphome non hodgkinien, un carcinome lobulaire infiltrant, un cancer intestinal, un cancer du rein, un cancer du larynx, un lentigo malin, un carcinome de la ligne médiane létal, une leucémie, une tumeur à cellules de Leydig, un liposarcome, un cancer du poumon, un lymphangiome, un lymphangiosarcome, un lymphoépithéliome, un lymphome, une leucémie aiguë lymphocytaire, une leucémie aiguë myéloïde, une leucémie chronique lymphocytaire, un cancer du foie, un cancer du poumon à petites cellules, un cancer du poumon non à petites cellules, un lymphome du MALT, un histiocytome fibreux malin, une tumeur maligne des gaines des nerfs périphériques, une tumeur triton maligne, un lymphome à cellules du manteau, un lymphome B de la zone marginale, une leucémie à mastocytes, une tumeur des cellules germinales du médiastin, un carcinome médullaire du sein, un cancer médullaire de la thyroïde, un médulloblastome, un mélanome, un méningiome, un cancer à cellules de Merkel, un mésothéliome, un carcinome urothélial métastatique, une tumeur müllérienne mixte, une tumeur mucineuse, un myélome multiple, un néoplasme de tissu musculaire, un mycosis fongoïde, un liposarcome myxoïde, un myxome, un myxosarcome, un carcinome du nasopharynx, un neurinome, un neuroblastome, un neurofibrome, un névrome, un mélanome nodulaire, un cancer oculaire, un oligoastrocytome, un oligodendrogliome, un oncocytome, un méningiome de la gaine du nerf optique, une tumeur du nerf optique, un cancer buccal, un ostéosarcome, un cancer de l'ovaire, une tumeur de Pancoast, un cancer papillaire de la thyroïde, un paragangliome, un pinéaloblastome, un pinéocytome, un pituicytome, un adénome pituitaire, une tumeur pituitaire, un plasmocytome, un polyembryome, un lymphome lymphoblastique à précurseurs T, un lymphome primitif du système nerveux central, un lymphome primitif à épanchement, un cancer péritonéal primitif, un cancer de la prostate, un cancer du pancréas, un cancer du pharynx, un pseudomyxome péritonéal, un carcinome à cellules rénales, un carcinome médullaire du rein, un rétinoblastome, un rhabdomyome, un rhabdomyosarcome, une transformation de Richter, un cancer rectal, un sarcome, une schwannomatose, un séminome, une tumeur à cellules de Sertoli, une tumeur des cordons sexuels et du stroma gonadique, un carcinome à cellules en bague à chaton, un cancer de la peau, les tumeurs à petites cellules rondes et bleues, un carcinome à petites cellules, un sarcome des tissus mous, un somatostatinome, une verrue de suie, une tumeur spinale, un lymphome de la zone marginale splénique, un carcinome à cellules squameuses, un sarcome synovial, la maladie de Sézary, un cancer de l'intestin grêle, un carcinome squameux, un cancer de l'estomac, un lymphome T, un cancer du testicule, un thécome, un cancer de la thyroïde, un carcinome à cellules transitionnelles, un cancer de la gorge, un cancer de l'ouraque, un cancer urogénital, un carcinome urothélial, un mélanome uvéal, un cancer de l'utérus, un carcinome verruqueux, un gliome des voies optiques, un cancer de la vulve, un cancer du vagin, une macroglobulinémie de Waldenström, une tumeur de Warthin et une tumeur de Wilms.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 62146808 A
- WO 62048757 A

### Non-patent literature cited in the description


- **ANDREW FERGUSSON et al.** STRUCTURE. ELSEVIER, 13 June 2011, vol. 19, 1262-1273
- *Pure & Appl. Chem,* 1996, vol. 68, 2193
- **M. CAIRA et al.** *J. Pharmaceut. Sci.,* 2004, vol. 93 (3), 601-611
- **E.C. VAN TONDER et al.** *AAPS Pharm. Sci. Tech.,* 2004, vol. 5 (1
- **A.L. BINGHAM et al.** *Chem. Commun.,* 2001, 603-604
- **ABU-FARHA et al.** *JMol Cell Biol,* 2011, vol. 3 (5), 301-308
- **HAMAMOTO et al.** *Nat Cell. Biol.,* 2004, vol. 6, 731-740
- **HU et al.** *Canncer Research,* 2009, 4067-4072
- **KOMATSU et al.** *Carcinogenesis,* 2009, 301139-1146
- **HAMAMOTO, R. et al.** *Nat. Cell Biol.,* 2004, vol. 6 (8), 731-40
- **HAMAMOTO, R. et al.** *Cancer Sci.,* 2006, vol. 97 (2), 113-8
- **VAN ALLER, G.S. et al.** *Epigenetics,* 2012, vol. 7 (4), 340-3
- **LIU, C. et al.** *J. Natl. Cancer Inst.,* 2013, vol. 105 (22), 1719-28
- **MAZUR, P.K. et al.** *Nature,* 2014, vol. 510 (7504), 283-7
- **PESERICO et al.** *Cell Physiol.,* 28 February 2015
- **DE ALMEIDA et al.** *Mucosal Immunol,* 11 February 2015
- **PROSERPIO et al.** *Genes Dev.,* 01 June 2013, vol. 27 (11), 1299-312
- **FUJII et al.** *PLoS One,* 2011, vol. 6 (8), e23491
- **KOMATSU et al.** *Carcinogenesis,* 2009, vol. 30, 1139
- **CHO et al.** *Neoplasia,* June 2012, vol. 14 (6), 476-86
- **KOMATSU et al.** *Br J Cancer,* 20 January 2015, vol. 112 (2), 357-64
- **SAKAMOTO et al.** *Leuk Res.,* April 2014, vol. 38 (4), 496-502
- **NGUYEN et al.** *J Biol Chem.,* 30 March 2015
- **XU et al.** *J. Biol. Chem.,* 27 February 2015, vol. 290 (9), 5414-23
- Remington's Pharmaceutical Sciences. Mack Publishing Co, 1995
- **WUTS, P. G. M. ; GREENE, T. W.** Greene's Protective Groups in Organic Synthesis. J. Wiley & Sons, NY, 2007